# FASCICULE DE BREVET EUROPEEN

(11) **EP 3 027 602 B1**
(45) Date de publication et mention de la délivrance du brevet: **13.06.2018**
(21) Numéro de dépôt: 14758604.4
(22) Date de dépôt: 22.07.2014
(51) Int. Cl.: C07D 401/14, C07D 405/14, A61K 31/4709, C07D 401/10, C07D 471/04, A61P 35/00, A61P 37/00

(54) **NOUVEAUX DERIVES D'INDOLE ET DE PYRROLE, LEUR PROCEDE DE PREPARATION ET LES COMPOSITIONS PHARMACEUTIQUES QUI LES CONTIENNENT**
NEUARTIGE INDOL- UND PYRROLDERIVATE, VERFAHREN ZUR HERSTELLUNG DAVON UND PHARMAZEUTISCHE ZUSAMMENSETZUNGEN DAMIT
NOVEL DERIVATIVES OF INDOLE AND PYRROLE, METHOD FOR THE PRODUCTION THEREOF AND PHARMACEUTICAL COMPOSITIONS CONTAINING SAME

(30) Priorité: 23.07.2013 FR 1357277
(43) Date de publication de la demande: 08.06.2016
(60) Demande divisionnaire: 16201228.0
(73) Titulaire: Les Laboratoires Servier, 92284 Suresnes (FR); Vernalis (R&D) Limited, Winnersh Berkshire RG41 5RD (GB)
(72) Inventeur: CASARA, Patrick, 67100 Strasbourg (FR); LE DIGUARHER, Thierry, F-45550 Saint Denis de l'Hôtel (FR); HENLIN, Jean-Michel, F-92150 Suresnes (FR); STARCK, Jérôme-Benoît, F-92500 Rueil-Malmaison (FR); LE TIRAN, Arnaud, F-78290 Croissy Sur Seine (FR); DE NANTEUIL, Guillaume, F-92150 Suresnes (FR); GENESTE, Olivier, F-92500 Rueil Malmaison (FR); DAVIDSON, James Edward Paul, Cambridge Cambridgeshire CB22 5DJ (GB); MURRAY, James Brooke, Linton Cambridgeshire CB21 4YL (GB); CHEN, I-Jen, Cambridge Cambridgeshire CB1 3JJ (GB); WALMSLEY, Claire, Ely Cambridgeshire CB6 3WQ (GB); GRAHAM, Christopher John, Newmarket Suffolk CB8 0AN (GB); RAY, Stuart, Milton Cambridgeshire CB24 6UD (GB); MADDOX, Daniel, Upper Cambourne Cambridgeshire CB23 6FP (GB); BEDFORD, Simon, Harlow Essex CM19 4HD (GB)
(86) Numéro de dépôt international: PCT/FR2014/051884
(87) Numéro de publication internationale: WO 2015/011396

(56) Documents cités:
- WO-A1-2012/162365
- WO-A1-2013/096055
- WO-A1-2013/096059
- WO-A1-2013/110890
- WO-A2-2006/023778
- PORTER J ET AL: "Tetrahydroisoquinoline amide substituted phenyl pyrazoles as selective Bcl-2 inhibitors", BIOORGANIC & MEDICINAL CHEMISTRY LETTERS, PERGAMON, AMSTERDAM, NL, vol. 19, no. 1, 1 janvier 2009 (2009-01-01), pages 230-233, XP025816913, ISSN: 0960-894X, DOI: 10.1016/J.BMCL.2008.10.113 [extrait le 2008-10-31]

## Description

La présente invention concerne de nouveaux dérivés d'indole et de pyrrole, leur procédé de préparation et les compositions pharmaceutiques qui les contiennent.

Les composés de la présente invention sont nouveaux et présentent des caractéristiques pharmacologiques très intéressantes dans le domaine de l'apoptose et de la cancérologie.

L'apoptose, ou mort cellulaire programmée, est un processus physiologique crucial pour le développement embryonnaire et le maintien de l'homéostasie tissulaire.
La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).
Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell, 2000, 100, 57-70).

Les protéines anti-apoptotiques de la famille Bcl-2 sont associées à de nombreuses pathologies. L'implication des protéines de la famille Bcl-2 est décrite dans de nombreux types de cancer, tel que le cancer colorectal, le cancer du sein, le cancer du poumon à petites cellules, le cancer du poumon non à petites cellules, le cancer de la vessie, le cancer de l'ovaire, le cancer de la prostate, la leucémie lymphoïde chronique, le lymphome folliculaire, le myélome... La surexpression des protéines anti-apoptotiques de la famille Bcl-2 est impliquée dans la tumorogenèse, dans la résistance à la chimiothérapie et dans le pronostique clinique des patients atteints de cancer. Il existe donc un besoin thérapeutique de composés inhibant l'activité anti-apoptotique des protéines de la famille Bcl-2.

Les composés de la présente invention outre leur nouveauté, présentent des propriétés pro-apoptotiques permettant de les utiliser dans les pathologies impliquant un défaut d'apoptose, comme par exemple dans le traitement du cancer, des maladies auto-immunes et du système immunitaire.

La présente invention concerne plus particulièrement les composés de formule (I): 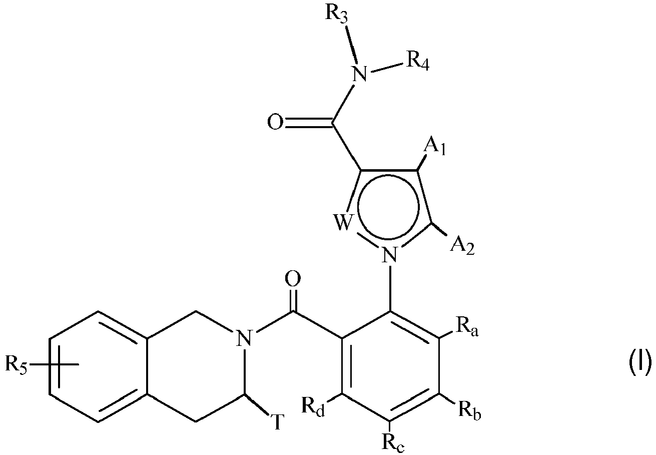 dans laquelle :
◆ W représente un groupe C-H tandis que A₁ et A₂ représentent respectivement un atome d'hydrogène et un groupement méthyle,
   ou bien W représente un groupe C-H tandis que A₁ et A₂ forment avec les atomes de carbone qui les portent un cyclohexenyle ou un cycle benzo éventuellement substitué par un atome d'halogène,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
   ou bien R₁ et R₂ forment avec l'atome d'azote qui les porte un hétérocycloalkyle,
◆ R₃ représente un groupement aryle ou hétéroaryle, étant entendu qu'un ou plusieurs atomes de carbone des groupements précédents, ou de leurs éventuels substituants, peut(vent) être deutéré(s),
◆ R₄ représente un groupement aryle ou hétéroaryle, étant entendu qu'un ou plusieurs atomes de carbone des groupements précédents, ou de leurs éventuels substituants, peut(vent) être deutéré(s),
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ, R_{b}, R_{c} et R_{d} représentent indépendamment les uns des autres R₇, un atome d'halogène, un groupement alkoxy (C₁-C₆) linéaire ou ramifié, un groupe hydroxy, un groupement polyhalogénoalkyle (C₁-C₆) linéaire ou ramifié, un groupe trifluorométhoxy, -NR₇R₇', nitro, ou bien les substituants de l'un des couples (Rₐ,R_{b}), (R_{b},R_{c}) ou (R_{c},R_{d}) forment ensemble avec les atomes de carbone qui les portent un cycle constitué de 5 à 7 chaînons, pouvant contenir de un à 2 hétéroatomes choisis parmi oxygène et soufre, étant aussi entendu qu'un ou plusieurs atomes de carbone du cycle précédemment défini peut(vent) être deutéré(s) ou substitués par un à 3 groupements choisis parmi halogène ou alkyle (C₁-C₆) linéaire ou ramifié,
   ou bien Rₐ et R_{d} représentent chacun un atome d'hydrogène, R_{b} représente un hydrogène, un halogène, un groupement hydroxy ou méthoxy, et R_{c} est choisi parmi l'un des groupes suivants : hydroxy, méthoxy, amino, 3-phénoxyazétidine, 2-(phénylsulfanyl)acétamide, ou 2-(phénoxy)acétamide,
◆ R₇ et R₇' représentent indépendamment l'un de l'autre un hydrogène, un groupement alkyle (C₁-C₆) linéaire ou ramifié, alkényle (C₂-C₆) linéaire ou ramifié, alkynyle (C₂-C₆) linéaire ou ramifié, aryle ou hétéroaryle, ou bien R₇ et R₇' forment ensemble avec l'atome d'azote qui les porte un hétérocycle constitué de 5 à 7 chaînons,
étant entendu que lorsque le composé de formule (I) contient un groupement hydroxy, ce dernier peut être éventuellement substitué par l'un des groupes suivants : -PO(OM)(OM'), -PO(OM)(O⁻M₁⁺), -PO(O⁻M₁⁺)(O⁻M₂⁺), -PO(O⁻)(O⁻)M₃²⁺, -PO(OM)(O[CH₂CH₂O]ₙCH₃), ou -PO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5,
étant aussi entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique constitué de 3 à 10 chaînons et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié, spiro (C₃-C₆), alkoxy (C₁-C₆) linéaire ou ramifié, (C₁-C₆)alkyl-S-, hydroxy, oxo (ou N-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tert-butylamine, etc...

Parmi les composés préférés de l'invention, on trouve les composés de formule (I) pour lesquels R₄ représente un phényle substitué en position *para* par un groupement de formule -OPO(OM)(OM'), -OPO(OM)(O⁻M₁⁺), -OPO(O⁻M₁⁺)(O⁻M₂⁺), -OPO(O⁻)(O⁻)M₃²⁺ -OPO(OM)(O[CH₂CH₂O]ₙCH₃), ou -OPO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyl (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5, étant entendu que le groupement phényle peut être optionnellement substitué par un ou plusieurs atomes d'halogène.

De manière préférentielle, T représente un groupement choisi parmi méthyle, aminométhyle, diméthylaminométhyle, morpholinylméthyle, (4-méthyl-1-pipérazinyl)méthyle, (3a*R*,6a*S*)-hexahydrocyclopenta[*c*]pyrrol-2(1*H*)-ylméthyle, (4,4-difluoropipéridin-1-yl)méthyle, (4-cyclopentylpipérazin-1-yl)méthyle, (4-cyclobutylpipérazin-1-yl)méthyle, pyrrolidin-1-ylméthyle, pipéridin-1-ylméthyle ou 2-(morpholin-4-yl)éthyle. Plus préférentiellement encore, T représente un groupement morpholinylméthyle ou (4-méthyl-1-pipérazinyl)méthyle.

Dans les composés préférés de l'invention, Rₐ et R_{d} représentent chacun un atome d'hydrogène et (R_{b},R_{c}) forment ensemble avec les atomes de carbone qui les portent l'un des groupes suivants : 1,3-dioxolane éventuellement substitué ; 1,4-dioxane éventuellement substitué ; cyclopentane ; tétrahydrofurane ; 2,3-dihydrofurane ; ou bien Rₐ, R_{c} et R_{d} représentent chacun un atome d'hydrogène et R_{b} représente un groupement hydroxy ou méthoxy, un atome d'halogène, un groupement trifluorométhyle ou trifluorométhoxy. Plus préférentiellement encore :
- Rₐ et R_{d} représentent chacun un atome d'hydrogène et (R_{b},R_{c}) forment ensemble avec les atomes de carbone qui les portent un groupe 1,3-dioxolane,
- ou bien Rₐ, R_{c} et R_{d} représentent chacun un atome d'hydrogène et R_{b} représente un atome d'halogène.

Alternativement, un à deux des groupements Rₐ, R_{b}, R_{c}, R_{d} représentent un atome d'halogène, tandis que les autres représentent un atome d'hydrogène.

Les groupements R₄ préférés sont les suivants : phényle ; 4-hydroxyphényle ; 4-méthoxyphényle; 4-méthylphényle ; 3-chloro-4-hydroxyphényle; 3-fluoro-4-hydroxyphényle. Plus préférentiellement encore, R₄ représente un groupement 4-hydroxyphényle.

Dans les composés préférés de l'invention, R₃ représente un groupement choisi parmi phényle, 1*H*-indole, benzothiophène, benzofurane, 2,3-dihydro-1*H*-indole, 1*H*-indazole, 2,3-dihydro-1*H*-isoindole, 1*H*-pyrrolo[2,3-*b*]pyridine, phénoxyphényle, 2,3-dihydro-1*H-*pyrrolo[2,3-*b*]pyridine, 1*H*-pyrrole, ces groupements comportant éventuellement un ou plusieurs substituants choisis parmi halogène, alkyle (C₁-C₆) linéaire ou ramifié, trifluorométhoxy, 4-méthylpipérazinyle, alkoxy (C₁-C₆) linéaire ou ramifié, ou cyano.

Les composés préférés selon l'invention sont compris dans le groupe suivant :
- *N*-(4-hydroxyphényl)-1-{6-[(3*S*)-3-(morpholin-4-ylméthyl)-1,2,3,4-tétrahydroisoquinoléine-2-carbonyl]-2*H*-1,3-benzodioxol-5-yl}-*N*-phényl-1*H-*indole-3-carboxamide,
- *N*-(4-hydroxyphényl)-*N*-(1-méthyl-1*H*-pyrrolo[2,3-*b*]pyridin-5-yl)-1-(6-{[(3*S*)-3-(morpholin-4-ylméthyl)-3,4-dihydroisoquinoléin-2(1*H*)-yl]carbonyl}-1,3-benzodioxol-5-yl)-4,5,6,7-tétrahydro-1*H*-indole-3-carboxamide,
- 1-(5-chloro-2-{[(3*S*)-3-(morpholin-4-ylméthyl)-3,4-dihydroisoquinoléin-2(1*H*)-yl]carbonyl}phényl)-*N*-(4-hydroxyphényl)-4,5-diméthyl-*N*-(1-méthyl-2,3-dihydro-1*H*-pyrrolo[2,3-*b*]pyridin-5-yl)-1*H*-pyrrole-3-carboxamide,
- 1-(5-chloro-2-{[(3*S*)-3-(morpholin-4-ylméthyl)-3,4-dihydroisoquinoléin-2(1*H*)-yl]carbonyl}phényl)-*N*-(5-cyano-1,2-diméthyl-1*H*-pyrrol-3-yl)-*N*-(4-hydroxyphényl)-4,5-diméthyl-1*H*-pyrrole-3-carboxamide,
- *N*-(4-hydroxyphényl)-*N*-(4-méthylphényl)-1-(6-{[(3*S*)-3-[(4-méthylpipérazin-1-yl)méthyl]-3,4-dihydroisoquinoléin-2(1*H*)-yl]carbonyl}-1,3-benzodioxol-5-yl)-1*H-*indole-3-carboxamide,
- *N*-(4-hydroxyphényl)-*N*-(1*H*-indol-5-yl)-1-(6-{[(3*S*)-3-[(4-méthylpipérazin-1-yl)méthyl]-3,4-dihydroisoquinoléin-2(1*H*)-yl]carbonyl}-1,3-benzodioxol-5-yl)-1*H-*indole-3-carboxamide,
- *N*-(4-hydroxyphényl)-*N*-(1-méthyl-1*H*-indol-5-yl)-1-(6-{[(3*S*)-3-[(4-méthylpipérazin-1-yl)méthyl]-3,4-dihydroisoquinoléin-2(1*H*)-yl]carbonyl}-1,3-benzodioxol-5-yl)-1*H*-indole-3-carboxamide,
- 1-(5-chloro-2-{[(3*S*)-3-[(4-méthylpipérazin-1-yl)méthyl]-3,4-dihydroisoquinoléin-2(1*H*)-yl]carbonyl}phényl)-*N*-(4-chlorophényl)-*N*-(4-hydroxyphényl)-5-méthyl-1*H*-pyrrole-3-carboxamide,
- 1-(5-chloro-2-{[(3*S*)-3-[(4-méthylpipérazin-1-yl)méthyl]-3,4-dihydroisoquinoléin-2(1*H*)-yl]carbonyl}phényl)-*N*-(4-hydroxyphényl)-*N*-(1*H*-indol-5-yl)-1*H*-indole-3-carboxamide,
- *N*-(4-hydroxyphényl)-1-{6-[(3*S*)-3-[(4-méthylpipérazin-1-yl)méthyl]-1,2,3,4-tétrahydroisoquinoléine-2-carbonyl]-2*H*-1,3-benzodioxol-5-yl}-*N*-phényl-1*H-*indole-3-carboxamide,
leurs énantiomères et diastéréoisomères, ainsi que leurs sels d'addition à un acide ou à une base pharmaceutiquement acceptable.

L'invention s'étend également au procédé de préparation d'un composé de formule (I) caractérisé en ce que l'on utilise comme produit de départ le composé de formule (II) : 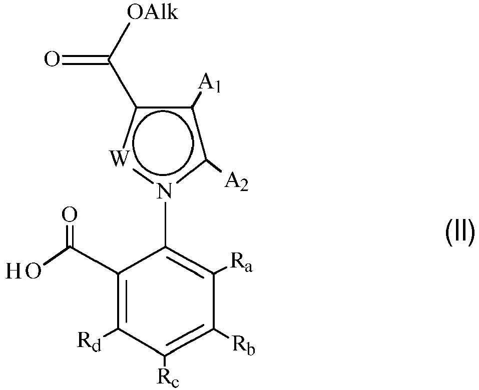 dans laquelle Alk représente un groupement alkyle (C₁-C₆) et W, A₁, A₂, Rₐ, R_{b}, R_{c}, R_{d} sont tels que définis dans la formule (I),
composé de formule (II) qui subit ensuite un couplage peptidique avec un composé de formule (III) : 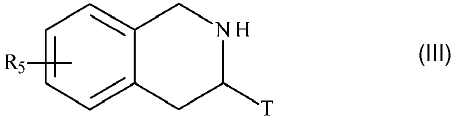 dans laquelle T et R₅ sont tels que définis dans la formule (I),
pour conduire au composé de formule (IV) : 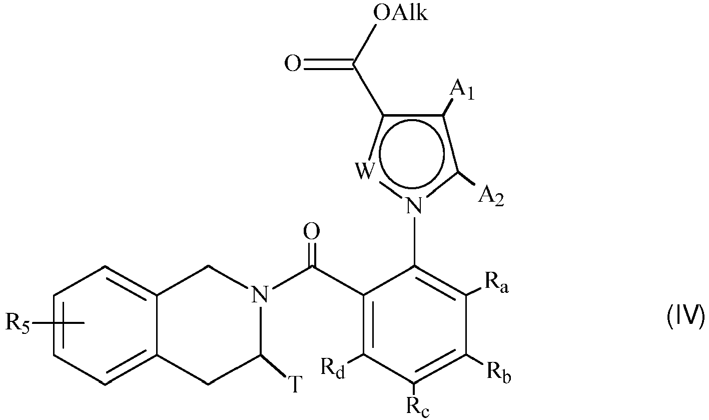 dans laquelle Alk est tel que défini précédemment et W, A₁, A₂, Rₐ, R_{b}, R_{c}, R_{d}, R₅ et T sont tels que définis dans la formule (I),
composé de formule (IV) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), qui peut être optionnellement soumis à l'action d'un dérivé pyrophosphate ou phosphonate dans des conditions basiques, pour conduire au composé de formule (I), composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

Plus particulièrement, lorsque l'un des groupements R₃ ou R₄ de l'amine NHR₃R₄ est substitué par une fonction hydroxy, cette dernière peut être préalablement soumise à une réaction de protection avant tout couplage avec l'acide carboxylique du composé de formule (IV), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

Alternativement, les composés de formule (I) peuvent être obtenus selon le procédé de préparation suivant, caractérisé en ce que l'on utilise comme produit de départ le composé de formule (V) tel que défini ci-dessous : 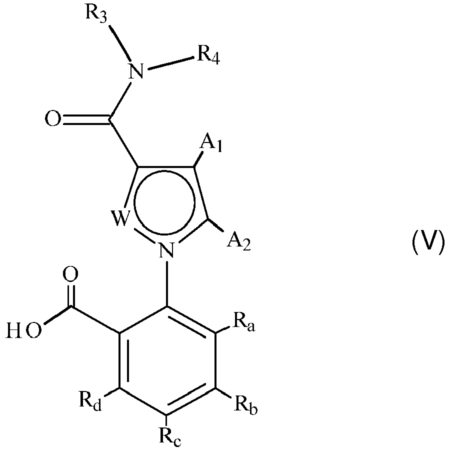 dans laquelle W, A₁, A₂, Rₐ, R_{b}, R_{c}, R_{d}, R₃ et R₄ sont tels que définis dans la formule (I),
composé de formule (V) qui subit ensuite un couplage peptidique avec un composé de formule (III) : 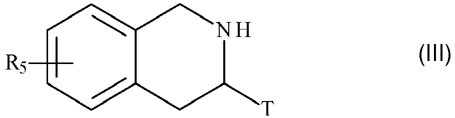 dans laquelle T et R₅ sont tels que définis dans la formule (I), le composé ainsi obtenu étant optionnellement soumis à l'action d'un dérivé pyrophosphate ou phosphonate dans des conditions basiques, pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

Les composés de formules (II), (III), (V), ainsi que l'amine NHR₃R₄, sont soit commerciaux, soit accessibles à l'homme du métier par des réactions chimiques classiques et décrites dans la littérature.

L'étude pharmacologique des dérivés de l'invention a montré qu'ils possédaient des propriétés pro-apoptotiques. La capacité à réactiver le processus apoptotique dans les cellules cancéreuses représente un intérêt thérapeutique majeur dans le traitement des cancers, des maladies auto-immunes et du système immunitaire.

Plus particulièrement, les composés selon l'invention seront utiles dans le traitement des cancers chimio ou radiorésistants, ainsi que dans les hémopathies malignes et le cancer du poumon à petites cellules.
Parmi les traitements des cancers envisagés on peut citer, sans s'y limiter, le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules. Parmi les lymphomes non hodgkiniens, on peut citer plus préférentiellement les lymphomes folliculaires, les lymphomes des cellules du manteau, les lymphomes diffus à grandes cellules B, les petits lymphomes lymphocytiques et les lymphomes à cellules B de la zone marginale.

La présente invention a également pour objet les compositions pharmaceutiques contenant au moins un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également l'association d'un composé de formule (I) avec un agent anticancéreux choisi parmi les agents génotoxiques, les poisons mitotiques, les anti-métabolites, les inhibiteurs du protéasome, les inhibiteurs de kinase, ou les anticorps, ainsi que les compositions pharmaceutiques contenant ce type d'association et leur utilisation pour la fabrication de médicaments utiles dans le traitement du cancer.

Les composés de l'invention peuvent également être utilisés en association avec une radiothérapie dans le traitement du cancer.

Enfin, les composés de l'invention peuvent être liés à des anticorps monoclonaux ou à des fragments de ceux-ci ou peuvent être liés à des protéines de charpente qui peuvent être apparentées, ou non, à des anticorps monoclonaux.
Par fragments d'anticorps, on doit entendre des fragments du type Fv, scFv, Fab, F(ab')2, F(ab'), scFv-Fc, ou des diabodies, qui en général ont la même spécificité de liaison que l'anticorps duquel ils sont issus. Selon la présente invention, les fragments d'anticorps peuvent être obtenus à partir d'anticorps par des méthodes telles que digestion par enzymes telles que la pepsine ou la papaïne, et/ou par clivage des ponts disulfures par réduction chimique. D'autre manière, les fragments d'anticorps compris dans la présente invention peuvent être obtenus par des techniques de recombinaison génétique également bien connues à l'homme du métier ou bien par synthèse de peptides au moyen de synthétiseurs automatiques de peptides par exemple, tels que ceux fournis par la société Applied Biosystems etc...
Par protéines de charpente qui peuvent être apparentées, ou non, à des anticorps monoclonaux, on entend une protéine qui comprend, ou non, un repliement d'immunoglobuline et qui livre une capacité de liaison similaire à celle d'un anticorps monoclonal. L'homme du métier sait comment sélectionner la protéine de charpente. Plus particulièrement, il est connu que, pour être sélectionné, une telle charpente devrait présenter plusieurs caractéristiques comme suivant (Skerra A., J. Mol. Recogn. 13, 2000, 167-187): une bonne conservation phylogénétique, une architecture robuste avec une organisation moléculaire en trois dimensions bien connue (telle que la cristallographie ou la RMN, par exemple), une petite taille, aucune ou peu de modification(s) post-traductionnelle(s), la facilité de production, d'expression et de purification. Une telle protéine de charpente peut être, sans s'y limiter, une structure choisie parmi le groupe consistant en la fibronectine et de préférence le dixième domaine type III de la fibronectine (FNfn10), la lipocaline, l'anticaline (Skerra A., J. Biotechnol., 2001, 74(4):257-75), la protéine Z dérivée du domaine B de la protéine A des staphylocoques, la thiorédoxine A ou une protéine quelconque avec un domaine répété tel qu'une "répétition ankyrine" (Kohl et al., PNAS, 2003, vol.100, no.4, 1700-1705), une "répétition armadillo", une "répétition riche en leucine" ou une "répétition tétratricopeptide". On pourrait aussi mentionner une charpente dérivée de toxines (telles que des toxines de scorpions, d'insectes, de plantes ou des mollusques, par exemple) ou des protéines inhibitrices de l'oxyde nitrique synthase (PIN).

Les Préparations et Exemples suivants illustrent l'invention et ne la limitent en aucune façon.

### Procédures générales

Tous les réactifs et les solvants anhydres sont commerciaux et ont été utilisés sans purification ou séchage supplémentaire. La chromatographie éclair est réalisée sur un appareil ISCO CombiFlash Rf 200i avec des cartouches prépackées remplies de gel de silice (SiliaSep™ F60 (40-63µm), 60Å). Les chromatographies sur couche mince ont été réalisées sur des plaques de 5 x 10 cm recouvertes de gel de silice Merck Type 60 F₂₅₄. Le chauffage par microonde a été réalisé avec un appareil CEM Discover® SP.

### LC-MS analytique

Les composés de l'invention ont été caractérisés par chromatographie liquide à haute performance couplée à la spectrométrie de masse (HPLC-MS) soit sur un appareil Agilent HP1200 à résolution rapide couplé à un détecteur de masse 6140 à source multimodale (intervalle m/z de 150 à 1000 unités de masse atomique ou uma), soit sur un appareil Agilent HP1100 couplé à un détecteur de masse 1946D à source d'ionisation par électrospray (intervalle m/z de 150 à 1000 uma). Les conditions et les méthodes listées ci-dessous sont identiques pour les deux machines.
Détection : Détection UV à 230, 254 et 270 nm.
Volume d'injection : 2µL
Phases Mobiles :
   A - Eau + 10 mMol / formate d'ammonium + 0,08% (v/v) acide formique à pH environ 3,5.
   B - 95% Acétonitrile + 5% A + 0,08% (v/v) acide formique

### Méthode A (3,75 min; soit ionisation positive (pos) soit ionisation positive et négative (pos/neg))

Colonne : Gemini 5µm, C18, 30 mm x 4,6mm (Phenomenex).
Température : 35°C.
Gradient :

| **Temps (min)** | **Solvant A (%)** | **Solvant B (%)** | **Débit (mL/min)** |
|---|---|---|---|
| 0 | 95 | 5 | 2 |
| 0,25 | 95 | 5 | 2 |
| 2,50 | 95 | 5 | 2 |
| 2,55 | 5 | 95 | 3 |
| 3,60 | 5 | 95 | 3 |
| 3,65 | 5 | 95 | 2 |
| 3,70 | 95 | 5 | 2 |
| 3,75 | 95 | 5 | 2 |

### Méthode B (1,9 min; soit ionisation positive (pos) soit ionisation positive et négative (pos /neg))

Colonne : Gemini 5µm, C18, 30 mm x 4,6mm (Phenomenex).
Température : 35°C.
Gradient:

| **Temps (min)** | **Solvant A (%)** | **Solvant B (%)** | **Débit (mL/min)** |
|---|---|---|---|
| 0 | 95 | 5 | 1,1 |
| 0,12 | 95 | 5 | 1,1 |
| 1,30 | 5 | 95 | 1,1 |
| 1,35 | 5 | 95 | 1,7 |
| 1,85 | 5 | 95 | 1,7 |
| 1,90 | 5 | 95 | 1,1 |
| 1,95 | 95 | 5 | 1,1 |

### HPLC préparative

Certains composés de l'invention ont été purifiés par HPLC préparative sur un appareil Waters FractionLynx MS avec système d'autopurification, équipé d'une colonne Gemini® 5 µm C18(2), 100 mm x 20 mm i.d. (Phenomenex), fonctionnant à un débit de 20 cm³.min⁻¹ avec détecteur UV à barrette de diodes (210-400 nm) et collecteur de fractions couplé à la spectrométrie de masse. Les gradients utilisés pour chacun des composés sont présentés dans le Tableau 1.

A pH 4 : solvant A = acétate d'ammonium 10 mM dans l'eau qualité HPLC + 0,08% v/v acide formique. Solvant B = 95% v/v acétonitrile qualité HPLC + 5% v/v solvant A + 0,08% v/v acide formique.

A pH 9 : solvant A = acétate d'ammonium 10 mM dans l'eau qualité HPLC + 0,08% v/v solution d'ammoniaque. Solvant B = 95% v/v acétonitrile qualité HPLC + 5% v/v solvant A + 0,08% v/v solution d'ammoniaque.

Le spectromètre de masse est un appareil Waters Micromass ZQ2000, fonctionnant par ionisation électrospray en mode positif ou négatif, avec un intervalle de détection du poids moléculaire allant de 150 à 1000.

### Préparation 1 : Acide 2-[4-(éthoxycarbonyl)-2-méthyl-1H-pyrrol-1-yl]benzoïque

A une solution de 8,3 g d'acide 2-aminobenzoïque (48 mmol) dans l'acide acétique (20 mL), on ajoute 8,7 g de 4-oxopentanoate d'éthyle (préparé selon la méthode décrite dans WO2005/040128). Puis, l'ensemble est chauffé au reflux durant une nuit. Le milieu réactionnel est ensuite évaporé à sec et purifié par chromatographie sur gel de silice (gradient dichlorométhane/ EtOH). On obtient le produit du titre sous la forme d'une huile.
**RMN ¹H :** δ (400 MHz ; dmso-d6; 300K) : 13.0 (m, 1H OH); 7.91 (d, 1H, aryl) ; 7.70 (t, 1H, aryl) ; 7.62 (t,1H, aryl) ; 7.40 (d, 1H, aryl) ; 7.30 (s, 1H, pyrrole) ; 6.30 (s, 1H, pyrrole) ; 4.18 (q, 2H, OC**H₂**CH₃); 1.95 (t, 3H, OCH₂C**H₃**).
**IR :** v : -OH : 2800-2000 cm⁻¹ acide ; v : C=O 1716 et 1667 cm⁻¹ ; v : C=C 1600 cm⁻¹

### Préparations 2 : Acide 4-(benzyloxy)-2-[4-(éthoxycarbonyl)-2-méthyl-1H-pyrrol-1-yl]beuzoïque

Le composé du titre est obtenu suivant le procédé de la Préparation 1 en remplaçant l'acide 2-aminobenzoïque par l'acide 2-amino-4-benzyloxy-benzoïque.

### Préparation 3: Acide 2-[4-(éthoxycarbonyl)-2-méthyl-1H-pyrrol-1-yl]-4-(trifluorométhyl)benzoïque

Le composé du titre est obtenu suivant le procédé de la Préparation 1 en remplaçant l'acide 2-aminobenzoïque par l'acide 2-amino-4-(trifluorométhyl)benzoïque.

### Préparation 4: Acide 4-chloro-2-[4-(éthoxycarbonyl)-2-méthyl-1H-pyrrol-1-yl]benzoïque

Le composé du titre est obtenu suivant le procédé de la Préparation 1 en remplaçant l'acide 2-aminobenzoïque par l'acide 2-amino-4-chlorobenzoïque.

### Préparation 5 : Acide 4-fluoro-2-[4-(éthoxycarbonyl)-2-méthyl-1H-pyrrol-1-yl]benzoïque

Le composé du titre est obtenu suivant le procédé de la Préparation 1 en remplaçant l'acide 2-aminobenzoïque par l'acide 2-amino-4-fluorobenzoïque.

### Préparation 6 : Acide 4,5-dibromo-2-[4-(éthoxycarbonyl)-2-méthyl-1H-pyrrol-1-yl]benzoïque

Le composé du titre est obtenu suivant le procédé de la Préparation 1 en remplaçant l'acide 2-aminobenzoïque par l'acide 2-amino-4,5-dibromobenzoïque.

### Préparation 7: Acide 2-chloro-6-[4-(éthoxycarbonyl)-2-méthyl-1H-pyrrol-1-yl]benzoïque

Le composé du titre est obtenu suivant le procédé de la Préparation 1 en remplaçant l'acide 2-aminobenzoïque par l'acide 2-amino-6-chlorobenzoïque.

### Préparation 8: Acide 2-[4-(éthoxycarbonyl)-2-méthyl-1H-pyrrol-1-yl]-5-(trifluorométhoxy)benzoïque

Le composé du titre est obtenu suivant le procédé de la Préparation 1 en remplaçant l'acide 2-aminobenzoïque par l'acide 2-amino-5-(trifluorométhoxy)benzoïque.

### Réparation 9 : Acide 2-[4-(éthoxycarbonyl)-2-méthyl-1H-pyrrol-1-yl]-4,5-difluorobenzoïque

Le composé du titre est obtenu suivant le procédé de la Préparation 1 en remplaçant l'acide 2-aminobenzoïque par l'acide 2-amino-4,5-difluorobenzoïque.

### Préparation 10 : Acide 4-bromo-2-[4-(éthoxycarbonyl)-2-méthyl-1H-pyrrol-1-yl]benzoïque

Le composé du titre est obtenu suivant le procédé de la Préparation 1 en remplaçant l'acide 2-aminobenzoïque par l'acide 2-amino-4-bromobenzoïque.

### Préparation 11 : Acide 6-[4-(éthoxycarbonyl)-2-méthyl-1H-pyrrol-1-yl]-1,3-benzodioxole-5-carboxylique

Le composé du titre est obtenu suivant le procédé de la Préparation 1 en remplaçant l'acide 2-aminobenzoïque par l'acide 6-amino-1,3-benzodioxole-5-carboxylique (obtenu à partir du 6-nitro-1,3-benzodioxole-5-carbaldéhyde selon un protocole de la littérature : N. Mahindoo et al, Med Chem. Res. 14(6), 347, 2006).

### Préparation 12: Acide 4-chloro-2-[4-(ethoxycarbonyl)-2-méthyl-1H-pyrrol-1-yl]-5-fluorobenzoïque

Le composé du titre est obtenu suivant le procédé de la Préparation 1 en remplaçant l'acide 2-aminobenzoïque par l'acide 2-amino-4-chloro-5-fluorobenzoïque.

### Préparation 13 : Acide 5-bromo-2-[4-(éthoxycarbonyl)-2-méthyl-1H-pyrrol-1-yl]-benzoïque

Le composé du titre est obtenu suivant le procédé de la Préparation 1 en remplaçant l'acide 2-aminobenzoïque par l'acide 2-amino-5-bromobenzoïque.

### Préparation 14: Acide 6-[3-(méthoxycarbonyl)-1H-indol-1-yl]-1,3-benzodioxole-5-carboxylique

A une solution de 2 g (7,8 mmol) de 2-(2-bromophényl)-3-oxopropanoate de méthyle (préparé selon Heterocycles, 2008 2973-2980) dans 20 mL de méthanol sont ajoutés 1,4 g (7,8 mmol) d'acide 6-amino-1,3-benzodioxole-5-carboxylique (obtenu selon un protocole de la littérature : N. Mahindoo et al, Med Chem. Res. 14(6), 347, 2006). Le milieu réactionnel est laissé sous agitation pendant 48 h à température ambiante, puis évaporé à sec. On ajoute alors successivement 73 mg de CuI (0,38 mmol), 3,3 g de K₃PO₄ (15,6 mmol), 0,9 mL (15,6 mmol) d'éthylène glycol et 31 mL de diméthylformamide (DMF). Le milieu réactionnel est ensuite chauffé à 80°C pendant 15 h. Les solvants sont évaporés et 200 mL d'une solution aqueuse d'acide chlorhydrique 1M est ajouté au résidu. Cette phase aqueuse est extraite à l'acétate d'éthyle (3x100 mL). Les phases organiques sont réunies et séchées sur sulfate de magnésium, puis filtrée et évaporée à sec. Le produit brut ainsi obtenu est alors purifié par chromatographie sur gel de silice (gradient dichlorométhane/ méthanol), puis trituré dans un mélange dichlorométhane/diisopropyléther pour conduirer au produit attendu sous la forme d'une poudre.
**RMN¹H :** δ (400 MHz; dmso-d6; 300K): 12-13 (m, 1H, CO₂H); 8.10 (s, 1H, H aromatique) ; 8.05 (dd, 1H, H indole) ; 7.45 (s, 1H, H aromatique) ; 7.25 (m, 2H, H indole) ; 7.05 (d, 1H, H indole); 6.25 (s, 2H, méthylenedioxy); 3.85 (s, 3H, OCH3).
**IR :** v OH : 3100-2500 cm⁻¹ ; v : >C=O : 1687 cm⁻¹ (bande dédoublée)

### Préparation 15: Acide 4-bromo-2-[3-(méthoxycarbonyl)-1H-indol-1-yl]benzoïque

Le composé du titre est obtenu suivant le procédé de la Préparation 14 en remplaçant l'acide 6-amino-1,3-benzodioxole-5-carboxylique par l'acide 2-amino-4-bromobenzoïque.

### Préparation 16 : Acide 4-chloro-2-[3-(méthoxycarbonyl)-1H-indol-1-yl]benzoïque

Le composé du titre est obtenu suivant le procédé de la Préparation 14 en remplaçant l'acide 6-amino-1,3-benzodioxole-5-carboxylique par l'acide 2-amino-4-chlorobenzoïque.

### Préparation 17 : Acide 2,4-dichloro-6-[4-(éthoxycarbonyl)-2-méthyl-1H-Pyrrol-1-yl]benzoïque

Le composé du titre est obtenu suivant le procédé de la Préparation 1 en remplaçant l'acide 2-aminobenzoïque par l'acide 2-amino-4,6-dichlorobenzoïque.

### Préparation 18 : Acide 6-[4-(éthoxycarbonyl)-2-méthyl-1H-pyrrol-1-yl]-2,3-dihydro-1H-indène-5-carboxylique

Ce dérivé acide anthranilique est préparé en 2 étapes selon le protocole de la littérature (T. Yoshino et al, Chemistry letters, 38(3), 200, 2009) à partir de 20 g (150 mmol) de 2,3-dihydro-1*H*-indén-5-amine. Le produit du titre est obtenu, ainsi que 8% de son régioisomère qui est éliminé par lavage acido-basique.
**RMN ¹H :** δ (400 MHz; dmso-d6; 300K): 9.5-7.5 (m,1H, **COOH**); 7.55 (s, 1H, H aromatique); 6.55 (s, 1H, H aromatique); 2.7 (m, 4H, *H*-indane); 1.95 (m, 2H, *H*-indane)
**IR :** v :NH₂ : 3494- 3384 cm⁻¹; v : OH : 3000-2200 cm⁻¹ (OH acide); cm⁻¹ ; v : >C=O: 1672 cm⁻¹

### Préparation 19 : Acide 6-[3-(éthoxycarbonyl)-4,5,6,7-tétrahydro-1H-indol-1-yl]-1,3-benzodioxole-5-carboxylique

### Stade A : Mélange de (2,2-diéthoxycyclohexyl)acétate d'éthyle et de (2-éthoxycyclohex-2-én-1-yl)acétate d'éthyle

Le mélange de composés du titre est obtenu selon la méthode décrite dans la littérature (WO2007/054739) à partir de 15 g (81,4 mmol) de (2-oxocyclohexyl)acétate d'éthyle en solution dans 25 mL d'éthanol anhydre en présence de triéthylorthoformate 40 mL (244 mmol) et d'APTS 1,4g (8,13 mmol) comme catalyseur. Le milieu réactionnel est chauffé pendant une nuit à 95°C, puis concentré à sec. Le mélange des 2 composés est obtenu sous la forme d'une huile qui est utilisée directement pour l'étape suivante.
**RMN¹H :** δ (300 MHz ; CDCl₃ ; 300K) : 4.60 (t, 1H); 4.17-4.09 (m, 4H) ; 3.76-3.58 (m, 4H) ; 3.47-3.41 (m, 3H) ; 3.11 (s, 1H) ; 2.71-2.63 (m, 2H); 2.58-2.40 (m, 1H); 2.37-2.09 (m, 4H); 2.07-1.97 (m, 4H); 1.91-1.33 (m, 8H) ; 1.31-1.16 (m, 14H)
**R :** v : >C=O : 1740 cm⁻¹ ester

### Stade B : 3-Oxo-2-(2-oxocyclohexyl)propanoate d'éthyle

A une suspension 2,15 g (89,5 mmol) d'hydrure de sodium (60% dans l'huile) dans 40 mL de THF anhydre placée à 0°C sous atmosphère inerte, on ajoute, goutte à goutte, 20,6 g (77,8 mmol) du mélange obtenu au Stade A et 12,6 mL (155,6 mmol) d'éthylformate en solution dans 25 mL de THF. L'ensemble est agité à 0°C pendant 2 h, puis 12 h à température ambiante. Le milieu réactionnel est hydrolysé. La phase aqueuse est acidifiée en ajoutant une solution d'HCl concentré puis extraite avec de l'acétate d'éthyle. La phase organique est ensuite séchée sur MgSO₄, filtrée et concentrée à sec. On obtient le produit attendu sous la forme d'une huile.
**RMN¹H :** δ (300 MHz; CDCl₃ ; 300K) : 8.4 (s, 1H, C**H**O); 4.25-4.1 (m, 2H, H aliphatiques, COO**CH₂**CH₃); 2.9-2.75 (m, 1H, H aliphatique, CHO**CH**COOEt) ; 2.5-1.45 (m, 9H, H aliphatiques, cyclohexanone) ; 1.3 (m, 3H, H aliphatiques, COOCH₂**CH₃**)

### Stade C : Acide 6-[3-(éthoxycarbonyl)-4,5,6,7-tétrahydro-1H-indol-1-yl]-1,3-benzodioxole-5-carboxylique

A une solution de 4 g (18,84 mmol) du composé du Stade B dans 20 mL d'acide acétique, on ajoute ensuite, par pelletés, 3,4g (18,84 mmol) d'acide 6-amino-1,3-benzodioxole-5-carboxylique (obtenu selon un protocole de la littérature : N. Mahindoo et al, Med Chem. Res. 14(6), 347, 2006). L'ensemble est chauffé à 110°C pendant 2 h. Le milieu réactionnel est ensuite concentré à sec en co-évaporant avec du toluène. Le solide ainsi obtenu est trituré dans un mélange de pentane et d'éther diisopropylique (50/50), filtré et séché pour conduire au produit du titre.
**RMN¹H :** δ (300 MHz ; CDCl₃ ; 300K) : 7.5 (s, 1H, *H*-pyrrole); 7.20 (s, 1H, H aromatique); 6.70 (s, 1H, H aromatique); 6.12 (s, 2H, O-**CH₂**-O); 4.28 (q, 2H, H aliphatiques, COO**CH₂**CH₃); 2.80 (m, 2H, H aliphatiques, tétrahydroindole) ; 2.20 (m, 2H, H aliphatiques, tétrahydroindole) ; 1.75 (m, 4H, H aliphatiques, tétrahydroindole) ; 1.30 (t, 3H, H aliphatiques, COOCH₂**CH₃**)
**IR :** v : OH acide : 2800-2300 cm⁻¹ ; v : >C=O: 1672 cm⁻¹ (bande dédoublée acide + ester) ; v : >C=C< : 1616 cm⁻¹ aromatique)

### Préparation 20 : Acide 6-[4-(éthoxycarbonyl)-2-méthyl-1H-pyrrol-1-yl]-2,3-dihydro-1-benzofuran-5-carboxylique

Le composé du titre est obtenu suivant le procédé de la Préparation 1 en remplaçant l'acide 2-aminobenzoïque par l'acide 6-amino-2,3-dihydro-1-benzofuran-5-carboxylique.
**RMN ¹H :** δ (400 MHz ; dmso-d6 ; 300K) : 8.00 (s, 1H, H dihydrobenzofurane) ; 7.25 (d, 1H, H pyrrole); 6.65 (s, 1H, H dihydrobenzofurane); 6.40 (d, 1H, H pyrrole) ; 4.75 (t, 2H, H dihydrobenzofurane) ; 4.30 (q, 2H, COO**CH₂**CH₃) ; 3.30 (t, 2H, H dihydrobenzofurane) ; 2.0 (s, 3H, CH₃-pyrrole) ; 1.30 (t, 3H, COOCH₂**CH₃**)
**IR :** v :-OH : 3000-2000 cm⁻¹ ; v : C=O : 1702-1669 cm⁻¹

### Préparation 21 : Acide 6-[4-(éthoxycarbonyl)-2-méthyl-1H-pyrrol-1-yl]-1-benzofuran-5-carboxylique

Le composé du titre est obtenu suivant le procédé de la Préparation 1 en remplaçant l'acide 2-aminobenzoïque par l'acide 6-amino-1-benzofuran-5-carboxylique.
**RMN ¹H :** δ (400 MHz ; dmso-d6; 300K) : 12.80 (large s, 1H, **COOH**) ; 8.25 (s, 1H, H Benzofurane) ; 8.20 (s, 1H, H pyrrole) ; 7.75 (s, 1H, H benzofurane); 7.30 (s, 1H, H pyrrole); 7.20 (s, 1H, H benzofurane) ; 6.30 (s, 1H, benzofurane) ; 4.20 (q, 2H, COO**CH₂**CH₃) ; 1.9 (s, 3H, CH₃-pyrrole) ; 1.25 (t, 3H, COOCH₂**CH₃**)
**IR :** v :-OH : 2720-2450 cm⁻¹ ; C=O : 1698-1682 cm⁻¹

### Préparation 22 : Acide 4-chloro-2-[4-(méthoxycarbonyl)-2,3-diméthyl-1H-pyrrol-1-yl]benzoïque

3,35 g (153,18 mmol) de 4,5-diméthyl-1*H*-pyrrole-3-carboxylate de méthyle préparé selon la littérature (Synthetic uses of tosylmethyl isocyanide (TosMIC) Organic Reactions (Hoboken, NJ, United States) (2001), 57, No 418) et 4,63g (16,4 mmol) d'acide 4-chloro-2-iodo-benzoïque sont mis en solution dans 50 mL d'acétonitrile. On y ajoute du cuivre en poudre (45 µ) (280 mg, 4,37 mmol), ainsi que du carbonate de césium (14,25 g, 43,74 mmol). Le milieu réactionnel est chauffé à reflux pendant 12 h. L'avancement de la réaction est suivi par chromatographie liquide (LC). On laisse la suspension revenir à température ambiante, puis elle est filtrée, lavée avec de l'acétonitrile et évaporée à sec. Le résidu est repris dans de l'acétate d'éthyle. La solution est ensuite lavée avec de l'acide chlorhydrique 1M, puis avec une solution saturée de chlorure de sodium, elle est séchée sur sulfate de magnésium, filtrée puis évaporée à sec. Le composé ainsi obtenu est purifié sur colonne de gel de silice en utilisant le dichlorométhane et l'éthanol comme solvants.

### Masse (ESI+) :

Formule brute : C₁₅H₁₄ClNO₂
masse monoisotopique = 307.07 Da
[M+H]⁺mesuré: 308.12
(rapports isotopiques en accord avec un atome de chlore)

### Préparation 1' : [(3S)-1,2,3,4-Tétrahydroisoquinoléin-3-ylméthyl]carbamate de tert-butyle

### Stade A : (3S)-3-(Hydroxyméthyl)-3,4-dihydro-1H-isoquinoléine-2-carboxylate de benzyle

Ce dérivé est obtenu en utilisant un protocole de la littérature (R. B. Kawthekar et al South Africa Journal of Chemistry 63, 195, 2009) à partir de 15 g de (3*S*)-1,2,3,4-tétrahydroisoquinoléin-3-ylméthanol (91,9 mmol) en présence de chloroformate de benzyle et de triéthylamine en solution dans le dichlorométhane. Après purification sur gel de silice (gradient éther de pétrole/ AcOEt), le produit du titre est obtenu sous la forme d'une huile.
**RMN¹H :** δ (300 MHz ; DMSO-d6 ; 300K) : 7.33 (m, 5H, H aromatiques, O-Benzyl); 7.15 (s, 4H, H aromatiques, H tétrahydroisoquinoléine) ; 5.13 (s, 2H, **CH₂**-Ph) ; 4.73 (d, 1H, H tétrahydroisoquinoléine) ; 4.47 (m, H, CH₂O**H**) ; 4.36 (m, 1H, H tétrahydroisoquinoléine) ; 4.28 (d, 1H, H tétrahydroisoquinoléine) ; 3.39 (dd, 1H, **CH₂**OH) ; 3.23 (dd, 1H, **CH₂**OH) ; 2.93 (dd, 1H, H tétrahydroisoquinoléine) ; 2.86 (dd, 1H, H tétrahydroisoquinoléine)
**IR :** v : OH : 3416 cm⁻¹ ; v : <C=O 1694 cm⁻¹ ; v : >C-H aromatique: 754 cm⁻¹

### Stade B : (3S)-3-(Azidométhyl)-3,4-dihydroisoquinoléine-2(1H)-carboxylate de benzyle

Ce dérivé est obtenu en utilisant un protocole de la littérature (D. Pagé et al J. Med. Chem, 44, 2387, 2001) à partir de 23 g du composé obtenu au Stade A (77,3 mmol) en présence de diphénylphosphorylazide et de triphénylphosphine en solution dans le THF. Après purification sur gel de silice (gradient éther de pétrole/ AcOEt), le produit du titre est obtenu sous la forme d'une huile.
**RMN¹H :** δ (400 MHz ; DMSO-d6 ; 300K) : 7.36 (m, 5H, H aromatiques, O-Benzyl); 7.19 (m, 4H, H aromatiques, H tétrahydroisoquinoléine) ; 5.16 (s, 2H, **CH₂**-Ph) ; 4.76 (d, 1H, H tétrahydroisoquinoléine) ; 4.53 (m, 1H, H tétrahydroisoquinoléine) ; 4.30 (m, 1H, H tétrahydroisoquinoléine) ; 3.28 (m, 2H, **CH₂**N₃) ; 3.06 (dd, 1H, H tétrahydroisoquinoléine) ; 2.78 (dd, 1H, H tétrahydroisoquinoléine)
**IR :** v : N₃ : 2095 cm⁻¹ ; v : <C=O :1694 cm⁻¹ ; >C-H aromatique : 754 cm⁻¹

### Stade C : (3S)-3-(Aminométhyl)-3,4-dihydroisoquinoléine-2(1H)-carboxylate de benzyle

A une solution de 20,9 g (64,5 mmol) du dérivé azido obtenu au Stade B dans 650 mL de THF, on ajoute successivement 25,5 g (97,2 mmol) de triphénylphosphine et 157 mL d'eau. L'ensemble est porté à reflux pendant 2h30. Le milieu réactionnel est ensuite concentré à sec, puis l'huile résiduelle est reprise avec de l'éther isopropylique. Un précipité blanc apparait; il est filtré et lavé avec de l'éther isopropylique. Le filtrat est alors concentré à sec, puis purifié par chromatographie sur gel de silice (gradient CH₂Cl₂/MeOH). On obtient le produit du titre sous la forme d'une huile.
**RMN¹H :** δ (400 MHz ; DMSO-d6 ; 300K) : 7.40 (m, 5H, H aromatiques, O-Benzyl); 7.20 (m, 4H, H aromatiques, H tétrahydroisoquinoléine) ; 5.15 (s, 2H, **CH₂**-Ph) ; 4.75-4.3 (m, 2H, H tétrahydroisoquinoléine) ; 4.30 (d, 1H, H tétrahydroisoquinoléine) ; 2.90 (m, 2H, **CH₂**NH₂); 2.45 (m, 2H, H tétrahydroisoquinoléine) ; 1.40 (m, 2H, NH₂)
**IR :** v : NH₂ : 3400-3300 cm⁻¹ ; v : <C=O : 1688 cm⁻¹

### Stade D : (3S)-3-{[(tert-Butoxycarbonyl)amino]méthyl}-3,4-dihydroisoquinoléine-2(1H)-carboxylate de benzyle

A une solution de 18,4 g (62,1 mmol) du composé obtenu au Stade C dans 630 mL de dichlorométhane, on ajoute successivement 17,5 mL (124 mmol) de triéthylamine et, par pelletés, 14,9 g (68,3 mmol) de dicarbonate de di-*tert*-butyle. L'ensemble est agité à température ambiante pendant 2 h. On concentre ensuite le milieu réactionnel, puis on ajoute de l'actétate d'éthyle. La phase organique est lavée successivement avec une solution HCl 1M, une solution saturée de NaCl, une solution saturée de NaHCO₃, puis une solution de saturée de NaCl. Après séchage, concentration à sec et purification par chromatographie sur gel de silice (gradient éther de pétrole/ AcOEt), le produit du titre est obtenu sous la forme d'une huile.
**RMN¹H :** δ (400 MHz ; DMSO-d6 ; 300K) : 7.35 (m, 5H, H aromatiques, O-Benzyl); 7.15 (m, 4H, H aromatiques, H tétrahydroisoquinoléine) ; 6.51 (m, 1H, **NH**Boc) ; 5.12 (s, 2H, **CH₂**-Ph) ; 4.76 (d, 1H, H tétrahydroisoquinoléine) ; 4.51 (m, 1H, H tétrahydroisoquinoléine) ; 4.36 (d, 1H, H tétrahydroisoquinoléine) ; 2.95 (m, 3H, H tétrahydroisoquinoléine + **CH₂**NHBoc); 2.71 (d, 1H, H tétrahydroisoquinoléine) ; 1.34 (s, 9H, NH**Boc**)
**IR :** v : NH : 3351 cm⁻¹; v : <C=O : 1686 cm⁻¹

### Stade E : [(3S)-1,2,3,4-Tétrahydroisoquinoléin-3-ylméthyl]carbamate de tert-butyle

A une solution de 21 g (53 mmol) du composé obtenu au Stade D dans 600 mL d'acétate d'éthyle, on ajoute 2,1 g de palladium sur charbon 10%. L'ensemble est agité à température ambiante sous 1,3 bar de pression de dihydrogène pendant 5 h. Le milieu réactionnel est ensuite filtré, puis concentré à sec. Le produit du titre est obtenu sous la forme d'un solide.
**RMN¹H :** δ (400 MHz ; DMSO-d6 ; 300K) : 7.15 (m, 4H, H aromatiques, H tétrahydro isoquinoléine) ; 6.85 (t, 1H, **NH**Boc); 3.90 (m, 2H, H tétrahydroisoquinoléine) ; 3.00 (m, 2H, **CH₂**NHBoc); 2.80 (m, 1H, H tétrahydroisoquinoléine) ; 2.65 (dd, 1H, H tétrahydro isoquinoléine) ; 2.40 (dd, 1H, H tétrahydro isoquinoléine) ; 1.40 (s, 9H, NH**Boc**)
**IR :** v : NH : 3386-3205 cm⁻¹ (NH amide); v : <C=O: 1688 cm⁻¹ ; v : NH : 1526 cm⁻¹ (NH amine)

### Préparation 2' : (3S)-3-(4-Morpholinylméthyl)-1,2,3,4-tétrahydroisoquinoléine

### Stade A : (3S)-3-(4-Morpholinylcarbonyl)-3,4-dihydro-2(1H)-isoquinoléine carboxylate de benzyle

A une solution de 5 g d'acide (3*S*)-2-[(benzyloxy)carbonyl]-1,2,3,4-tétrahydro-3-isoquinoléinecarboxylique (16 mmol) dans 160 mL de dichlorométhane sont ajoutés 1,5 mL de morpholine (17,6 mmol), puis 9 mL de *N*,*N*,*N*-triéthylamine (64 mmol), 3,3 g de 1-éthyl-3-(3'-diméthylaminopropyl)-carbodiimide (EDC) (19,2 mmol), et 2,6 g d'hydroxybenzotriazole (HOBT) (19,2 mmol). Le milieu réactionnel est agité à température ambiante pendant une nuit, puis il est versé sur une solution de chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient dichlorométhane/ méthanol). Le produit est obtenu sous la forme d'une mousse.
**RMN¹H:** δ (400 MHz; dmso-d6; 353°K) : 7.30 (m, 5H benzyl); 7.15 (m, 4H aromatiques) ; 5.2-5.0 (m, 3H, 2H benzyl, 1H dihydroisoquinoléine); 4.75-4.5 (2d, 2H dihydroisoquinoléine); 3.55-3.3 (m, 8H morpholine); 3.15-2.9 (2dd, 2H dihydroisoquinoléine)
**IR :** v : >C=O : 1694-1650 cm⁻¹

### Stade B : (3S)-3-(4-Morpholinylméthyl)-3,4-dihydro-2(1H)-isoquinoléine carboxylate de benzyle

A une solution de 5,3 g du produit obtenu au Stade A (13,9 mmol) dans 278 mL de tétrahydrofurane sont ajoutés 14 mL de BH₃Me₂S (27,8 mmol) à température ambiante. Le tout est chauffé pendant 4 heures à 80°C. On laisse revenir à température ambiante, puis on ajoute 7 mL (14 mmol) de BH₃Me₂S. Le milieu réactionnel est de nouveau chauffé à 80°C pendant 2 heures. On évapore ensuite le tétrahydrofurane, puis on ajoute lentement du méthanol, puis 5,6 mL d'acide chlorhydrique 5N (27,8 mmol). Le mélange est agité à température ambiante pendant une nuit, puis à 80°C pendant 1h. On ajoute ensuite une solution de NaHCO₃ saturée sur le milieu réactionnel placé à 0°C jusqu'à atteindre un pH=8, puis on extrait avec de l'acétate d'éthyle. La phase organique est ensuite séchée sur sulfate de magnésium, puis filtrée et évaporée à sec. Le produit du titre est obtenu sous la forme d'une huile.
**RMN¹H :** δ (400 MHz ; dmso-d6 ; 353°K) : 7.43-7.30 (massif, 5H benzyl) ; 7.19 (m, 4H aromatiques) ; 5.16 (m, 2H, 2H benzyl) ; 4.79-4.29 (d, 2H dihydroisoquinoléine) ; 4.58 (m, 1H dihydroisoquinoléine) ; 3.50 (m, 4H morpholine) ; 3.02-2.80 (dd, 2H dihydroisoquinoléine) ; 2.42-2.28 (massif, 5H, 4H morpholine, 1H morpholine) ; 2.15 (dd, 1H morpholine)
**IR :** v : >CH : 2810 cm⁻¹ ; v : >C=O : 1694 cm⁻¹ ; v : >C-O-C< : 1114 cm⁻¹ ; v : >CH-Ar : 751 ; 697 cm⁻¹

### Stade C : (3S)-3-(4-Morpholinylméthyl)-1,2,3,4-tétrahydroisoquinoléine

A une solution de 4,9 g du composé du Stade B (13,4 mmol) dans 67 mL d'éthanol est ajouté 0,980 g de dihydroxyde de palladium (20% massique) à température ambiante. Le milieu réactionnel est placé sous 1,2 bar de d'hydrogène à température ambiante pendant 4 heures. Il est ensuite passé sur une filtre Wathman, puis le palladium est rincé plusieurs fois avec de l'éthanol. Le filtrat est évaporé à sec. Le produit du titre est obtenu sous la forme d'une huile.
**RMN¹H :** δ (400 MHz ; dmso-d6; 300K) : 7.12-7.0 (massif, 4H aromatiques) ; 3.92 (s, 2H tétrahydroisoquinoléine) ; 3.60 (t, 4H morpholine) ; 2.98 (m, 1H tétrahydroisoquinoléine) ; 2.68 (dd, 1H tétrahydroisoquinoléine) ; 2.5-2.3 (massif, 8H, 1H tétrahydroisoquinoléine, 6H morpholine, 1H NH)
**IR :** v : >NH : 3322 cm⁻¹; v : >C-O-C< : 1115 cm⁻¹; v : >CH-Ar : 742 cm⁻¹

### Préparation 3' : (3S)-3-[(4-Méthyl-1-piperazinyl)méthyl]-1,2,3,4-tétrahydroisoquinoléine

On procède selon le procédé de la Préparation 2' en remplaçant la morpholine utilisée au Stade A par la 1-méthyl-pipérazine.

### Préparation 4': (3S)-3-[(3aR,6aS)-Hexahydrocyclopenta[c]pyrrol-2(1H)-ylméthyl]-1,2,3,4-tétrahydroisoquinoléine

On procède selon le procédé de la Préparation 2' en remplaçant la morpholine utilisée au Stade A par la (3a*R*,6a*S*)-octahydrocyclopenta[*c*]pyrrole.
**RMN¹H :** δ (400 MHz ; DMSO-d6 ; 300K) : 7.05 (m, 4H, H aromatiques, H tétrahydroisoquinoléine) ; 3.90 (s, 2H, H tétrahydroisoquinoléine) ; 2.85 (m, 1H, H tétrahydroisoquinoléine) ; 2.70 (dd, 1H, H tétrahydroisoquinoléine) ; 2.7-2.3 (m, 6H) ; 2 ;4-2.3 (2dd, 2H) ; 2.2-2.1 (2dd, 2H, amine bicyclique) ; 1.7-1.3 (2m, 6H, amine bicyclique)

### Préparation 5' : (3S)-3-[(4,4-Difluoropipéridin-1-yl)méthyl]-1,2,3,4-tétrahydroisoquinoléine

On procède selon le procédé de la Préparation 2' en remplaçant la morpholine utilisée au Stade A par la 4,4-difluoro-1-pipéridine.
**RMN¹H :** δ (300 MHz ; DMSO-d6 ; 300K) : 10.2 (large s, 1H, NH₂⁺); 7.25 (m, 4H, H aromatiques, H tétrahydroisoquinoléine) ; 4.40 (s, 2H, H tétrahydroisoquinoléine) ; 4.20 (m, 1H, H tétrahydroisoquinoléine) ; 3.75-3.35 (m, 6H, H difluoropipéridine) ; 3.3-3.1 (2dd, 2H, H tétrahydroisoquinoléine) ; 2.4 (m, 4H, H difluoropipéridine)
**IR :** v : NH₂⁺ : 2782-2381 cm⁻¹

### Préparation 6' : (3S)-3-[(4-Cyclopentylpipérazin-1-yl)méthyl]-1,2,3,4-tétrahydroisoquinoléine

On procède selon le procédé de la Préparation 2' en remplaçant la morpholine utilisée au Stade A par la 1-cyclopentylpipérazine.
**RMN¹H :** δ (400 MHz ; DMSO-d6 ; 300K) : 7.20 (m, 4H, H aromatiques, H tétrahydroisoquinoléine) ; 4.30 (large s, 2H, H tétrahydroisoquinoléine) ; 3.70 (m, 1H, H tétrahydroisoquinoléine) ; 3.50 (m, 3H, H pipérazine) ; 3.10 (m, 4H) ; 3.1-2.75 (2 m, 4H, H pipérazine) ; 2.85 (dd, 1H) ; 2.60 (m, 1H) ; 2.00 (m, 2H, H cyclopentyl) ; 1.75 (m, 4H , H cyclopentyl) ; 1.55 (m, 2H, H cyclopentyl)
**IR :** v : NH⁺/NH₂⁺ : 3550-2000 cm⁻¹ ; v : >C-H aromatique : 761 cm⁻¹

### Préparation 7': (3S)-3-[(4-Cyclobutylpipérazin-1-yl)méthyl]-1,2,3,4-tétrahydroisoquinoléine

On procède selon le procédé de la Préparation 2' en remplaçant la morpholine utilisée au Stade A par la 1-cyclobutylpipérazine.
**RMN¹H :** δ (400 MHz ; DMSO-d6 ; 300K) : 7.20 (m, 4H, H aromatiques, H tétrahydroisoquinoléine) ; 4.20 (2 d, 2H, H tétrahydroisoquinoléine) ; 3.55 (m, 1H, H tétrahydroisoquinoléine) ; 3.00 (dd, 1H, H tétrahydroisoquinoléine) ; 2.85 (m, 1H) ; 2.75 (dd, 1H, H tétrahydroisoquinoléine) ; 2.7-2.2 (m, 8H, H pipérazine); 2.65 (dd, 1H) ; 2.55 (dd, 1H) ; 2.1-1.5 (m, 6H, H cyclobutyl)
**IR :** v : NH⁺ : 2900-2050 cm⁻¹ ; v : <C=C< : 1603 cm⁻¹; v : >C-H aromatique : 754 cm⁻¹

### Préparation 8' : (3S)-3-(Pyrrolidin-1-ylméthyl)-1,2,3,4-tétrahydroisoquinoléine

On procède selon le procédé de la Préparation 2' en remplaçant la morpholine utilisée au Stade A par la pyrrolidine.
**RMN¹H :** δ (400 MHz; DMSO-d6; 300K): 7.10 (m, 4H, H aromatiques, H tétrahydroisoquinoléine) ; 3.90 (s, 2H, H tétrahydroisoquinoléine) ; 2.90 (m, 1H, H tétrahydroisoquinoléine) ; 2.70 (dd, 1H, H tétrahydroisoquinoléine) ; 2.5-2.4 (m, 7H) ; 1.7 (m, 4H, pyrrolidine)
**IR :** v : NH : 3400-3300 cm⁻¹

### Préparation 9' : Chlorhydrate de (3R)-3-méthyl-1,2,3,4-tétrahydroisoquinoléine

### Stade A : {(3S)-2-[(4-Méthylphényl)sulfonyl]-1,2,3,4-tétrahydroisoquinoléin-3-yl}méthyl 4-méthylbenzènesulfonate

A une solution de 30,2 g de [(3*S*)-1,2,3,4-tétrahydroisoquinoléin-3-yl]méthanol (185 mmol) dans 750 mL de dichlorométhane sont ajoutés successivement 91,71 g de chlorure de tosyle (481 mmol), puis, au goutte à goutte, 122,3 mL de *N,N,N*-triéthylamine (740 mmol). Le milieu réactionnel est ensuite agité à température ambiante durant 20 h. Il est ensuite dilué avec du dichlorométhane, lavé successivement avec une solution HCl 1M, une solution saturée de NaHCO₃, puis une solution saturée de NaCl jusqu'à neutralité. La phase organique est alors séchée sur MgSO₄, filtrée, concentrée à sec. Le solide obtenu est alors solubilisé dans un volume minimum de dichlorométhane, puis est ajouté du cyclohexane jusqu'à formation d'un précipité. Ce précipité est alors filtré et lavé avec du cyclohexane. Après séchage, le produit du titre est obtenu sous forme de cristaux.
**RMN ¹H :** δ (400 MHz ; dmso-d6; 300K) : 7.75 (d, 2H , H aromatiques, *ortho* O-tosyle); 7.6 (d, 2H , H aromatiques, *ortho* N-tosyle); 7.5 (d, 2H , H aromatiques, *méta* O-tosyle); 7.3 (d, 2H , H aromatiques, *méta* N-tosyle); 7.15-6.9 (m, 4H, H aromatiques, tétrahydro isoquinoléine); 4.4-4.15 (dd, 2H, H aliphatiques, tétrahydroisoquinoléine); 4.25 (m, 1H, H aliphatique, tétrahydroisoquinoléine); 4.0-3.8 (2dd, 2H, H aliphatiques, **CH₂**-O-tosyle); 2.7 (2dd, 2H, H aliphatiques, tétrahydroisoquinoléine) ; 2.45 (s, 3H, O-SO₂-Ph-**CH₃**); 2.35 (s, 3H, *N*-SO₂-Ph-**CH₃**)
**IR :** v : -SO₂ : 1339-1165 cm⁻¹

### Stade B : (3R)-3-Méthyl-2-[(4-méthylphényl)sulfonyl]-1,2,3,4-tétrahydroisoquinoléine

A une suspension de 8,15 g (214,8 mmol) de LiAlH₄ dans 800 mL de méthyl *tert*-butyl éther (MTBE), on ajoute 101,2 g du dérivé ditosylé obtenu au Stade A (214,8 mmol) en solution dans 200 mL de MTBE. L'ensemble est ensuite chauffé à 50°C pendant 2 h. On laisse refroidir, on se place à 0°C, puis on ajoute, goutte à goutte, 12 mL d'une solution de NaOH 5N. L'ensemble est agité à température ambiante pendant 45 minutes. Le solide ainsi obtenu est alors filtré, lavé avec du MTBE, puis avec du dichlorométhane. Le filtrat est ensuite concentré à sec. On obtient alors le produit du titre sous la forme d'un solide
**RMN ¹H :** δ (400 MHz ; dmso-d6; 300K) : 7.70 (d, 2H , H aromatiques, *ortho* N-tosyle); 7.38 (d, 2H , H aromatiques, *méta* N-tosyle); 7.2-7.0 (m, 4H, H aromatiques, tétrahydroisoquinoléine); 4.4 (m, 2H, H aliphatiques, tétrahydroisoquinoléine); 4.3 (m, 1H, H aliphatique, tétrahydroisoquinoléine) ; 2.85-2.51 (2dd, 2H, H aliphatiques, tétrahydro isoquinoléine); 2.35 (s, 3H, *N*-SO₂-Ph-**CH₃**); 0.90 (d, 3H, tétrahydroisoquinoléine-**CH₃**)
**IR :** v : -SO₂ : 1332-1154 cm⁻¹

### Stade C : (3R)-3-Méthyl-1,2,3,4-tétrahydroisoquinoléine

A une solution de 31,15 g (103,15 mmol) du dérivé monotosylé obtenu au Stade B dans 500 mL de méthanol anhydre, on ajoute 3,92 g (161 mmol) de tournure de magnésium par pelletés. L'ensemble est agité en présence d'ultra-sons pendant 96 h. Le milieu réactionnel est ensuite filtré et le solide est lavé plusieurs fois avec du méthanol. Le filtrat est ensuite concentré à sec. Après purification par colonne chromatographique sur gel de silice (dichlorométhane /EtOH /NH₄OH), on obtient le produit du titre sous la forme d'une huile.
**RMN ¹H :** δ (400 MHz ; dmso-d6; 300K) : 7.05 (m, 4H, H aromatiques, tétrahydroisoquinoléine); 3.90 (m, 2H, H aliphatiques, tétrahydroisoquinoléine); 2.85 (m, 1H, H aliphatique, tétrahydroisoquinoléine); 2.68-2.4 (2dd, 2H, H aliphatiques, tétrahydro isoquinoléine); 1.12 (d, 3H, tétrahydroisoquinoléine-**CH₃**); 2.9-2.3 (m, large, 1H, **HN**(tétrahydroisoquinoléine))
**IR :** v : -NH : 3248 cm⁻¹

### Stade D : Chlorhydrate de (3R)-3-méthyl-1,2,3,4-tétrahydroisoquinoléine

A une solution de 14,3 g (97.20 mmol) du composé obtenu au Stade C dans 20 mL d'éthanol anhydre, on ajoute, goutte à goutte, 100 mL d'une solution d'éther chlorhydrique 1M. L'ensemble est agité à température ambiante pendant 1h, puis filtré. Les cristaux ainsi obtenus sont lavés avec de l'éther éthylique. Après séchage, le produit du titre est obtenu sous la forme de cristaux.
**RMN ¹H :** δ (400 MHz ; dmso-d6; 300K) : 9.57 (m, large, 2H, **NH₂⁺**(tétrahydro isoquinoléine)); 7.22 (m, 4H, H aromatiques, tétrahydroisoquinoléine); 4.27 (s, 2H, H aliphatiques, tétrahydroisoquinoléine); 3.52 (m, 1H, H aliphatique, tétrahydroisoquinoléine); 3.03-2.85 (2dd, 2H, H aliphatiques, tétrahydroisoquinoléine); 1.39 (d, 3H, tétrahydroisoquinoléine-**CH₃**)
**IR :** v : -NH₂⁺ : 3000-2300 cm⁻¹ ; v : -CH aromatique : 766 cm⁻¹

### Préparation 10' : Chlorhydrate de (3S)-3-[2-(morpholin-4-yl)éthyl]-1,2,3,4-tétrahydroisoquinoléine

### Stade A : (3S)-3-(2-Morpholino-2-oxo-éthyl)-3,4-dihydro-1H-isoquinoléine-2-carboxylate de tert-butyle

A une solution de 3 g (10,30 mmol) d'acide [(3*S*)-2-(*tert*-butoxycarbonyl)-1,2,3,4-tétrahydroisoquinoléin-3-yl]acétique dans 100 mL de dichlorométhane, on ajoute goutte à goutte 1,10 mL (11,32 mmol) de morpholine, puis, toujours goutte à goutte, 4,3 mL (30,9 mmol) de triéthylamine, 2,20 g (12,40 mmol) de 1,2-dichlorométhane et 1,70g (1,68 mmol) d'hydroxybenzotriazole. L'ensemble est agité à température ambiante pendant 15 h. Le milieu réactionnel est ensuite dilué avec du dichlorométhane, lavé successivement avec une solution HCl 1M, une solution saturée de NaHCO₃, puis une solution saturée de NaCl jusqu'à neutralité. La phase organique est alors séchée sur MgSO₄, filtrée et concentrée à sec. Après purification par colonne chromatographique sur gel de silice (dichlorométhane /MeOH), on obtient le produit du titre sous la forme d'une huile.
**RMN ¹H :** δ (400 MHz; dmso-d6; 300K): 7.20-7.10 (m, 4H, H aromatiques, tétrahydroisoquinoléine); 4.70 (m, 1H, H aliphatiques, CH tétrahydroisoquinoléine); 4.75-4.20 (2m, 2H, H aliphatiques, CH₂ en alpha du N, tétrahydroisoquinoléine); 3.60 (m, 8H, H aliphatiques, morpholine); 3.00 et 2.70 (2dd, 2H, H aliphatique, tétrahydroisoquinoléine); 2.50-2.20 (2d, 2H, H aliphatiques, CH₂CO); 1.40 (s, 9H, *^{t}*Bu)
**IR :** v : C=O : 1687 ;1625 cm⁻¹

### Stade B : Chlorhydrate de 1-(morpholin-4-yl)-2-[(3S)-1,2,3,4-tétrahydroisoquinoléin-3-yl]ethanone

A une solution de 2,88 g (7,18 mmol) du composé obtenu au Stade A dans 16 mL de dichlorométhane, on ajoute goutte à goutte 80 mL (80 mmol) d'une solution d'éther chlorhydrique 1M. L'ensemble est agité à température ambiante pendant 15 h, puis la suspension est filtrée et le précipité lavé à l'éther. Après séchage, on obtient le produit du titre sous la forme d'un solide.
**RMN ¹H :** δ (400 MHz ; dmso-d6; 300K) : 9.80-9.50 (m, 2H, NH₂⁺); 7.30-7.10 (m, 4H, H aromatiques, tétrahydroisoquinoléine); 4.30 (m, 2H, H aliphatiques, CH₂ en alpha du N, tétrahydroisoquinoléine); 3.80 (m, 1H, H aliphatiques, CH tétrahydroisoquinoléine); 3.70-3.40 (2m, 8H, H aliphatiques, morpholine); 3.15 et 2.8 (m, 4H, H aliphatique, CH₂ tétrahydroisoquinoléine et CH₂CO)
**IR :** v : -NH₂⁺ : 2800-1900 cm⁻¹ ; v : C=O : 1620 cm⁻¹

### Stade C : Chlorhydrate de (3S)-3-[2-(morpholin-4-yl)éthyl]-1,2,3,4-tétrahydroisoquinoléine

On prépare une solution de 2,2 g (7,44 mmol) du composé obtenu au Stade B dans 22 mL de MTBE et 5 mL de dichlorométhane. Après refroidissement dans un bain de glace à 0°C, on y ajoute goutte à goutte 15 mL (15 mmol) d'une solution de LiAlH₄ 1M dans le tétrahydrofurane. L'ensemble est ensuite agité à température ambiante pendant 6 h. On se place à 0°C, puis on ajoute goutte à goutte 1 mL d'une solution de NaOH 5N. L'ensemble est agité à température ambiante pendant 45 minutes. Le solide est alors filtré et lavé avec du MTBE, puis avec du dichlorométhane et le filtrat est concentré à sec. L'huile ainsi obtenue est diluée au dichlorométhane et on ajoute goutte à goutte 6,3 mL d'une solution d'éther chlorhydrique 1M. L'ensemble est agité à température ambiante pendant 1h, puis filtré. Les cristaux ainsi obtenus sont lavés avec de l'éther éthylique. Après séchage, on obtient le produit du titre sous la forme d'un solide.
**RMN ¹H :** δ (400 MHz ; dmso-d6 ; 300K) : 11.35 + 9.80 (2m, 2H, NH₂⁺); 10.00 (m, H, NH⁺); 7.20 (m, 4H, H aromatiques, tétrahydroisoquinoléine); 4.30 (s, 2H, H aliphatiques, CH₂ en alpha du N, tétrahydroisoquinoléine); 4.00 + 3.85 (2m, 4H, H aliphatiques, CH₂ en alpha du N, morpholine); 3.70 (m, 1H, H aliphatiques, CH tétrahydroisoquinoléine); 3.55-3.30 (m, 4H, H aliphatiques, CH en alpha du O morpholine et CH₂ morpholine); 3.15 (dd, 1H, H aliphatique, CH₂ tétrahydroisoquinoléine); 3.10 (m, 2H, H aliphatique, CH en alpha du O, morpholine) ; 2.90 (dd, 1H, H aliphatique, CH₂ tétrahydroisoquinoléine); 2.30 + 2.15 (2m, 2H, H aliphatique, CH₂ tétrahydroisoquinoléine)
**IR :** v : NH⁺ / -NH₂⁺ : entre 3500 et 2250 cm⁻¹ ; v : C=C : faible 1593 cm⁻¹ ; v : C-H aromatique : 765 cm⁻¹

### Préparation 11' : (3S)-3-(Pipéridin-1-ylméthyl)-1,2,3,4-tétrahydroisoquinoléine

On procède selon le procédé de la Préparation 2' en remplaçant la morpholine utilisée au Stade A par la pipéridine.
**RMN¹H :** δ (400 MHz; DMSO-d6; 300K): 7.15 (m, 4H, H aromatiques, H tétrahydroisoquinoléine) ; 3.85 (s, 2H, H tétrahydroisoquinoléine) ; 2.90 (m, 1H, H tétrahydroisoquinoléine) ; 2.75 (dd, 1H, H tétrahydroisoquinoléine) ; 2.5-2.4 (m, 7H) ; 1.7 (m, 6H, pipéridine)

### Préparation 12' : N,N-Diméthyl-1-[(3S)-1,2,3,4-tétrahydroisoquinoléin-3-yl] méthanamine

On procède selon le procédé de la Préparation 2' en remplaçant la morpholine utilisée au Stade A par la *N,N*-diméthylamine.

### Préparation 1" : 4-Benzyloxy-N-phényl-aniline

A une solution de 4-hydroxy-N-phényl-aniline (30 g ; 162 mmol) dans de l'acétonitrile (400 mL), on ajoute 58 g de Cs₂CO₃ (178 mmol) et on agite pendant 15 minutes à température ambiante. Le bromure de benzyle (22.5 mL ; 178 mmol) est ensuite ajouté au goutte-à-goutte, et le milieu réactionnel est chauffé au reflux pendant 4 h. Après filtration et rinçage à l'acétonitrile, le filtrat est concentré et chromatographié sur gel de silice (gradient éther de pétrole / AcOEt). Le produit du titre est alors obtenu sous la forme d'un solide incolore.
**RMN¹H :** δ (400 MHz ; dmso-d6; 300K) : 7.80 (m, 1H, NH) ; 7.45 (m, 2H, aryl) ; 7.40 (m, 2H, aryl) ; 7.30 (m, 1H, aryl) ; 7.15 (s, 2H, aryl); 7.05 (d, 2H, aryl) ; 6.9-7.0 (m, 4H, aryl) ; 6.70 (t, 1H, aryl) ; 5.05 (s, 2H, benzyle)
**IR :** v : >NH : 3408 cm⁻¹

### Préparation 2" : N-(4-{[tert-Butyl(diméthyl)silyl]oxy}phényl)-1-benzothiophén-5-amine

### Stade A : 4-{[tert-Butyl(diméthyl)silyl]oxy}aniline

Le composé du titre est obtenu à partir du 4-aminophénol dans le THF en présence d'imidazole et de chlorure de *tert*-butyl(diméthyl)silyle selon le protocole décrit dans la littérature (S. Knaggs et al, Organic & Biomolecular Chemistry, 3(21), 4002-4010; 2005).
**RMN ¹H :** δ (400 MHz ; dmso-d6; 300K) : 6.45-6.55 (dd, 4H, H aromatiques); 4.60 (m, 2H, **NH₂**-Ph) ; 0.90 (s, 9H, Si(CH₂)₂CH(**CH₃**)₂); 0.10 (s, 6H, Si(**CH₂**)₂CH(CH₃)₂)
**IR :** v : -NH₂⁺ : 3300-3400 cm⁻¹

### Stade B : N-(4-{[tert-Butyl(diméthyl)silyl]oxy}phényl)-1-benzothiophén-5-amine

A une solution de 6 g (26,9 mmol) du composé obtenu au Stade A dans 250 mL de toluène anhydre, on ajoute successivement le *tert*-butylate de sodium (2,8 g ; 23,5 mmol), le Pd(OAc)₂ (500 mg ; 2,3 mmol), le 1,1-bis(diphénylphosphino)ferrocène (2,6 g ; 4,7 mmol) et le 5-bromobenzothiophène (5 g ; 23,5 mmol). L'ensemble est dégazé par bullage d'argon durant 30 minutes, puis chauffé à reflux pendant 17 h. On laisse refroidir. Le milieu réactionnel est concentré à sec, puis repris dans le dichlorométhane, filtré sur célite puis de nouveau concentré à sec. Le résidu est alors purifié par chromatographie sur gel de silice (gradient CH₂Cl₂/AcOEt) pour fournir le produit attendu sous la forme d'un solide.
**RMN ¹H :** δ (400 MHz ; dmso-d6; 300K) : 7.90 (s, 1H, NH); 7.75 (d, 1H, CH aromatique); 7.65 (d, 1H, CH thiophène); 7.40 (d, 1H, CH aromatique); 7.30 (d, 1H, CH thiophène); 7.05 (d, 2H, CH aromatique); 7.00 (dd, 1H, CH aromatique); 6.80 (d, 2H, CH aromatique); 0.95 (s, 9H, *^{t}*Bu); 0.20 (s, 6H, SiCH₃)
**IR :** v >NH : 3397 cm⁻¹

### Préparation 3" : 5-(Phénylamino)-1H-indole-1-carboxylate de tert-butyle

On procède selon le procédé du Stade B de la Préparation 2" en remplaçant le 5-bromobenzothiophène utilisé par le 5-bromo-1*H*-indole-1-carboxylate de *tert*-butyle et la 4-{[*tert*-butyl(diméthyl)silyl]oxy}aniline par l'aniline.
**RMN ¹H :** δ (400 MHz ; dmso-d6; 300K) : 8.04 (s, 1H); 7.92 (d, 1H); 7.59 (s, 1H); 7.31 (d, 1H); 7.20 (t, 2H); 7.07 (dd, 1H); 7.03 (d, 2H); 6.76 (t, 1H); 6.61 (d, 1H); 1.63 (s, 9H)

### Préparation 4" : 6-(Phénylamino)-1H-indole-1-carboxylate de tert-butyle

On procède selon le procédé du Stade B de la Préparation 2" en remplaçant le 5-bromobenzothiophène utilisé par le 6-bromo-1*H*-indole-1-carboxylate de *tert*-butyle et la 4-{[*tert*-butyl(diméthyl)silyl]oxy}aniline par l'aniline.
**RMN ¹H :** δ (400 MHz ; CDCl₃ ; 300K) : 8.00 (s, 1H); 7.92 (d, 1H); 7.50 (s, 1H); 7.31 (d, 1H); 7.20-7.03 (m, 4H); 6.76 (t, 1H); 6.80 (d, 1H); 1.65 (s, 9H)

### Préparation 5" : 5-[(4-{[tert-Butyl(diméthyl)silyl]oxy}phényl)amino]-1H-indole-1-carboxylate de tert-butyle

On procède selon le procédé de la Préparation 2" en remplaçant le 5-bromobenzothiophène utilisé par le 5-bromo-1*H*-indole-1-carboxylate de *tert*-butyle.
**RMN ¹H :** δ (400 MHz ; dmso-d6; 300K): 7.85 (d, 1H); 7.78 (s, 1H); 7.55 (d, 1H); 7.15 (d, 1H); 6.95 (m, 3H); 6.75 (d, 2H); 6.58 (d, 1H); 1.65 (s, 9H); 1.00 (s, 9H); 0.2 (s, 6H)

### Préparation 6" : 4-{[tert-Butyl(diméthyl)silyl]oxy}-3-chloro-N-phénylaniline

On procède selon le procédé du Stade B de la Préparation 2" en remplaçant le 5-bromobenzothiophène utilisé par le bromobenzène et la 4-{[*tert-*butyl(diméthyl)silyl]oxy}aniline par la 4-{[*tert*-butyl(diméthyl)silyl]oxy}-3-chloroaniline (obtenue selon un protocole de la littérature : Bioorg Med Chem Lett 22(14), 4839-4843).
**RMN ¹H :** δ (400 MHz ; dmso-d6; 300K) : 7.2-7.1 (m, 3H); 7.15-7.05 (m, 2H); 7-6.8 (m, 4 H); (s, 9H); 0.20 (s, 6H)

### Préparation 7" : N-(4-{[tert-Butyl(diméthyl)silyl]oxy}phényl)-3-phénoxyaniline

On procède selon le procédé de la Préparation 2" en remplaçant le 5-bromobenzothiophène utilisé au Stade B par le 1-bromo-3-phénoxybenzène.
**RMN ¹H :** δ (400 MHz ; dmso-d6; 300K) : 7.98 (s, 1H, NH); 7.38 (d, 1H); 7.15 (m, 2H); 7.00 (d, 2H) ; 6.95 (d, 2H); 6.75 (d, 2H); 6.70 (d, 2H); 6.50 (t, 1H); 6.35 (dd, 1H); 0.95 (s, 9H, *^{t}*Bu); 0.20 (s, 6H, SiCH₃)
**IR :** v >NH : 3300 cm⁻¹

### Préparation 8" : 4-{[tert-Butyl(diméthyl)silyl]oxy}-N-(2-phényléthyl)aniline

A une solution de 4-[*tert*-butyl(diméthyl)silyl]oxyaniline (6 g ; 26,9 mmol) et de phénylacétaldéhyde (4g ; 33,6 mmol) dans un mélange d'isopropanol (270 mL) et d'eau (27 mL), sont ajoutés 17 g de formiate d'ammonium (270 mmol) et du Pd/C (2 g). Après 17 h d'agitation à température ambiante, le milieu réactionnel est filtré sur célite puis concentré à sec. Le résidu est alors dilué avec une solution saturée de bicarbonate de soude, puis extrait au dichlorométhane. La phase organique est séchée au MgSO₄, concentrée à sec et le résidu est purifié par chromatographie sur gel de silice (gradient heptane/AcOEt) pour fournir le produit attendu sous la forme d'huile.
**IR :** v >NH : 3300 cm⁻¹

### Préparation 9" : N-(4-{[tert-Butyl(diméthyl)silyl]oxy}phényl)-1-benzofuran-5-amine

On procède selon le procédé de la Préparation 2" en remplaçant le 5-bromobenzothiophène utilisé au Stade B par le 5-bromo-1-benzofurane.
**RMN ¹H :** δ (400 MHz ; dmso-d6 ; 300K) : 7.78 (s, 1H, H furane); 7.70 (s, 1H, NH); 7.40 (d, 1H, H aromatique); 7.20 (s, 1H, H aromatique); 6.95 (dd, 1H, H aromatique); 6.95 (d, 2H, H aromatique); 6.80 (d, 1H, H furane); 6.70 (d, 2H, H aromatique); 0.95 (s, 9H, *^{t}*Bu); 0.20 (s, 6H, SiCH₃)
IR : v >NH : 3401 cm⁻¹

### Préparation 10" : 4-{[tert-Butyl(diméthyl)silyl]oxy}-N-(4-méthoxyphényl)aniline

On procède selon le procédé de la Préparation 2" en remplaçant le 5-bromobenzothiophène utilisé au Stade B par le 1-bromo-4-méthoxybenzène.
**RMN ¹H :** δ (400 MHz ; dmso-d6; 300K) : 4.52 (s, 1H, NH); 6.95 (d, 2H, H aromatique); 6.85 (d, (d, 1H, H aromatique); 7.20 (s, 1H, H aromatique); 6.95 (dd, 1H, H aromatique); 6.95 (d, H, H aromatique) ; 6.85 (d, 2H, H aromatique); 6.70 (d, 2H, H aromatique) ; 3.70 (s, 3H, OCH₃); 1.00 (s, 9H, *^{t}*Bu); 0.20 (s, 6H, SiCH₃)

### Préparation 11" : 4-{[tert-Butyl(diméthyl)silyl]oxy}-N-[4-(trifluorométhoxy) phényl]aniline

On procède selon le procédé de la Préparation 2" en remplaçant le 5-bromobenzothiophène utilisé au Stade B par le 4-bromophényl trifluorométhyl éther.
**RMN ¹H :** δ (400 MHz ; CDCl₃ ; 300K) : 7.1-6.8 (m, 8H); 5.70 (s, 1H); 1 (s, 9H); 0.25 (s, 6H)

### Préparation 12" : 5-[(4-Méthylphényl)amino]-1H-indole-1-carboxylate de tert-butyle

On procède selon le procédé du Stade B de la Préparation 2" en remplaçant le 5-bromobenzothiophène utilisé par le 5-bromo-1*H*-indole-1-carboxylate de *tert*-butyle et la 4-{[*tert*-butyl(diméthyl)silyl]oxy}aniline par la 4-méthylaniline.
**RMN ¹H** : δ (400 MHz ; CDCl₃ ; 300K) :8.0 (s, 1H); 7.5 (m, 1H); 7.1-6.9 (m, 6H); 6.45 (d, 1H); 2.25 (s, 3H); 1.65 (s, 9H)

### Préparation 13" : N-(4-{[tert-Butyl(diméthyl)silyl]oxy}phényl)-3-chloro-4-fluoroaniline

On procède selon le procédé de la Préparation 2" en remplaçant le 5-bromobenzothiophène utilisé au Stade B par le 4-bromo-2-chloro-1-fluorobenzène.
**RMN ¹H** : δ (400 MHz ; dmso-d6 ; 300K) :8.0 (s, 1H); 7.3 (t, 1H); 7.0-6.7 (m, 6H); 1.00 (s, 9H); 0.20 (s, 6H)

### Préparation 14" : 4-{[tert-Butyl(diméthyl)silyl]oxy}-3-chloro-N-(4-méthylphényl) aniline

On procède selon le procédé du Stade B de la Préparation 2" en remplaçant le 5-bromobenzothiophène utilisé par le 1-bromo-4-méthylbenzène et la 4-{[*tert-*butyl(diméthyl)silyl]oxy}aniline par la 4-{[*tert*-butyl(diméthyl)silyl]oxy}-3-chloroaniline (obtenue selon un protocole de la littérature : Bioorg Med Chem Lett 22(14), 4839-4843).
**RMN ¹H** : δ (400 MHz ; dmso-d6 ; 300K) : 7.90 (s, 1H, NH); 7.00 (m, 3H) ; 6.90 (m, 4H) ; 2.20 (s, 3H) ; 1.00 (s, 9H, *^{t}*Bu); 0.20 (s, 6H, SiCH₃)

### Préparation 15" : 4-{[tert-Butyl(diméthyl)silyl]oxy}-3-chloro-N-[4-(propan-2-yl)phényl]aniline

On procède selon le procédé du Stade B de la Préparation 2" en remplaçant le 5-bromobenzothiophène utilisé par le 1-bromo-4-(propan-2-yl)benzène et la *4-*{[*tert-*butyl(diméthyl)silyl]oxy}aniline par la 4-{[*tert*-butyl(diméthyl)silyl]oxy}-3-chloroaniline (obtenue selon un protocole de la littérature : Bioorg Med Chem Lett 22(14), 4839-4843).
**RMN ¹H** : δ (400 MHz ; dmso-d6 ; 300K) :7.12 (s, 1H, NH); 7.10 (d, 2H) ; 6.95 (d, 2H); 6.85 (m, 1H); 6.80 (d, 1H); 2.85 (m, 1H); 1.25 (d, 6H); 1.05 (s, 9H, *^{t}*Bu); 0.25 (s, 6H, SiCH₃)

### Préparation 16" : 5-[(4-Méthylphényl)amino]-1H-indazole-1-carboxylate de tert-butyle

On procède selon le procédé du Stade B de la Préparation 2" en remplaçant le 5-bromobenzothiophène utilisé par le 5-bromo-1*H*-indazole-1-carboxylate de *tert*-butyle et la 4-{[*tert*-butyl(diméthyl)silyl]oxy}aniline par la 4-méthylaniline.
**RMN ¹H** : δ (400 MHz ; dmso-d6 ; 300K) :8.25 (s, 1H); 8.15 (s, 1H); 7.95 (d, 1H); 7.40 (d, 1H); 7.30 (dd, 1H); 7.08 (d, 2H); 7.02 (d, 2H); 2.25 (s, 3H); 1.65 (s, 9H)

### Préparation 17" : N-(4-{[tert-Butyl(diméthyl)silyl]oxy}-3-chlorophényl)-1-méthyl-1H-indol-5-amine

On procède selon le procédé du Stade B de la Préparation 2" en remplaçant le 5-bromobenzothiophène utilisé par le 5-bromo-1-méthyl-1*H*-indole et la 4-{[*tert-*butyl(diméthyl)silyl]oxy}aniline par la 4-{[*tert*-butyl(diméthyl)silyl]oxy}-3-chloroaniline (obtenue selon un protocole de la littérature : Bioorg Med Chem Lett 22(14), 4839-4843).
**RMN ¹H** : δ (400 MHz ; dmso-d6 ; 300K) :7.70 (s, 1H); 7.35 (d, 1H); 7.25 (s, 1H);7.28 (d, 1H); 6.92 (d, 1H); 6.85 (m, 3H); 6.30 (d, 1H); 3.75 (s, 3H); 1.00 (s, 9H); 0.20 (s, 6H)

### Préparation 18" : 5-[(4-{[tert-Butyl(diméthyl)silyl]oxy}-3-chlorophényl)amino]-1H-indole-1-carboxylate de tert-butyle

On procède selon le procédé du Stade B de la Préparation 2" en remplaçant le 5-bromobenzothiophène utilisé par le 5-bromo-1*H*-indole-1-carboxylate de *tert*-butyle et la 4-{[*tert*-butyl(diméthyl)silyl]oxy}aniline par la 4-{[*tert*-butyl(diméthyl)silyl]oxy}-3-chloroaniline (obtenue selon un protocole de la littérature : Bioorg Med Chem Lett 22(14), 4839-4843).
**RMN ¹H** : δ (400 MHz ; dmso-d6 ; 300K) :8.0 (s, 1H); 7.9 (d, 1H); 7.6 (d, 1H); 7.25 (d, 1H); 7.1-6.8 (m, 4H); 6.60 (d, 1H); 1.60 (s, 9H), 1.00 (s, 9H); 0.20 (s, 6H)

### Préparation 19" : 4-(4-Méthylphénylamino)pipéridine-1-carboxylate de tert-butyle

A une solution de 4 g (20 mmol) de 4-aminopipéridine-1-carboxylate de *tert*-butyle dans le dichlorométhane est ajouté de l'acide (4-méthylphényl)boronique (5,4 g ; 40 mmol), de la triéthylamine (5,6 mL ; 40 mmol) et de l'acétate de cuivre (3,6 g ; 20 mmol). Le milieu réactionnel est agité pendant 24 h à température ambiante puis pendant 17 h au reflux. Après évaporation à sec, le résidu est repris dans l'acétate d'éthyle, lavé à l'acide chlorhydrique 1N, à l'eau, et à la saumure. La phase organique est séchée au MgSO₄, puis concentrée à sec et purifiée par chromatographie sur gel de silice (gradient CH₂Cl₂/AcOEt). Le produit du titre est obtenu sous la forme d'un solide.
**RMN ¹H :** δ (400 MHz ; dmso-d6 ; 300K) :6.85 (d, 2H); 6.50 (d, 2H); 5.65 (d, 1H); 3.85 (m, 2H); 3.35 (m, 1H); 2.90 (m, 2H); 2.15 (s, 3H); 2.15 (s, 3H); 1.85 (m, 2H); 1.40 (s, 9H), 1.20 (m, 2H)

### Préparation 20" : N-(4-{[tert-Butyl(diméthyl)silyl]oxy}phényl)-1-méthylpipéridin-4-amine

A une solution de 4-[*tert*-butyl(diméthyl)silyl]oxyaniline (5g ; 22,4 mmol) et de 1-méthylpipéridin-4-one (2.5g ; 22,4 mmol) dans du dichlorométhane est ajouté, par portions, le triacétoxyborohydrure de sodium (4,8 g ; 22,4 mmol). Après 24 h d'agitation à température ambiante, le milieu réactionnel est lentement coulé sur une solution de bicarbonate de sodium. La phase aqueuse est extraite au dichlorométhane. La phase organique est séchée au MgSO₄, concentrée à sec et le résidu est purifié par chromatographie sur gel de silice (gradient CH₂Cl₂/MeOH) pour conduire au produit attendu sous la forme d'un solide.
**RMN ¹H** : δ (400 MHz ; dmso-d6 ; 300K) : 6.62 (2d, 4H); 3.15 (m, 1H); 2.88-2.18 (m, 4H); 2.30 (s, 3H) ; 1.98-1.48 (m, 4H); 0.98 (s, 9H); 0.15(s, 6H).

### Préparation 21" : N-(4-{[tert-Butyl(diméthyl)silyl]oxy}phényl)-3-(4-méthyl pipérazin-1-yl)aniline

On procède selon le procédé de la Préparation 2" en remplaçant le 5-bromobenzothiophène utilisé au Stade B par le 1-(3-bromophényl)-4-méthylpipérazine.
**RMN ¹H** : δ (400 MHz ; dmso-d6 ; 300K) : 7.68 (s, 1H); 7.00 (t, 1H); 6.96 (d, 2H); 6.74 (d, 2H); 6.46 (m, 1H); 6.4-6.35 (m, 2H); 3.04 (m, 4H); 2.42 (m, 4H); 2.20 (s, 3H); 0.95 (s, 9H); 0.16 (s, 6H)

### Préparation 22" : N-(4-{[tert-Butyl(diméthyl)silyl]oxy}phényl)-2-méthyl-2,3-dihydro-1H-isoindol-5-amine

On procède selon le procédé de la Préparation 2" en remplaçant le 5-bromobenzothiophène utilisé au Stade B par le 5-bromo-2-méthyl-2,3-dihydro-1*H-*isoindole.
**RMN ¹H** : δ (400 MHz ; dmso-d6 ; 300K) : 7.02 (d, 1H); 6.95 (d, 2H); 6.75 (m, 4H); 5.45 (s, 1H); 3.85 (s, 4H); 2.78 (s, 3H); 1.00 (s, 9H); 0.20 (s, 6H)

### Préparation 23" : N-(4-{[tert-Butyl(diméthyl)silyl]oxy}phényl)-1-[2-(morpholin-4-yl)éthyl]-1H-indol-5-amine

### Stade A : 5-Bromo-1-[2-(morpholin-4-yl)éthyl]-1H-indole

A une suspension de NaH (4.5 g; 112 mmol) dans le THF anhydre (300 mL) est ajouté à 0°C et par portions le 5-bromoindole (10.4 g ; 51 mmol). Après 20 minutes d'agitation à 0°C, le chlorhydrate de 4-(2-chloroéthyl)morpholine (10.4 g ; 56 mmol) est ajouté par portions en 1 h. Après une nuit d'agitation à température ambiante, puis 5 h à 80°C, le milieu réactionnel est coulé sur un mélange de bicarbonate de sodium aqueux et de dicholorométhane. La phase aqueuse est extraite au dichlorométhane. La phase organique est séchée au MgSO₄, concentrée à sec et le résidu est purifié par chromatographie sur gel de silice (gradient CH₂Cl₂/MeOH) pour fournir le produit attendu sous la forme d'une huile légèrement jaune.
**RMN ¹H** : δ (400 MHz ; CDCl3 ; 300K) :7.75 (d, 1H) ; 7.30 (dd, 1H) ; 7.20 (d, 1H) ; 7.15 (d, 1H) ; 6.40 (d, 1H) ; 4.20 (t, 2H) ; 3.70 (m, 4H) ; 2.75 (t, 2H) ; 2.45 (m, 4H)

### Stade B : 5-Bromo-1-[2-(morpholin-4-yl)éthyl]-1H-indole

On procède selon le procédé de la Préparation 2" en remplaçant le 5-bromobenzothiophène utilisé au Stade B par le composé obtenu au Stade A.
**RMN ¹H** : δ (400 MHz ; dmso-d6 ; 300K) : 7.35 (d, 1H); 7.15 (s, 1H); 6.85 (d, 3H); 6.70 (d, 2H); 7.30 (d, 1H); 6.25 (d, 1H); 4.20 (t, 2H); 3.55 (m, 4H); 2.65 (t, 2H); 2.45 (m, 4H); 1.45 (s, 9H), 0.15 (s, 6H)

### Préparation 24" : N-(4-{[tert-Butyl(diméthyl)silyl]oxy}phényl)-1-méthyl-1H-pyrrolo[2,3-b]pyridin-5-amine

On procède selon le procédé de la Préparation 2" en remplaçant le 5-bromobenzothiophène utilisé au Stade B par le 5-bromo-1-méthyl-1*H*-pyrrolo[2,3-*b*]pyridine (obtenu selon un protocole de la littérature : Heterocycles, 60(4), 865, 2003).
**IR :** v :-NH- : 3278 cm⁻¹; v :-C=C- aromatiques: 1605 cm⁻¹

### Préparation 25" : 4-{[tert-Butyl(diméthyl)silyl]oxy}-N-phénylaniline

A une solution de 12 g de 4-anilinophénol (64,7 mmol) dans 200 mL d'acétonitrile sont ajoutés à température ambiante 6,7 g d'imidazole (97,05 mmol) et 11,7 g de *tert-*butyl(chloro)diméthylsilane (77,64 mmol). L'ensemble est mis sous agitation à 70°C pendant 4 heures. Puis, le milieu réactionnel est versé sur de l'eau et extrait avec de l'éther. La phase organique est ensuite séchée sur sulfate de magnésium, puis filtrée et évaporée à sec. Le produit brut ainsi obtenu est alors purifié par chromatographie sur gel de silice (gradient éther de pétrole / dichlorométhane). Le produit du titre est obtenu sous la forme d'une poudre.
**RMN¹H** : δ (400 MHz ; dmso-d6 ; 300°K) : 7.84 (s, 1H NH) ; 7.17 (t, 2H aniline) ; 6.98 (d, 2H phénoxy) ; 6.94 (d, 2H aniline) ; 6.76 (d, 2H phénoxy) ; 6.72 (t, 1H aniline) ; 0.95 (s, 9H *tert-*butyl) ; 0.15 (s, 6H diméthyl)
**IR :** v : >NH : 3403 cm⁻¹ ; v :>Ar : 1597 cm⁻¹

### Préparation 26" : 4-({4-[(tert-Butyl(diméthylsilyl]oxy}phényl}amino)-1,5-diméthyl-1H-pyrrole-2-carbonitrile

### Stade A : 4-Bromo-1,5-diméthyl-1H-pyrrole-2-carbonitrile

Une solution de brome (6,58 mL, 0,13 mol) dans l'acide acétique (60 mL) est ajoutée goutte-à-goutte à l'aide d'une ampoule à addition dans une solution de 1,5-diméthyl-1H-pyrrole-2-carbonitrile (15,0 g, 0,12 mol) dans l'acide acétique (300 mL). L'ensemble est agité à la température ambiante pendant 24 h. Le mélange réactionnel est ensuite versé dans un bécher contenant 300 mL d'eau. Le solide formé est filtré et rincé avec de l'eau. Puis, il est solubilisé dans le dichlorométhane (300 mL) et la phase organique est lavée avec de la saumure, séchée avec du sulfate de sodium, filtrée et concentrée sous vide pour donner le produit attendu sous la forme d'un solide.
**RMN ¹H** (CDCl₃) δ ppm : 2.25 (s, 3 H), 3.67 (s, 3 H), 6.74 (s, 1 H)

### Stade B : 4-({4-[(tert-Butyldiméthylsilyl)oxy]phényl}amino)-1,5-diméthyl-1H-pyrrole-2-carbonitrile

Une solution du composé du stade précédent (1,5 g, 7,53 mmol), de 4-[(*tert-*butyldiméthylsilyl)oxy]aniline (2,02 g, 9,04 mmol), de *tert*-butoxide de sodium (1,45 g, 15,06 mmol) et de 2-di-*tert*-butylphosphino-2',4',6'-triisopropylbiphényl (0,13 g, 0,30 mmol) dans le toluène (20 mL) est purgée à l'azote. Le tris(dibenzylideneacetone)dipalladium(0) (0,28 g, 0,30 mmol) est ajouté, puis le mélange réactionnel est chauffé à 90°C jusqu'à ce que la réaction soit complète (suivi par CCM). Le chauffage est arrêté et on laisse le mélange revenir à la température ambiante. De l'eau (75 mL) est ajoutée et le mélange est extrait avec de l'acétate d'éthyle (3 x 75 mL). Les phases organiques réunies sont lavées avec de la saumure puis concentrées. Le produit brut est absorbé sur gel de silice et purifié par chromatographie éclair sur gel de silice avec un mélange d'acétate d'éthyle et d'heptane (0 à 30%). Le produit ainsi obtenu est solubilisé à chaud dans l'heptane et est laissé précipiter sous agitation à température ambiante, puis à 0°C. Le solide est filtré et l'opération est répétée sur le filtrat pour donner le composé attendu sous la forme d'un solide.
**RMN ¹H** (400 MHz, CDCl₃) δ ppm : 0.15 (s, 6 H), 0.97 (s, 9 H), 2.13 (s, 3 H), 3.66 (s, 3 H), 4.68 (br. s, 1 H), 6.49 (d, *J* = 8.5 Hz, 2 H), 6.64 (s, 1 H), 6.66 (d, *J* = 8.7 Hz, 2 H)
**RMN ¹³C** (100 MHz, CDCl₃) δ ppm: 4.34, 9.72, 18.30, 25.88, 32.94, 101.27, 114.37, 114.70, 116.41, 120.73, 124.52, 131.23, 141.54, 148.27
**MS** (**ESI**+)**:** [M+H]⁺ mesuré : 342.3

Les amines NHR₃R₄ dans lesquelles R₃ et R₄ représentent indépendamment l'une de l'autre un groupement aryle ou hétéroaryle sont obtenues selon les procédés décrits dans la littérature (Surry D.S. et al., Chemical Science, 2011, 2, 27-50, Charles M.D. et al., Organic Letters, 2005, 7, 3965-3968). La réaction de protection de la fonction hydroxy du 4-anilinophénol décrite à la Préparation 25" peut être appliquée à diverses amines secondaires NHR₃R₄ (telles que définies précédemment), comportant une ou plusieurs fonctions hydroxy, lorsque celles-ci sont disponibles commercialement. Alternativement, les amines secondaires comportant au moins un substituant hydroxy peuvent être synthétisées directement sous une forme protégée, i.e. à partir de réactifs dont la fonction hydroxy a été préalablement protégée. Parmi les groupements protecteurs, le *tert-*butyl(diméthyl)silyloxy et le benzyloxy sont particulièrement préférés.
Parmi les amines NHR₃R₄ comportant un substituant hydroxy qui sont utilisées pour synthétiser les composés de l'invention, on peut citer : le 4-(4-toluidino)phénol, le 4-(4-chloroanilino)phénol, le 4-(3-fluoro-4-méthylanilino)phénol, le 4-[4-(trifluorométhoxy)anilino]phénol, le 4-[4-hydroxyanilino]phénol, le {4-[(1-méthyl-1*H-*indol-6-yl)amino]phényl}méthanol, le 4-(2,3-dihydro-1*H*-indol-6-ylamino)phénol, le 4-[(1-méthyl-2,3-dihydro-1*H*-indol-6-yl)amino]phénol, 4-[(1-méthyl-1*H*-indol-6-yl)amino]phénol, le 4-[(1-méthyl-1*H*-indol-6-yl)amino]cyclohexanol, le 4-[(1-méthyl-1,2,3,4-tétrahydro-6-quinolinyl)amino]phénol, le 4-[(4-méthyl-3,4-dihydro-2*H*-1,4-benzoxazin-7-yl)amino]phénol, le 4-[4-(diéthylamino)anilino]phénol, le 4-(2,3-dihydro-1*H*-indén-5-ylamino)phénol, le 4-[(1-méthyl-1*H*-indazol-5-yl)amino]phénol, le 4-[(1'-méthyl-1',2'-dihydrospiro[cyclopropane-1,3'-indol]-5'-yl)amino]phénol, le 4-[(1,3,3-triméthyl-2,3-dihydro-1*H*-indol-5-yl)amino]phénol, le 4-[4-méthoxy-3-(trifluorométhyl)anilino]phénol, le 4-[4-(méthylsulfanyl)-3-(trifluorométhyl)anilino] phénol, le 2-fluoro-4-[(1-méthyl-1*H*-indol-5-yl)amino]phénol, le 4-[(1-éthyl-1*H*-indol-5-yl)amino]phénol, le 4-[(1-éthyl-2,3-dihydro-1*H*-indol-5-yl)amino]phénol, le 4-[(1-isopropyl-2,3-dihydro-1*H*-indol-5-yl)amino]phénol, le 4-(butylamino)phénol, le 3-[(1-méthyl-1*H*-indol-5-yl)amino]-1-propanol, le 4-[(1-méthyl-1*H*-indol-5-yl)amino]-1-butanol, le 4-[(3-fluoro-4-méthylphényl)amino]phénol, le 4-[(3-chloro-4-méthylphényl)amino]phénol, le 4-[(4-fluorophényl)amino]phénol, le 4-[(1-méthyl-1*H-*pyrrolo[2,3-*b*]pyridin-5-yl)amino]phénol, le 4-[(4-fluorophényl)amino]phénol, le 4-[(2-fluorophényl)amino]phénol, le 4-[(3-fluorophényl)amino]phénol, le 4-[(2,4-difluorophényl)amino]phénol, le 4-[(3,4-difluorophényl)amino]phénol, le 3-[(4-hydroxyphényl)amino]benzonitrile, le 4-[(3-méthoxyphényl)amino]phénol, le 4-[(3,5-difluorophényl)amino]phénol, le 4-[(3-méthylphényl)amino]phénol, le 4-[(4-hydroxyphényl)amino]benzonitrile, le 4-[(3-chlorophényl)amino]phénol, le 4-(pyrimidin-2-ylamino)phénol, le 4-[(cyclobutylméthyl)amino]phénol, le 2-[(4-hydroxyphényl)amino]benzonitrile, le 4-{[(1-méthyl-1*H*-pyrazol-4-yl)méthyl]amino} phénol, le 4-[(cyclopropylméthyl)amino]phénol, le 4-{[(1-méthyl-1*H*-pyrazol-3-yl)méthyl]amino}phénol, le 4-(but-2-yn-1-ylamino)phénol, le 4-(pyrazin-2-ylamino) phénol, le 4-(pyridin-2-ylamino)phénol, le 4-(pyridazin-3-ylamino)phénol, le 4-(pyrimidin-5-ylamino)phénol, le 4-(pyridin-3-ylamino)phénol, le 4-[(3,5-difluoro-4-méthoxyphényl) amino]phénol, le 4-(pyridin-4-ylamino)phénol, le 4-[(3-fluoro-4-méthoxyphényl)amino] phénol, le 2-(phénylamino)pyrimidin-5-ol, le 5-[(4-hydroxyphényl)amino]-2-méthoxy benzonitrile et le 4-{[3-(trifluorométhyl)phényl]amino}phénol.
La ou les fonction(s) hydroxy des amines secondaires listées ci-dessus est (sont) préalablement protégée(s) par un groupement protecteur adapté avant tout couplage à un dérivé d'acide du composé de formule (IV) tel que défini dans le procédé général précédent.

### Préparation 2a : N-[4-(Benzyloxy)phényl]-N-phényl-1H-indole-3-carboxamide

### Stade A : 1-{[2-(Triméthylsilyl)éthoxy]méthyl}-1H-indole-3-carboxylate de méthyle

On refroidit jusqu'à 0°C du tétrahydrofurane anhydre (200 ml) et du 1*H*-indole-3-carboxylate de méthyle (20 g, 114,17 mmol), puis l'on ajoute de l'hydrure de sodium à 60 % dans de l'huile (9,12 g, 228 mmol) et on laisse le tout sous agitation pendant 10 minutes sous azote. On ajoute du [2-(chlorométhoxy)éthyl]triméthylsilane (24,25 ml, 137 mmol) et l'on agite le mélange réactionnel à température ambiante sous azote pendant environ 16 h. Le mélange réactionnel est inactivé avec de l'eau et extrait dans de l'acétate d'éthyle, séché sur sulfate de magnésium, filtré et concentré. Le produit brut est purifié par chromatographie sur gel de silice selon un gradient allant de iso-hexane à un mélange iso-hexane (10 %)/acétate d'éthyle pour livrer le composé sous forme d'une huile.
**LC/MS** (C₁₆H₂₃NO₃Si) 306 [M+H]⁺ ; RT 2,75 (Méthode A) étant entendu que RT indique le temps de rétention

### Stade B : N-[4-(Benzyloxy)phényl]-N-phényl-1-{[2-(triméthylsilyl)éthoxy]méthyl}-1H-indole-3-carboxamide

A une solution de 4-(benzyloxy)-*N*-phénylaniline (8,59 g, 31,2 mmol) dans du tétrahydrofurane (40 ml), refroidie jusqu'à -78°C sous azote, on ajoute du bis(triméthylsilyl)amidure de lithium (1M, 43 ml, 43 mmol). Après 10 minutes, on ajoute l'indole obtenu au Stade A dans 40 ml de tétrahydrofurane, et on laisse le mélange réactionnel se réchauffer jusqu'à la température ambiante, avant de chauffer jusqu'à 50°C sous azote pendant environ 16 h. Le mélange réactionnel est réparti selon sa solubilité entre l'acétate d'éthyle et l'eau, et les phases organiques sont séchées sur sulfate de magnésium, filtrées et concentrées. Le produit brut est purifié sur gel de silice selon un gradient allant de iso-hexane à un mélange iso-hexane (20 %)/acétate d'éthyle pour livrer une huile.
**LC/MS** (C₃₄H₃₆N₂O₃Si) 549 [M+H]⁺ ; RT 2,95 (Méthode A)

### Stade C : N-[4-(Benzyloxy)phényl]-N-phényl-1H-indole-3-carboxamide

On ajoute du TBAF (1M, 78 ml, 78 mmol) à une solution du composé obtenu au Stade B (14,24 g, 25,95 mmol) et d'éthylènediamine (5,22 ml, 77,85 mmol) dans du tétrahydrofurane et l'on chauffe le mélange à reflux sous azote pendant environ 16 h. Le mélange réactionnel est concentré et réparti selon sa solubilité entre l'acétate d'éthyle et la saumure. Les phases organiques sont ensuite séchées sur sulfate de magnésium, filtrées et agitées pendant 1 heure avec du CaCO₃ (6,5 g) et une résine échangeuse d'ions DOWEX 50WX8-100 (5 g). Le mélange est ensuite filtré, concentré et trituré avec de l'éther. Le produit solide est isolé par filtration et séché sous vide.
**LC/MS** (C₂₈H₂₂N₂O₂) 419 [M+H]⁺ ; RT 2,36 (Méthode A)

### Préparation 2b : N,N-Diphényl-1H-indole-3-carboxamide

Le mode opératoire est le même que dans la Préparation 2a, en remplaçant la 4-(benzyloxy)-*N*-phénylaniline utilisé au Stade B par de la N-phénylaniline. On obtient le produit sous forme d'un solide.
**LC/MS** (C₂₁H₁₆N₂O) 313,2 [M+H]⁺ ; RT 2,47 (Méthode A)

### Préparation 2c : N,N-Dibutyl-1H-indole-3-carboxamide

On ajoute de la dibutylamine (3,14 ml, 18,62 mmol), de la DIPEA (1,95 ml, 11,17 mmol) et du HBTU (4,24 g, 11,17 mmol) à une solution d'acide 1*H*-indole-3-carboxylique (1,5 g, 9,31 mmol) dans du *N*,*N*-diméthylformamide (60 ml) et l'on agite le mélange réactionnel à température ambiante pendant 16 heures. Le mélange réactionnel est concentré sous vide, dilué avec une solution aqueuse saturée de NaHCO₃ et extrait avec de l'acétate d'éthyle. Les phases organiques sont lavées avec une solution saturée de NaHCO₃, à la saumure, séchées sur sulfate de magnésium et concentrées sous vide. Le matériau résultant est dissous dans du dichlorométhane et agité avec 5 g de résine MP-Carbonate pré-lavée pendant 20 minutes, filtré et lavé au dichlorométhane, puis concentré sous vide et purifié par chromatographie sur gel de silice dans de l'iso-hexane, puis un mélange 3:1, puis 3:2, iso-hexane:acétate d'éthyle pour livrer le produit sous forme d'un solide.
**LC/MS** (C₁₇H₂₄N₂O) 273 [M+H]⁺ ; RT 2,53 (Méthode A)

### Préparation 2d : N-[4-(Benzyloxy)phényl]-5-fluoro-N-phényl-1H-indole-3-carboxamide

Le mode opératoire est le même que dans la Préparation 2a, en remplaçant le 1*H*-indole-3-carboxylate de méthyle utilisé au Stade A par du 5-fluoro-1*H*-indole-3-carboxylate de méthyle. On obtient le produit sous forme d'un solide.
**LC/MS** (C₂₈H₂₁N₂O₂F) 437 [M+H]⁺ ; RT 2,71 (Méthode A)

### Préparation 2e : 6-(Benzyloxy)-N,N-diphényl-1H-indole-3-carboxamide

### Stade A : 1-(Benzènesulfonyl)-6-(benzyloxy)-3-iodo-1H-indole

On ajoute une solution de *n*-butyllithium (4,02 ml, 8,44 mmol) à une solution de 6-(benzyloxy)-1*H*-indole (942 mg, 4,22 mmol) dans du tétrahydrofurane (20 ml), refroidie jusqu'à -78°C, puis l'on agite le tout à 0°C pendant 1 heure. On transfère ensuite la solution résultante par le biais d'une canule dans une solution d'iode (1,07 g, 4,22 mmol) dans du tétrahydrofurane (20 ml), refroidie jusqu'à -78°C, et l'on agite le mélange résultant à 0°C pendant 2 heures. Le mélange réactionnel est inactivé par addition de méthanol (1 goutte), refroidi jusqu'à -78°C, puis l'on ajoute une solution de diisopropylamidure de lithium préparée selon le procédé suivant : on traite avec une solution de *n*-butyllithium (2,01 ml, 4,22 mmol) une solution de diisopropylamine (0,596 ml, 4,22 mmol) dans 10 ml de tétrahydrofurane, refroidie jusqu'à -78°C, et l'on agite le mélange résultant à température ambiante pendant 1 heure.

Après addition de la solution de diisopropylamidure de lithium, on ajoute du chlorure de phénylsulfonyle (0,565 ml, 4,43 mmol) et, après 30 minutes, on laisse le mélange réactionnel se réchauffer jusqu'à la température ambiante et on l'agite pendant environ 16 h. Le mélange réactionnel est inactivé avec de l'eau et réparti selon sa solubilité entre l'acétate d'éthyle et une solution aqueuse saturée de bicarbonate de sodium, et les phases organiques sont lavées avec une solution de thiosulfate de sodium, séchées sur sulfate de magnésium, filtrées et concentrées sous vide. Le matériau brut est purifié par chromatographie sur gel de silice dans un mélange acétate d'éthyle (10 %)/iso-hexane pour livrer une huile.
**LC/MS** (C₂₁H₁₆NO₃SI) 490 [M+H]⁺ ; RT 2,83 (Méthode A)

### Stade B : 1-(Benzènesulfonyl)-6-(benzyloxy)-N,N-diphényl-1H-indole-3-carboxamide

On ajoute une solution de *n*-butyllithium (0,41 ml, 0,82 mmol) à une solution de l'iodure issu du Stade A (200 mg, 0,41 mmol) dans du tétrahydrofurane (8 ml) sous azote, refroidie jusqu'à -78°C. Après 5 minutes, on ajoute du chlorure de *N*,*N*-diphénylcarbamoyle (142 mg, 0,61 mmol) dans du tétrahydrofurane (4 ml) et l'on agite le mélange réactionnel à -78°C pendant 90 minutes. Le mélange réactionnel est inactivé avec de l'eau et extrait dans de l'acétate d'éthyle, séché sur sulfate de magnésium, filtré et évaporé. Le matériau brut est purifié par chromatographie sur gel de silice dans un mélange acétate d'éthyle (10 %)/isohexane, puis 5:1 iso-hexane/acétate d'éthyle pour livrer une gomme.
**LC/MS** (C₃₄H₂₆N₂O₄S) 559 [M+H]⁺ ; RT 2,84 (Méthode A)

### Stade C : 6-(Benzyloxy)-N,N-diphényl-1H-indole-3-carboxamide

On ajoute une solution de TBAF (3,63 ml, 3,63 mmol) à une solution du produit issu du Stade B (0,68 g, 1,21 mmol) dans du tétrahydrofurane (8 ml) et l'on chauffe le mélange réactionnel par irradiation micro-onde à 100°C pendant 45 minutes. Le mélange réactionnel est concentré sous vide, réparti selon sa solubilité entre l'acétate d'éthyle et l'eau et séché sur sulfate de magnésium. Le solvant est éliminé sous vide et le produit brut est purifié par chromatographie sur gel de silice, en éluant avec un gradient allant d'isohexane à un mélange 1:2 acétate d'éthyle/isohexane pour livrer un solide.
**LC/MS** (C₂₈H₂₂N₂O₂) 419 [M+H]⁺ ; RT 2,62 (Méthode A)

### Préparation 2f : N-(4-Fluorophényl)-N-phényl-1H-indole-3-carboxamide

Le mode opératoire est le même que dans la Préparation 2a, en remplaçant la 4-(benzyloxy)-N-phénylaniline utilisée au Stade B par de la 4-fluoro-*N*-phénylaniline. On obtient le produit sous forme d'un solide.

### Préparation 2g : N-(4-Méthylphényl)-N-phényl-1H-indole-3-carboxamide

Le mode opératoire est le même que dans la Préparation 2a, en remplaçant la 4-(benzyloxy)-*N*-phénylaniline utilisée au Stade B par de la 4-méthyl-*N*-phénylaniline. On obtient le produit sous forme d'un solide.

### Préparation 3a : Acide 5-(benzyloxy)-2-(3-{[4-(benzyloxy)phényl](phényl) carbamoyl}-1H-indol-1-yl)-4-méthoxybenzoïque

Le *N*-[4-(benzyloxy)phényl]-*N*-phényl-1*H*-indole-3-carboxamide issu de la Préparation 2a (1,17 g, 2,79 mmol), de l'acide 5-benzyloxy-2-bromo-4-méthoxybenzoïque (940 mg, 2,79 mmol) et du carbonate de potassium (771 mg, 5,58 mmol) sont combinés et mis en suspension dans du *N*,*N*-diméthylformamide (10 ml). On dégaze le mélange réactionnel avec de l'azote, puis l'on ajoute de l'iodure de cuivre (55 mg, 0,28 mmol) et l'on chauffe le tout jusqu'à 80°C sous azote pendant environ 16 h. Le mélange réactionnel est dilué avec de l'eau et acidifié avec du HCl aqueux 2 M. Le précipité résultant est filtré et lavé à l'eau, dissous dans du dichlorométhane et séché sur sulfate de magnésium, filtré et évaporé. Le matériau brut est purifié par chromatographie sur gel de silice selon un gradient allant d'iso-hexane à acétate d'éthyle pour livrer le produit sous forme d'une mousse.
**LC/MS** (C₄₃H₃₄N₂O₆) 675 [M+H]⁺ ; RT 2,89 (Méthode A)

### Préparation 3b : Acide 2-[6-(benzyloxy)-3-(diphénylcarbamoyl)-1H-indol-1-yl]benzoïque

Le mode opératoire est le même que dans la Préparation 3a, en remplaçant le *N*-[4-(benzyloxy)phényl]-*N*-phényl-1*H*-indole-3-carboxamide par du *N*,*N*-diphényl-1*H*-indole-3-carboxamide issu de la Préparation 2b, et en remplaçant l'acide 5-benzyloxy-2-bromo-4-méthoxybenzoïque par de l'acide 2-bromobenzoïque.

### Préparation 3c : Acide 5-(benzyloxy)-2-[3-(diphénylcarbamoyl)-1H-indol-1-yl]-4-méthoxybenzoïque

Le mode opératoire est le même que dans la Préparation 3a, en remplaçant le *N*-[4-(benzyloxy)phényl]-*N*-phényl-1*H*-indole-3-carboxamide par du *N*,*N*-diphényl-1*H*-indole-3-carboxamide issu de la Préparation 2b.

### Préparation 3d : Acide 2-(3-{[4-(benzyloxy)phényl](phényl)carbamoyl}-1H-indol-1-yl)benzoïque

### Stade A : Acide 2-[3-(méthoxycarbonyl)-1H-indol-1-yl]benzoïque

On ajoute du carbonate de potassium (1,3 g, 9,42 mmol) et de l'acide 2-iodobenzoïque (1,56 g, 6,28 mmol) à une solution de 1*H*-indole-3-carboxylate de méthyle (1,1 g, 6,28 mmol) dans du *N*,*N*-diméthylformamide (10 ml) et l'on dégaze le mélange réactionnel avec de l'azote avant d'ajouter de l'iodure de cuivre (120 mg, 0,628 mmol). On chauffe le mélange réactionnel à 90°C pendant environ 16 h, puis on le laisse refroidir jusqu'à la température ambiante, on le dilue avec de l'eau et on l'extrait dans de l'acétate d'éthyle. Le pH de la phase aqueuse est ajusté jusqu'à ce qu'il devienne acide avec une solution aqueuse de HCl 2M, et on l'extrait à nouveau avec de l'acétate d'éthyle. Les extraits organiques combinés sont séchés sur sulfate de magnésium, filtrés et évaporés, et le matériau brut est purifié par chromatographie sur gel de silice selon un gradient allant d'iso-hexane à acétate d'éthyle pour livrer le produit sous forme d'un solide.
**LC/MS** (C₁₇H₁₃NO₄) 296 [M+H]⁺ ; RT 2,25 (Méthode A)

### Stade B : Acide 2-(3-{[4-(benzyloxy)phényl](phényl)carbamoyl}-1H-indol-1-yl)benzoïque

On ajoute du bis(triméthylsilyl)amidure de lithium (1M dans du THF, 3,1 ml, 3,11 mmol) à une solution de 4-(benzyloxy)-*N*-phénylaniline (570 mg, 2,07 mmol) dans du tétrahydrofurane anhydre (10 ml), refroidie jusqu'à -78°C sous azote, et l'on agite le mélange réactionnel à -78°C pendant 20 minutes. Une solution du composé obtenu au Stade A (610 mg, 2,07 mmol) dans du tétrahydrofurane (5 ml) est ensuite ajoutée au mélange réactionnel, qu'on laisse se réchauffer jusqu'à la température ambiante, avant de le chauffer jusqu'à 50°C pendant environ 16 h, durée au cours de laquelle on ajoute en 2 portions 5,1 ml supplémentaires de bis(triméthylsilyl)amidure de lithium. On laisse le mélange réactionnel refroidir jusqu'à la température ambiante, on le dilue avec de l'eau et on l'extrait avec de l'acétate d'éthyle. La phase aqueuse est acidifiée et extraite à nouveau avec de l'acétate d'éthyle, et les extraits organiques combinés sont séchés sur sulfate de magnésium, filtrés et évaporés. Le matériau brut est purifié par chromatographie sur gel de silice selon un gradient allant de dichlorométhane à un mélange méthanol (5%)/dichlorométhane pour livrer une huile que l'on utilise ensuite sans autre purification.
**LC/MS** (C₃₅H₂₆N₂O₄) 539 [M+H]⁺ ; RT 2,72 (Méthode A)

### Préparation 3e : Acide 2-(3-{[4-(benzyloxy)phényl](phényl)carbamoyl}-1H-indol-1-yl)-4-méthoxybenzoïque

### Stade A : 2-(3-{[4-(Benzyloxy)phényl](phényl)carbamoyl}-1H-indol-1-yl)-4-méthoxybenzoate de méthyle

Le mode opératoire est le même que dans le procédé de Préparation 3a, en remplaçant l'acide 5-benzyloxy-2-bromo-4-méthoxybenzoïque par du 2 bromo-4-méthoxybenzoate de méthyle.
**LC/MS** (C₃₇H₃₀N₂O₅) 583 [M+H]⁺ ; RT 2,85 (Méthode A)

### Stade B : Acide 2-(3-{[4-(benzyloxy)phényl](phényl)carbamoyl}-1H-indol-1-yl)-4-méthoxybenzoïque

On ajoute du NaOH aqueux (2 M, 1 ml) à une solution du composé obtenu au Stade A (314 mg, 0,54 mmol) dans du tétrahydrofurane/méthanol (1:1, 4 ml) et l'on agite le mélange réactionnel à température ambiante toute une nuit. La solution résultante est acidifiée avec du HCl aqueux 2 M et extraite dans de l'acétate d'éthyle, séchée sur sulfate de magnésium, filtrée et évaporée pour livrer le produit sous forme d'une huile qui est utilisée sans autre purification.
**LC/MS** (C₃₆H₂₈N₂O₅) 569 [M+H]⁺ ; RT 2,74 (Méthode A)

### Préparation 3f : Acide 2-[3-(diphénylcarbamoyl)-1H-indol-1-yl]benzoïque

Le mode opératoire est le même que dans la Préparation 3d, en remplaçant la 4-(benzyloxy)-N-phénylaniline du Stade B par de la N-phénylaniline.

### Préparation 3g : Acide 2-[3-(diphénylcarbamoyl)-1H-indol-1-yl]-4-méthoxybenzoïque

Le mode opératoire est le même que dans la Préparation 3a, en remplaçant le *N*-[4-(benzyloxy)phényl]-*N*-phényl-1*H*-indole-3-carboxamide par du *N*,*N*-diphényl-1*H*-indole-3-carboxamide issu de la Préparation 2b, et en remplaçant l'acide 5-benzyloxy-2-bromo-4-méthoxybenzoïque par de l'acide 2-iodo-4-méthoxybenzoïque.

### Préparation 3h : Acide 6-(3-{[4-(benzyloxy)phényl](phényl)carbamoyl}-1H-indol-1-yl)-2H-1,3-benzodioxole-5-carboxylique

Le mode opératoire est le même que dans la Préparation 3a, en remplaçant l'acide 5-benzyloxy-2-bromo-4-méthoxybenzoïque par de l'acide 6-bromo-2*H*-1,3-benzodioxole-5-carboxylique.

### Préparation 3i : Acide 2-(3-{[4-(benzyloxy)phényl](phényl)carbamoyl}-1H-indol-1-yl)-4-chlorobenzoïque

Le mode opératoire est le même que dans la Préparation 3a, en remplaçant l'acide 5-benzyloxy-2-bromo-4-méthoxybenzoïque par de l'acide 4-chloro-2-iodobenzoïque.

### Préparation 3j : Acide 6-(3-{[4-(benzyloxy)phényl](phényl)carbamoyl}-1H-indol-1-yl)-2-(2-méthylpropyl)-2H-1,3-benzodioxole-5-carboxylique

Le mode opératoire est le même que dans la Préparation 3a, en remplaçant l'acide 5-benzyloxy-2-bromo-4-méthoxybenzoïque par de l'acide 6-bromo-2-(2-méthylpropyl)-2*H-*1,3-benzodioxole-5-carboxylique.

### Préparation 3k : Acide 2-(3-{[4-(benzyloxy)phényl](phényl)carbamoyl}-1H-indol-1-yl)-4-,5-diméthoxybenzoïque

Le mode opératoire est le même que dans la Préparation 3a, en remplaçant l'acide 5-benzyloxy-2-bromo-4-méthoxybenzoïque par de l'acide 2-bromo-4,5-diméthoxybenzoïque.

### Préparation 3l: Acide 2-(3-{[4-(benzyloxy)phényl](phényl)carbamoyl}-1H-indol-1-yl)-4-chloro-5-méthoxybenzoïque

### Stade A : 2-(3-{[4-(Benzyloxy)phényl](phényl)carbamoyl}-1H-indol-1-yl)-4-chloro-5-méthoxybenzoate de méthyle

Le mode opératoire est le même que dans le procédé de Préparation 3e, en remplaçant le 2-bromo-4-méthoxybenzoate de méthyle par du 2-bromo-4-chloro-5-méthoxybenzoate de méthyle.

### Stade B : Acide 2-(3-{[4-(benzyloxy)phényl](phényl)carbamoyl}-1H-indol-1-yl)-4-chloro-5-méthoxybenzoïque

On chauffe à reflux le composé issu du Stade A (110 mg, 0,18 mmol) dans du tétrahydrofurane (2 ml) et du NaOH aqueux 2M (2 ml, 4 mmol) pendant 1,5 heures. Le mélange réactionnel est réparti selon sa solubilité entre l'acétate d'éthyle et l'eau, et les phases organiques sont séchées sur sulfate de magnésium, filtrées et concentrées. Le matériau brut est chargé sur une colonne PE-AX, balayé avec du dichlorométhane et le composé est élué avec un mélange acide formique (10 %)/dichlorométhane et soumis à une distillation azéotropique avec du toluène pour livrer une gomme.
**LC/MS** (C₃₆H₂₇N₂O₅Cl) 603,2 [M+H]⁺ ; RT 2,58 (Méthode A)

### Préparation 3m : Acide 7-(3-{[4-(benzyloxy)phényl](phényl)carbamoyl}-1H-indol-1-yl)-3-éthyl-2,3-dihydro-1,4-benzodioxine-6-carboxylique

### Step A: 2-Bromo-4,5-dihydroxybenzoate de méthyle

A une solution de 2-bromo-4,5-diméthoxybenzoate de méthyle (2,41 g, 10 mmol) dans le dichlorométhane, refroidie à -78 °C sous azote, est additionné du tribromure de bore (1M; 77 mL, 77 mmol), et on laisse le milieu réactionnel revenir à la température ambiante. Le mélange est versé sur du MeOH (200 mL) refroidi dans un bain de glace, puis évaporé et réparti selon sa solubilité entre l'acétate d'éthyle et l'eau. La phase organique est séchée sur sulfate de magnésium, filtrée et évaporée pour conduire au produit attendu qui est engagé dans le stade suivant sans purification.
**LC/MS** (C₈H₇BrO₄) 244 [M-H]⁻; RT 1,82 (Méthode A)

### Step B: 7-Bromo-3-éthyl-2,3-dihydro-1,4-benzodioxine-6-carboxylate de méthyle

A une solution du composé du Stade A (200 mg, 0,81 mmol) dans le DMF (3 mL) sont additionnés du carbonate de césium (792 mg, 2,43 mmol) et du 1,2-dibromobutane (295 µL, 2,43 mmol). Le mélange résultant est chauffé dans un appareil à micro-ondes à 150 °C pendant 20 min. Le mélange est réparti selon sa solubilité entre l'acétate d'éthyle et l'eau, et la phase organique est séchée sur sulfate de magnésium, filtrée et concentrée sous pression réduite. Le matériau brut est purifié par chromatographie éclair sur colonne de gel de silice, élué avec de l'hexane jusqu'à un mélange 4:1 hexane/ acétate d'éthyle pour conduire au produit attendu sous la forme d'une huile.
**LC/MS** (C₁₂H₁₃BrO₄) 301 [M+H]⁺; RT 2,62 (Méthode A)

### Step C: Acide 7-(3-{[4-(Benzyloxy)phényl](phényl)carbamoyl}-1H-indol-1-yl)-3-éthyl-2,3-dihydro-1,4-benzodioxine-6-carboxylique

Le mode opératoire est le même que celui de la Préparation 3e, en remplaçant le 2-bromo-4-méthoxybenzoate de méthyle avec le composé du Stade B.

### Préparation 3n : Acide 3-(benzyloxy)-6-(3-{[4-(benzyloxy)phényl](phényl)-carbamoyl}-1H-indol-1-yl)-2-chloro-4-méthoxybenzoïque

Le mode opératoire est le même que dans la Préparation 3a, en remplaçant l'acide 5-benzyloxy-2-bromo-4-méthoxybenzoïque par de l'acide 3-(benzyloxy)-6-bromo-2-chloro-4-méthoxybenzoïque.

### Préparation 3o : Acide 6-(3-{[4-(benzyloxy)phényl](phényl)carbamoyl}-5-fluoro-1H-indol-1-yl)-2H-1,3-benzodioxole-5-carboxylique

Le mode opératoire est le même que dans la Préparation 3a, en remplaçant l'acide 5-benzyloxy-2-bromo-4-méthoxybenzoïque par de l'acide 6-bromo-2*H*-1,3-benzodioxole-5-carboxylique, et en remplaçant le composé de la Préparation 2a par le composé de la Préparation 2d.

### Préparation 3p : Acide 6-[3-(diphénylcarbamoyl)-1H-indol-1-yl]-2H-1,3-benzodioxole-5-carboxylique

Le mode opératoire est le même que dans la Préparation 3a, en remplaçant l'acide 5-benzyloxy-2-bromo-4-méthoxybenzoïque par de l'acide 6-bromo-2*H*-1,3-benzodioxole-5-carboxylique, et en remplaçant le composé de la Préparation 2a par le composé de la Préparation 2b.

### Préparation 3q : Acide 4-chloro-2-[3-(diphénylcarbamoyl)-1H-indol-1-yl]benzoïque

Le mode opératoire est le même que dans la Préparation 3a, en remplaçant l'acide 5-benzyloxy-2-bromo-4-méthoxybenzoïque par de l'acide 2-bromo-4-chlorobenzoïque, et en remplaçant le composé de la Préparation 2a par le composé de la Préparation 2b.

### Préparation 3r : Acide 4,5-bis(benzyloxy)-2-(3-{[4-(benzyloxy)phényl] (phényl)carbamoyl}-1H-indol-1-yl)benzoïque

Le mode opératoire est le même que dans la Préparation 3e, en remplaçant le 2-bromo-4-méthoxybenzoate de méthyle par du 4,5-bis(benzyloxy)-2-bromobenzoate de méthyle.

### Préparation 3s : Acide 2-[3-(dibutylcarbamoyl)-1H-indol-1-yl]-5-nitrobenzoïque

Une solution du composé de la Préparation 2c (250 mg, 0,92 mmol) et de 2-fluoro-5-nitrobenzoate de méthyle (365,5 mg, 1,84 mmol) dans du *N*,*N*-diméthylformamide (5 ml) est traitée avec du carbonate de potassium (317,12 mg, 2,29 mmol) et chauffé à reflux pendant 20 heures sous azote. Le mélange réactionnel est refroidi jusqu'à la température ambiante, concentré sous vide, dilué avec de l'eau, acidifié jusqu'à pH 1 avec du HCl aqueux 2M et extrait avec de l'acétate d'éthyle. Les extraits organiques sont lavés à la saumure, séchés sur sulfate de magnésium, filtrés et concentrés sous vide. Le matériau brut, isolé sous forme d'acide, est chargé à sec sur une colonne de silice de 20 g et élué avec de l'iso-hexane, puis un mélange 1:1 iso-hexane:acétate d'éthyle, puis avec de l'acétate d'éthyle, et enfin avec un mélange 9:1 acétate d'éthyle:méthanol. Les fractions contenant le produit sont combinées, concentrées sous vide et chargées sur une cartouche PEAX de 10 g pré-lavée, en lavant avec du dichlorométhane, puis du méthanol, puis du dichlorométhane et en éluant le produit avec un mélange 9:1 dichlorométhane:acide formique, qui est ensuite concentré sous vide, dilué avec de la saumure et extrait avec de l'acétate d'éthyle. Les phases organiques sont ensuite lavées à la saumure, séchées sur sulfate de magnésium, filtrées et concentrées sous vide pour livrer un solide.
**LC/MS** (C₂₄H₂₇N₃O₅) 438 [M+H]⁺ ; RT 2,64 (Méthode A)

### Préparation 3t : Acide 5-(benzyloxy)-2-[3-(diphénylcarbamoyl)-1H-indol-1-yl]-4-méthoxybenzoïque

Le mode opératoire est le même que dans la Préparation 3a, en remplaçant l'acide 5-benzyloxy-2-bromo-4-méthoxybenzoïque par de l'acide 5-(benzyloxy)-2-bromo-4-méthoxybenzoïque, et en remplaçant le composé de la Préparation 2a par le composé de la Préparation 2b.

### Préparation 3u : Acide 5-(benzyloxy)-2-{3-[(4-fluorophényl)(phényl)carbamoyl]-1H-indol-1-yl}-4-méthoxybenzoïque

Le mode opératoire est le même que dans la Préparation 3a, en remplaçant le composé de la Préparation 2a par le composé de la Préparation 2f.

### Préparation 3v: Acide 5-(benzyloxy)-4-méthoxy-2-{3-[(4-méthylphényl)(phényl) carbamoyl]-1H-indol-1-yl}benzoïque

Le mode opératoire est le même que dans la Préparation 3a, en remplaçant le composé de la Préparation 2a par le composé de la Préparation 2g.

**Les composés des Préparations 4a, 4b et 4c sont synthétisés selon des modes opératoires analogues à ceux décrits aux Préparations 1" à 26".**

### Préparation 4a: 4-[(tert-Butyldimethylsilyl)oxy]-N-cyclohexylaniline

### Préparation 4b: 4-(Benzyloxy)-N-butylaniline

### Préparation 4c: N-(4-{[tert-Butyl(diméthyl)silyl]oxy}phényl)-1-méthyl-1H-indol-5-amine

### Préparation 5a: N,N-Diphényl-1H-indazole-3-carboxamide

### Stade A: 3-Iodo-1H-indazole

On ajoute de l'iode (51,56 g, 0,2 mol) et du KOH pilé (8,62 g, 0,375 mol) à une solution de 1*H*-indazole (12 g, 0,1 mol) dans le *N*,*N*-diméthylformamide (190 ml) et l'on agite le mélange réactionnel à température ambiante pendant 1 heure. Le mélange réactionnel est réparti selon sa solubilité entre l'éther diéthylique et une solution à 10 % de thiosulfate de sodium, puis la phase aqueuse est lavée deux fois à l'éther diéthylique. Les phases organiques sont combinées et lavées à la saumure, séchées sur sulfate de magnésium, filtrées, évaporées et séchées sous vide pour livrer le produit sous forme d'un solide.
**LC/MS** (C₇H₅N₂I) 245 [M+H]⁺; RT 2,13 (Méthode A)

### Stade B: 3-Iodo-2-{[2-(triméthylsilyl)éthoxy]méthyl}-2H-indazole

On ajoute de la *N*-méthyl dicyclohexylamine (18,3 ml, 0,09 mol) et du chlorure de 2-(triméthylsilyl)éthoxyméthyle (15,3 ml, 0,09 mol) à une solution de 3-iodo-1*H*-indazole (15 g, 0,06 mol) dans du tétrahydrofurane (150 ml) et l'on agite le mélange réactionnel à température ambiante sous azote pendant le week-end. Le mélange réactionnel est réparti selon sa solubilité entre l'acétate d'éthyle et la NaOH 2M, puis la phase organique est lavée à la saumure, séchée sur sulfate de magnésium, filtrée et concentrée. On la purifie par chromatographie éclair sur colonne de gel de silice selon un gradient allant d'iso-hexane à un mélange acétate d'éthyle (40 %)/iso-hexane pour obtenir le produit sous forme d'une huile.
**LC/MS** (C₁₃H₁₉N₂OSiI) 375 [M+H]⁺; RT 2,78 (Méthode A)

### Stade C: N,N-Diphényl-2-{[2-(triméthylsilyl)éthoxy]méthyl}-2H-indazole-3-carboxamide

On ajoute goutte à goutte une solution du composé obtenu au Stade B (5,0 g, 13,36 mmol) dans le tétrahydrofurane anhydre (25ml) à une solution de tétrahydrofurane anhydre (100 ml) et de *n*-butyllithium (2,2 M, 12,15 ml, 26,72 mmol) refroidie jusqu'à -78°C sous azote. Après 5 minutes, on ajoute goutte à goutte une solution de chlorure de diphénylcarbamyle (4,64 g, 20,04 mmol) dans du tétrahydrofurane anhydre (25 ml) et on laisse le mélange réactionnel se réchauffer jusqu'à la température ambiante pendant 4 heures. Le mélange réactionnel est réparti selon sa solubilité entre l'eau et l'acétate d'éthyle, et les phases organiques sont séchées sur sulfate de magnésium, filtrées et concentrées jusqu'à livrer une huile, qui est purifiée par chromatographie éclair sur gel de silice selon un gradient allant d'iso-hexane à un mélange acétate d'éthyle (25 %)/iso-hexane pour livrer le produit sous forme d'un solide.
**LC/MS** (C₂₆H₂₉N₃O₂Si) pas de m/z observé; RT 2,87 (Méthode A)

### Stade D: N,N-Diphényl-1H-indazole-3-carboxamide

On ajoute de l'éthylènediamine (0,48 ml, 7,16 mmol), puis une solution de TBAF 1M dans du tétrahydrofurane (7,16 ml, 7,16 mmol), à une solution du composé obtenu au Stade C (3,03 g, 6,82 mmol) dans du tétrahydrofurane anhydre (50 ml) et l'on chauffe le mélange réactionnel à reflux à 80°C toute une nuit sous azote. On ajoute une quantité supplémentaire d'éthylènediamine (0,17 ml, 2,54 mmol) et de TBAF (1,36 ml, 1,36 mmol) et l'on chauffe le mélange réactionnel à reflux pendant une heure de plus. On laisse le mélange réactionnel refroidir jusqu'à la température ambiante, puis on le répartit selon sa solubilité entre l'eau et l'acétate d'éthyle. Les phases organiques sont séchées sur sulfate de magnésium, filtrées et concentrées. On les purifie par chromatographie éclair sur gel de silice selon un gradient allant d'iso-hexane à un mélange acétate d'éthyle (80 %)/iso-hexane pour obtenir un solide.
**LC/MS** (C₂₀H₁₅N₃O) 314 [M+H]⁺; RT 2,36 (Méthode A)

### Préparation 5b: 5-(Benzyloxy)-N,N-diphényl-1H-indazole-3-carboxamide

Le mode opératoire est le même que dans le procédé de la Préparation 5a, en remplaçant le 1*H*-indazole du Stade A par du 5-(benzyloxy)-1*H*-indazole.
**LC/MS** (C₂₇H₂₁N₃O₂) 420 [M+H]⁺; RT 2,63 (Méthode A)

### Préparation 5c: 6-Méthoxy-N,N-Diphényl-1H-indazole-3-carboxamide

Le mode opératoire est le même que dans le procédé de la Préparation 5a, en remplaçant le 1*H*-indazole du Stade A par du 6-(méthoxy)-1*H*-indazole.
**LC/MS** (C₂₁H₁₇N₃O₂) 344 [M+H]⁺; RT 2,33 (Méthode A)

### Préparation 5d: N,N-Dibutyl-1H-indazole-3-carboxamide

On ajoute de la DIPEA (1,1 ml, 6,16 mmol) et de la dibutylamine (627 µl, 3,70 mmol), puis du HBTU (1,4 g, 3,70 mmol), à une solution d'acide 1*H*-indazole-3-carboxylique (500 mg, 3,08 mmol) dans du *N*,*N*-diméthylformamide (10 ml) sous azote, et l'on agite le mélange réactionnel à température ambiante pendant 16 heures. On ajoute davantage de dibutylamine (2,61 ml, 15,4 mmol) et l'on chauffe le mélange réactionnel à 50°C pendant 48 heures. Le mélange réactionnel est inactivé avec de l'eau, concentré, puis dilué avec une solution saturée de bicarbonate de sodium et les phases organiques sont extraites dans de l'acétate d'éthyle, lavées à la saumure, séchées sur sulfate de magnésium, filtrées et concentrées. Le matériau brut est purifié par chromatographie sur gel de silice dans de l'iso-hexane, jusque dans un mélange 9:1 iso-hexane/acétate d'éthyle, puis jusque dans un mélange 3:1 iso-hexane/acétate d'éthyle, pour livrer le produit sous forme d'un solide.
**LC/MS** (C₁₆H₂₃N₃O) 274 [M+H]⁺; RT 2,51 (Méthode A)

### Préparation 5e: 6-(Benzyloxy)-1H-indazole-3-carboxylate d'éthyle

### Stade A: 6-(Benzyloxy)-3-iodo-2-{[2-(triméthylsilyl)éthoxy]méthyl}-2H-indazole

Le mode opératoire est le même que dans le procédé des Stades A -B de la Préparation 5a, en remplaçant le 1*H*-indazole utilisé au Stade A par le 6-(benzyloxy)-1*H*-indazole.

### Stade B: Acide 6-(Benzyloxy)-2-{[2-(triméthylsilyl)éthoxy]méthyl}-2H-indazole-3-carboxylique

On ajoute une solution du composé obtenu au Stade A (0,97 g, 2,02 mmol) dans du tétrahydrofurane (5 ml) à une solution de tétrahydrofurane (10 ml), refroidie jusqu'à -78°C, et de *n*-butyllithium (2,5 M, 1,62 ml, 4,04 mmol), puis on laisse le tout se réchauffer jusqu'à la température ambiante pendant 30 minutes. On ajoute 0,41 ml supplémentaire de *n*-butyllithium au mélange réactionnel, qui est agité pendant 30 minutes, avant un nouveau refroidissement jusqu'à -78°C. On ajoute du dioxyde de carbone solide pilé, puis on laisse le mélange réactionnel se réchauffer jusqu'à la température ambiante et l'on l'agite toute une nuit. Le mélange réactionnel est inactivé avec de l'eau et le pH est ajusté à 4 avec du HCl 2M, on l'extrait avec de l'acétate d'éthyle et les phases organiques sont séchées sur sulfate de magnésium, filtrées et évaporées. Le matériau brut est chargé sur une colonne PE-AX, balayé avec du méthanol et le composé est élué avec un mélange 5:1 dichlorométhane/acide acétique pour livrer un solide.
**LC/MS** (C₂₁H₂₆N₂O₄Si) 397,1 [M-H]⁻; RT 2,69 (Méthode A)

### Stade C: 6-(Benzyloxy)-1H-indazole-3-carboxylate d'éthyle

On ajoute de l'acide sulfurique (0,25 ml) à une solution du composé obtenu au Stade B (76 mg, 0,19 mmol) dans de l'éthanol (5 ml) et l'on chauffe le mélange réactionnel à reflux toute une nuit. Le mélange réactionnel est alcalinisé avec du NaOH 2N, puis extrait dans de l'acétate d'éthyle et les phases organiques sont séchées sur sulfate de magnésium, filtrées et concentrées pour livrer un solide. Le produit est utilisé à l'étape suivante sans aucune autre purification.
**LC/MS** (C₁₇H₁₆N₂O₃) 297,1 [M+H]⁺; RT 2,47 (Méthode A)

### Préparation 6a: Acide 6-[3-(Diphénylcarbamoyl)-1H-indazol-1-yl]-2H-1,3-benzodioxole-5-carboxylique

On dégaze une solution de *N*,*N*-diphényl-1*H*-indazole-3-carboxamide issu de la Préparation 5a (300 mg, 0,96 mmol), de carbonate de potassium (200 mg, 1,44 mmol) et d'acide 6-bromo-2*H*-1,3-benzodioxole-5-carboxylique (235 mg, 0,96 mmol) dans du *N,N-*diméthylformamide (3 ml) avec de l'azote, puis l'on ajoute de l'iodure de cuivre (I) (20 mg, 0,096 mmol) et l'on agite le mélange réactionnel à 100°C sous azote pendant 4,5 heures. Le mélange réactionnel est refroidi jusqu'à la température ambiante, dilué avec de l'acétate d'éthyle et lavé à l'eau. Les phases organiques sont séchées sur sulfate de magnésium, filtrées et évaporées et le matériau brut est purifié par chromatographie sur colonne de gel de silice selon un gradient allant de dichlorométhane à un mélange méthanol (10 %)/dichlorométhane pour livrer le produit sous forme d'une huile.
**LC/MS** (C₂₈H₁₉N₃O₅) 478 [M+H]⁺; RT 2,54 (Méthode A)

### Préparation 6b: Acide 2-[3-(diphénylcarbamoyl)-1H-indazol-1-yl]-5-nitrobenzoïque

### Stade A: 2-[3-(Diphénylcarbamoyl)-1H-indazol-1-yl]-5-nitrobenzoate de méthyle

On ajoute du carbonate de potassium (514 mg, 3,72 mmol) à une solution du composé obtenu à la Préparation 5a (777 mg, 2,48 mmol) et de 2-fluoro-5-nitrobenzoate de méthyle (593 mg, 2,98 mmol) dans du *N*,*N*-diméthylformamide (30 ml) et l'on chauffe le mélange réactionnel jusqu'à 100°C sous azote toute une nuit. Le mélange réactionnel est concentré et repris dans de l'acétate d'éthyle, lavé à la saumure, puis séché sur sulfate de magnésium, filtré et concentré. Le matériau brut est purifié par chromatographie sur gel de silice dans un mélange 6:1 iso-hexane/acétate d'éthyle, puis dans un mélange 2:1 iso-hexane/acétate d'éthyle pour livrer un solide.
**LC/MS** (C₂₈H₂₀N₄O₅) 493 [M+H]⁺; RT 2,69 (Méthode A)

### Stade B: Acide 2-[3-(diphénylcarbamoyl)-1H-indazol-1-yl]-5-nitrobenzoïque

On ajoute du NaOH 2N (8 ml) à une solution du composé obtenu au Stade A (816 mg, 1,66 mmol) dans du tétrahydrofurane (8 ml) et l'on agite le mélange réactionnel à température ambiante toute une nuit. Le mélange réactionnel est acidifié avec du HCl 2M et le produit est extrait dans de l'acétate d'éthyle, séché sur sulfate de magnésium, filtré et concentré pour livrer un solide, qui est utilisé directement, sans autre purification, à l'étape suivante.
**LC/MS** (C₂₇H₁₈N₄O₅) 479 [M+H]⁺; RT 2,5 (Méthode A)

### Préparation 6c: Acide 2-[6-(benzyloxy)-3-(diphénylcarbamoyl)-1H-indazol-1-yl]benzoïque

### Stade A: Acide 2-[6-(benzyloxy)-3-(éthoxycarbonyl)-1H-indazol-1-yl]benzoïque

On ajoute de l'iodure de cuivre (I) (15 mg, 0,081 mmol) et de l'acide 2-bromobenzoïque (196 mg, 0,973 mmol) au composé obtenu à la Préparation 5e (240 mg, 0,811 mmol) dans du *N*,*N*-diméthylformamide (10 ml) et du carbonate de potassium (160 mg, 1,217 mmol) et l'on agite le mélange réactionnel à 140°C toute une nuit. Le mélange réactionnel est concentré et le résidu est repris dans de l'acétate d'éthyle et lavé successivement à l'eau et à la saumure, puis séché sur sulfate de magnésium, filtré et concentré. Le matériau brut est purifié par chromatographie sur colonne dans un mélange 96:4 dichlorométhane/méthanol pour livrer une huile.
**LC/MS** (C₂₄H₂₀N₂O₅) 417 [M+H]⁺; RT 2,55 (Méthode A)

### Stade B: Acide 2-[6-(benzyloxy)-3-(diphénylcarbamoyl)-1H-indazol-1-yl]benzoïque

On ajoute du *n*-butyllithium (2,5 M, 0,2 ml, 0,49 mmol) à une solution de N-phénylaniline (83 mg, 0,49 mmol) dans du tétrahydrofurane (5 ml), refroidie jusqu'à -78°C, et l'on laisse le mélange réactionnel se réchauffer jusqu'à la température ambiante pendant 1 heure. Le composé obtenu au Stade A (68 mg, 0,163 mmol) dissous dans du tétrahydrofurane (5 ml) est refroidi jusqu'à -78°C et l'on ajoute la solution de N-phénylaniline. On laisse le mélange réactionnel se réchauffer jusqu'à la température ambiante. Après 1,5 heure, le mélange réactionnel est inactivé avec de l'eau et neutralisé avec du HCl 2M. Les phases organiques sont extraites dans de l'acétate d'éthyle, puis séchées sur sulfate de magnésium, filtrées et évaporées. Le matériau brut est purifié par chromatographie sur colonne dans un mélange 96:4 dichlorométhane/méthanol.
**LC/MS** (C₃₅H₂₆N₂O₄) 540 [M+H]⁺; RT 2,67 (Méthode A)

### Préparation 6d: Acide 2-[3-(dibutylcarbamoyl)-1H-indazol-1-yl]benzoïque

Le mode opératoire est le même que dans le procédé de la Préparation 6a, en remplaçant le composé de la Préparation 5a par le composé de la Préparation 5d, ainsi que l'acide 6-bromo-2*H*-1,3-benzodioxole-5-carboxylique par l'acide 2-iodobenzoïque.

### Préparation 6e: Acide 2-[3-(diphénylcarbamoyl)-1H-indazol-1-yl]benzoïque

Le mode opératoire est le même que dans le procédé de la Préparation 6a, en utilisant l'acide 2-iodobenzoïque.

### Préparation 6f: Acide 2-(3-{[4-(Benzyloxy)phényl](phényl)carbamoyl}-1H-indazol-1-yl)benzoïque

### Stade A: Acide 2-[3-(éthoxycarbonyl)-1H-indazol-1-yl]benzoïque

On ajoute du carbonate de potassium (218 mg, 1,58 mmol) et de l'acide 2-iodobenzoïque (313 mg, 1,26 mmol) à une solution de 1*H*-indazole-3-carboxylate d'éthyle (200 mg, 1,05 mmol) dans du *N*,*N*-diméthylformamide (20 ml). On dégaze la solution avec de l'azote, puis l'on ajoute de l'iodure de cuivre (I) (20 mg, 0,11 mmol), avant de chauffer le tout à 100°C toute une nuit. Le mélange réactionnel est réparti selon sa solubilité entre l'acétate d'éthyle et le HCl 2M, puis les phases organiques sont lavées à la saumure, séchées sur sulfate de magnésium, filtrées et évaporées pour livrer une huile, qui est purifiée par une colonne PE-AX pré-lavée avec du méthanol. Le composé est chargé dans du méthanol minimal avec quelques gouttes de triéthylamine. La colonne est lavée au méthanol et le produit est élué avec un mélange acide formique (10%)/dichlorométhane pour livrer une huile, qui est utilisée directement sans autre purification.
**LC/MS** (C₁₇H₁₄N₂O₄) 311 [M+H]⁺; RT 2,22 (Méthode A)

### Stade B: Acide 2-(3-{[4-(benzyloxy)phényl](phényl)carbamoyl}-1H-indazol-1-yl)benzoïque

On ajoute du LiHMDS (0,8 ml, 0,8 mmol) à une solution de 4-(benzyloxy)-*N-*phénylaniline (0,15 g, 0,53 mmol) dans du tétrahydrofurane anhydre (5 ml), refroidie jusqu'à -78°C sous azote, et on laisse le mélange réactionnel sous agitation à -78°C pendant 10 minutes. On ajoute une solution du composé obtenu au Stade A (0,17 g, 0,53 mmol) dans du tétrahydrofurane anhydre (5 ml) et on laisse le mélange réactionnel se réchauffer jusqu'à la température ambiante, puis on le chauffe jusqu'à 50°C pendant le week-end. On ajoute une quantité supplémentaire de LiHMDS (0,80 ml) et l'on chauffe le mélange réactionnel à 50°C pendant 4 heures de plus. Le mélange réactionnel est réparti selon sa solubilité entre l'acétate d'éthyle et l'eau, puis les phases organiques sont lavées à la saumure, séchées sur sulfate de magnésium, filtrées et concentrées jusqu'à livrer une huile, qui est utilisée directement, sans autre purification, pour l'étape suivante.
**LC/MS** (C₃₄H₂₅N₃O₄) 538 [M-H]⁻; RT 2,72 (Méthode A)

### Préparation 6g: Acide 2-[3-(diphénylcarbamoyl)-6-méthoxy-1H-indazol-1-yl]benzoïque

On applique au composé obtenu à la Préparation 5c le mode opératoire de la Préparation 6a en présence d'acide 2-iodobenzoïque, en chauffant à 140°C.

### Préparation 6h: Acide 2-[3-(diphénylcarbamoyl)-6-hydroxy-1H-indazol-1-yl]benzoïque

Le composé de la Préparation 6g (464 mg, 1 mmol) dans du dichlorométhane (20 ml), refroidi jusqu'à 0°C sous azote, est traité goutte à goutte avec du tribromure de bore (1M, 0,95 ml, 10 mmol) et l'on agite le mélange réactionnel à température ambiante sous azote toute une nuit. Le mélange réactionnel est dilué avec du méthanol et neutralisé avec de la triéthylamine, puis concentré. Le résidu est extrait avec de l'acétate d'éthyle et lavé au HCl dilué, et les phases organiques sont séchées sur sulfate de magnésium, filtrées et concentrées. Le matériau brut est purifié par chromatographie sur gel de silice selon un gradient allant de dichlorométhane à un mélange méthanol (8 %)/dichlorométhane pour livrer un solide.
**LC/MS** (C₂₇H₁₉N₃O₄) 450 [M+H]⁺; RT 2,34 (Méthode A)

### Préparation 6i: Acide 2-[3-(diphénylcarbamoyl)-6-hydroxy-1H-indazol-1-yl]benzoïque

Le mode opératoire est le même que dans le procédé de la Préparation 6a en utilisant l'acide 5-(benzyloxy)-2-bromo-4-méthoxybenzoïque.

### Préparation 6j: Acide 2-[5-(benzyloxy)-3-(diphénylcarbamoyl)-1H-indazol-1-yl]benzoïque

Le mode opératoire est le même que dans le procédé de la Préparation 6a, en remplaçant le composé de la Préparation 5a par le composé de la Préparation 5b, ainsi que l'acide 6-bromo-2*H*-1,3-benzodioxole-5-carboxylique par l'acide 2-iodobenzoïque.

### Exemple 1. Chlorhydrate de 1-(2-{[(3S)-3-(aminométhyl)-3,4-dihydroisoquinoléin-2(1H)-yl]carbonyl}phényl)-N,N-dibutyl-5-méthyl-1H-pyrrole-3-carboxamide

### Stade A : {[(3S)-2-{2-[4-(Dibutylcarbamoyl)-2-méthyl-1H-pyrrol-1-yl]benzoyl}-1,2,3,4-tétrahydroisoquinoléin-3-yl]méthyl}carbamate de tert-butyle

Le composé du titre est obtenu suivant les Stades A-C du procédé de l'Exemple 8 en utilisant le [(3*S*)-1,2,3,4-tétrahydroisoquinoléin-3-ylméthyl]carbamate de *tert*-butyle (voir Préparation 1') au Stade A et la *N*,*N*-dibutylamine au Stade C.

### Stade B : Chlorhydrate de 1-(2-{[(3S)-3-(Aminométhyl)-3,4-dihydroisoquinoléin-2(1H)-yl]carbonyl}phényl)-N,N-dibutyl-5-méthyl-1H-pyrrole-3-carboxamide

A une solution du dérivé NH-Boc du Stade A dans le dichlorométhane (10 mL/mmol) placée à 0°C, on ajoute goutte à goutte l'acide trifluoroacétique (10 équivalents molaires). L'ensemble est agité progressivement à température ambiante pendant 12 h. Le milieu réactionnel est ensuite concentré à sec, repris et co-évaporé 2 fois avec du toluène, puis concentré à sec. Le résidu est alors solubilisé dans le dichlorométhane. Cette phase organique est lavée avec une solution saturée de NaHCO₃, puis avec une solution saturée de NaCl, séchée sur MgSO₄ et concentrée à sec. Le résidu obtenu est alors solubilisé dans un minimum de dichlorométhane. L'ensemble est placé à 0°C et on ajoute une solution HCl/Et₂O 1M (2 équivalents molaires). Le milieu réactionnel est agité 1h à température ambiante, concentré à sec, repris avec un mélange CH₃CN/H₂O et lyophilisé à basse température pour conduire au produit attendu
***Microanalyse élémentaire : (% théorique : mesuré)***
%C=69.32:69.32; %H=7.69:7.68; %N=10.43:10.43; %Cl=6.6:6.79; %Cl-=6.6:6.66

**Les composés des Exemples 2 à 4 sont obtenus suivant le procédé de l'Exemple 1 en utilisant l'amine NHR₃R₄ adéquate.**

### Exemple 2. Chlorhydrate de 1-(2-{[(3S)-3-(aminométhyl)-3,4-dihydroisoquinoléin-2(1H)-yl]carbonyl}phényl)-N-butyl-5-méthyl-N-(2-phényléthyl)-1H-pyrrole-3-carboxamide

***Microanalyse élémentaire : (% théorique : mesuré)***
%C=71.84:71.44; %H=7.06:6.73; %N=9.57:9.42; %Cl-=6.06:6.16

### Exemple 3. Chlorhydrate de 1-(2-{[(3S)-3-(aminométhyl)-3,4-dihydroisoquinoléin-2(1H)-yl]carbonyl}phényl)-N-butyl-5-méthyl-N-(3-phénylpropyl)-1H-pyrrole-3-carboxamide

***Microanalyse élémentaire :** (% **théorique : mesuré)***
%C=72.16:70.89; %H=7.23:6.96; %N=9.35:9.21; %Cl-=5.92:8.01

### Masse haute résolution (ESI+) :

Formule brute : C₃₆H₄₂N₄O₂
[M+H]⁺calculé : 563.3386
[M+H]⁺ mesuré : 563.3373

### Exemple 4. Chlorhydrate de 1-(2-{[(3S)-3-(aminométhyl)-3,4-dihydroisoquinoléin-2(1H)-yl]carbonyl}phényl)-N-butyl-5-méthyl-N-(4-phénylbutyl)-1H-pyrrole-3-carboxamide

***Microanalyse élémentaire : (% théorique : mesuré)***
%C=72.47:73.11; %H=7.4:6.95; %N=9.14:9.03; %Cl-=5.78:5.81

### Exemple 5. Trifluoroacétate de 1-(2-{[(3S)-3-(aminométhyl)-3,4-dihydroisoquinoléin-2(1H)-yl]carbonyl}phényl)-5-méthyl-N,N-diphényl-1H-pyrrole-3-carboxamide

### Stade A : {[(3S)-2-{2-[4-(Diphénylcarbamoyl)-2-méthyl-1H-pyrrol-1-yl]benzoyl}-1,2,3,4-tétrahydroisoquinoléin-3-yl]méthyl}carbamate de tert-butyle

Le composé du titre est obtenu selon le procédé de l'Exemple 8 en utilisant le [(3*S*)-1,2,3,4-tétrahydroisoquinoléin-3-ylméthyl]carbamate de *tert*-butyle (voir Préparation 1') au Stade A et la *N*-phénylaniline au Stade C.

### Stade B : Trifluoroacétate de 1-(2-{[(3S)-3-(aminométhyl)-3,4-dihydroisoquinoléin-2(1H)-yl]carbonyl}phényl)-5-méthyl-N,N-diphényl-1H-pyrrole-3-carboxamide

Une solution du dérivé NH-Boc du Stade A dans le dichlorométhane est placée à 0°C. On y ajoute, goutte à goutte, 10 équivalents molaires d'acide trifluoroacétique. L'ensemble est agité à température ambiante pendant 4 h jusqu'à totale disparition du produit de départ. Le milieu réactionnel est ensuite concentré à sec, repris et co-évaporé 2 fois avec du toluène, puis repris avec un mélange acétonitrile/H₂O et enfin lyophilisé.

### Masse haute résolution (ESI+) :

Formule brute : C₃₅H₃₂N₄O₂
[M+H]⁺ calculé : 541.2604
[M+H]⁺ mesuré: 541.2612

### Exemple 6. 1-(2-{[(3S)-3-(Aminométhyl)-3,4-dihydroisoquinoléin-2(1H)-yl]carbonyl}-5-hydroxyphényl)-5-méthyl-N,N-diphényl-1H-pyrrole-3-carboxamide

### Stade A : {[(3S)-2-{4-(Benzyloxy)-2-[4-(diphénylcarbamoyl)-2-méthyl-1H-pyrrol-1-yl]benzoyl}-1,2,3,4-tétrahydroisoquinoléin-3-yl]méthyl}carbamate de tert-butyle

Le composé du titre est synthétisé selon le procédé de l'Exemple 8 en utilisant au Stade A l'acide obtenu à la Préparation 2 et la [(3*S*)-1,2,3,4-tétrahydroisoquinoléin-3-ylméthyl]carbamate de *tert*-butyle obtenue à la Préparation l', ainsi que la N-phénylaniline au Stade C.

### Stade B : {[(3S)-2-{2-[4-(Diphénylcarbamoyl)-2-méthyl-1H-pyrrol-1-yl]-4-hydroxybenzoyl}-1,2,3,4-tétrahydroisoquinoléin-3-yl]méthyl}carbamate de tert-butyle

Le composé du Stade A est solubilisé dans le méthanol (5mL/mmol), et on ajoute du Pd/C (10% massique). Après une nuit sous 1 bar d'hydrogène, le milieu réactionnel est filtré, rincé à l'éthanol. Le filtrat est concentré à sec. On obtient le produit désiré que l'on engage dans l'étape suivant sans purification.

### Stade C : 1-(2-{[(3S)-3-(Aminométhyl)-3,4-dihydroisoquinoléin-2(1H)-yl]carbonyl}-5-hydroxyphényl)-5-méthyl-N,N-diphényl-1H-pyrrole-3-carboxamide

A une solution de l'intermédiaire NH-Boc dans le dichlorométhane (10 mL/mmol) placée à 0°C, on ajoute, goutte à goutte, l'acide trifluoroacétique (10 équivalents molaires). L'ensemble est ensuite agité à température ambiante pendant 12 h. Le milieu réactionnel est concentré à sec, repris et coévaporé 2 fois avec du toluène, puis concentré à sec. Le résidu est alors solubilisé dans le dichlorométhane. Cette phase organique est lavée avec une solution saturée de NaHCO₃, puis avec une solution saturée de NaCl, séchée sur MgSO4 et concentrée à sec. Le résidu obtenu est alors solubilisé dans un minimum de dichlorométhane. L'ensemble est placé à 0°C et on ajoute une solution de HCl/Et₂O 1M (2 équivalents molaires). Le milieu réactionnel est agité 1h à température ambiante, concentré à sec, repris avec un mélange CH₃CN/H₂O et lyophilisé à basse température pour conduire au produit attendu.
***Microanalyse élémentaire : (% théorique : mesuré)***
%C=75.52:74.91; %H=5.79:5.17; %N=10.06:10.07

### Exemple 7. Chlorhydrate de 1-(2-{[(3S)-3-(aminométhyl)-3,4-dihydroisoquinoléin-2(1H)-yl]carbonyl}-5-hydroxyphényl)-N-(4-hydroxyphényl)-5-méthyl-N-phényl-1H pyrrole-3-carboxamide

Le composé du titre est synthétisé selon le procédé de l'Exemple 6 en remplaçant la *N-*phénylaniline par la 4-benzyloxy-*N*-phényl-aniline.
***Microanalyse élémentaire : (% théorique : mesuré)***
%C=69.01:67.55; %H=5.46:4.86; %N=9.2:9.26; %Cl-=5.82:6.56

### Masse haute résolution (ESI+) :

Formule brute : C₃₅H₃₂N₄O₄
[M+H]⁺ calculé : 573.2502
[M+H]⁺ mesuré : 573.2493

### Exemple 8. 1-(2-{[(3S)-3-[(Diméthylamino)méthyl]-3,4-dihydroisoquinoléin-2(1H)-yl]carbonyl}phényl)-N-(4-hydroxyphényl)-5-méthyl-N-phényl-1H-pyrrole-3-carboxamide

### Stade A : 1-(2-{[(3S)-3-[(Diméthylamino)méthyl]-3,4-dihydroisoquinoléin-2(1H)-yl]carbonyl}phényl)-5-méthyl-1H-pyrrole-3-carboxylate d'éthyle

A une solution de 1,7 g d'acide obtenu à la Préparation 1 (6,3 mmol) dans 50 mL de dichlorométhane sont ajoutés 2 g de *N*,*N*-diméthyl-1-[(3*S*)-1,2,3,4-tétrahydroisoquinoléin-3-yl]méthanamine (7,6 mmol), 4 mL de *N*,*N*,*N*-triéthylamine (28,5 mmol), 1,46 g de 1-éthyl-3-(3'-diméthylaminopropyl)-carbodiimide (EDC) (7,6 mmol mmol), et 1,03g d'hydroxybenzotriazole (HOBT) (7,6 mmol). Le milieu réactionnel est agité à température ambiante pendant une nuit, puis il est versé sur une solution aqueuse saturée de NaHCO₃ et extrait avec du chlorure de méthylène. La phase organique est ensuite séchée sur sulfate de magnésium, puis filtrée et évaporée à sec. Le produit brut ainsi obtenu est alors purifié par chromatographie sur gel de silice (gradient dichlorométhane/ méthanol) pour donner le produit attendu sous la forme d'une poudre.
**IR :** v :>C=O 1703 cm⁻¹ ester ; v :>C=O 1630 cm⁻¹ amide; v : CHN 2768 cm⁻¹ bande de Bolhmann

### Stade B : 1-(2-{[(3S)-3-[(Diméthylamino)méthyl]-3,4-dihydroisoquinoléin-2(1H)-yl] carbonyl}phényl)-5-méthyl-1H-pyrrole-3-carboxylate de lithium

Le composé du Stade A (0,5 g ; 1,1 mmol) est solubilisé dans 4 mL de dioxane. Une solution de LiOH (50 mg ; 1,2 mmol) dans l'eau (1 mL) est ajoutée. L'ensemble est chauffé dans un appareil à micro-ondes pendant 70 min à 140°C (puissance 100 W). Après retour à température ambiante, le milieu réactionnel est évaporé à sec et utilisé tel quel pour l'étape suivante.
**RMN¹H** : δ :(400 MHz ; dmso-d6 ; 353K) : 7.6-6.8 (m, 9H, aryls+1H pyrrole) ; 6.05 (s1, 1H, pyrrole) ; 5.0-4.0 (m, 3H, Cₜₑᵣₜᵢₐᵢᵣₑ THIQ + 2C_{secondaire} THIQ) ; 2.68 (m, 2H C_{secondaire} THIQ); 2.2-1.8 (m, 11H, NMe₂ + CH₂N + CH₃pyrrole)
**IR :** v : >C=O 1626 cm⁻¹ amide; v : >C=O-O 1573 cm⁻¹

### Stade C : N-[4-(Benzyloxy)phényl]-1-(2-{[(3S)-3-[(diméthylamino)méthyl]-3,4-dihydro isoquinoléin-2(1H)-yl]carbonyl}phényl)-5-méthyl-N-phényl-1H-pyrrole-3-carboxamide

A une suspension de 475 mg (1,1 mmol) du carboxylate de lithium obtenu au Stade B dans 10 mL de dichlorométhane, on ajoute 0,3 mL (3,4 mmol) de chlorure d'oxalyle et une goutte de DMF. Après 3 h d'agitation, le milieu réactionnel est concentré à sec, puis repris dans du dichlorométhane (10 ml). Cette solution est alors coulée sur une solution de dichlorométhane (10 mL) contenant du composé de la Préparation 1" (470 mg ; 1,7 mmol) et de la pyridine (0,15 mL ; 1,7 mmol). Après l'addition, le milieu réactionnel est chauffé au reflux pendant 10 heures. Après refroidissement, il est versé sur une solution aqueuse saturée de NaHCO₃ et extrait avec du chlorure de méthylène. La phase organique est ensuite séchée sur sulfate de magnésium, puis filtrée et évaporée à sec. Le produit brut ainsi obtenu est alors purifié par chromatographie sur gel de silice (gradient dichlorométhane/ méthanol) pour donner le produit attendu sous la forme d'une mousse.
**RMN¹H** : δ (400 MHz ; dmso-d6 ; 350K) : 7.6-6.3 (m, 23H, H aromatiques) ; 5.5 (s1, 1H, H pyrrole) ; 5.05 (s, 2H, H benzyliques) ; 4.0-5.0 (m, 3H, H THIQ) ; 2.9-2.5 (m, 2H, H THIQ); 2.3-1.6 (m, 8H, NME₂ + CH₂N) ; 1.85 (s1, 3H, CH₃pyrrole)
**IR :** v : >C=O : 1623 cm⁻¹ amide

### Stade D : 1-(2-{[(3S)-3-[(Diméthylamino)méthyl]-3,4-dihydroisoquinoléin-2(1H)-yl] carbonyl}phényl)-N-(4-hydroxyphényl)-5-méthyl-N-phényl-1H-pyrrole-3-carboxamide

A une solution du produit issu du Stade C (510 mg ; 0,75 mmol) dans de l'éthanol (8 mL) est ajouté du cyclohexène (0,5 mL) et du Pd/C à 10% de Pd (100 mg). Le milieu réactionnel est chauffé à 80°C une nuit. Après refroidissement et filtration sur Whatman®, le filtrat est concentré à sec et purifié par chromatographie sur gel de silice (gradient dichlorométhane/ méthanol) pour donner le produit attendu sous la forme d'une mousse incolore.
**RMN¹H** : δ (400 MHz ; dmso-d6 ; 350K) : 9.5 (s1, 1H, OH) ; 7.55-6.3 (m, 14H, H aromatiques + H pyrrole) ; 6.9 (d, 2H, H aromatiques) ; 6.67 (d, 2H, H aromatiques) ; 5.50 (s, 1H, H pyrrole) ; 4.80 (m, 1H, H THIQ); 4.15 (m, 2H, H THIQ) ; 2.80 (m, 2H, H THIQ) ; 2.3-1.6 (m, 11H, NMe₂ + CH₂N + CH₃pyrrole).
**IR :** v : OH (H₂O et OH (phénol) : 3500-2200 cm⁻¹ v : >C=O : 1630 cm⁻¹ amide
***Microanalyse élémentaire : (% théorique : mesuré)***
%C=76:75.62; %H=6.21:5.83; %N=9.58:9.68

### Exemple 9. 1-(2-{[(3S)-3-[(Diméthylamino)méthyl]-3,4-dihydroisoquinoléin-2(1H)-yl]carbonyl}phényl)-N,N-bis(4-méthoxyphényl)-5-méthyl-1H-pyrrole-3-carboxamide

Le composé est obtenu suivant les Stades A-C du procédé de l'Exemple 8 en utilisant la 4-méthoxy-*N*-(4-méthoxyphényl)aniline au Stade C.
***Microanalyse élémentaire : (% théorique : mesuré)***
%C=74.5:73.91; %H=6.41:6.06; %N=8.91:8.9

### Exemple 10. 1-(2-{[(3S)-3-[(Diméthylamino)méthyl]-3,4-dihydroisoquinoléin-2(1H)-yl]carbonyl}phényl)-N-(1H-indol-5-yl)-5-méthyl-N-phényl-1H-pyrrole-3-carboxamide

Le composé est obtenu suivant les Stades A-C du procédé de l'Exemple 8 en utilisant le composé de la Préparation 3" au Stade C. Le groupement 1*H*-indol-5-yl est ensuite déprotégé à l'aide de potasse méthanolique selon le protocole du Stade B de l'Exemple 48.
***Microanalyse élémentaire : (% théorique : mesuré)***
%C=77.08:76.34; %H=6.14:5.76; %N=11.52:11.3

### Exemple 11. 1-(2-{[(3S)-3-[(Diméthylamino)méthyl]-3,4-dihydroisoquinoléin-2(1H)-yl]carbonyl}phényl)-N-(1H-indol-6-yl)-5-méthyl-N-phényl-1H-pyrrole-3-carboxamide

Le composé est obtenu suivant les Stades A-C du procédé de l'Exemple 8 en utilisant le composé de la Préparation 4" au Stade C. Le groupement 1*H*-indol-5-yl est ensuite déprotégé à l'aide de potasse méthanolique selon le protocole du Stade B de l'Exemple 48.
***Microanalyse élémentaire : (% théorique : mesuré)***
%C=77.08:76.59; %H=6.14:5.63; %N=11.52:11.01

### Exemple 12. 1-[2-{[(3S)-3-[(Diméthylamino)méthyl]-3,4-dihydroisoquinoléin-2(1H)-yl]carbonyl}-5-(trifluorométhyl)phényl]-N-(4-hydroxyphényl)-5-méthyl-N-phényl-1H-pyrrole-3-carboxamide

### Stade A : N-(4-{[tert-Butyl(diméthyl)silyl]oxy}phényl)-1-(2-{[(3S)-3-[(diméthylamino)méthyl]-3,4-dihydroisoquinoléin-2(1H)-yl]carbonyl}-5-(trifluorométhyl) phényl]-5-méthyl-N-phényl-1H-pyrrole-3-carboxamide

Le composé du titre est synthétisé suivant les Stades A-C du procédé de l'Exemple 8 en utilisant au Stade A l'acide obtenu à la Préparation 3 et au Stade C le composé de la Préparation 25".

### Stade B : 1-(2-{[(3S)-3-[(Diméthylamino)méthyl]-3,4-dihydroisoquinoléin-2(1H)-yl]carbonyl}-5-(trifluorométhyl)phényl]-N-(4-hydroxyphényl)-5-méthyl-N-phényl-1H-pyrrole-3-carboxamide

A une solution de 2,3 mmol du composé obtenu au Stade A dans 4 mL de méthanol, on ajoute 0,646 g (11,5 mmol) d'hydroxyde de potassium solubilisé dans 8 mL de méthanol. L'ensemble est agité à température ambiante pendant 30 min. Le milieu réactionnel est ensuite dilué dans le dichlorométhane et lavé successivement avec une solution HCl 1N, une solution de NaHCO₃ saturée, puis une solution de NaCl saturée jusqu'à atteindre un pH neutre. La phase organique est ensuite séchée sur sulfate de magnésium, filtrée et évaporée. Le produit brut ainsi obtenu est purifié sur gel de silice (gradient dichlorométhane/méthanol) pour conduire au produit du titre.
***Microanalyse élémentaire : (% théorique : mesuré)***
%C=69.93:69.34; %H=5.4:5.09; %N=8.58:8.09

### Exemple 13. 1-(5-Chloro-2-{[(3S)-3-[(diméthylamino)méthyl]-3,4-dihydro isoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 4.
***Microanalyse élémentaire : (% théorique : mesuré)***
%C=71.78:69.85; %H=5.7:5.39; %N=9.05:8.54

### Masse haute résolution (ESI+) :

Formule brute : C₃₇H₃₅³⁵ClN₄O₃
[M+H]⁺ calculé : 619.2476
[M+H]⁺ mesuré: 619.2458

### Exemple 14. 1-(2-{[(3S)-3-[(Diméthylamino)méthyl]-3,4-dihydroisoquinoléin-2(1H)-yl]carbonyl}-5-fluorophényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 5.
***Microanalyse élémentaire : (% théorique : mesuré)***
%C=73.74:72.75; %H=5.85:5.73; %N=9.3:9

### Exemple 15. 1-(5-Hydroxy-2-{[(3S)-3-(morpholin-4-ylméthyl)-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 7 en utilisant la (3*S*)-3-(4-morpholinylméthyl)-1,2,3,4-tétrahydroisoquinoléine obtenue à la Préparation 2'.
***Microanalyse élémentaire : (% théorique : mesuré)***
%C=68.97:68.74; %H=5.79:5.65; %N=8.25:8.16; %Cl-=5.22:4.74

### Exemple 16. 1-(4,5-Dibromo-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 6 et la (3*S*)-3-[(4-méthyl-1-pipérazinyl)méthyl]-1,2,3,4-tétrahydroisoquinoléine obtenue à la Préparation 3'.
***Microanalyse élémentaire : (% théorique : mesuré)***
%C=60.24:59.6; %H=4.93:4.56; %N=8.78:8.31; %Br=20.04:19.65

### Exemple 17. 1-(5-Hydroxy-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 7 en utilisant au Stade A la (3*S*)-3-[(4-méthyl-1-pipérazinyl)méthyl]-1,2,3,4-tétrahydroisoquinoléine obtenue à la Préparation 3'.
***Microanalyse élémentaire : (% théorique : mesuré)***
%C=65.93:66.7; %H=5.95:5.61; %N=9.61:9.72; %Cl-=9.73:7.55

### Exemple 18. 1-(3-Chloro-2-{[(3S)-3-[(diméthylamino)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 7.
***Microanalyse élémentaire : (% théorique : mesuré)***
%C=71.78:70.82; %H=5.7:5.31; %N=9.05:8.89

### Exemple 19. N-(4-Hydroxyphényl)-5-méthyl-1-[2-{[(3S)-3-[(4-méthylpipérazin-1-yl)méthyl]-3,4-dihydroisoquinoléin-2(1H)-yl]carbonyl}-4-(trifluorométhoxy)phényl]-N-phényl-1H-pyrrole-3-carboxamide

Le composé du titre est synthétisé selon le procédé de l'Exemple 12 en utilisant au Stade A l'acide obtenu à la Préparation 8 et la (3*S*)-3-[(4-méthyl-1-pipérazinyl)méthyl]-1,2,3,4-tétrahydroisoquinoléine obtenue à la Préparation 3'.
***Microanalyse élémentaire : (% théorique : mesuré)***
%C=68.04:67.57; %H=5.57:5.48; %N=9.68:9.73

### Exemple 20. 1-(4,5-Difluoro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 9 et la (3*S*)-3-[(4-méthyl-1-pipérazinyl)méthyl]-1,2,3,4-tétrahydroisoquinoléine obtenue à la Préparation 3'.
***Microanalyse élémentaire : (% théorique : mesuré)***
%C=71.09:70.09; %H=5.82:5.28; %N=10.36:10.24

### Exemple 21. 1-(5-fluoro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 5 et la (3*S*)-3-[(4-méthyl-1-pipérazinyl)méthyl]-1,2,3,4-tétrahydroisoquinoléine obtenue à la Préparation 3'.
***Microanalyse élémentaire : (% théorique : mesuré)***
%C=73.04:72.28; %H=6.13:5.73; %N=10.65:10.52

### Exemple 22. 1-(5-Bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 10 et la (3S)-3-[(4-méthyl-1-pipérazinyl)méthyl]-1,2,3,4-tétrahydroisoquinoléine obtenue à la Préparation 3'.
***Microanalyse élémentaire : (% théorique : mesuré)***
%C=66.85:66.38; %H=5.61:5.41; %N=9.74:9.73; %Br=11.12:11.09

### Exemple 23. 1-(2-{[(3S)-3-[(Diméthylamino)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

### Stade A : 5-[(4-{[tert-Butyl(diméthyl)silyl]oxy}phényl){[1-(2-{[(3S)-3-[(diméthylamino) méthyl]-3,4-dihydroisoquinoléin-2(1H)-yl]carbonyl}phényl)-5-méthyl-1H-pyrrol-3-yl]carbonyl}amino]-1H-indole-1-carboxylate de tert-butyle

Le composé du titre est synthétisé suivant les Stades A-C du procédé de l'Exemple 8 en remplaçant le composé de la Préparation 1" par le composé de la Préparation 5".

### Stade B : 1-(2-{[(3S)-3-[(Diméthylamino)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

Le produit du Stade A est déprotégé suivant le protocole du Stade B de l'Exemple 48.
***Microanalyse élémentaire : (*% *théorique : mesuré)***
%C=75.1:72.56; %H=5.98:5.42; %N=11.23:10.71

### Masse haute résolution (ESI+) :

Formule brute : C₃₉H₃₇N₅O₃
[M+H]⁺ calculé : 624.2975
[M+H]⁺ mesuré : 624.2994

### Exemple 24. N-(4-Hydroxyphényl)-5-méthyl-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-phényl-1H-pyrrole-3-carboxamide

Le composé du titre est synthétisé selon le procédé de l'Exemple 12 en utilisant au Stade A l'acide obtenu à la Préparation 11 et la (3*S*)-3-[(4-méthyl-1-pipérazinyl)méthyl]-1,2,3,4-tétrahydroisoquinoléine obtenue à la Préparation 3'.

### Masse haute résolution (ESI+) :

Formule brute : C₄₁H₄₁N₅O₅
[M+H]⁺ calculé : 684.3186
[M+H]⁺ mesuré : 684.3163

**Les composés des Exemples 25 à 27 sont obtenus suivant le procédé de l'Exemple 13 en utilisant le dérivé de 1,2,3,4-tétrahydroisoquinoléine et l'amine NHR₃R₄ adéquate.**

### Exemple 25. 1-(4-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

***Microanalyse élémentaire : (% théorique : mesuré)***
%C=71.26:69.72; %H=5.98:5.35; %N=10.39:10.09

### Masse haute résolution (ESI+) :

Formule brute : C₄₀H₄₀³⁵ClN₅O₃
[M+H]⁺ calculé : 674.2898
[M+H]⁺ mesuré : 674.2873

### Exemple 26. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

***Microanalyse élémentaire : (*% *théorique : mesuré)***
%C=71.26:70.06; %H=5.98:5.19; %N=10.39:10.05; %Cl=5.26:5.49

### Masse haute résolution (ESI+) :

Formule brute : C₄₀H₄₀³⁵ClN₅O₃
[M+H]⁺ calculé : 674.2898
[M+H]⁺ mesuré : 674.2878

### Exemple 27. Chlorhydrate de N-(3-chloro-4-hydroxyphényl)-1-(5-chloro-2-{[(3S)-3-[(4-méthylpipérazin-1-yl)méthyl]-3,4-dihydroisoquinoléin-2(1H)-yl]carbonyllphényl)-5-méthyl-N-phényl-1H-pyrrole-3-carboxamide

***Microanalyse élémentaire : (*% *théorique : mesuré)***
%C=64.48:65.67; %H=5.41:5.4; %N=9.4:9.45; %Cl=14.27:11.82

### Masse haute résolution (ESI+) :

Formule brute : C₄₀H₃₉³⁵Cl₂N₅O₃
[M+H]⁺ calculé : 708.2508
[M+H]⁺ mesuré : 708.2509

### Exemple 28. 1-(5-Chloro-4-fluoro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 12 et la (3*S*)-3-[(4-méthyl-1-pipérazinyl)méthyl]-1,2,3,4-tétrahydroisoquinoléine obtenue à la Préparation 3'.
***Microanalyse élémentaire : (*% *théorique : mesuré)***
%C=69.4:68.27; %H=5.68:5.08; %N=10.12:9.81

### Masse haute résolution (ESI+) :

Formule brute : C₄₀H₃₉³⁵ClFN₅O₃
[M+H]⁺ calculé : 692.2804
[M+H]⁺ mesuré: 692.2818

### Exemple 29. 1-(4-Bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 13 et la (3*S*)-3-[(4-méthyl-1-pipérazinyl)méthyl]-1,2,3,4-tétrahydroisoquinoléine obtenue à la Préparation 3'.
***Microanalyse élémentaire : (*% *théorique : mesuré)***
%C=66.85:64.98; %H=5.61:5.2; %N=9.74:9.38

### Masse haute résolution (ESI+) :

Formule brute : C₄₀H₄₀⁷⁹BrN₅O₃
[M+H]⁺ calculé : 718.2393
[M+H]⁺ mesuré : 718.2380

### Exemple 30. N,N-bis(4-Chlorophényl)-1-(6-{[(3S)-3-[(4-méthylpipérazin-1-yl) méthyl]-3,4-dihydroisoquinoléin-2(1H)-yl]carbonyl}-1,3-benzodioxol-5-yl)-1H-indole-3-carboxamide

Le composé du titre est synthétisé selon le procédé de l'Exemple 12 en utilisant au Stade A l'acide obtenu à la Préparation 14 et la (3*S*)-3-[(4-méthyl-1-pipérazinyl)méthyl]-1,2,3,4-tétrahydroisoquinoléine obtenue à la Préparation 3', ainsi que l'amine NHR₃R₄ adéquate.

### Masse haute résolution (ESI+) :

Formule brute : C₄₄H₃₉Cl₂N₅O₄
[M+H]⁺ calculé : 772.2457
[M+H]⁺ mesuré : 772.2477

### Exemple 31. N-(4-Chlorophényl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

### Stade A : 1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxylate de méthyle

On applique le procédé décrit au Stade A de l'Exemple 8 en utilisant l'acide obtenu à la Préparation 14 et la (3S)-3-[(4-méthyl-1-pipérazinyl)méthyl]-1,2,3,4-tétrahydroisoquinoléine obtenue à la Préparation 3'.

### Stade B : N-(4-{[tert-butyl(diméthyl)silyl]oxy}phényl)-N-(4-chlorophényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

On applique le procédé décrit aux Stade B et C de l'Exemple 8 en remplaçant au Stade C la 4-benzyloxy-N-phényl-aniline par la 4-[*tert*-butyl(diméthyl)silyl]oxy-*N*-(4-chlorophényl)aniline.

### Stade C : N-(4-chlorophényl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

A une suspension de 250 mg (0,29 mmol) du produit obtenu au Stade B dans 1,5 mL de THF, on ajoute 0,32 mL de solution molaire de fluorure de tétrabutylammonium (0,32 mmol). Après 2 h d'agitation, le milieu réactionnel est concentré à sec et purifié par chromatographie sur gel de silice (gradient dichlorométhane/ méthanol) pour donner le produit attendu sous la forme d'une mousse.
***Microanalyse élémentaire : (*% *théorique : mesuré)***
%C=70.06:69.54; %H=5.34:4.93; %N=9.28:9.29

**Les composés des Exemples 32 à 36 sont obtenus suivant le procédé de l'Exemple 31 en utilisant l'amine NHR₃R₄ adéquate.**

### Exemple 32. N-(4-Hydroxyphényl)-N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=73.65:73.41; %H=5.91:5.47; %N=9.54:9.58

### Exemple 33. N-Butyl-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=72.08:71; %H=6.48:6.06; %N=10.01:10.02

### Masse haute résolution (ESI+) :

Formule brute : C₄₂H₄₅N₅O₅
[M+H]⁺ calculé : 700.3499
[M+H]⁺ mesuré : 700.3495

### Exemple 34. N-(3-Fluoro-4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-phényl-1H-indole-3-carboxamide

***Microanalyse élémentaire : (*% *théorique : mesuré)***
%C=71.63:69.22; %H=5.46:4.77; %N=9.49:9.36

### Masse haute résolution (ESI+) :

Formule brute : C₄₄H₄₀FN₅O₅
[M+H]⁺calculé : 738.3092
[M+H]⁺ mesuré : 738.3083

### Exemple 35. N-(4-Fluorophényl)-N-(4-hydroxyphényl)-1-(6-{[(3S)3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=71.63:69.88; %H=5.46:4.72; %N=9.49:9.5

### Masse haute résolution (ESI+) :

Formule brute : C₄₄H₄₀FN₅O₅
[M+H]⁺calculé : 738.3092
[M+H]⁺ mesuré : 738.3086

### Exemple 36. N-(4-Chlorophényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-(2-phényléthyl)-1H-indole-3-carboxamide

***Microanalyse élémentaire : (*% *théorique : mesuré)***
%C=72.1:71.1; %H=5.79:5.25; %N=9.14:9.34

### Exemple 37. N-(4-Hydroxyphényl)-N-(1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

### Stade A : 5-[(4-{[tert-Butyl(diméthyl)silyl]oxy}phényl){[1-(6-{[3S)-3-[(4-méthylpipérazin-1-yl)méthyl]-3,4-dihydroisoquinoléin-2(1H)-yl]carbonyl}-1,3-benzodioxol-5-yl)-1H-indol-3-yl]carbonyl}amino]-1H-indole-1-carboxylate de tert-butyle

Le composé du titre est synthétisé suivant les Stades A-B du procédé de l'Exemple 31 en remplaçant le composé de la Préparation 6" par le composé de la Préparation 5".

### Stade B : N-(4-hydroxyphényl)-N-(1H-indol-5-yl)-1-(6-{[(3S)-3-[(4-méthylpipérazin-1-yl)méthyl]-3,4-dihydroisoquinoléin-2(1H)-yl]carbonyl)-1,3-benzodioxol-5-yl)-1H-indole-3-carboxamide

A une solution de 0,49 mmol du produit obtenu au Stade A dans 5 mL de méthanol, est ajouté 135 mg (2,5 mmol) de KOH. Après 3 h d'agitation à température ambiante, le milieu réactionnel est concentré, traité par une solution aqueuse saturée de NaHCO₃ et extrait au chlorure de méthylène. La phase organique est ensuite séchée sur sulfate de magnésium, filtrée et évaporée à sec. Le produit brut ainsi obtenu est alors purifié par chromatographie sur gel de silice (gradient dichlorométhane/ méthanol) pour donner le produit attendu sous la forme d'une mousse.
***Microanalyse élémentaire : (*% *théorique : mesuré)***
%C=72.81:72.42; %H=5.58:5.34; %N=11.07:10.84

### Exemple 38. 1-(5-Bromo-2-{[(3S)-3-[(3aR,6aS)-hexahydrocyclopenta[c]pyrrol-2(1H)-ylméthyl]-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 13 et la (3*S*)-3-[(3a*R*,6a*S*)-hexahydrocyclopenta[*c*]pyrrol-2(1H)-ylméthyl]-1,2,3,4-tétrahydroisoquinoléine obtenue à la Préparation 4'.
***Microanalyse élémentaire : (*% *théorique : mesuré)***
%C=69.13:67.94; %H=5.66:5.25; %N=7.68:7.46; %Br=10.95:10.49

### Masse haute résolution (ESI+) :

Formule brute : C₄₂H₄₁⁷⁹BrN₄O₃
[M+H]⁺ calculé : 729.2440
[M+H]⁺ mesuré : 729.2454

**Les composés des Exemples 39 à 41 sont obtenus suivant le procédé de l'Exemple 31 en utilisant l'amine NHR₃R₄ adéquate.**

### Exemple 39. Chlorhydrate de N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-(3-phénoxyphényl)-1H-indole-3-carboxamide

***Microanalyse élémentaire : (*% *théorique : mesuré)***
%C=73.97:71.81; %H=5.59:5.26; %N=8.63:8.1

### Masse haute résolution (ESI+) :

Formule brute : C₅₀H₄₅N₅O₆
[M+H]⁺ calculé : 812.3448
[M+H]⁺ mesuré: 812.3431

### Exemple 40. N-(4-Hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-(2-phényléthyl)-1H-indole-3-carboxamide

***Microanalyse élémentaire : (*% *théorique : mesuré)***
%C=73.88:72.92; %H=6.06:5.64; %N=9.36:9.38

### Exemple 41. N,N-bis(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=71.82:70.67; %H=5.62:5.33; %N=9.52:8.97

### Masse haute résolution (ESI+) :

Formule brute : C₄₄H₄₁N₅O₆
[M+H]⁺ calculé : 736.3135
[M+H]⁺ mesuré: 736.3138

### Exemple 42. 1-(5-Bromo-2-{[(3S)-3-(morpholin-4-ylméthyl)-3,4-dihydroisoquinoléin-2(1H)-yl]carbonyl}phényl)-N-(4-hydroxyphényl)-5-méthyl-N-phényl-1H-pyrrole-3-carboxamide

Le composé du titre est synthétisé selon le procédé de l'Exemple 12 en utilisant au Stade A l'acide obtenu à la Préparation 10 et la (3*S*)-3-(4-morpholinylméthyl)-1,2,3,4-tétrahydroisoquinoléine obtenue à la Préparation 2'.
***Microanalyse élémentaire : (*% *théorique : mesuré)***
%C=66.38:65.51; %H=5.28:4.91; %N=7.94:7.95; %Br=11.32:10.2

**Les composés des Exemples 43 à 45 sont obtenus suivant le procédé de l'Exemple 31 en utilisant l'amine NHR₃R₄ adéquate.**

### Exemple 43. N-(1-Benzothiophén-5-yl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=71.21:66.87; %H=5.33:4.87; %N=9.03:8.3;S=4.13:5.03

### Masse haute révolution (ESI+) :

Formule brute : C₄₆H₄₁N₅O₅S
[M+H]⁺ calculé : 776.2907
[M+H]⁺ mesuré : 776.2922

### Exemple 44. N-(1-Benzofuran-5-yl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (% théorique : mesuré)***
%C=72.71:70.11; %H=5.44:5.08; %N=9.22:8.78

### Masse haute révolution (ESI+) :

Formule brute : C₄₆H₄₁N₅O₆
[M+H]⁺ calculé : 760.3135
[M+H]⁺ mesuré: 760.3135

### Exemple 45. N-(4-Hydroxyphényl)-N-(1-méthyl-1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (% théorique : mesuré)***
%C=73.04:70.78; %H=5.74:5.26; %N=10.87:10.28

### Masse haute révolution (ESI+) :

Formule brute : C₄₇H₄₄N₆O₅
[M+H]⁺ calculé : 773.3451
[M+H]⁺ mesuré : 773.3459

### Exemple 46. 1-(5-Bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

Le composé du titre est synthétisé selon le procédé de l'Exemple 12 en utilisant au Stade A l'acide obtenu à la Préparation 10, la (3S)-3-[(4-méthyl-1-pipérazinyl)méthyl]-1,2,3,4-tétrahydroisoquinoléine obtenue à la Préparation 3' et le composé de la Préparation 5".
***Microanalyse élémentaire : (*% *théorique : mesuré)***
%C=66.58:64.54; %H=5.45:4.96; %N=11.09:10.48; %Br=10.55:10.02

### Masse haute résolution (ESI+) :

Formule brute : C₄₂H₄₁⁷⁹BrN₆O₃
[M+H]⁺ calculé : 757.2502
[M+H]⁺ mesuré : 757.2480

### Exemple 47. N-(2,3-Dihydro-1H-indol-5-yl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

Le composé du titre est synthétisé suivant le procédé de l'Exemple 31 en utilisant l'amine NHR₃R₄ adéquate.
***Microanalyse élémentaire : (*% *théorique : mesuré)***
%C=72.61:70.73; %H=5.83:5.16; %N=11.05:10.51

### Masse haute révolution (ESI+) :

Formule brute : C₄₆H₄₄N₆O₅
[M+H]⁺ calculé : 761.3451
[M+H]⁺ mesuré: 761.3418

### Exemple 48. 1-(5-bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1H-indol-5-yl)-1H-indole-3-carboxamide

### Stade A : 5-[{[1-(5-Bromo-2-{[(3S)-3-[(4-méthylpipérazin-1-yl)méthyl]-3,4-dihydroisoquinoléin-2(1H)-yl]carbonyl}phényl)-1H-indol-3-yl]carbonyl}(4-{[tert-butyl(diméthyl)silyl]oxy}phényl)amino]-1H-indole-1-carboxylate de tert-butyle

Le composé du titre est synthétisé selon le procédé de l'Exemple 37 en utilisant l'acide obtenu à la Préparation 15.

### Stade B : 1-(5-Bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1H-indol-5-yl)-1H-indole-3-carboxamide

A une solution de 495 mg (0,49 mmol) du produit obtenu au Stade A dans 5 mL de méthanol, est ajouté 135 mg (2,5 mmol) de KOH. Après 3 h d'agitation à température ambiante, le milieu réactionnel est concentré, traité par une solution aqueuse saturée de NaHCO₃ et extrait au chlorure de méthylène. La phase organique est ensuite séchée sur sulfate de magnésium, filtrée et évaporée à sec. Le produit brut ainsi obtenu est alors purifié par chromatographie sur gel de silice (gradient dichlorométhane/ méthanol) pour donner le produit attendu sous la forme d'une mousse.
***Microanalyse élémentaire : (*% *théorique : mesuré)***
%C=68.09:66.77; %H=5.21:4.73; %N=10.59:10.29; %Br=10.07:9.76

### Masse haute résolution (ESI+) :

Formule brute : C₄₅H₄₁⁷⁹BrN₆O₃
[M+H]⁺ calculé : 793.2502
[M+H]⁺ mesuré : 793.2539

**Sauf mention contraire, les composés des Exemples suivants sont synthétisés selon le procédé de l'Exemple 31 en utilisant au Stade A : (i) l'acide approprié obtenu selon l'une des Préparations 1 à 22 et (ii) le dérivé de tétrahydroisoquinoléine approprié obtenu selon l'une des Préparations 1' à 12', ainsi qu'au Stade B : (iii) l'amine NHR₃R₄ adéquate (une liste non exhaustive est proposée aux Préparations 1" à 26"). Les composés ainsi obtenus peuvent être isolés sous la forme d'un chlorhydrate en utilisant le protocole de salification utilisant de l'éther méthanolique présenté à la fin du Stade C de l'Exemple 1.**

### Exemple 49. N-(4-Hydroxyphényl)-N-(4-méthoxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=72.08:71.86; %H=5.78:5.49; %N=9.34:9.31

### Exemple 50. 1-(5-Bromo-2-{[(3S)-3-[(4-méthylpipérazin-1-yl)méthyl]-3,4-dihydroisoquinoléin-2(1H)-yl]carbonyl}phényl)-N-(4-fluorophényl)-N-(4-hydroxyphényl)-5-méthyl-1H-pyrrole-3-carboxamide

***Microanalyse élémentaire : (*% *théorique : mesuré)***
%C=65.22:65.4; %H=5.34:4.89; %N=9.51:9.53; %Br=10.85:10.86

### Exemple 51. N-(4-Hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-[4-(trifluorométhoxy)phényl]-1H-indole-3-carboxamide

***Microanalyse élémentaire : (*% *théorique : mesuré)***
%C=67.24:67.28; %H=5.02:4.73; %N=8.71:8.78

### Exemple 52. N-(3-Fluoro-4-méthylphényl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=71.89:71.45; %H=5.63:5.15; %N=9.31:9.34

### Exemple 53. 1-(5-Bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-(4-méthylphényl)-1H-pyrrole-3-carboxamide

***Microanalyse élémentaire : (*% *théorique : mesuré)***
%C=67.21:67.28; %H=5.78:5.62; %N=9.56:9.49; %Br=10.91:10.87

### Exemple 54. 1-(5-Bromo-2-{[(3S)-3-[(4-méthylpipérazin-1-yl)méthyl]-3,4-dihydroisoquinoléin-2(1H)-yl]carbonyl}phényl)-N,N-bis(4-hydroxyphényl)-1H-indole-3-carboxamide

***Microanalyse élémentaire : (*% *théorique : mesuré)***
%C=67.01:66.03; %H=5.23:5.23; %N=9.09:8.74

### Exemple 55. N-(1H-Indol-5-yl)-N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=74.58:73.82; %H=5.86:5.69; %N=11.1:10.87

### Exemple 56. N-(3,4-Difluorophényl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=69.92:69.53; %H=5.2:4.98; %N=9.27:9.07

### Exemple 57. 1-(5-Bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(4-méthylphényl)-1H-indole-3-carboxamide

***Microanalyse élémentaire : (*% *théorique : mesuré)***
%C=68.75:68.42; %H=5.51:5.88; %N=9.11:8.83; %Br=10.39:10.16

### Exemple 58. Chlorhydrate de N-(3-chloro-4-fluorophényl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=65.35:66.91; %H=4.99:4.83; %N=8.66:8.8; %Cl=8.77:7.03; %Cl-=4.38:2.37

### Exemple 59. 1-(5-Bromo-2-{[(3S)-3-[(4-méthylpipérazin-1-yl)méthyl]-3,4-dihydroisoquinoléin-2(1H)-yl]carbonyl}phényl)-N-(4-chlorophényl)-N-(4-hydroxyphényl)-1H-indole-3-carboxamide

***Microanalyse élémentaire : (*% *théorique : mesuré)***
%H=4.98:5.64; %N=8.87:8.4; %C=65.45:65.99

### Exemple 60. 4-[(4-Méthylphényl){[1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indol-3-yl]carbonyl}amino]phényl acétate

***Microanalyse élémentaire : (*% *théorique : mesuré)***
%C=72.76:71.25; %H=5.85:5.71; %N=9.03:8.92

### Masse haute résolution (ESI+) :

Formule brute : C₄₇H₄₅N₅O₆
[M+H]⁺ calculé : 776.3448
[M+H]⁺ mesuré : 776.3398

### Exemple 61. 1-(5-Bromo-2-{[(3S)-3-[(4-méthylpipérazin-1-yl)méthyl]-3,4-dihydroisoquinoléin-2(1H)-yl]carbonyl}phényl)-N,N-bis(4-hydroxyphényl)-5-méthyl-1H-pyrrole-3-carboxamide

***Microanalyse élémentaire : (*% *théorique : mesuré)***
%C=65.39:63.91; %H=5.49:5.43; %N=9.53:9.12

### Masse haute résolution (ESI+) :

Formule brute : C₄₀H₄₀⁷⁹BrN₅O₄
[M+H]⁺ calculé : 734.2342
[M+H]⁺ mesuré : 734.2298

### Exemple 62. 1-(5-bromo-2-{[(3S)-3-[(4-méthylpipérazin-1-yl)méthyl]-3,4-dihydroisoquinoléin-2(1H)-yl]carbonyl}phényl)-N-(4-chlorophényl)-N-(4-hydroxyphényl)-5-méthyl-1H-pyrrole-3-carboxamide

***Microanalyse élémentaire : (*% *théorique : mesuré)***
%C=63.79:63.87; %H=5.22:5.13; %N=9.3:9.58

### Exemple 63. Chlorhydrate de N-(3-chloro-4-hydroxyphényl)-N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=64.25:64.13; %H=5.27:5.01; %N=8.33:8.18; %Cl-=8.43:7.25

### Exemple 64. Chlorhydrate de N-(3-chloro-4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-[4-(propan-2-yl)phényl]-1H-indole-3-carboxamide

***Microanalyse élémentaire : (*% *théorique : mesuré)***
%C=64.94:65.36; %H=5.57:5.2; %N=8.06:7.95; %Cl-=8.16:6.8

### Exemple 65. 1-(5-Chloro-2-{[(3S)-3-[(4-méthylpipérazin-1-yl)méthyl]-3,4-dihydroisoquinoléin-2(1H)-yl]carbonyl}phényl)-N-(4-chlorophényl)-N-(4-hydroxyphényl)-5-méthyl-1H-pyrrole-3-carboxamide

***Microanalyse élémentaire : (*% *théorique : mesuré)***
%C=67.79:67.6; %H=5.55:5.52; %N=9.88:9.98

### Exemple 66. 1-(5-Chloro-2-{[(3S)-3-[(4-méthylpipérazin-1-yl)méthyl]-3,4-dihydroisoquinoléin-2(1H)-yl]carbonyl}phényl)-N-(4-fluorophényl)-N-(4-hydroxyphényl)-5-méthyl-1H-pyrrole-3-carboxamide

***Microanalyse élémentaire : (*% *théorique : mesuré)***
%C=69.4:68.97; %H=5.68:5.64; %N=10.12:10.08

### Exemple 67. 1-(5-Chloro-2-{[(3S)-3-[(4-méthylpipérazin-1-yl)méthyl]-3,4-dihydroisoquinoléin-2(1H)-yl]carbonyl}phényl)-N,N-bis(4-hydroxyphényl)-5-méthyl-1H-pyrrole-3-carboxamide

***Microanalyse élémentaire : (*% *théorique : mesuré)***
%C=69.6:69.52; %H=5.84:5.73; %N=10.15:10.31

### Exemple 68. Chlorhydrate de N-(1H-indazol-5-yl)-N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=66.5:66.14; %H=5.46:5.3; %N=11.8:11.86; %Cl-=8.53:8.06

### Exemple 69. Chlorhydrate de 1-(5-bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1-méthyl-1H-indol-5-yl)-1H-indole-3-carboxamide

***Microanalyse élémentaire : (*% *théorique : mesuré)***
%C=62.73:63.57; %H=5.15:5.04; %N=9.54:9.64; %Cl-=8.05:6.7

### Exemple 70. Chlorhydrate de 1-(5-bromo-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-(1-méthyl-1H-indol-5-yl)-1H-pyrrole-3-carboxamide

***Microanalyse élémentaire : (*% *théorique : mesuré)***
%C=61.14:62.23; %H=5.37:5.27; %N=9.95:10.13; %Cl-=8.39:7

### Masse haute résolution (ESI+) :

Formule brute : C₄₃H₄₃⁷⁹BrN₆O₃
[M+H]⁺ calculé : 771.2658
[M+H]⁺ mesuré: 771.2645

### Exemple 71. 1-(5-Bromo-2-{[(3S)-3-[(4-méthylpipérazin-1-yl)méthyl]-3,4-dihydroisoquinoléin-2(1H)-yl]carbonyl}phényl)-N-(4-fluorophényl)-N-(4-hydroxyphényl)-1H-indole-3-carboxamide

***Microanalyse élémentaire : (*% *théorique : mesuré)***
%C=66.84:66.66; %H=5.09:5.23; %N=9.06:8.81; %Br=10.34:9.95

### Exemple 72. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1H-indol-5-yl)-1H-indole-3-carboxamide

Le composé du titre est synthétisé selon le procédé de l'Exemple 37 en utilisant l'acide obtenu à la Préparation 16.

***Microanalyse élémentaire : (*% *théorique : mesuré)***
%C=72.13:71.39; %H=5.51:5.45; %N=11.22:10.75; %Cl=4.73:5.46

### Exemple 73. 1-(5-Chloro-2-{[(3S)-3-[(4-méthylpipérazin-1-yl)méthyl]-3,4-dihydroisoquinoléin-2(1H)-yl]carbonyl}phényl)-N,N-bis(4-hydroxyphényl)-1H-indole-3-carboxamide

***Microanalyse élémentaire : (*% *théorique : mesuré)***
%C=71.11:70.47; %H=5.55:5.45; %N=9.64:9.44

### Exemple 74. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(4-méthylphényl)-1H-indole-3-carboxamide

***Microanalyse élémentaire : (*% *théorique : mesuré)***
%C=72.96:72.95; %H=5.84:5.74; %N=9.67:9.57

### Exemple 75. 1-(5-Chloro-2-{[(3S)-3-[(4-méthylpipérazin-1-yl)méthyl]-3,4-dihydroisoquinoléin-2(1H)-yl]carbonyl}phényl)-N-(4-fluorophényl)-N-(4-hydroxyphényl)-1H-indole-3-carboxamide

***Microanalyse élémentaire : (*% *théorique : mesuré)***
%C=70.92:70.28; %H=5.4:5.29; %N=9.62:9.44

### Exemple 76. 1-(5-Chloro-2-{[(3S)-3-[(4-méthylpipérazin-1-yl)méthyl]-3,4-dihydroisoquinoléin-2(1H)-yl]carbonyl}phényl)-N-(4-chlorophényl)-N-(4-hydroxyphényl)-1H-indole-3-carboxamide

***Microanalyse élémentaire : (*% *théorique : mesuré)***
%C=69.35:69.01; %H=5.28:5.16; %N=9.4:9.17; %Cl=9.52:9.74

### Exemple 77. Chlorhydrate de 1-(5-chloro-4-fluoro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-(1-méthyl-1H-indol-5-yl)-1H-pyrrole-3-carboxamide

***Microanalyse élémentaire : (*% *théorique : mesuré)***
%C=63.12:63.39; %H=5.42:5.16; %N=10.27:10.04; %Cl=13:12.21; %Cl-=8.67:7

### Exemple 78. Chlorhydrate de 1-(5-chloro-4-fluoro-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1-méthyl-1H-indol-5-yl)-1H-indole-3-carboxamide

***Microanalyse élémentaire : (*% *théorique : mesuré)***
%C=64.68:64.96; %H=5.19:4.89; %N=9.84:9.63; %Cl=12.45:12.01; %Cl-=8.3:6.83

### Exemple 79. 1-(5-Bromo-2-{[(3S)-3-[(4,4-difluoropipéridin-1-yl)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

***Microanalyse élémentaire : (*% *théorique : mesuré)***
%C=64.78:63.92; %H=4.92:4.98; %N=8.99:8.97; %Br=10.26:10.8

### Exemple 80. 1-(5-Bromo-2-{[(3S)-3-[(4-cyclopentylpipérazin-1-yl)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

***Microanalyse élémentaire : (*% *théorique : mesuré)***
%C=68.06:66.99; %H=5.84:5.75; %N=10.35:10.1; %Br=9.84:9.81

### Exemple 81. Chlorhydrate de N-(3-chloro-4-hydroxyphényl)-N-(1-méthyl-1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=64.13:64.93; %H=5.15:4.92; %N=9.55:9.57; %Cl=12.08:11; %Cl-=8.06:6.45

### Exemple 82. Chlorhydrate de N-(3-chloro-4-hydroxyphényl)-N-(1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=63.78:64.52; %H=5:4.87; %N=9.7:9.72; %Cl=12.28:11.49; %Cl-=8.19:6.77

### Exemple 83. N,N-bis(4-Hydroxyphényl)-5-méthyl-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=70.37:70.32; %H=5.91:5.77; %N=10.01:9.91

### Exemple 84. N-(4-Fluorophényl)-N-(4-hydroxyphényl)-5-méthyl-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=70.17:68.82; %H=5.74:5.5; %N=9.98:9.81

### Masse haute résolution (ESI+) :

Formule brute : C₄₁H₄₀FN₅O₅
[M+H]⁺ calculé : 702.3092
[M+H]⁺ mesuré : 702.3096

### Exemple 85. N-(4-Chlorophényl)-N-(4-hydroxyphényl)-5-méthyl-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=68.56:68.13; %H=5.61:5.53; %N=9.75:9.66

### Exemple 86. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

***Microanalyse élémentaire : (*% *théorique : mesuré)***
%C=70.72:70.45; %H=5.79:5.69; %N=11.78:11.56; %Cl=4.97:5.5

### Exemple 87. N-(4-Hydroxyphényl)-N-(1H-indol-5-yl)-5-méthyl-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=71.45:71.1; %H=5.86:5.86; %N=11.63:11.52

### Exemple 88. 5-Chloro-N-(4-hydroxyphényl)-N-(1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=69.64:69.34; %H=5.21:5.21; %N=10.59:10.45

### Exemple 89. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-(1-méthyl-1H-indol-5-yl)-1H-pyrrole-3-carboxamide

***Microanalyse élémentaire : (*% *théorique : mesuré)***
%C=71.01:69.93; %H=5.96:5.84; %N=11.56:11.2

### Masse haute résolution (ESI+) :

Formule brute : C₄₃H₄₃³⁵ClN₆O₃
[M+H]⁺ calculé : 727.3163
[M+H]⁺ mesuré: 727.3126

### Exemple 90. N-(4-Hydroxyphényl)-5-méthyl-N-(1-méthyl-1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=71.72:69.98; %H=6.02:6.02; %N=11.41:11.05

### Masse haute résolution (ESI+) :

Formule brute : C₄₄H₄₄N₆O₅
[M+H]⁺ calculé : 737.3451
[M+H]⁺ mesuré : 737.3444

### Exemple 91. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-5-méthyl-N-(4-méthylphényl)-1H-pyrrole-3-carboxamide

***Microanalyse élémentaire : (*% *théorique : mesuré)***
%C=71.55:71.06; %H=6.15:6.08; %N=10.18:10.02

### Exemple 92. 1-(3,5-Dichloro-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

Le composé du titre est synthétisé selon le procédé de l'Exemple 12 en utilisant au Stade A l'acide obtenu à la Préparation 17, la (3*S*)-3-[(4-méthyl-1-pipérazinyl)méthyl]-1,2,3,4-tétrahydroisoquinoléine obtenue à la Préparation 3' et le composé de la Préparation 5".
***Microanalyse élémentaire : (*% *théorique : mesuré)***
%C=67.47:67.28; %H=5.39:5.84; %N=11.24:10.07; %Cl=9.48:9.21

### Exemple 93. 1-(5-Chloro-2-{[(3S)-3-[(4-méthylpipérazin-1-yl)méthyl]-3,4-dihydroisoquinoléin-2(1H)-yl]carbonyl}phényl)-N-(4-hydroxyphényl)-N-(1-méthyl-1H-indol-5-yl)-1H-indole-3-carboxamide

***Microanalyse élémentaire : (*% *théorique : mesuré)***
%C=72.38:71.93; %H=5.68:5.94; %N=11.01:10.28

### Exemple 94. Chlorhydrate de 1-(6-{[(3S)-3-[(diméthylamino)méthyl]-3,4-dihydroisoquinoléin-2(1H)-yl]carbonyl}-1,3-benzodioxol-5-yl)-N-(4-hydroxyphényl)-5-méthyl-N-(1-méthyl-1H-indol-5-yl)-1H-pyrrole-3-carboxamide

***Microanalyse élémentaire : (*% *théorique : mesuré)***
%C=68.56:68.21; %H=5.61:5.43; %N=9.75:9.56; %Cl=4.94:5.41; %Cl-=4.94:5.17

### Exemple 95. Chlorhydrate de 1-(5-chloro-2-{[(3S)-3-[(diméthylamino)méthyl]-3,4-dihydroisoquinoléin-2(1H)-yl]carbonyl}phényl)-N-(4-hydroxyphényl)-5-méthyl-N-(1-méthyl-1H-indol-5-yl)-1H-pyrrole-3-carboxamide

***Microanalyse élémentaire : (*% *théorique : mesuré)***
%C=67.79:67.07; %H=5.55:5.34; %N=9.88:9.58; %Cl=10.01:10.09; %Cl-=5:5.41

### Exemple 96. N-(4-Hydroxyphényl)-5-méthyl-N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=72.29:72.01; %H=6.21:6.05; %N=10.04:9.95

### Exemple 97. Chlorhydrate de N-(3-fluoro-4-hydroxyphényl)-N-(1-méthyl-1H-indol-5-yl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=65.35:65.8; %H=5.25:5.15; %N=9.73:9.57; %Cl-=8.21:6.82

### Exemple 98. Bischlorhydrate de N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-(pipéridin-4-yl)-1H-indole-3-carboxamide

### Stade A : 4-[(4-Méthylphényl){[1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indol-3-yl]carbonyl)amino]pipéridine-1-carboxylate de tert-butyle

Le composé du titre est synthétisé suivant les Stades A-B du procédé de l'Exemple 31 en utilisant l'acide obtenu à la Préparation 14 et le composé de la Préparation 19".

### Stade B : Bischlorhydrate de N-(4-méthylphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-(pipéridin-4yl)-1H-indole-3-carboxamide

A une solution de produit de départ (615 mg ; 0,74 mmol) dans du chlorure de méthylene (7 mL) est ajouté de l'acide trifluoroacétique (0,5 mL; 6 mmol). Après 17h d'agitation à température ambiante, le milieu réactionnel est coulé sur un mélange constitué d'une solution aqueuse de bicarbonate de sodium et de dichlorométhane. La phase aqueuse est extraite au dichlorométhane, puis séchée au MgSO₄, concentrée à sec et le résidu est purifié par chromatographie sur gel de silice (gradient CH₂Cl₂/MeOH) pour fournir la base libre sous la forme d'une poudre blanche. Ce composé est mis en solution dans du CH₂Cl₂, puis traité à température ambiante avec une solution d'HCl 1M dans l'éther (2 mL ; 2 mmol). La solution est alors mise à sec et le résidu est lyophilisé.
***Microanalyse élémentaire : (*% *théorique : mesuré)***
%C=63.35:63.51; %H=6.16:6.04; %N=10.07:10.14; %Cl=12.75:11.53; %Cl-=12.75:11.92

### Exemple 99. N-(1-Éthyl-1H-indol-5-yl)-N-(4-hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-indole-3-carboxamide

***Microanalyse élémentaire : (*% *théorique : mesuré)***
%C=73.26:72.93; %H=5.89:5.74; %N=10.68:10.69

### Exemple 100. N-(4-Hydroxyphényl)-1-(6-{[(3S)-3-[(4-méthylpipérazin-1-yl)méthyl]-3,4-dihydroisoquinoléin-2(1H)-yl]carbonyl}-1,3-benzodioxol-5-yl)-N-[1-(propan-2-yl)-1H-indol-5-yl]-1H-indole-3-carboxamide

***Microanalyse élémentaire : (*% *théorique : mesuré)***
%C=73.48:74.43; %H=6.04:5.81; %N=10.49:10.71

### Exemple 101. 1-(5-Bromo-2-{[(3S)-3-[(4-méthylpipérazin-1-yl)carbonyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

***Microanalyse élémentaire : (*% *théorique : mesuré)***
%C=65.37:65.39; %H=5.09:4.89; %N=10.89:10.67; %Br=10.35:10.47

### Exemple 102. 1-(5-Bromo-2-{[(3S)-3-[(4-cyclobutylpipérazin-1-yl)méthyl]-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

***Microanalyse élémentaire : (*% *théorique : mesuré)***
%C=67.75:67.81; %H=5.69:5.63; %N=10.53:10.32; %Br=10.02:9.89

### Exemple 103. Chlorhydrate de N-(2,3-dihydro-1H-indol-5-yl)-N-(4-hydroxyphényl)-5-méthyl-1-(6-{[(3S)-3-[(4-méthylpipérazin-1-yl)méthyl]-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=64.74:63.93; %H=5.81:6.29; %N=10.53:8.92; %Cl=8.89:10.68; %Cl-=8.89:10.58

### Exemple 104. 1-(5-Bromo-2-{[(3S)-3-(pyrrolidin-1-ylméthyl)-3,4-dihydroisoquinoléin-2(1H)-yl]carbonyl}phényl)-N-(4-hydroxyphényl)-N-(1H-indol-5-yl)-5-méthyl-1H-pyrrole-3-carboxamide

***Microanalyse élémentaire : (*% *théorique : mesuré)***
%C=67.58:67.45; %H=5.26:5.09; %N=9.61:9.36; %Br=10.97:10.84

### Exemple 105. Chlorhydrate de N-(4-hydroxyphényl)-5-méthyl-N-(1-méthyl-1H-indol-5-yl)-1-(6-{[(3S)-3-(pyrrolidin-1-ylméthyl)-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%H=5.69:5.51; %N=9.41:9.2; %Cl=4.76:5.06; %Cl-=4.76:4.8; %C=69.39:69.24

### Exemple 106. Chlorhydrate de N-(4-hydroxyphényl)-5-méthyl-N-(4-méthylphényl)-1-(6-{[(3S)-3-(morpholin-4-ylméthyl)-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=68.28:67.63; %H=5.73:5.65; %N=7.77:7.53; %Cl-=4.92:4.81

### Exemple 107. Chlorhydrate de N-(4-hydroxyphényl)-5-méthyl-1-(6-{[(3S)-3-(morpholin-4-ylméthyl)-3,4-dihydroisoquinoléin-2(1H)-yl]carbonyl}-2,3-dihydro-1H-indén-5-yl)-N-phényl-1H-pyrrole-3-carboxamide

***Microanalyse élémentaire : (*% *théorique : mesuré)***
%C=71.73:71.34; %H=6.16:6.15; %N=7.97:7.87; %Cl-=5.04:4.73

### Exemple 108. Chlorhydrate de N-(4-hydroxyphényl)-5-méthyl-1-(6-{[(3S)-3-(morpholin-4-ylméthyl)-3,4-dihydroisoquinoléin-2(1H)-yl]carbonyl}-1,3-benzodioxol-5-yl)-N-phényl-1H-pyrrole-3-carboxamide

***Microanalyse élémentaire : (*% *théorique : mesuré)***
%C=67.93:68.17; %H=5.56:5.43; %N=7.92:8.09; %Cl-=5.01:4.77

### Exemple 109. Chlorhydrate de N-(4-hydroxyphényl)-5-méthyl-N-(1-méthyl-1H-indol-5-yl)-1-(6-{[(3S)-3-(morpholin-4-ylméthyl)-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=67.93:66.48; %H=5.57:5.52; %N=9.21:9.24; %Cl-=4.66:5.67

### Masse haute résolution (ESI+) :

Formule brute : C₄₃H₄₁N₅O₆
[M+H]⁺ calculé : 724.3135
[M+H]⁺ mesuré : 724.3073

### Exemple 110. Chlorhydrate de N-(2,3-dihydro-1H-indol-5-yl)-N-(4-hydroxyphényl)-5-méthyl-1-(6-{[(3S)-3-(morpholin-4-ylméthyl)-3,4-dihydroisoquinoléin-2(1H)-yl]carbonyl}-2,3-dihydro-1H-indén-5-yl)-1H-pyrrole-3-carboxamide

***Microanalyse élémentaire : (*% *théorique : mesuré)***
%C=67.69:67.78; %H=6.07:6.04; %N=8.97:9.39; %Cl=9.08:7.96; %Cl-=9.08:8.09

### Exemple 111. Chlorhydrate de N-(4-hydroxyphényl)-5-méthyl-1-(6-{[(3R)-3-méthyl-3,4-dihydroisoquinoléin-2(1H)-yl]carbonyl}-1,3-benzodioxol-5-yl)-N-(1-méthylpipéridin-4-yl)-1H-pyrrole-3-carboxamide

***Microanalyse élémentaire : (*% *théorique : mesuré)***
%C=67.23:66.34; %H=6.11:6.01; %N=8.71:8.72; %Cl-=5.51:5.67

### Exemple 112. Chlorhydrate de N-(4-hydroxyphényl)-5-méthyl-1-(6-{[(3R)-3-méthyl-3,4-dihydroisoquinoléin-2(1H)-yl]carbonyl}-1,3-benzodioxol-5-yl)-N-[3-(4-méthylpipérazin-1-yl)phényl]-1H-pyrrole-3-carboxamide

***Microanalyse élémentaire : (*% *théorique : mesuré)***
%C=68.37:68.91; %H=5.88:5.34; %N=9.72:9.78; %Cl-=4.92:5.24

### Exemple 113. Chlorhydrate de N-(4-hydroxyphényl)-1-(6-{[(3S)-3-(morpholin-4-ylméthyl)-3,4-dihydroisoquinoléin-2(1H)-yl]carbonyl}-1,3-benzodioxol-5-yl)-N-phényl-4,5,6,7-tétrahydro-1H-indole-3-carboxamide

Le composé du titre est synthétisé selon le procédé de l'Exemple 12 en utilisant au Stade A l'acide obtenu à la Préparation 19 et la (3*S*)-3-(4-morpholinylméthyl)-1,2,3,4-tétrahydroisoquinoléine obtenue à la Préparation 2'.
***Microanalyse élémentaire : (*% *théorique : mesuré)***
%C=69.11:68.82; %H=5.8:5.8; %N=7.5:7.45; %Cl-=4.74:4.46

### Masse haute résolution (ESI+) :

Formule brute : C₄₃H₄₂N₄O₆
[M+H]⁺ calculé: 711.3183
[M+H]⁺ mesuré: 711.3140

### Exemple 114. Chlorhydrate de N-(4-hydroxyphényl)-5-méthyl-N-(2-méthyl-2,3-dihydro-1H-isoindol-5-yl)-1-(6-{[(3R)-3-méthyl-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=69.17:68.95; %H=5.51:5.33; %N=8.27:8.05; %Cl-=5.24:5.23

### Exemple 115. Chlorhydrate de N-(4-hydroxyphényl)-5-méthyl-N-(1-méthyl-2,3-dihydro-1H-indol-5-yl)-1-(6-{[(3R)-3-méthyl-3,4-dihydroisoquinoléin-2(1H)-yl]carbonyl}-1,3-benzodioxol-5-yl)-1H-pyrrole-3-carboxamide

***Microanalyse élémentaire : (*% *théorique : mesuré)***
%C=69.17:69.32; %H=5.51:5.27; %N=8.27:8.48; %Cl-=5.24:5.18

### Exemple 116. Chlorhydrate de N-(4-hydroxyphényl)-5-méthyl-1-(6-{[(3R)-3-méthyl-3,4-dihydroisoquinoléin-2(1H)-yl]carbonyl}-1,3-benzodioxol-5-yl)-N-{1-[2-(morpholin-4-yl)éthyl]-1H-indol-5-yl}-1H-pyrrole-3-carboxamide

***Microanalyse élémentaire : (*% *théorique : mesuré)***
%C=68.25:67.87; %H=5.73:5.42; %N=9.04:8.8; %Cl-=4.58:4.81

### Exemple 117. Chlorhydrate de N-(4-hydroxyphényl)-5-méthyl-N-(1-méthyl-1H-indol-5-yl)-1-(6-{[(3S))-3-(morpholin-4-ylméthyl)-3,4-dihydroisoquinoléin-2(1H)-yl]carbonyl}-2,3-dihydro-1H-indén-5-yl)-1H-pyrrole-3-carboxamide

***Microanalyse élémentaire : (*% *théorique : mesuré)***
%C=71.46:70.88; %H=6.13:5.89; %N=9.26:9.12; %Cl-=4.69:4.37

### Exemple 118. N-(4-Hydroxyphényl)-5-méthyl-N-(1-méthyl-1H-indol-5-yl)-1-(5-{[(3S)-3-(morpholin-4-ylméthyl)-3,4-dihydroisoquinoléin-2(1H)-yl]carbonyl}-2,3-dihydro-1-benzofuran-6-yl)-1H-pyrrole-3-carboxamide

Le composé du titre est synthétisé selon le procédé de l'Exemple 12 en utilisant au Stade A l'acide obtenu à la Préparation 20 et la (3S)-3-(4-morpholinylméthyl)-1,2,3,4-tétrahydroisoquinoléine obtenue à la Préparation 2' et l'amine NR₃R₄ adéquate.
***Microanalyse élémentaire : (*% *théorique : mesuré)***
%C=73.21:72.64; %H=6:6.03; %N=9.7:9.57

### Exemple 119. Chlorhydrate de N-(4-hydroxyphényl)-5-méthyl-N-(1-méthyl-1H-indol-5-yl)-1-(5-{[(3S)-3-(morpholin-4-ylméthyl)-3,4-dihydroisoquinoléin-2(1H)-yl]carbonyl}-1-benzofuran-6-yl)-1H-pyrrole-3-carboxamide

Le composé du titre est synthétisé selon le procédé de l'Exemple 12 en utilisant au Stade A l'acide obtenu à la Préparation 21, la (3S)-3-(4-morpholinylméthyl)-1,2,3,4-tétrahydroisoquinoléine obtenue à la Préparation 2' et l'amine NR₃R₄ adéquate.
***Microanalyse élémentaire : (% théorique : mesuré)***
%C=69.88:69.87; %H=5.6:5.29; %N=9.26:9.18; %Cl-=4.69:4.44

### Exemple 120. Chlorhydrate de N-(4-hydroxyphényl)-5-méthyl-1-(6-{[(3S)-3-[2-(morpholin-4-yl)éthyl]-3,4-dihydroisoquinoléin-2(1H)-yl]carbonyl}-1,3-benzodioxol-5-yl)-N-phényl-1H-pyrrole-3-carboxamide

***Microanalyse élémentaire : (% théorique : mesuré)***
%C=68.28:67.77; %H=5.73:5.59; %N=7.77:7.69; %Cl-=4.92:5

### Exemple 121. 1-{2-[(3S)-3-(Aminométhyl)-1,2,3,4-tétrahydroisoquinoléine-2-carbonyl]phényl}-N,N-dibutyl-1H-indazole-3-carboxamide (exemple de référence)

Le composé de la Préparation 6d est traité selon le mode opératoire décrit à l'Exemple 126.
**LC/MS** (C₃₃H₃₉N₅O₂) 538 [M+H]⁺; RT 2.33 (Méthode A)

### Masse haute résolution (ESI+):

Formule empirique: C₃₃H₃₉N₅O₂
[M+H]+ calculé: 538.3177
[M+H]+ mesuré: 538.3169

### Exemple 122. 1-{2-[(3S)-3-(Aminométhyl)-1,2,3,4-tétrahydroisoquinoléine-2-carbonyl]phényl}-N,N-diphényl-1H-indazole-3-carboxamide (exemple de référence)

Le composé de la Préparation 6e est traité selon le mode opératoire décrit à l'Exemple 126, et le produit ainsi obtenu est purifié par chromatographie sur gel de silice selon un gradient allant du dichlorométhane à un mélange 94:6 de dichlorométhane/méthanol.
**LC/MS** (C₃₇H₃₁N₅O₂) 578 [M+H]⁺; RT 1.15 (Méthode B)

### Masse haute révolution (ESI+):

Formule empirique: C₃₇H₃₁N₅O₂
[M+H]+ calculé: 578.2551
[M+H]+ mesuré: 578.2548

### Exemple 123. 1-{2-[(3S)-3-(Aminométhyl)-1,2,3,4-tétrahydroisoquinoléine-2-carbonyl]phényl}-N,N-diphényl-1H-indole-3-carboxamide

Le mode opératoire est le même que dans les Stades A et C de l'Exemple 131, en remplaçant le composé issu de la Préparation 3d par le composé issu de la Préparation 3f.
**LC/MS** (C₃₈H₃₂N₄O₂) 577 [M+H]⁺ ; RT 2,24 (Méthode A)

### Masse haute révolution (ESI+) :

Formule empirique : C₃₈H₃₂N₄O₂
[M+H]⁺ calculé : 577,2598
[M+H]⁺ mesuré : 577,2600

### Exemple 124. 1-{2-[(3S)-3-(Aminométhyl)-1,2,3,4-tétrahydroisoquinoléine-2-carbonyl]phényl}-6-(benzyloxy)-N,N-diphényl-1H-indazole-3-carboxamide (exemple de référence)

Le composé de la Préparation 6c est traité selon le mode opératoire tel que décrit dans l'Exemple 126, puis purifié par chromatographie sur gel de silice selon un gradient allant de dichlorométhane à un mélange méthanol (5 %)/dichlorométhane.
**LC/MS** (C₄₄H₃₇N₅O₃) 684 [M+H]⁺; RT 2.45 (Méthode A)

### Masse haute révolution (ESI+):

Formule empirique: C₄₄H₃₇N₅O₃
[M+H]+ calculé: 684.2969
[M+H]+ mesuré: 684.2982

### Exemple 125. 1-{2-[(3S)-3-(Aminométhyl)-1,2,3,4-tétrahydroisoquinoléine-2-carbonyl]phényl}-6-hydroxy-N,N-diphényl-1H-indazole-3-carboxamide (exemple de référence)

Le composé obtenu dans l'Exemple 124 (30 mg, 0,04 mmol) est dissous dans du dichlorométhane (5 ml), refroidi jusqu'à -78°C sous azote, puis traité avec du trichlorure de bore (1M, 0,02 ml, 0,2 mmol). On laisse le mélange réactionnel se réchauffer jusqu'à la température ambiante et on l'agite pendant 3 heures. Le mélange réactionnel est inactivé avec du méthanol, neutralisé avec de la triéthylamine et concentré. Le résidu est extrait avec de l'acétate d'éthyle et lavé à l'eau, séché sur sulfate de magnésium, filtré et concentré. Le matériau brut est purifié par chromatographie dans du dichlorométhane, un mélange 95:5 dichlorométhane/méthanol, puis un mélange 94:6 dichlorométhane/méthanol.
**LC/MS** (C₃₇H₃₁N₅O₃) 594 [M+H]⁺; RT 2.15 (Méthode A)

### Masse haute révolution (ESI+):

Formule empirique: C₃₇H₃₁N₅O₃
[M+H]+ calculé: 594.2500
[M+H]+ mesuré: 594.2526

### Exemple 126. 1-{2-[(3S)-3-(Aminométhyl)-1,2,3,4-tétrahydroisoquinoléine-2-carbonyl]phényl}-6-méthoxy-N,N-diphényl-1H-indazole-3-carboxamide (exemple de référence)

### Stade A: N-{[(3S)-2-{2-[3-(diphénylcarbamoyl)-6-méthoxy-1H-indazol-1-yl]benzoyl}-1,2,3,4-tétrahydroisoquinoléin-3-yl]méthyl}carbamate de tert-butyle

Le composé de la Préparation 6g (39 mg, 0,08 mmol) dans du dichlorométhane (8 ml) et du *N*,*N*-diméthylformamide (1 goutte) est refroidi jusqu'à 0°C sous azote, puis l'on ajoute du chlorure d'oxalyle 2M (14 µl, 0,17 mmol) et l'on agite le mélange réactionnel pendant 1 heure, avant de le concentrer sous vide. On refroidit jusqu'à 0°C sous azote de la *N*-Boc-aminométhyl THIQ (44,2 mg, 0,17 mmol) et de la triéthylamine (60 µl, 0,4 mmol) dissous dans du dichlorométhane (5 ml), puis l'on ajoute goutte à goutte le chlorure d'acide dans du dichlorométhane et l'on agite le mélange réactionnel à température ambiante toute une nuit. Le mélange réactionnel est réparti selon sa solubilité entre le dichlorométhane et l'eau, et les phases organiques sont séparées, séchées sur sulfate de magnésium, filtrées et concentrées. Le matériau brut est purifié par chromatographie sur gel de silice selon un gradient allant d'iso-hexane à un mélange 1:1 iso-hexane/acétate d'éthyle pour livrer une gomme.
**LC/MS** (C₄₃H₄₁N₅O₅) 608 [M-Boc]⁺; RT 2.78 (Méthode A)

### Stade B: 1-{2-[(3S)-3-(Aminométhyl)-1,2,3,4-tétrahydroisoquiuoléine-2-carbonyl]phényl}-6-méthoxy-N,N-diphényl-1H-indazole-3-carboxamide

On agite à température ambiante, pendant 1 heure, une solution du composé obtenu au Stade A (58 mg, 0,08 mmol) dans du HCl 4M dans du dioxane (3 ml). Le mélange réactionnel est neutralisé avec du NaOH 2M et le produit est extrait dans de l'acétate d'éthyle, séché sur sulfate de magnésium, filtré et concentré. Le produit est purifié par chargement sur une colonne SCX, en balayant avec du méthanol et en éluant avec un mélange 1:1 méthanol/ammoniaque méthanolique 7N. Une purification finale est mise en oeuvre par HPLC préparative.
**LC/MS** (C₃₈H₃₃N₅O₃) 608 [M+H]⁺; RT 2.27 (Méthode A)

### Masse haute résolution (ESI+):

Formule empirique: C₃₈H₃₃N₅O₃
[M+H]+ calculé: 608.2656
[M+H]+ mesuré: 608.2650

### Exemple 127. 1-{2-[(3S)-3-(Aminométhyl)-1,2,3,4-tétrahydroisoquinoléine-2-carbonyl]phényl}-6-hydroxy-N,N-diphényl-1H-indole-3-carboxamide

### Stade A : N-{[(3S)-2-{2-[6-(benzyloxy)-3-(diphénylcarbamoyl)-1H-indol-1-yl]benzoyl}-1,2,3,4-tétrahydroisoquinoléin-3-yl]méthyl}carbamate de tert-butyle

On refroidit jusqu'à 0°C le composé obtenu dans la Préparation 3b (238 mg, 0,44 mmol) dans du dichlorométhane (8 ml) et une goutte de *N*,*N*-diméthylformamide, puis l'on ajoute du chlorure d'oxalyle (0,07 ml, 0,44 mmol). On agite le mélange réactionnel pendant 1 heure, puis on le concentre sous vide. On ajoute ce matériau sous forme d'une suspension / solution dans du dichlorométhane (5 ml) à une solution de *N*-[(3*S*)-1,2,3,4-tétrahydroisoquinoléin-3-ylméthyl]carbamate de *tert*-butyle (232 mg, 0,88 mmol) issu de la Préparation 1' et de triéthylamine (0,31 ml, 2,2 mmol) dans 5 ml de dichlorométhane, on refroidit le tout jusqu'à 0°C et l'on agite le mélange réactionnel pendant environ 16 h sous azote. Le mélange réactionnel est extrait dans de l'acétate d'éthyle, lavé à l'eau, séché sur sulfate de magnésium, filtré et évaporé et purifié par chromatographie sur gel de silice selon un gradient allant d'iso-hexane à un mélange 1:1 iso-hexane/acétate d'éthyle pour livrer une huile.
**LC/MS** (C₅₀H₄₆N₄O₅) 783 [M+H]⁺ ; RT 2,86 (Méthode A)

### Stade B : 1-{2-[(3S)-3-(aminométhyl)-1,2,3,4-tétrahydroisoquinoléine-2-carbonyl]phényl}-6-hydroxy-N,N-diphényl-1H-indole-3-carboxamide

Le produit issu du Stade A (155 mg, 0,20 mmol) est dissous dans du dichlorométhane (8 ml) et refroidi jusqu'à -78°C sous azote, puis l'on ajoute un excès de solution de trichlorure de bore et on laisse le mélange réactionnel se réchauffer jusqu'à la température ambiante pendant 2 h. Le mélange réactionnel est inactivé par addition de méthanol, neutralisé par addition de triéthylamine, évaporé et réparti selon sa solubilité entre l'acétate d'éthyle et l'eau. La phase organique est séchée sur sulfate de magnésium, le solvant est éliminé sous vide et le produit brut est purifié d'abord par chromatographie sur colonne de gel de silice, en éluant avec du dichlorométhane jusqu'à un mélange 95:5 dichlorométhane/méthanol, puis par HPLC préparative pour livrer le produit sous forme d'un solide.
**LC/MS** (C₃₈H₃₂N₄O₃) 593 [M+H]⁺ ; RT 1,06 (Méthode A)

### Masse haute résolution (ESI+) :

Formule empirique : C₃₈H₃₂N₄O₃
[M+H]⁺ calculé : 593,2547
[M+H]⁺ mesuré : 593,2550

### Exemple 128. 2-[(1-{2-[(3S)-3-(Aminométhyl)-1,2,3,4-tétrahydroisoquinoléine-2-carbonyl]phényl}-3-(diphénylcarbamoyl)-1H-indazol-6-yl)oxy]acetate de méthyle (exemple de référence)

### Stade A: 2-[(1-{2-[(3S)-3-({[(tert-Butoxy)carbonyl]amino}méthyl)-1,2,3,4-tétrahydroisoquinoléine-2-carbonyl]phényl}-3-(diphénylcarbamoyl)-1H-indazol-6-yl)oxy]acetate d'éthyle

Le composé du Stade B de l'Exemple 133 (175 mg, 0,25 mmol) est dissous dans de l'acétone (10 ml), puis l'on ajoute du carbonate de potassium (46 mg, 0,33 mmol) et de l'éthyl-2-bromoacétate (36 µl, 0,33 mmol) et l'on chauffe le mélange réactionnel à reflux sous azote toute une nuit. Le mélange réactionnel est concentré, extrait avec de l'acétate d'éthyle et lavé à l'eau, et les phases organiques sont séchées sur sulfate de magnésium, filtrées et concentrées. Le matériau brut est purifié par chromatographie selon un gradient allant d'iso-hexane à un mélange 1:1 iso-hexane/acétate d'éthyle pour livrer une gomme.
**LC/MS** (C₄₆H₄₅N₅O₇) 680.3 [M-Boc]⁺; RT 2,77 (Méthode A)

### Stade B: 2-[(1-{2-[(3S)-3-(Aminométhyl)-1,2,3,4-tétrahydroisoquinoléine-2-carbonyl]phényl}-3-(diphénylcarbamoyl)-1H-indazol-6-yl)oxylacetate de méthyle

On dissout le composé obtenu au Stade A (102 mg, 0,13 mmol) dans du dichlorométhane (4 ml) et l'on ajoute 1 ml d'acide trifluoroacétique, et l'on agite le mélange réactionnel à température ambiante pendant 1 heure. Le mélange réactionnel est neutralisé avec de la triéthylamine, concentré et le résidu est extrait dans de l'acétate d'éthyle et lavé avec une solution saturée de bicarbonate de sodium. Les phases organiques sont séchées sur sulfate de magnésium, filtrées et chargées sur une colonne SCX, qui est balayée avec du méthanol et les composés sont élués avec un mélange 3:1 méthanol/ammoniaque méthanolique 7N. Les produits sont purifiés encore par chromatographie sur gel de silice dans du dichlorométhane, jusque dans un mélange 98:2 dichlorométhane/méthanol, puis 96:4 dichlorométhane/méthanol, puis 9:1 dichlorométhane/méthanol pour conduire au composé déprotégé de type trans-ester (Exemple 128) et au composé complètement déprotégé (Exemple 129).
**LC/MS** (C₄₀H₃₅N₅O₅) 666 [M+H]⁺; RT 2,26 (Méthode A)

### Masse haute résolution (ESI+):

Formule empirique: C₄₀H₃₅N₅O₅
[M+H]+ calculé: 666.2711
[M+H]+ mesuré: 666.2745

### Exemple 129. Acide 2-[(1-{2-[(3S)-3-(aminométhyl)-1,2,3,4-tétrahydroisoquinoléine-2-carbonyl]phényl}-3-(diphénylcarbamoyl)-1H-indazol-6-yl)oxy]acétique (exemple de référence)

Le composé de l'Exemple 129 est isolé en tant que produit secondaire à partir du Stade B de l'Exemple 128.

### Masse haute révolution (ESI+):

Formule empirique: C₃₉H₃₃N₅O₅
[M+H]+ calculé: 652.2554
[M+H]+ mesuré: 652.2584

### Exemple 130. 1-{2-[(3S)-3-(aminométhyl)-1,2,3,4-tétrahydroisoquinoléine-2-carbonyl]phényl}-N-[4-(benzyloxy)phényl]-N-phényl-1H-indole-3-carboxamide

On ajoute de l'acide trifluoroacétique (0,5 ml) à une solution du composé obtenu au Stade A de la Préparation 131 (50 mg, 0,06 mmol) dans du dichlorométhane (3 ml) et l'on agite le mélange réactionnel à température ambiante pendant environ 16 h. Le mélange réactionnel est dilué avec de l'eau, alcalinisé avec une solution aqueuse de NaOH 2M, et le produit est extrait dans du dichlorométhane. Les phases organiques sont séchées sur sulfate de magnésium, filtrées et évaporés, et le matériau brut est purifié par chromatographie sur gel de silice selon un gradient allant de dichlorométhane à un mélange méthanol (5 %)/dichlorométhane pour livrer le produit sous forme d'un solide.
**LC/MS** (C₄₅H₃₈N₄O₃) 683 [M+H]⁺ ; RT 2,44 (Méthode A)

### Masse haute révolution (ESI+) :

Formule empirique : C₄₅H₃₈N₄O₃
[M+H]⁺ calculé : 683,3017
[M+H]⁺ mesuré : 683,2997

### Exemple 131. 1-{2-[(3S)-3-(aminométhyl)-1,2,3,4-tétrahydroisoquinoléine-2-carbonyl]phényl}-N-(4-hydroxyphényl)-N-phényl-1H-indole-3-carboxamide

### Stade A : N-{[(3S)-2-[2-(3-{[4-(benzyloxy)phényl](phényl)carbamoyl}-1H-indol-1-yl)benzoyl]-1,2,3,4-tétrahydroisoquinoléin-3-yl]méthyl}carbamate de tert-butyle

On ajoute de la triéthylamine (0,16 ml, 1,18 mmol), du HOBT (100 mg, 0,65 mmol), de l'EDAC (125 mg, 0,65 mmol) et du *N*-[(3*S*)-1,2,3,4-tétrahydroisoquinoléin-3-ylméthyl]carbamate de *tert*-butyle (186 mg, 0,71 mmol) issu de la Préparation 1' à une solution de l'acide issu de la Préparation 3d (318 mg, 0,59 mmol) dans du tétrahydrofurane (5 ml), et l'on agite le mélange réactionnel à température ambiante pendant environ 16 h. Le mélange réactionnel est dilué avec de l'acétate d'éthyle, lavé à l'eau, et les phases organiques sont séchées sur sulfate de magnésium, filtrées et évaporées. Le matériau brut est purifié par chromatographie sur gel de silice selon un gradient allant d'iso-hexane à un mélange 1:1 acétate d'éthyle/iso-hexane pour livrer le produit sous forme d'un solide.
**LC/MS** (C₅₀H₄₆N₄O₅) 783 [M+H]⁺ ; RT 2,92 (Méthode A)

### Stade B : N-{[(3S)-2-(2-{3-[(4-hydroxyphényl)(phényl)carbamoyl]-1H-indol-1-yl}benzoyl)-1,2,3,4-tétrahydroisoquiuoléin-3-yl]méthyl}carbamate de tert-butyle

On ajoute du palladium à 10 % sur carbone catalytique à une solution du matériau obtenu au Stade A (210 mg, 0,268 mmol) dans de l'éthanol (10 ml), vidée et balayée avec de l'azote, et l'on agite le mélange réactionnel par oscillation sous hydrogène à température ambiante pendant environ 72 h, en ajoutant, pendant ce temps, une quantité supplémentaire de palladium à 10 % sur carbone catalytique. Le mélange réactionnel est filtré au travers de Célite, lavé au méthanol et évaporé pour livrer une huile. Le produit est utilisé sans autre purification pour l'étape suivante.
**LC/MS** (C₄₃H₄₀N₄O₅) 693 [M+H]⁺ ; RT 2,68 (Méthode A)

### Stade C : 1-{2-[(3S)-3-(aminométhyl)-1,2,3,4-tétrahydroisoquinoléine-2-carbonyl]phényl}-N-(4-hydroxyphényl)-N-phényl-1H-indole-3-carboxamide

On ajoute de l'acide trifluoroacétique (0,5 ml) à une solution du composé obtenu au Stade B (200 mg, 0,29 mmol) dans du dichlorométhane (5 ml) et l'on agite le mélange réactionnel à température ambiante pendant 2 heures. Le mélange réactionnel est dilué avec de l'eau, alcalinisé avec du NaOH 2M et le produit est extrait dans du dichlorométhane. Les extraits organiques sont séchés sur sulfate de magnésium, filtrés et évaporés, et le matériau brut est purifié par chromatographie sur gel de silice selon un gradient allant de dichlorométhane à un mélange méthanol (10%)/dichlorométhane pour livrer le produit sous forme d'une poudre.
**LC/MS** (C₃₈H₃₂N₄O₃) 593 [M+H]⁺ ; RT 2,12 (Méthode A)

### Masse haute résolution (ESI+) :

Formule empirique : C₃₈H₃₂N₄O₃
[M+H]⁺ calculé : 593,2547
[M+H]⁺ mesuré : 593,2545

### Exemple 132. 1-{6-[(3S)-3-(Aminométhyl)-1,2,3,4-tétrahydroisoquinoléine-2-carbonyl]-2H-1,3-benzodioxol-5-yl}-N,N-diphényl-1H-indazole-3-carboxamide (exemple de référence)

### Stade A: N-{[(3S)-2-{6-[3-(diphénylcarbamoyl)-1H-indazol-1yl]-2H-1,3-benzodioxole-5-carbonyl}-1,2,3,4-tétrahydroisoquinoléin-3-yl]méthyl}carbamate de tert-butyle

On ajoute de la triéthylamine (0,26 ml, 1,88 mmol), du HOBT (158 mg, 1,03 mmol) et de l'EDAC (197 mg, 1,03 mmol), puis le composé de la Préparation 1' (247 mg, 0,94 mmol), à une solution de l'acide obtenu au Stade A de la Préparation 6a (450 mg, 0,94 mmol) dans du tétrahydrofurane (5 ml). On agite le mélange réactionnel à température ambiante pendant le week-end, après quoi on ajoute une quantité supplémentaire de triéthylamine (0,13 ml, 0,94 mmol), de HOBT (79 mg, 0,52 mmol) et d'EDAC (99 mg, 0,5 mmol) et l'on continue d'agiter le mélange réactionnel toute une nuit. Le mélange réactionnel est dilué avec de l'acétate d'éthyle et lavé à l'eau et avec une solution saturée de bicarbonate de sodium. Les phases organiques sont séchées sur sulfate de magnésium, filtrées et évaporées et le matériau brut est purifié par chromatographie sur gel de silice selon un gradient allant d'iso-hexane à un mélange 1:1 acétate d'éthyle/iso-hexane pour livrer le produit sous forme d'une huile.
**LC/MS** (C₄₃H₃₉N₅O₆) 622 [M-Boc]⁺ observé; RT 2,77 (Méthode A)

### Stade B: 1-{6-[(3S)-3-(Aminométhyl)-1,2,3,4-tétrahydroisoquinoléine-2-carbonyl]-2H-1,3-benzodioxol-5-yl}-N,N-diphényl-1H-indazole-3-carboxamide

On ajoute de l'acide trifluoroacétique (0,5 ml) à une solution du composé obtenu au Stade A (288 mg, 0,4 mmol) dans du dichlorométhane (3 ml). On a agité le mélange réactionnel à température ambiante pendant 1 heure. Le mélange réactionnel est dilué à l'eau et alcalinisé avec du NaOH 2M. Les phases organiques sont extraites dans du dichlorométhane, séchées sur sulfate de magnésium, filtrées et évaporées et le matériau brut est purifié par chromatographie sur gel de silice selon un gradient allant de dichlorométhane à un mélange méthanol (5 %)/dichlorométhane.
**LC/MS** (C₃₈H₃₁N₅O₄) 622 [M-H]⁺ observé; RT 2,27 (Méthode A)

### Masse haute résolution (ESI+):

Formule empirique: C₃₈H₃₁N₅O₄
[M+H]⁺ calculé: 622.2449
[M+H]⁺ mesuré: 622.2457

### Exemple 133. Acide 1-{2-[(3S)-3-(Aminométhyl)-1,2,3,4-tétrahydroisoquinoléine-2-carbonyl]phényl}-3-(diphénylcarbamoyl)-1H-indazole-6-carboxylique (exemple de référence)

### Stade A: N-{[(3S)-2-{2-[3-(Diphénylcarbamoyl)-6-hydroxy-1H-indazol-1-yl]benzoyl}-1,2,3,4-tétrahydroisoquinoléin-3-yl]méthyl}carbamate de tert-butyle

Le composé obtenu dans la Préparation 6h est traité selon le mode opératoire décrit au Stade A de l'Exemple 132.

### Stade B: N-{[(3S)-2-{2-[3-(Diphénylcarbamoyl)-6-(trifluorométhanesulfonyloxy)-1H-indazol-1-yl]benzoyl}-1,2,3,4-tétrahydroisoquinoléin-3-yl]méthyl}carbamate de tert-butyle

Le composé du Stade A (170 mg, 0,25 mmol) est dissous dans du dichlorométhane (10 ml), refroidi jusqu'à 0°C sous azote, puis l'on ajoute de la triéthylamine (0,28 ml, 2 mmol) et de l'anhydride triflique (62 µl, 0,37 mmol), avant d'agiter le tout à température ambiante pendant 1 heure. Le mélange réactionnel est dilué avec du dichlorométhane et lavé à l'eau, séché sur sulfate de magnésium, filtré et concentré. Le matériau brut est purifié par chromatographie sur gel de silice selon un gradient allant d'iso-hexane à un mélange 3:2 iso-hexane/acétate d'éthyle pour livrer une gomme.
**LC/MS** (C₄₃H₃₈N₅O₇F₃S) 726,2 [M-Boc]⁺ observé; RT 2,87 (Méthode A)

### Stade C: Acide 1-{2-[(3S)-3-({[(tert-butoxy)carbonyl]amino}méthyl)-1,2,3,4-tétrahydroisoquinoléine-2-carbonyl]phényl}-3-(diphénylcarbamoyl)-1H-indazole-6-carboxylique

On ajoute du DPPP (9 mg ,0,022 mmol) à une solution du composé obtenu au Stade B (92 mg, 0,11 mmol) dans un mélange *N*,*N*-diméthylformamide/eau (9 ml:3 ml) et l'on dégaze le mélange ainsi obtenu avec de l'azote. On ajoute de l'acétate de palladium (II) (2,5 mg, 0,011 mmol), puis on dégaze. Le mélange réactionnel est ensuite balayé avec du monoxyde de carbone, puis l'on ajoute de la triéthylamine (30 µl, 0,22 mmol) avant de chauffer le tout jusqu'à 80°C, pendant 3 heures, sous une atmosphère de CO. Le mélange réactionnel est concentré, le résidu est extrait dans de l'acétate d'éthyle et lavé au HCl dilué. Les phases organiques sont séchées sur sulfate de magnésium, filtrées et concentrées et chargées sur une colonne PE-AX, balayée avec du méthanol, et le produit est élué avec un mélange 9:1 dichlorométhane/acide formique pour livrer un solide.
**LC/MS** (C₄₃H₃₉N₅O₆) 622 [M-Boc]⁺ observé; RT 2,67 (Méthode A)

### Stade D: Acide 2-[3-(diphénylcarbamoyl)-6-hydroxy-1H-indazol-1-yl]benzoïque

Le composé du Stade C est traité selon le procédé décrit au Stade B de l'Exemple 132.
**LC/MS** (C₃₈H₃₁N₅O₄) 622 [M+H]⁺; RT 2,17 (Méthode A)

### Masse haute résolution (ESI+):

Formule empirique: C₃₈H₃₁N₅O₄
[M+H]+ calculé: 622.2449
[M+H]+ mesuré: 622.2439

### Exemple 134. 1-{2-[(3S)-3-(Aminométhyl)-1,2,3,4-tétrahydroisoquinoléine-2-carbonyl]-4-(benzyloxy)-5-méthoxyphényl}-N,N-diphényl-1H-indazole-3-carboxamide (exemple de référence)

On ajoute de la triéthylamine (0,06 ml, 0,46 mmol), du HOBT (39 mg, 0,253 mmol) et de l'EDAC (49 mg, 0,253 mmol), puis le composé de la Préparation 1' (60 mg, 0,23 mmol), à une solution de l'acide obtenu dans la Préparation 6i (130 mg, 0,23 mmol) dans du tétrahydrofurane (3 ml). On agite le mélange réactionnel à température ambiante pendant 7 heures, puis on le dilue avec de l'acétate d'éthyle et on le lave à l'eau et avec une solution saturée de bicarbonate de sodium. Les phases organiques sont séchées sur sulfate de magnésium, filtrées et évaporées et le matériau brut est purifié par chromatographie sur gel de silice selon un gradient allant d'iso-hexane à un mélange 1:1 acétate d'éthyle/iso-hexane pour livrer le produit sous forme d'un solide.

La déprotection qui s'ensuit est mise en oeuvre selon le Stade B de l'Exemple 132.
**LC/MS** (C₄₅H₃₉N₅O₄) 714 [M+H]⁺; RT 2,47 (Méthode A)

### Masse haute résolution (ESI+):

Formule empirique: C₄₅H₃₉N₅O₄
[M+H]+ calculé: 714.3075
[M+H]+ mesuré: 714.3077

### Exemple 135. 1-{2-[(3S)-3-(Aminométhyl)-1,2,3,4-tétrahydroisoquinoléine-2-carbonyl]-4-hydroxy-5-méthoxyphényl}-N,N-diphényl-1H-indazole-3-carboxamide (exemple de référence)

On ajoute du palladium à 10 % sur carbone catalytique à une solution du composé de l'Exemple 134 (146 mg, 0,18 mmol) dans de l'éthanol (5 ml), dégazée et balayée avec de l'azote. Le mélange réactionnel est agité sous hydrogène, à température ambiante, toute une nuit. Le mélange réactionnel est filtré au travers d'une cartouche de Célite, lavé au méthanol et concentré. Le produit est utilisé à l'étape suivante comme décrit au Stade B de l'Exemple 132, sans aucune autre purification.
**LC/MS** (C₃₈H₃₃N₅O₄) 624 [M+H]⁺; RT 2,23 (Méthode A)

### Masse haute révolution (ESI+):

Formule empirique: C₃₈H₃₃N₅O₄
[M+H]+ calculé: 624.2605
[M+H]+ mesuré: 624.2607

### Exemple 136. 1-{2-[(3S)-3-(Aminométhyl)-1,2,3,4-tétrahydroisoquinoléine-2-carbonyl]phényl}-5-hydroxy-N,N-diphényl-1H-indazole-3-carboxamide (exemple de référence)

Le mode opératoire est tel que décrit au Stade A de l'Exemple 134 en utilisant le composé de la Préparation 6j. Le produit ainsi obtenu est déprotégé selon le procédé décrit à l'Exemple 135.
**LC/MS** (C₃₇H₃₁N₅O₃) 594 [M+H]⁺; RT 2,15 (Méthode A)

### Masse haute révolution (ESI+):

Formule empirique: C₃₇H₃₁N₅O₃
[M+H]+ calculé: 594.2500
[M+H]+ mesuré: 594.2482

### Exemple 137. 1-{2-[(3S)-3-(Aminométhyl)-1,2,3,4-tétrahydroisoquinoléine-2-carbonyl]-4-nitrophényl}-N,N-diphényl-1H-indazole-3-carboxamide (exemple de référence)

La procédure est la même que celle décrite pour l'Exemple 132, en remplaçant le composé de la Préparation 6a au Stade A par le composé de la Préparation 6b.
**LC/MS** (C₃₇H₃₀N₆O₄) 623 [M+H]⁺; RT 2,25 (Méthode A)

### Masse haute révolution (ESI+):

Formule empirique: C₃₇H₃₀N₆O₄
[M+H]⁺ calculé: 623.2401
[M+H]⁺ mesuré: 623.2380

### Exemple 138. 1-{4-Amino-2-[(3S)-3-(aminométhyl)-1,2,3,4-tétrahydroisoquinoléine-2-carbonyl]phényl}-N,N-diphényl-1H-indazole-3-carboxamide (exemple de référence)

### Stade A: N-{[(3S)-2-{5-Amino-2-[3-(diphénylcarbamoyl)-1H-indazol-1-yl]benzoyl}-1,2,3,4-tétrahydroisoquinoléin-3-yl]méthyl}carbamate de tert-butyle

On ajoute du zinc (327 mg, 5 mmol) et du chlorure d'ammonium (267,5 mg, 5 mmol) à une solution du composé obtenu au Stade A de l'Exemple 137 (360 mg, 0,5 mmol) dans du méthanol (10 ml) et l'on chauffe le mélange réactionnel à reflux pendant 10 minutes sous azote. Le mélange réactionnel est refroidi jusqu'à la température ambiante, filtré au travers de Célite, lavé au méthanol chaud et concentré. Le résidu est extrait avec de l'acétate d'éthyle et lavé à la saumure, séché sur sulfate de magnésium, filtré et concentré. Le matériau est utilisé sans autre purification.
**LC/MS** (C₄₂H₄₀N₆O₄) 593.3 [M-Boc]⁺; RT 2,66 (Méthode A)

### Stade B: 1-{4-Amino-2-[(3S)-3-(aminométhyl)-1,2,3,4-tétrahydroisoquinoléine-2-carbonyl]phényl}-N,N-diphényl-1H-indazole-3-carboxamide

Le composé obtenu au Stade A est déprotégé selon le mode opératoire du Stade B de l'Exemple 132, puis purifié par une colonne SCX, en éluant le composé dans un mélange 4:1 méthanol/ammoniaque méthanolique 7N.
**LC/MS** (C₃₇H₃₂N₆O₂) 593 [M+H]⁺; RT 2,15 (Méthode A)

### Masse haute résolution (ESI+):

Formule empirique: C₃₇H₃₂N₆O₂
[M+H]⁺ calculé: 593.2660
[M+H]⁺ mesuré: 593.2676

### Exemple 139. 1-{2-[(3S)-3-(Aminométhyl)-1,2,3,4-tétrahydroisoquinoléine-2-carbonyl]-5-hydroxyphényl}-6-hydroxy-N,N-diphényl-1H-indazole-3-carboxamide (exemple de référence)

Le mode opératoire est tel que dans le procédé de la Préparation 6a, en remplaçant le composé de la Préparation 5a par le composé de la Préparation 5c, et en utilisant du 2-bromo-4-méthoxybenzoate de méthyle. On applique ensuite le mode opératoire décrit au Stade B de la Préparation 6b, puis le procédé décrit au Stade A de l'Exemple 132.

Le composé est dissous dans du dichlorométhane (3 ml), refroidi jusqu'à 0°C, puis l'on ajoute du tribromure de bore (1M dans du dichlorométhane, 0,4 ml, 0,4 mmol) et l'on agite le mélange réactionnel à température ambiante sous azote toute une nuit. Le mélange réactionnel est refroidi jusqu'à 0°C et inactivé avec du méthanol. Le matériau brut est purifié par HPLC préparative.
**LC/MS** (C₃₇H₃₁N₅O₄) 610 [M+H]⁺; RT 2,08 (Méthode A)

### Masse haute résolution (ESI+):

Formule empirique: C₃₇H₃₁N₅O₄
[M+H]+ calculé: 610.2449
[M+H]+ mesuré: 610.2458

### Exemple 140. 1-{2-[(3S)-3-(Aminométhyl)-1,2,3,4-tétrahydroisoquinoléine-2-carbonyl]-4-(méthylamino)phényl}-N,N-diphényl-1H-indazole-3-carboxamide (exemple de référence)

### Stade A: N-{[(3S)-2-{2-[3-(diphénylcarbamoyl)-1H-indazol-1-yl]-5-(méthylamino)benzoyl}-1,2,3,4-tétrahydroisoquinoléin-3-yl]méthyl}carbamate de tert-butyle

On ajoute une solution à 37 % de formaldéhyde (12,4 µl, 0,09 mmol), puis du triacétoxyborohydrure (54 mg, 0,255 mmol) et, après 5 minutes, 2 gouttes d'acide acétique, au composé préparé au Stade A de l'Exemple 138 (59 mg, 0,09 mmol) dans du dichlorométhane (5 ml), puis l'on agite le mélange réactionnel à température ambiante toute une nuit sous azote. Le mélange réactionnel est réparti selon sa solubilité entre le dichlorométhane et la saumure, séché sur sulfate de magnésium, filtré et évaporé. Le mélange brut de produits mono- et di-alkylés est purifié par chromatographie sur gel de silice dans de l'iso-hexane, jusque dans un mélange 1:1 iso-hexane /acétate d'éthyle, puis dans un mélange 3:2 iso-hexane /acétate d'éthyle.
**Composé 1: LC/MS** (C₄₃H₄₂N₆O₄) 607.3 [M-Boc]⁺; RT 2,7 (Méthode A)
**Composé 2: LC/MS** (C₄₄H₄₄N₆O₄) 621.3 [M-Boc]⁺; RT 2,78 (Méthode A)

### Stade B: 1-{2-[(3S)-3-(Aminométhyl)-1,2,3,4-tétrahydroisoquinoléine-2-carbonyl]-4-(méthylamino)phényl}-N,N-diphényl-1H-indazole-3-carboxamide

Le composé obtenu au Stade A est déprotégé selon le mode opératoire du Stade B de l'Exemple 132, puis purifié par une colonne SCX, en éluant le composé dans de l'ammoniaque méthanolique 7N.
**LC/MS** (C₃₈H₃₄N₆O₂) 607 [M+H]⁺; RT 2,23 (Méthode A)

### Masse haute résolution (ESI+):

Formule empirique: C₃₈H₃₄N₆O₂
[M+H]⁺ calculé: 607.2816
[M+H]⁺ mesuré: 607.2788

### Exemple 141. 1-{2-[(3S)-3-(Aminométhyl)-1,2,3,4-tétrahydroisoquinoléine-2-carbonyl]-4-(diméthylamino)phényl}-N,N-diphényl-1H-indazole-3-carboxamide (exemple de référence)

Le composé 2 obtenu au Stade A de l'Exemple 140 est déprotégé selon le mode opératoire du Stade B de l'Exemple 132, puis purifié par une colonne SCX, en éluant le composé dans de l'ammoniaque méthanolique 7N.
**LC/MS** (C₃₉H₃₆N₆O₂) 621 [M+H]⁺; RT 2,31 (Méthode A)

### Masse haute révolution (ESI+):

Formule empirique: C₃₉H₃₆N₆O₂
[M+H]⁺ calculé: 621.2973
[M+H]⁺ mesuré: 621.2955

### Exemple 142. 1-{2-[(3S)-3-(Aminométhyl)-1,2,3,4-tétrahydroisoquinoléine-2-carbonyl]-4-(benzylamino)phényl}-N,N-diphényl-1H-indazole-3-carboxamide (exemple de référence)

Le composé est préparé selon le mode opératoire décrit au Stade A de l'Exemple 140, en utilisant le benzaldéhyde, puis il est déprotégé selon le mode opératoire du Stade B de l'Exemple 132 et purifié par une colonne SCX, en éluant le composé dans un mélange 4 :1 méthanol /ammoniaque méthanolique 7N.
**LC/MS** (C₄₄H₃₈N₆O₂) 683 [M+H]⁺; RT 2,37 (Méthode A)

### Masse haute révolution (ESI+):

Formule empirique: C₄₄H₃₈N₆O₂
[M+H]⁺ calculé: 683.3129
[M+H]⁺ mesuré: 683.3127

### Exemple 143. 1-{2-[(3S)-3-[(diméthylamino)méthyl]-1,2,3,4-tétrahydroisoquinoléine-2-carbonyl]phényl}-N-(4-hydroxyphényl)-N-phényl-1H-indole-3-carboxamide

### Stade A : N-(4-(benzyloxy)phényl)-1-{2-[(3S)-3-[(diméthylamino)méthyl]-1,2,3,4-tétrahydroisoquinoléine-2-carbonyl]phényl}-N-phényl-1H-indole-3-carboxamide

Le mode opératoire est le même que dans le procédé du Stade A de l'Exemple 131, en remplaçant le composé de la Préparation 1' par le composé de la Préparation 12'.
**LC/MS** (C₄₇H₄₂N₄O₃) 711 [M+H]⁺ ; RT 2,44 (Méthode A)

### Stade B : 1-{2-[(3S)-3-[(diméthylamino)méthyl]-1,2,3,4-tétrahydroisoquinoléine-2-carbonyl]phényl}-N-(4-hydroxyphényl)-N-phényl-1H-indole-3-carboxamide

On ajoute du palladium à 10 % sur carbone catalytique à une solution du composé obtenu au Stade A (200 mg, 0,28 mmol) dans de l'éthanol (10 ml), qui est dégazée avec de l'azote. On agite le mélange réactionnel sous hydrogène à température ambiante pendant environ 16 h. Le mélange réactionnel est filtré au travers de Célite et lavé à l'éthanol, le solvant est évaporé et le matériau brut est purifié par chromatographie sur gel de silice selon un gradient allant de dichlorométhane à un mélange méthanol (5 %)/dichlorométhane pour livrer le produit sous forme d'un solide.
**LC/MS** (C₄₀H₃₆N₄O₃) 621 [M+H]⁺ ; RT 2,09 (Méthode A)

### Masse haute résolution (ESI+) :

Formule empirique : C₄₀H₃₆N₄O₃
[M+H]⁺ calculé: 621,2860
[M+H]⁺ mesuré: 621,2850

### Exemple 144. 1-{2-[(3S)-3-(Aminométhyl)-1,2,3,4-tétrahydroisoquinoléine-2-carbonyl]-4-(2-phénoxyacétamido)phényl}-N,N-diphényl-1H-indazole-3-carboxamide (exemple de référence)

### Stade A: N-{[(3S)-2-{2-[3-(Diphénylcarbamoyl)-1H-indazol-1-yl]-5-(2-phénoxyacétamido)benzoyl}-1,2,3,4-tétrahydroisoquinoléin-3-yl]méthyl}carbamate de tert-butyle

On ajoute de la DIPEA (40 µl, 0,225 mmol), puis du chlorure de 2-phénoxyacétyle (25 µl, 0,18 mmol) à une solution du composé obtenu au Stade A de l'Exemple 138 (104 mg, 0,15 mmol) dans du dichlorométhane (5 ml) et l'on agite le mélange réactionnel à température ambiante toute une nuit sous azote. Le mélange réactionnel est dilué avec du dichlorométhane et lavé à l'eau, séché sur sulfate de magnésium, filtré et concentré, puis purifié par chromatographie sur gel de silice selon un gradient allant d'iso-hexane à un mélange 2:1 acétate d'éthyle/iso-hexane pour livrer le produit sous forme d'un solide.
**LC/MS** (C₅ₒH₄₆N₆O₆) 727.3 [M-Boc]⁺; RT 2,79 (Méthode A)

### Stade B: 1-{2-[(3S)-3-(Aminométhyl)-1,2,3,4-tétrahydroisoquinoléine-2-carbonyl]-4-(2-phénoxyacétamido)phényl}-N,N-diphényl-1H-indazole-3-carboxamide

Le composé obtenu au Stade A est déprotégé selon le mode opératoire du Stade B de l'Exemple 132, puis purifié par une colonne SCX, en éluant le composé dans un mélange 4:1 méthanol/ammoniaque 7N.
**LC/MS** (C₄₅H₃₈N₆O₄) 727 [M+H]⁺; RT 2,33

### Masse haute révolution (ESI+):

Formule empirique: C₄₅H₃₈N₆O₄
[M+H]⁺ calculé: 727.3027
[M+H]⁺ mesuré: 727.3004

### Exemple 145. 1-{2-[(3S)-3-(aminométhyl)-1,2,3,4-tétrahydroisoquinoléine-2-carbonyl]-4-hydroxy-5-méthoxyphényl}-N-(4-hydroxyphényl)-N-phényl-1H-indole-3-carboxamide

Le mode opératoire est le même que dans le procédé de l'Exemple 131, en remplaçant le composé de la Préparation 3d par le composé de la Préparation 3a.
**LC/MS** (C₃₉H₃₄N₄O₅) 639 [M+H]⁺ ; RT 2,03 (Méthode A)

### Masse haute révolution (ESI+) :

Formule empirique : C₃₉H₃₄N₄O₅
[M+H]⁺ calculé : 639,2602
[M+H]⁺ mesuré : 639,2619

### Exemple 146. 1-{2-[(3S)-3-(aminométhyl)-1,2,3,4-tétrahydroisoquinoléine-2-carbonyl]-5-méthoxyphényl}-N-(4-hydroxyphényl)-N-phényl-1H-indole-3-carboxamide

Le mode opératoire est le même que dans le procédé de l'Exemple 131, en remplaçant le composé issu de la Préparation 3d par le composé issu de la Préparation 3e.
**LC/MS** (C₃₉H₃₄N₄O₄) 623 [M+H]⁺ ; RT 2,12 (Méthode A)

### Masse haute révolution (ESI+) :

Formule empirique : C₃₉H₃₄N₄O₄
[M+H]⁺ calculé : 623,2653
[M+H]⁺ mesuré : 623,2632

### Exemple 147. 1-{2-[(3S)-3-(aminométhyl)-1,2,3,4-tétrahydroisoquinoléine-2-carbonyl]-5-méthoxyphényl}-N,N-diphényl-1H-indole-3-carboxamide

Le mode opératoire est le même que dans les Stades A et C de l'Exemple 131, en remplaçant le composé issu de la Préparation 3d par le composé issu de la Préparation 3g, et en purifiant le produit final par HPLC préparative.
**LC/MS** (C₃₉H₃₄N₄O₃) 607 [M+H]⁺ ; RT 1,14 (Méthode A)

### Masse haute révolution (ESI+) :

Formule empirique : C₃₉H₃₄N₄O₃
[M+H]⁺ calculé : 607,2704
[M+H]⁺ mesuré : 607,2687

### Exemple 148. 1-{2-[(3S)-3-(Aminométhyl)-1,2,3,4-tétrahydroisoquinoléine-2-carbonyl]phényl}-N-(4-hydroxyphényl)-N-phényl-1H-indazole-3-carboxamide (exemple de référence)

### Stade A: 1-{2-[(3S)-3-(Aminométhyl)-1,2,3,4-tétrahydroisoquinoléine-2-carbonyl]phényl}-N-[4-(benzyloxy)phényl]-N-phényl-1H-indazole-3-carboxamide

Le composé obtenu à la Préparation 6f est traité selon le mode opératoire du Stade A de l'Exemple 132, puis déprotégé par la suite selon le mode opératoire du Stade B de l'Exemple 132.

### Stade B: 1-{2-[(3S)-3-(Aminométhyl)-1,2,3,4-tétrahydroisoquinoléine-2-carbonyl]phényl}-N-(4-hydroxyphényl)-N-phényl-1H-indazole-3-carboxamide

On ajoute du palladium à 10 % sur carbone catalytique à une solution du composé (43 mg, 0,06 mmol) dans du méthanol (4 ml) et l'on balaye le mélange réactionnel avec de l'azote. Le mélange réactionnel est ensuite vidé, rempli d'hydrogène et agité sous une atmosphère d'hydrogène pendant 2,5 heures. On ajoute une quantité supplémentaire de palladium à 10 % sur carbone et l'on continue d'agiter le mélange réactionnel pendant 3 heures de plus. Le mélange réactionnel est filtré au travers d'une cartouche de Célite, lavé au méthanol, puis concentré sous vide pour livrer un solide, qui est purifié par chromatographie éclair sur gel de silice, selon un gradient allant de dichlorométhane jusqu'à un mélange méthanol (10 %)/dichlorométhane, puis purifié encore par SCX. La colonne est pré-lavée avec du méthanol, avant le chargement du composé dans du dichlorométhane, puis un lavage successif avec du dichlorométhane, du méthanol et une élution avec un mélange 1:4 ammoniaque méthanolique/méthanol.
**LC/MS** (C₃₇H₃₁N₅O₃) 594 [M+H]⁺; RT 2,11 (Méthode A)

### Masse haute résolution (ESI+):

Formule empirique: C₃₇H₃₁N₅O₃
[M+H]+ calculé: 594.2500
[M+H]+ mesuré: 594.2475

### Exemple 149. 1-{2-[(3S)-3-(Aminométhyl)-1,2,3,4-tétrahydroisoquinoléine-2-carbonyl]-4-[2-(phénylsulfanyl)acétamido]phényl}-N,N-diphényl-1H-indazole-3-carboxamide (exemple de référence)

Le mode opératoire est le même que celui décrit à l'Exemple 144, en utilisant le chlorure de 2-(phénylsulphanyl)acétyl au Stade A , puis en déprotégeant le composé obtenu selon la procédure décrite au Stade B de l'Exemple 132.
**LC/MS** (C₄₅H₃₈N₆O₃S) 743 [M+H]⁺; RT 2,39 (Méthode A)

### Masse haute résolution (ESI+):

Formule empirique: C₄₅H₃₈N₆O₃S
[M+H]⁺ calculé: 743.2799
[M+H]⁺ mesuré: 743.2772

### Exemple 150. 1-{2-[(3S)-3-(Aminométhyl)-1,2,3,4-tétrahydroisoquinoléine-2-carbonyl]-5-méthoxyphényl}-N,N-diphényl-1H-indazole-3-carboxamide (exemple de référence)

Le mode opératoire est tel que dans le procédé de la Préparation 6a, en remplaçant l'acide 6-bromo-2*H*-1,3-benzodioxole-5-carboxylique par l'acide 2-iodo-4-méthoxybenzoïque. On applique ensuite le procédé décrit au Stade A de l'Exemple 132.

Le composé ainsi obtenu (0,18 g, 0,25 mmol) est ensuite dissous dans du dichlorométhane anhydre (5 ml) sous azote et refroidi jusqu'à 0°C, traité goutte à goutte avec du trichlorure de bore (0,09 ml, 1 mmol) et on le laisse se réchauffer jusqu'à la température ambiante toute une nuit. Le mélange réactionnel est alors refroidi jusqu'à 0°C et traité avec du tribromure de bore (0,09 ml, 1 mmol), puis 0,18 ml supplémentaires après 4 heures. Le mélange réactionnel est inactivé avec du méthanol, concentré sous pression réduite, repris dans de l'acétate d'éthyle et lavé à l'eau. Les phases organiques sont séchées sur sulfate de magnésium, filtrées et concentrées pour livrer une huile, qui est purifiée par chromatographie éclair selon un gradient allant de dichlorométhane à un mélange méthanol (10 %)/ dichlorométhane.
**LC/MS** (C₃₈H₃₃N₅O₃) 608.2 [M+H]⁺; RT 2,25 (Méthode A)

### Masse haute résolution (ESI+):

Formule empirique: C₃₈H₃₃N₅O₃
[M+H]+ calculé: 608.2656
[M+H]+ mesuré: 608.2639

### Exemple 151. 1-{2-[(3S)-3-[(Diméthylamino)méthyl]-1,2,3,4-tétrahydroisoquinoléine-2-carbonyl]phényl}-N,N-diphényl-1H-indole-3-carboxamide

### Exemple 152. 1-{4-Amino-2-[(3S)-3-(aminométhyl)-1,2,3,4-tétrahydroisoquinoléine-2-carbonyl]-5-méthoxyphényl}-N,N-diphényl-1H-indazole-3-carboxamide (exemple de référence)

### Stade A: N-{[(3S)-2-{2-[3-(diphénylcarbamoyl)-1H-indazol-1-yl]-5-hydroxy-4-méthoxybenzoyl}-1,2,3,4-tétrahydroisoquinoléin-3-yl]méthyl}carbamate de tert-butyle

Le mode opératoire est tel que dans le procédé de la Préparation 6a, en remplaçant l'acide 6-bromo-2*H*-1,3-benzodioxole-5-carboxylique par l'acide 5-(benzyloxy)-2-bromo-4-méthoxybenzoïque. On applique ensuite le procédé décrit au Stade A de l'Exemple 132. Le composé ainsi obtenu (367 mg, 0,45 mmol) est dissous dans de l'acétate d'éthyle (10 ml) et dégazé, puis l'on ajoute du palladium à 10 % sur carbone catalytique. Le mélange réactionnel est dégazé, puis agité sous une atmosphère d'hydrogène, à température ambiante, toute une nuit. Le mélange réactionnel est filtré au travers de Célite, lavé à l'acétate d'éthyle, puis concentré pour livrer un solide, qui est utilisé à l'étape suivante sans autre purification.
**LC/MS** (C₄₄H₄₂N₄O₆) 723.3 [M+H]⁺; RT 2,68 (Méthode A)

### Stade B: N-{[(3S)-2-{2-[3-(Diphénylcarbamoyl)-1H-indazol-1-yl]-4-méthoxy-5-(trifluorométhanesulfonyloxy)benzoyl}-1,2,3,4-tétrahydroisoquinoléin-3-yl]méthyl}carbamate de tert-butyle

On ajoute de la triéthylamine (0,14 ml, 1 mmol) à une solution du composé obtenu au Stade A (236 mg, 0,33 mmol) dans du dichlorométhane (10 ml). On refroidit le mélange réactionnel jusqu'à 0°C sous azote, puis l'on ajoute de l'anhydride triflique (82,3 µl, 0,49 mmol), avant d'agiter le tout à température ambiante sous azote pendant 5 heures. Le mélange réactionnel est dilué avec du dichlorométhane, lavé à l'eau, séché sur sulfate de magnésium, filtré et concentré. Le matériau brut est purifié par chromatographie sur gel de silice, selon un gradient allant d'iso-hexane à un mélange 6:4 iso-hexane/acétate d'éthyle pour livrer le produit sous forme d'une gomme.
**LC/MS** (C₄₄H₄₀N₅O₈F₃S) pas de m/z observé; RT 2,85 (Méthode A)

### Stade C: N-{[(3S)-2-{2-[3-(diphénylcarbamoyl)-1H-indazol-1-yl]-5-[(diphénylméthylidene)amino]-4-méthoxybenzoyl}-1,2,3,4-tétrahydroisoquinoléin-3-yl]méthyl}carbamate de tert-butyle

On met en suspension de la diphénylméthanimine (22,4 µl, 0,13 mmol), de l'acétate de palladium (II) (4,04 mg, 0,018 mmol), du BINAP (11,2 mg, 0,018 mmol) et du carbonate de césium (58,6 mg, 0,18 mmol) dans du toluène (1 ml), puis l'on ajoute une solution du composé obtenu au Stade B (76 mg, 0,09 mmol) dans du toluène (4 ml). Le mélange réactionnel est dégazé avec de l'azote pendant 5 minutes, puis soumis à des micro-ondes pendant 1,5 heure à 150°C. On ajoute davantage de diphénylméthanimine (22,4 µl, 0,13 mmol) et l'on soumet le mélange réactionnel à des micro-ondes pendant 6 heures. Le mélange réactionnel est concentré, extrait avec de l'acétate d'éthyle et lavé à la saumure, séché sur sulfate de magnésium, filtré et concentré. Le matériau brut est purifié par chromatographie dans de l'iso-hexane, jusque dans un mélange 2:1 iso-hexane/acétate d'éthyle, 2:1 iso-hexane/acétate d'éthyle, puis 1:1 iso-hexane/acétate d'éthyle, pour livrer le produit sous forme d'un solide.
**LC/MS** (C₅₆H₅₀N₆O₅) 787.3 [M-Boc]⁺; RT 2,91 (Méthode A)

### Stade D: 1-{4-Amino-2-[(3S)-3-(aminométhyl)-1,2,3,4-tétrahydroisoquinoléine-2-carbonyl]-5-méthoxyphényl}-N,N-diphényl-1H-indazole-3-carboxamide

On ajoute de l'acide trifluoroacétique (0,5 ml) au composé obtenu au Stade C (4 mg, 0,005 mmol) dans du dichlorométhane (3 ml) et l'on agite le mélange réactionnel à température ambiante. Après 30 minutes, on ajoute un supplément d'acide trifluoroacétique (0,5 ml), puis quelques gouttes de HCl 2N après 30 nouvelles minutes. Après 15 minutes, le mélange réactionnel est alcalinisé avec du NaOH 2N et la phase organique est séparée, séchée sur sulfate de magnésium, filtrée et évaporée. Le matériau brut est purifié par chromatographie sur gel de silice dans du dichlorométhane, jusque dans un mélange 9:1 dichlorométhane /méthanol.
**LC/MS** (C₃₈H₃₄N₆O₃) 623 [M+H]⁺; RT 2,17 (Méthode A)

### Masse haute résolution (ESI+):

Formule empirique: C₃₈H₃₄N₆O₃
[M+H]+ calculé: 623.2765
[M+H]+ mesuré: 623.2768

### Exemple 153. 1-{6-[(3S)-3-(Aminométhyl)-1,2,3,4-tétrahydroisoquinoléine-2-carbonyl]-2H-1,3-benzodioxol-5-yl}-N-(4-hydroxyphényl)-N-phényl-1H-indole-3-carboxamide

Le mode opératoire est le même que dans le procédé de l'Exemple 131, en remplaçant le composé issu de la Préparation 3d par le composé issu de la Préparation 3h.
**LC/MS** (C₃₉H₃₂N₄O₅) 637 [M+H]⁺ ; RT 2,11 (Méthode A)

### Exemple 154. 1-{2-[(3S)-3-(Aminométhyl)-1,2,3,4-tétrahydroisoquinoléine-2-carbonyl]-5-méthoxy-4-(3-phénoxyazétidin-1-yl)phényl}-N,N-diphényl-1H-indole-3-carboxamide

### Stade A : N-{[(3S)-2-[5-(Benzyloxy)-2-[3-(diphénylcarbamoyl)-1H-indol-1-yl]-4-méthoxybenzoyl]-1,2,3,4-tétrahydroisoquinoléin-3-yl]méthyl}carbamate de tert-butyle

Le mode opératoire est le même que dans le procédé du Stade A de l'Exemple 131, en remplaçant le composé de la Préparation 3d par le composé de la Préparation 3t.

### Stade B : N-{[(3S)-2-{2-[3-(Diphénylcarbamoyl)-1H-indol-1-yl]-4-méthoxy-5-(trifluorométhanesulfonyloxy)benzoyl}-1,2,3,4-tétrahydroisoquinoléin-3-yl]méthyl}carbamate de tert-butyle

On ajoute de l'anhydride trifluoroacétique (85,5 µl, 0,51 mmol) à une solution du composé obtenu au Stade A (245 mg, 0,34 mmol) et de triéthylamine (0,14 ml, 1,02 mmol) dans du dichlorométhane (15 ml), refroidie jusqu'à 0°C, et l'on agite le mélange réactionnel à température ambiante sous azote. Après 1 heure, le mélange réactionnel est réparti selon sa solubilité entre le dichlorométhane et l'eau, séché sur sulfate de magnésium, filtré et concentré, puis purifié par chromatographie sur gel de silice selon un gradient allant d'iso-hexane à un mélange 6/4 iso-hexane/acétate d'éthyle pour livrer le produit sous forme d'une huile.
**LC/MS** (C₄₅H₄₁N₄OF₃S) pas de m/z observé ; RT 2,87 (Méthode A)

### Stade C : N-{[(3S)-2-{2-[3-(Diphénylcarbamoyl)-1H-indol-1-yl]-4-méthoxy-5-(3-phénoxyazétidin-1-yl)benzoyl}-1,2,3,4-tétrahydroisoquinoléin-3-yl]méthyl}carbamate de tert-butyle

On a ajouté une solution du composé obtenu au Stade B (58 mg, 0,07 mmol dans du tétrahydrofurane (2 ml), dégazée avec de l'azote, à un mélange de *tert*-butanolate de sodium (19,8 mg, 0,2 mmol), de chlorhydrate de 3-phénoxyazétidine (18,9 mg, 0,1 mmol) et de bis(tri-*tert*-butylphosphine)palladium(0) (3,47 mg, 0,01 mmol) et l'on a chauffé le mélange réactionnel par irradiation micro-onde pendant 30 minutes à 120°C. Le mélange réactionnel est réparti selon sa solubilité entre l'acétate d'éthyle et l'eau, séché sur sulfate de magnésium, filtré et concentré, puis purifié par chromatographie sur gel de silice selon un gradient allant d'iso-hexane à un mélange 1:1 iso-hexane/acétate d'éthyle.

### Stade D : 1-{2-[(3S)-3-(Aminométhyl)-1,2,3,4-tétrahydroisoquinoléine-2-carbonyl]-5-méthoxy-4-(3-phénoxyazétidin-1-yl)phényl}-N,N-diphényl-1H-indole-3-carboxamide

Le mode opératoire est le même que dans le procédé du Stade C de l'Exemple 131.
**LC/MS** (C₄₈H₄₃N₅O₄) 754 [M+H]⁺ ; RT 2,5 (Méthode A)

### Masse haute résolution (ESI+) :

Formule empirique : C₄₈H₄₃N₅O₄
[M+H]⁺ calculé : 754,3388
[M+H]⁺ mesuré : 754,3376

### Exemple 155. N-(4-Hydroxyphényl)-1-{2-[(3S)-3-[(4-méthylpipérazin-1-yl)méthyl]-1,2,3,4-tétrahydroisoquinoléine-2-carbonyl]phényl}-N-phényl-1H-indole-3-carboxamide

### Stade A : N-[4-(Benzyloxy)phényl]-1-{2-[(3S)-3-[(4-méthylpipérazin-1-yl)méthyl]-1,2,3,4-tétrahydroisoquinoléine-2-carbonyl]phényl}-N-phényl-1H-indole-3-carboxamide

Le mode opératoire est le même que dans le procédé du Stade A de l'Exemple 131, en remplaçant le composé de la Préparation 1' par le composé de la Préparation 3'.

### Stade B : N-(4-Hydroxyphényl)-1-{2-[(3S)-3-[(4-méthylpipérazin-1-yl)méthyl]-1,2,3,4-tétrahydroisoquinoléine-2-carbonyl]phényl}-N-phényl-1H-indole-3-carboxamide

On ajoute du trichlorure de bore (1M dans du dichlorométhane, 0,03 ml) à une solution du composé préparé au Stade A (122 mg, 0,16 mmol) dans du dichlorométhane (3 ml), refroidie jusqu'à 0°C sous azote. On laisse le mélange réactionnel se réchauffer jusqu'à la température ambiante et on l'agite pendant 2 jours, durée au cours de laquelle on procède à 2 additions successives de quantités aliquotes de 0,15 ml de trichlorure de bore. Le mélange réactionnel est inactivé avec du méthanol, concentré, et le résidu est purifié par chromatographie sur colonne dans du dichlorométhane, jusqu'à un mélange méthanol (5 %)/dichlorométhane.
**LC/MS** (C₄₃H₄₁N₅O₃) 676 [M+H]⁺ ; RT 2,15 (Méthode A)

### Masse haute résolution (ESI+) :

Formule empirique : C₄₃H₄₁N₅O₃
[M+H]⁺ calculé : 676,3282
[M+H]⁺ mesuré : 676,3259

### Exemple 156. 1-{2-[(3S)-3-(Aminométhyl)-1,2,3,4-tétrahydroisoquinoléine-2-carbonyl]-5-chlorophényl}-N-(4-hydroxyphényl)-N-phényl-1H-indole-3-carboxamide

### Stade A : N-{[(3S)-2-[2-(3-{[4-(Benzyloxy)phényl](phényl)carbamoyl}-1H-indol-1yl)-4-chlorobenzoyl]-1,2,3,4-tétrahydroisoquinoléin-3-yl]méthyl}carbamate de tert-butyle

Le composé issu de la Préparation 3i (0,48 g, 0,84 mmol) est dissous dans 10 ml de dichlorométhane anhydre. On ajoute du chlorure d'oxalyle (0,63 ml, 1,26 mmol), puis quelques gouttes de *N*,*N*-diméthylformamide et on laisse le mélange réactionnel sous agitation à température ambiante pendant environ 16 h. Le mélange réactionnel est concentré jusqu'à siccité, puis dissous à nouveau dans 10 ml de dichlorométhane anhydre, refroidi jusqu'à 0°C, et l'on ajoute de la triéthylamine (0,21 ml, 1,53 mmol), suivie du composé issu de la Préparation 1' (0,24 g, 0,92 mmol) et on laisse le mélange réactionnel sous agitation pendant 1,5 heures. Le mélange réactionnel est réparti selon sa solubilité entre le dichlorométhane et l'eau et la phase organique est lavée à la saumure, séchée sur sulfate de magnésium et concentrée. Le matériau brut est purifié par chromatographie sur colonne de silice, en éluant avec de l'iso-hexane jusqu'à de l'acétate d'éthyle pour livrer le produit sous forme d'une huile.
**LC/MS** (C₅₀H₄₅N₄O₅Cl) 817 [M+H]⁺ ; RT 2,96 (Méthode A)

### Stade B : 1-{2-[(3S)-3-(Aminométhyl)-1,2,3,4-tétrahydroisoquinoléine-2-carbonyl]-5-chlorophényl}-N-(4-hydroxyphényl)-N-phényl-1H-indole-3-carboxamide

On ajoute une solution de trichlorure de bore (0,21 ml, 2,4 mmol), goutte à goutte, à une solution du composé obtenu au Stade A (0,20g, 0,24 mmol) dans du dichlorométhane anhydre (5 ml), sous azote et refroidie jusqu'à 0°C. On laisse ensuite le mélange réactionnel se réchauffer jusqu'à la température et on le laisse sous agitation pendant environ 16 h. La solution est refroidie jusqu'à 0°C et l'on ajoute une solution de trichlorure de bore supplémentaire (0,21 ml, 2,4 mmol). Après 6 heures, on refroidit le mélange réactionnel, puis l'on ajoute du tribromure de bore (0,23 ml, 2,4 mmol). On laisse de nouveau le mélange réactionnel se réchauffer jusqu'à la température ambiante et on l'agite pendant environ 16 h. On ajoute deux autres portions de 0,23 ml de solution de tribromure de bore, en refroidissant pendant 7 autres heures. Le mélange réactionnel est inactivé avec du méthanol, concentré, puis réparti selon sa solubilité entre l'acétate d'éthyle et l'eau. Les phases organiques sont séchées sur sulfate de magnésium, filtrées et concentrées. Le produit brut est purifié par chromatographie sur silice en utilisant un gradient allant de dichlorométhane jusqu'à un mélange méthanol (20 %)/dichlorométhane, puis l'on procède à une autre purification par HPLC préparative.
**LC/MS** (C₃₈H₃₁N₄O₃Cl) 627 [M+H]⁺ ; RT 1,07 (Méthode B)

### Masse haute résolution (ESI+) :

Formule empirique : C₃₈H₃₁N₄O₃Cl
[M+H]⁺ calculé : 627,2157
[M+H]⁺ mesuré : 627,2178

### Exemple 157. 1-{2-[(3S)-3-(Aminométhyl)-1,2,3,4-tétrahydroisoquinoléine-2-carbonyl]-4-[2-(phénylsulfanyl)acétamido]phényl}-N-(4-hydroxyphényl)-N-phényl-1H-indole-3-carboxamide

### Stade A: Acide 2-(3-{[4-(Benzyloxy)phényl](phényl)carbamoyl}-1H-indol-1-yl)-5-nitrobenzoïque

A une solution du composé obtenu dans la Préparation 2a (175 mg, 0,42 mmol) dans le *N*,*N*-diméthylformamide (10 mL), on additionne du carbonate de potassium (87 mg, 0,63 mmol) et du 2-fluoro-5-nitrobenzoate de méthyle (93 mg, 0,5 mmol) et la réaction est agitée à 110 °C sous azote. On ajoute à nouveau du carbonate de potassium (29 mg, 0,21 mmol) et du 2-fluoro-5-nitrobenzoate de méthyle (46,5 mg, 0,25 mmol) et la température est augmentée à 130 °C. Le milieu réactionnel est agité toute la nuit. Il est ensuite concentré et extrait avec de l'acétate d'éthyle, lavé avec du HCl 1M et les phases organiques sont séchées sur sulfate de magnésium, filtrées et concentrées. Le résidu brut est purifié par chromatographie selon un gradient allant de dichlorométhane jusqu'à un mélange méthanol (10%)/dichlorométhane pour conduire à une huile.
**LC/MS** (C₃₅H₂₅N₃O₆) 584,2 [M+H]⁺; RT 2,69 (Méthode A)

### Stade B: N-{[(3S)-2-[5-Amino-2-(3-{[4-(benzyloxy)phényl](phényl)carbamoyl}-1H-indol-1-yl)benzoyl]-1,2,3,4-tétrahydroisoquinoléin-3-yl]méthyl}carbamate de tert-butyle

Le composé obtenu au stade A est traité selon le mode opératoire du Stade A de l'Exemple 131. Le composé ainsi obtenu (300 mg, 0,36 mmol) est dissous dans du méthanol (10 mL). On y ajoute du zinc (235 mg, 3,6 mmol) et du chlorure d'ammonium (193 mg, 3,6 mmol). Le milieu réactionnel est porté au reflux pendant 5 minutes sous azote, et on le laisse refroidir jusqu'à la température ambiante. Il est filtré sur célite, lavé avec de l'éthanol chaud, puis concentré. Le résidu est repris dans de l'acétate d'éthyle et lavé avec une solution saturée de bicarbonate de sodium, séché sur sulfate de magnésium, filtré et concentré pour conduire à un solide qui est utilisé sans autre purification dans le stade suivant.
**LC/MS** (C₅₀H₄₇N₅O₅) 798,4 [M+H]⁺; RT 2,83

### Stade C: N-{[(3S)-2-[2-(3-{[4-(Benzyloxy)phényl](phényl)carbamoyl}-1H-indol-1-yl)-5-[2-(phénylsulfanyl)acétamido]benzoyl]-1,2,3,4-tétrahydroisoquinoléin-3-yl]méthyl}carbamate de tert-butyle

A une solution du composé obtenu au Stade B (304 mg, 0,38 mmol) dans du dichlorométhane (10 mL) et de la DIPEA (0,1 mL, 0,6 mmol) est ajouté le chlorure de 2-(phénylsulfanyl)acétyle (68 µL, 0,46 mmol), et la réaction est agitée à température ambiante pendant une nuit. Le milieu réactionnel est réparti selon sa solubilité entre le dichlorométhane et la saumure, séché sur sulfate de magnésium, filtré et concentré. Le matériau brut est purifié par chromatographie dans l'iso-hexane, jusqu'à un mélange 6:4 d'acétate d'éthyle/iso-hexane, suivi d'un mélange 2:1 d'acétate d'éthyle/iso-hexane pour conduire au produit du titre sous la forme d'un solide.
**LC/MS** (C₅₈H₅₃N₅O₆S) pas de m/z observé; RT 2,93 (Méthode A)

### Stade D: 1-{2-[(3S)-3-(Aminométhyl)-1,2,3,4-tétrahydroisoquinoléine-2-carbonyl]-4-[2-(phénylsulfanyl)acétamido]phényl}-N-(4-hydroxyphényl)-N-phényl-1H-indole-3-carboxamide

Le composé obtenu au Stade C est traité selon le mode opératoire du Stade C de l'Exemple 131. Le composé ainsi obtenu (100 mg, 0,12 mmol) est dissous dans du dichlorométhane (10 mL), refroidi à -78 °C. On y additionne du trichlorure de bore (1M, 0,6 mL, 0,6 mmol). On laisse le milieu réactionnel se réchauffer jusqu'à la température ambiante et on l'agite pendant 4 heures. Il est inactivé avec du méthanol, neutralisé avec de la triéthylamine et lavé avec de la saumure. Les phases organiques sont séchées sur sulfate de magnésium, filtrées et concentrées. Le résidu obtenu est purifié par chromatographie sur gel de silice selon un gradient allant de dichlorométhane jusqu'à un mélange méthanol (8%)/dichlorométhane. Le produit obtenu est dissous dans de l'acétate d'éthyle et lavé avec de l'HCl dilué, séché sur sulfate de magnésium, filtré et concentré.
**LC/MS** (C₄₆H₃₉N₅O₄S) 758,2 [M+H]⁺; RT 1,83

### Exemple 158. 1-{6-[(3S)-3-(Aminométhyl)-1,2,3,4-tétrahydroisoquinoléine-2-carbonyl]-2-(2-méthylpropyl)-2H-1,3-benzodioxol-5-yl}-N-(4-hydroxyphényl)-N-phényl-1H-indole-3-carboxamide

Le mode opératoire est le même que dans le procédé de l'Exemple 131, en remplaçant le composé issu de la Préparation 3d par le composé issu de la Préparation 3j. Le produit est isolé sous forme d'un mélange de diastéréoisomères.
**LC/MS** (C₄₃H₄₀N₄O₅) 693 [M+H]⁺ ; RT 2,39 (Méthode A)

### Masse haute résolution (ESI+) :

Formule empirique : C₄₃H₄₀N₄O₅
[M+H]⁺ calculé: 693,3071
[M+H]⁺ mesuré : 693,3096

### Exemple 159. 1-{5-Chloro-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

### Stade A : N-[4-(Benzyloxy)phényl]-1-{5-chloro-2-[(3S)-3-[(4-méthylpipérazin-1-yl)méthyl]-1,2,3,4-tétrahydroisoquinoléine-2-carbonyl]phényl}-N-phényl-1H-indole-3-carboxamide

Le mode opératoire est le même que dans le procédé du Stade A de l'Exemple 131, en remplaçant le composé issu de la Préparation 3d par le composé issu de la Préparation 3i, et en remplaçant le composé issu de la Préparation 1' par le composé issu de la Préparation 3'.

### Stade B : 1-{5-Chloro-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

Le composé obtenu au Stade A (0,39 g, 0,48 mmol) est dissous dans du dichlorométhane anhydre (10 ml) et refroidi jusqu'à 0°C. On ajoute goutte à goutte une solution de tribromure de bore (0,45 ml, 4,8 mmol) et on laisse le mélange réactionnel se réchauffer jusqu'à la température ambiante, avant de l'agiter pendant environ 16 h. On ajoute une solution supplémentaire de tribromure de bore de 0,23 ml à 0°C et on laisse le mélange réactionnel se réchauffer jusqu'à la température ambiante. Après 4 heures, le mélange réactionnel est inactivé avec du méthanol, concentré, puis réparti selon sa solubilité entre l'acétate d'éthyle, le méthanol minimal et la saumure. La phase aqueuse est extraite avec du dichlorométhane, et les extraits organiques combinés sont séchés sur sulfate de magnésium et concentrés sous vide. Le matériau brut est d'abord purifié par chromatographie éclair sur colonne de gel de silice en utilisant un gradient allant de dichlorométhane jusqu'à un mélange méthanol (10 %)/ dichlorométhane, puis encore purifié par HPLC préparative (pH4).
**LC/MS** (C₄₃H₄₀N₅O₃Cl) 710 [M+H]⁺ ; RT 1,14 (Méthode B)

### Masse haute résolution (ESI+) :

Formule empirique : C₄₃H₄₀N₅O₃Cl
[M+H]⁺ calculé: 710,2892
[M+H]⁺ mesuré : 710,2923

### Exemple 160. 1-{4,5-Diméthoxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

Le mode opératoire est le même que dans le procédé du Stade A de l'Exemple 131, en remplaçant le composé de la Préparation 3d par le composé de la Préparation 3k, et en remplaçant le composé de la Préparation 1' par le composé de la Préparation 3'. La déprotection se fait selon le mode opératoire du Stade B de l'Exemple 155.
**LC/MS** (C₄₅H₄₅N₅O₅) 736 [M+H]⁺ ; RT 2,17 (Méthode A)

### Masse haute révolution (ESI+) :

Formule empirique : C₄₅H₄₅N₅O₅
[M+H]⁺ calculé : 736,3493
[M+H]⁺ mesuré : 736,3465

### Exemple 161. 1-{2-[(3S)-3-(Aminométhyl)-1,2,3,4-tétrahydroisoquinoléine-2-carbonyl]-4,5-diméthoxyphényl}-N-(4-hydroxyphényl)-N-phényl-1H-indole-3-carboxamide

Le mode opératoire est le même que dans le procédé de l'Exemple 131, en remplaçant le composé de la Préparation 3d par le composé de la Préparation 3k.
**LC/MS** (C₄₀H₃₆N₄O₅) 653 [M+H]⁺ ; RT 2,11 (Méthode A)

### Masse haute révolution (ESI+) :

Formule empirique : C₄₀H₃₆N₄O₅
[M+H]⁺ calculé : 653,2758
[M+H]⁺ mesuré : 653,2754

### Exemple 162. 1-{2-[(3S)-3-(Aminométhyl)-1,2,3,4-tétrahydroisoquinoléine-2-carbonyl]-5-méthoxy-4-[2-(phénylsulfanyl)acétamido]phényl}-N-(4-hydroxyphényl)-N-phényl-1H-indole-3-carboxamide

### Stade A: Acide 2-(3-{[4-(benzyloxy)phényl](phényl)carbamoyl}-1H-indol-1-yl)-4-méthoxy-5-nitrobenzoïque

La procédure est analogue à celle de la Préparation 3a, en utilisant l'acide 4-chloro-2-fluoro-5-nitrobenzoïque. Le composé ainsi obtenu (162 mg, 0,26 mmol) dans du méthanol (3 mL) est ajouté à une solution de méthoxyde de sodium (0,1 mL) dans du méthanol (2 mL), et le milieu réactionnel est porté à reflux sous azote pendant une nuit. La réaction est diluée avec de l'acétate d'éthyle, neutralisée avec du HCl 2M, puis les phases organiques sont lavées avec de l'eau, séchées sur sulfate de magnésium, filtrées et concentrées pour livrer une gomme, qui est utilisée sans autre purification.
**LC/MS** (C₃₆H₂₇N₃O₇) 612 [M-H]⁻; RT 2,7 (Méthode A)

### Stade B: N-{[(3S)-2-(5-Amino-2-{3-[(4-hydroxyphényl)(phényl)carbamoyl]-1H-indol-1-yl}-4-méthoxybenzoyl)-1,2,3,4-tétrahydroisoquinoléin-3-yl]méthyl}carbamate de tert-butyle

Le composé obtenu au Stade A est traité selon la procédure du Stade C de l'Exemple 166 en utilisant le composé de la Préparation 1'. Le composé ainsi obtenu (92 mg, 0,11 mmol) est dissous dans de l'acétate d'éthyle (10 mL) et purgé sous d'azote. On additionne ensuite une quantité catalytique de palladium sur charbon à 10%. Le milieu réactionnel est dégazé, puis agité sous atmosphère d'hydrogène pendant 2 jours. Le milieu réactionnel est filtré sur célite, lavé avec de l'acétate d'éthyle chaud, concentré et purifié par chromatographie sur gel de silice selon un gradient allant de dichlorométhane jusqu'à un mélange méthanol (3%) /dichlorométhane pour livrer un solide.
**LC/MS** (C₄₄H₄₃N₅O₆) 738.3 [M+H]⁺; RT 2,59 (Méthode A)

### Stade C: 4-(N-Phényl-1-{2-[(3S)-3-({[(tert-butoxy)carbonyl]amino}méthyl)-1,2,3,4-tétrahydroisoquinoléine-2-carbonyl]-5-méthoxy-4-[2-(phénylsulfanyl)acétamido]phényl}-1H-indole-3-amido)phényl 2-(phénylsulfanyl)acétate

A une solution du composé obtenu au Stade B (44 mg, 0,06 mmol) dans du dichlorométhane (4 mL) sont ajoutés de la DIPEA (0,04 mL, 0,24 mmol) et du chlorure de 2-(phénylsulphanyl)acétyle (33,4 mg, 0,18 mmol), et le milieu réactionnel est agité à température ambiante, sous azote, pendant un week-end. Le milieu réactionnel est dilué avec du dichlorométhane, lavé avec de l'eau, séché sur sulfate de magnésium, filtré et concentré. Le produit brut est purifié par chromatographie sur gel de silice selon un gradient allant de l'iso-hexane jusqu'à un mélange 6:4 d'acétate d'éthyle / iso-hexane.
**LC/MS** (C₆₀H₅₅N₅O₈S₂) pas de m/z observé; RT 2,72 (Méthode A)

### Stade D: 1-{2-[(3S)-3-(Aminométhyl)-1,2,3,4-tétrahydroisoquinoléine-2-carbonyl]-5-méthoxy-4-[2-(phénylsulfanyl)acétamido]phényl}-N-(4-hydroxyphényl)-N-phényl-1H-iudole-3-carboxamide

A une solution du composé obtenu au Stade C (45 mg, 0,04 mmol) dans du dichlorométhane (5 mL), est ajouté l'acide trifluoroacétique (1 mL), et le milieu réactionnel est agité à température ambiante pendant 1 heure. Le milieu réactionnel est alcalinisé avec du NaOH 2M et agité pendant 15 minutes. Le milieu réactionnel est extrait avec du dichlorométhane, séché sur sulfate de magnésium, filtré et concentré. Le produit intermédiaire est purifié par chromatographie sur gel de silice selon un gradient allant de dichlorométhane jusqu'à un mélange méthanol (5%)/dichlorométhane. Le produit est ensuite remis en suspension dans du tétrahydrofurane (4 mL) et du NaOH 2M (2 mL), et agité pendant 2 heures à température ambiante. Le milieu réactionnel est extrait avec du dichlorométhane, séché sur sulfate de magnésium, filtré et concentré, puis purifié par chromatographie sur gel de silice selon un gradient allant de dichlorométhane jusqu'à un mélange méthanol (10%)/dichlorométhane.
**LC/MS** (C₄₇H₄₁N₅O₅S) 788 [M+H]⁺; RT 2,29 (Méthode A)

### Masse haute résolution (ESI+):

Formule empirique: C₄₇H₄₁N₅O₅S
[M+H]+ calculé: 788.2901
[M+H]+ mesuré: 788.2939

### Exemple 163. N-(4-Hydroxyphényl)-1-{2-[(3S)-3-[(4-méthylpiperazin-1-yl)méthyl]-1,2,3,4-tétrahydroisoquinoléine-2-carbonyl]-4-[2-(phénylsulfanyl)acétamido]phényl}-N-phényl-1H-indole-3-carboxamide

### Stade A: Acide 5-amino-2-{3-[(4-hydroxyphényl)(phényl)carbamoyl]-1H-indol-1-yl}benzoïque

Le composé obtenu au Stade A de l'Exemple 157 (180 mg, 0,31 mmol) dans l'acétate d'éthyle (10 mL) est dégazé avec de l'azote. On y additionne une quantité catalytique de palladium sur charbon à 10%. Le milieu réactionnel est dégazé, puis agité sous atmosphère d'hydrogène pendant une nuit. On ajoute à nouveau du catalyseur, et le milieu réactionnel est agité sous atmosphère d'hydrogène pendant 2 jours de plus à 28°C. On rajoute du catalyseur et la réaction est maintenue sous agitation sous atmosphère d'hydrogène pendant une journée supplémentaire. La réaction incomplète est filtrée sur célite, lavée avec de l'acétate d'éthyle chaud et concentrée. Le matériau brut est purifié par chromatographie sur gel de silice selon un gradient allant de dichlorométhane jusqu'à un mélange méthanol (15%)/dichlorométhane, suivi de DMAW 240 pour livrer le composé désiré (Composé 2), ainsi qu'un peu de composé dont l'aniline est toujours protégée (groupement O-Bn) (Composé 1).
**Composé 1 LC/MS** (C₃₅H₂₇N₃O₄) 554,2 [M+H]⁺; RT 2,6 (Méthode A)
**Composé 2 LC/MS** (C₂₈H₂₁N₃O₄) 464,2 [M+H]⁺; RT 2,22 (Méthode A)

### Stade B: Acide 2-{3-[phényl(4-{[2-(phénylsulfanyl)acétyl]oxy}phényl)carbamoyl]-1H-indol-1-yl}-5-[2-(phénylsulfanyl)acétamido]benzoïque

Le composé 2 obtenu au Stade A est traité selon la procédure décrite au Stade C de l'Exemple 162. Le composé ainsi obtenu est purifié avec une colonne PE-AX, lavé avec du dichlorométhane et élué dans un mélange acide formique (10%)/dichlorométhane pour conduire à une gomme.
**LC/MS** (C₄₄H₃₃N₃O₆S₂) 764,2 [M+H]⁺; RT 2,75 (Méthode A)

### Stade C: 4-(N-Phényl-1-{2-[(3S)-3-[(4-méthylpipérazin-1-yl)méthyl]-1,2,3,4-tétrahydroisoquinoléine-2-carbonyl]-4-[2-(phénylsulfanyl)acétamido]phényl}-1H-indole-3-amido)phényl 2-(phénylsulfanyl)acétate

Le composé obtenu au Stade B est traité selon la procédure décrite au Stade C de l'Exemple 166 en utilisant la Préparation 3', et purifié par chromatographie sur gel de silice selon un gradient allant de dichlorométhane jusqu'à un mélange méthanol (5%)/dichlorométhane. Le composé est engagé dans le stade suivant sans autre purification.
**LC/MS** (C₅₉H₅₄N₆O₅S₂) 991,4 [M+H]⁺; RT 2,16 (Méthode A)

### Stade D: N-(4-Hydroxyphényl)-1-{2-[(3S)-3-[(4-méthylpiperazin-1-yl)méthyl]-1,2,3,4-tétrahydroisoquinoléine-2-carbonyl]-4-[2-(phénylsulfanyl)acétamido]phényl}-N-phényl-1H-indole-3-carboxamide

A une solution du composé obtenu au Stade C (19 mg, 0,02 mmol) dans du tétrahydrofurane (1,5 mL) est ajouté du NaOH 2M (1,5 mL), et le milieu réactionnel est agité à température ambiante pendant 1 heure, puis encore 1 heure à 32 °C. Le milieu réactionnel est réparti selon sa solubilité entre l'acétate d'éthyle et l'eau, et les phases organiques sont séchées sur sulfate de magnésium, filtrées et concentrées. Le produit brut est purifié par chromatographie sur gel de silice selon un gradient allant de dichlorométhane jusqu'à un mélange méthanol (5%)/dichlorométhane.
**LC/MS** (C₅₁H₄₈N₆O₄S) 841,4 [M+H]⁺; RT 1,87 (Méthode A)

### Exemple 164. Dichlorhydrate de N-(4-hydroxyphényl)-1-{6-[(3S)-3-[(4-méthylpipérazin-1-yl)méthyl]-1,2,3,4-tétrahydroisoquinoléine-2-carbonyl]-2H-1,3-benzodioxol-5-yl}-N-phényl-1H-indole-3-carboxamide

### Stade A : N-[4-(Benzyloxy)phényl]-1-{6-[(3S)-3-[(4-méthylpipérazin-1-yl)méthyl]-1,2,3,4-tétrahydroisoquinoléine-2-carbonyl]-2H-1,3-benzodioxol-5-yl}-N-phényl-1H-indole-3-carboxamide

Le mode opératoire est le même que dans le procédé du Stade A de l'Exemple 131, en remplaçant le composé de la Préparation 3d par le composé de la Préparation 3h, et en remplaçant le composé de la Préparation 1' par le composé de la Préparation 3'.

### Stade B : Dichlorhydrate de N-(4-hydroxyphényl)-1-{6-[(3S)-3-[(4-méthylpipérazin-1-yl)méthyl]-1,2,3,4-tétrahydroisoquinoléine-2-carbonyl]-2H-1,3-benzodioxol-5-yl}-N-phényl-1H-indole-3-carboxamide

On dissout le composé issu du Stade A (1,44 g, 1,79 mmol) dans du dichlorométhane anhydre (15 ml), on le refroidit jusqu'à 0°C sous azote, puis l'on ajoute du trichlorure de bore (1M, 3,6 ml, 3,58 mmol) et on laisse le mélange réactionnel se réchauffer jusqu'à la température ambiante, avant de l'agiter pendant environ 16 h. Le mélange réactionnel est refroidi jusqu'à 0°C, inactivé avec du méthanol, puis concentré. Le résidu est dissous dans du dichlorométhane, lavé avec une solution aqueuse saturée de bicarbonate de sodium, séché sur sulfate de magnésium, et évaporé. Le matériau brut est purifié par chromatographie sur gel de silice selon un gradient allant de dichlorométhane jusqu'à un mélange méthanol (10 %)/ dichlorométhane pour livrer une mousse. Le matériau est dissous dans de l'alcool isopropylique chaud (5 ml), et la solution résultante est agitée vigoureusement pendant que l'on ajoute goutte à goutte un excès de HCl (2M dans l'éther, 4 ml). La bouillie résultante est agitée pendant 5 minutes, puis filtrée et lavée à l'éther pour livrer le produit sous forme d'un solide.
**LC/MS** (C₄₄H₄₁N₅O₅) 720,3 [M+H]⁺ ; RT 2,19 (Méthode A)

### Masse haute résolution (ESI+) :

Formule empirique : C₄₄H₄₁N₅O₅
[M+H]⁺ calculé: 720,3180
[M+H]⁺ mesuré : 720,3151

### Exemple 165. Dichlorhydrate de 1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

### Stade A : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-N-[4-(benzyloxy)phényl]-N-phényl-1H-indole-3-carboxamide

On ajoute du HBTU (531 mg, 1,4 mmol) et de la DIPEA (0,49 ml, 2,8 mmol), puis de la (3*S*)-3-[(4-méthylpipérazin-1-yl)méthyl]-1,2,3,4-tétrahydroisoquinoléine (343 mg, 1,4 mmol) issue de la Préparation 3' à une solution de l'acide obtenu dans la Préparation 3a (944 mg, 1,4 mmol) dans du *N*,*N*-diméthylformamide (10 ml). Le mélange réactionnel est agité à température ambiante pendant 3 heures, puis dilué avec de l'eau et le précipité résultant est filtré et lavé à l'eau, dissous dans du dichlorométhane et lavé avec une solution saturée de bicarbonate de sodium. Les phases organiques sont séchées sur sulfate de magnésium, filtrées et évaporées et le matériau brut est purifié par chromatographie sur gel de silice selon un gradient allant de dichlorométhane jusqu'à un mélange méthanol (5 %)/dichlorométhane pour livrer le produit sous forme d'une gomme.
**LC/MS** (C₅₈H₅₅N₅O₅) pas de m/z observé ; RT 2,63 (Méthode A)

### Stade B : 1-{4-Hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

On ajoute du formiate d'ammonium (793 mg, 12,6 mmol), puis du palladium à 10 % sur carbone (130 mg) à une solution du produit obtenu au Stade A (1,14 g, 1,26 mmol) dans du méthanol (20 ml) et l'on chauffe le mélange réactionnel à reflux sous azote pendant 2,5 heures. On laisse le mélange réactionnel refroidir jusqu'à la température ambiante, et on le filtre au travers de Célite, on le lave au méthanol et on le concentre sous vide. Le matériau est dissous dans du dichlorométhane, lavé à l'eau, puis séché sur sulfate de magnésium, filtré et évaporé. Le produit brut est purifié par chromatographie sur gel de silice selon un gradient allant de dichlorométhane à un mélange méthanol (10%)/dichlorométhane pour livrer le produit sous forme d'une mousse.
**LC/MS** (C₄₄H₄₃N₅O₅) 722 [M+H]⁺ ; RT 2,17 (Méthode A)

### Stade C : Dichlorhydrate de 1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

On ajoute du HCl 2M dans de l'éther (3,70 ml, 7,4 mmol) à une solution du composé obtenu au Stade B (535 mg, 0,74 mmol) dans de l'alcool isopropylique (5 ml). La suspension résultante est diluée avec de l'éther (5 ml), puis refroidie jusqu'à 0°C pendant 30 minutes, avant filtration. Le solide est lavé à l'éther froid et séché sous vide.
**LC/MS** (C₄₄H₄₃N₅O₅) 722 [M+H]⁺ ; RT 2,16 (Méthode A)

### Masse haute résolution (ESI+) :

Formule empirique : C₄₄H₄₃N₅O₅
[M+H]⁺ calculé : 722,3337
[M+H]⁺ mesuré : 722,3344

### Exemple 166. N-(4-Hydroxyphényl)-1-{2-[(3S)-3-(4-méthylpipérazine-1-carbonyl)-1,2,3,4-tétrahydroisoquinoléine-2-carbonyl]phényl}-N-phényl-1H-indole-3-carboxamide

### Stade A: (3S)-3-(4-Méthylpiperazine-1-carbonyl)-1,2,3,4-tétrahydroisoquinoléine-2-carboxylate de tert-butyle

A une solution d'acide (3*S*)-2-[(*tert*-butoxy)carbonyl]-1,2,3,4-tétrahydroisoquinoléine-3-carboxylique (15 g, 54,1 mmol) dans le *N*,*N*-diméthylformamide (600 mL) sous azote, sont ajoutés la DIPEA (11,3 mL, 65 mmol), la N-méthylpipérazine (12 mL, 108 mmol) puis le HBTU (24,6 g, 65 mmol) par portion et le milieu réactionnel est agité à température ambiante pendant 16 heures. Le milieu est concentré sous vide, dilué avec une solution saturée de NaHCO₃ et extrait avec de l'acétate d'éthyle. Les phases organiques sont lavées avec une solution saturée de NaHCO₃, de la saumure, puis séchées sur sulfate de magnésium, filtrées et concentrées sous vide. Le matériau brut est chargé à sec sur une colonne de silice, et élué avec de l'iso-hexane, de l'acétate d'éthyle puis un mélange 19:1 acétate d'éthyle : ammoniaque 7M dans du méthanol pour livrer le produit sous la forme d'un solide.
**LC/MS** (C₂₀H₂₉N₃O₃) 360 [M+H]⁺; RT 1,83 (Méthode A)

### Stade B: (3S)-3-(4-Méthylpipérazine-1-carbonyl)-1,2,3,4-tétrahydroisoquinoléine

A une solution du composé obtenu au Stade A (19,68 g, 54,75 mmol) dans du dichlorométhane est ajouté lentement de l'acide trifluoroacétique (42,2 mL, 547,5 mmol) à température ambiante, puis le milieu réactionnel est chauffé à 40 °C, agité pendant 6 heures, puis refroidi jusqu'à la température ambiante et agité de nouveau pendant 16 heures. Le milieu réactionnel est ensuite concentré sous vide, dilué dans de l'eau, refroidi à 0°C et alcalinisé à pH=12 avec du NH₄OH concentré. La phase aqueuse est extraite avec de l'acétate d'éthyle et les phases organiques sont séchées sur sulfate de magnésium, filtrées et concentrées sous vide. Le matériau brut est chargé à sec sur une colonne de silice, et élué avec de l'iso-hexane, de l'acétate d'éthyle, puis un mélange 19:1 acétate d'éthyle : ammoniaque 7M dans du méthanol, et enfin un mélange 9:1 d'acétate d'éthyle : ammoniaque 7M dans du méthanol. Le produit est redissous dans du dichlorométhane et lavé avec une solution de NH₄OH 5%, de la saumure, puis séché sur sulfate de magnésium, filtré et concentré sous vide, pour conduire à un verre.
**LC/MS** (C₁₅H₂₁N₃O) 260 [M+H]⁺; RT 0,18 (Méthode A)

### Stade C: N-[4-(Benzyloxy)phényl]-1-{2-[(3S)-3-(4-méthylpipérazine-1-carbonyl)-1,2,3,4-tétrahydroisoquinoléine-2-carbonyl]phényl}-N-phényl-1H-indole-3-carboxamide

A une solution du composé obtenu selon la Préparation 3a, en utilisant l'acide 2-iodobenzoïque (135 mg, 251 µmol) dans du *N*,*N*-diméthylformamide (4 mL) sont ajoutés la DIPEA (52 µL, 301 µmol), le composé obtenu au Stade B (71,5 mg, 275,7 µmol) suivi du HBTU (114 mg, 300,8 µmol), et la réaction est agitée à température ambiante pendant 16 heures. Le milieu réactionnel est dilué avec une solution saturée de NaHCO₃ et extrait à l'acétate d'éthyle. Les phases organiques sont ensuite lavées avec de la saumure, séchées sur sulfate de magnésium, filtrées et concentrées sous vide. Le matériau brut est chargé à sec sur une colonne de silice, et élué avec de l'iso-hexane, de l'acétate d'éthyle, puis un mélange 19:1 acétate d'éthyle : méthanol, puis un mélange 19:1 acétate d'éthyle : ammoniaque 7M dans du méthanol pour conduire à une mousse.
**LC/MS** (C₅₀H₄₅N₅O₄) 780 [M+H]⁺; RT 2,5 (Méthode A)

### Stade D: N-(4-Hydroxyphényl)-1-{2-[(3S)-3-(4-méthylpipérazine-1-carbonyl)-1,2,3,4-tétrahydroisoquinoléine-2-carbonyl]phényl}-N-phényl-1H-indole-3-carboxamide

Le composé obtenu dans le Stade C (146 mg, 187 µmol) est dissous dans du méthanol. On y additionne du formiate d'ammonium (118 mg, 1,87 mmol) et du palladium sur charbon à 10% (106 mg, 18,7 µmol), et le milieu réactionnel est porté à reflux sous azote pendant 3 heures. Il est ensuite dilué avec de la saumure, et extrait avec de l'acétate d'éthyle. Les phases organiques sont lavées à l'eau, séchées sur sulfate de magnésium, filtrées sur célite et concentrées sous vide. Le matériau brut est chargé à sec sur une colonne de silice, et élué avec de l'iso-hexane, de l'acétate d'éthyle, puis un mélange 19:1 d'acétate d'éthyle : ammoniaque 7M dans du méthanol, et enfin un mélange 9:1 d'acétate d'éthyle : ammoniaque 7M dans du méthanol.
**LC/MS** (C₄₃H₃₉N₅O₄) 690 [M+H]⁺; RT 2,17 (Méthode A)

### Masse haute révolution (ESI+):

Formule empirique: C₄₃H₃₉N₅O₄
[M+H]+ calculé: 690.3075
[M+H]+ mesuré: 690.3063

### Exemple 167. 1-{5-Chloro-4-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

Le mode opératoire est le même que dans le procédé du Stade A de l'Exemple 131, en remplaçant le composé de la Préparation 3d par le composé de la Préparation 31, et en remplaçant le composé de la Préparation 1' par le composé de la Préparation 3'. La déprotection se fait selon le mode opératoire de l'Exemple 155.
**LC/MS** (C₄₄H₄₂N₅O₄Cl) 740,4 [M+H]⁺ ; RT 1,85 (Méthode A)

### Masse haute révolution (ESI+) :

Formule empirique : C₄₄H₄₂N₅O₄Cl
[M+H]⁺ calculé : 740,2998
[M+H]⁺ mesuré : 740,2977

### Exemple 168. Dichlorhydrate de N-(4-hydroxyphényl)-1-{5-méthoxy-2-[(3S)-3-[(4-méthylpipérazin-1-yl)méthyl]-1,2,3,4-tétrahydroisoquinoléine-2-carbonyl]phényl}-N-phényl-1H-indole-3-carboxamide

Le mode opératoire est le même que dans le procédé de l'Exemple 164, en remplaçant le composé issu de la Préparation 3h par le composé issu de la Préparation 3e.
**LC/MS** (C₄₄H₄₃N₅O₄) 706 [M+H]⁺ ; RT 1,75 (Méthode A)

### Masse haute révolution (ESI+) :

Formule empirique : C₄₄H₄₃N₅O₄
[M+H]⁺ calculé : 706,3388
[M+H]⁺ mesuré : 706,3415

### Exemple 169. Chlorhydrate de N-(4-hydroxyphényl)-1-{5-méthoxy-2-[(3S)-3-(pipéridin-1-ylméthyl)-1,2,3,4-tétrahydroisoquinoléine-2-carbonyl]phényl}-N-phényl-1H-indole-3-carboxamide

Le mode opératoire est le même que dans le procédé de l'Exemple 164, en remplaçant le composé issu de la Préparation 3h par le composé issu de la Préparation 3e, et en remplaçant le composé de la Préparation 3' par le composé de la Préparation 11'.
**LC/MS** (C₄₄H₄₂N₄O₄) 691 [M+H]⁺ ; RT 2,25 (Méthode A)

### Masse haute révolution (ESI+) :

Formule empirique : C₄₄H₄₂N₄O₄
[M+H]⁺ calculé: 691,3279
[M+H]⁺ mesuré: 691,3256

### Exemple 170. N-(4-Hydroxyphényl)-1-{7-[(3S)-3-[(4-méthylpipérazin-1-yl)méthyl]-1,2,3,4-tétrahydroisoquinoléine-2-carbonyl]-2,3-dihydro-1,4-benzodioxin-6-yl}-N-phényl-1H-indole-3-carboxamide

Le composé est préparé selon le mode opératoire de la Préparation 3a, en utilisant l'acide 7-bromo-2,3-dihydro-1,4-benzodioxine-6-carboxylique et en mettant l'ensemble dans un appareil à micro-ondes à 150 °C pendant 40 minutes. On applique ensuite le mode opératoire décrit au Stade C de l'Exemple 166 en utilisant le composé de la Préparation 3'. Le matériau est purifié par chromatographie sur gel de silice dans du dichlorométhane, jusqu'à un mélange à 5% de méthanol/dichlorométhane, et finalement déprotégé selon le mode opératoire du Stade D de l'Exemple 166 et purifié par chromatographie sur gel de silice dans du dichlorométhane, jusqu'à un mélange à 8% de méthanol/dichlorométhane.
**LC/MS** (C₄₅H₄₃N₅O₅) 734 [M+H]⁺; RT 2,2 (Méthode A)

### Masse haute révolution (ESI+):

Formule empirique: C₄₅H₄₃N₅O₅
[M+H]+ calculé: 734.3337
[M+H]+ mesuré: 734.3323

### Exemple 171. 1-{5-Chloro-2-[(3S)-3-(pipéridin-1-ylméthyl)-1,2,3,4-tétrahydroisoquinoléine-2-carbonyl]phényl}-N-(4-hydroxyphényl)-N-phényl-1H-indole-3-carboxamide

Le mode opératoire est le même que dans le procédé du Stade A de l'Exemple 131, en remplaçant le composé de la Préparation 3d par le composé de la Préparation 3i, et en remplaçant le composé de la Préparation 1' par le composé de la Préparation 11'. La déprotection se fait selon le mode opératoire du Stade B de l'Exemple 155.
**LC/MS** (C₄₃H₃₉N₄O₃Cl) 695 [M+H]⁺ ; RT 2,31 (Méthode A)

### Masse haute révolution (ESI+) :

Formule empirique : C₄₃H₃₉N₄O₃Cl
[M+H]⁺ calculé : 695,2783
[M+H]⁺ mesuré: 695,2771

### Exemple 172. 1-{2-[(3S)-3-(Hydroxyméthyl)-1,2,3,4-tétrahydroisoquinoléine-2-carbonyl]phényl}-N-(4-hydroxyphényl)-N-phényl-1H-indole-3-carboxamide

Le mode opératoire est le même que dans le procédé du Stade A de l'Exemple 131, en remplaçant le composé de la Préparation 1' par le (3*S*)-1,2,3,4-tétrahydroisoquinoléin-3-ylméthanol. La déprotection se fait selon le mode opératoire du Stade B de l'Exemple 155.
**LC/MS** (C₃₈H₃₁N₃O₄) 594 [M+H]⁺ ; RT 2,47 (Méthode A)

### Masse haute révolution (ESI+) :

Formule empirique : C₃₈H₃₁N₃O₄
[M+H]⁺ calculé : 594,2387
[M+H]⁺ mesuré : 594,2368

### Exemple 173. N-(4-Hydroxyphényl)-N-phényl-1-{2-[(3S)-3-(pipéridin-1-ylméthyl)-1,2,3,4-tétrahydroisoquinoléine-2-carbonyl]phényl}-1H-indole-3-carboxamide

Le mode opératoire est le même que dans le procédé du Stade A de l'Exemple 131, en remplaçant le composé de la Préparation 1' par le composé de la Préparation 11'. La déprotection se fait selon le mode opératoire du Stade B de l'Exemple 155.
**LC/MS** (C₄₃H₄₀N₄O₃) 661 [M+H]⁺ ; RT 2,21 (Méthode A)

### Masse haute révolution (ESI+) :

Formule empirique : C₄₃H₄₀N₄O₃
[M+H]⁺ calculé : 661,3173
[M+H]⁺ mesuré: 661,3156

### Exemple 174. N-(4-Hydroxyphényl)-N-phényl-1-{6-[(3S)-3-(pipéridin-1-ylméthyl)-1,2,3,4-tétrahydroisoquinoléine-2-carbonyl]-2H-1,3-benzodioxol-5-yl}-1H-indole-3-carboxamide

Le mode opératoire est le même que dans le procédé de l'Exemple 164, en remplaçant le composé de la Préparation 3' par le composé de la Préparation 11'.
**LC/MS** (C₄₄H₄₀N₄O₅) 705 [M+H]⁺ ; RT 2,24 (Méthode A)

### Masse haute révolution (ESI+) :

Formule empirique : C₄₄H₄₀N₄O₅
[M+H]⁺ calculé: 705,3071
[M+H]⁺ mesuré : 705,3041

### Exemple 175.1-{2-[(3S)-3-[(Diméthylamino)méthyl]-1,2,3,4-tétrahydroisoquinoléine-2-carbonyl]-5-méthoxyphényl}-N-(4-hydroxyphényl)-N-phényl-1H-indole-3-carboxamide

Le mode opératoire est le même que dans le procédé de l'Exemple 164, en remplaçant le composé issu de la Préparation 3h par le composé issu de la Préparation 3e, et en remplaçant le composé de la Préparation 3' par le composé de la Préparation 12'.
**LC/MS** (C₄₄H₃₈N₄O₄) 651 [M+H]⁺ ; RT 2,18 (Méthode A)

### Masse haute révolution (ESI+) :

Formule empirique : C₄₁H₃₈N₄O₄
[M+H]⁺ calculé: 651,2966
[M+H]⁺ mesuré: 651,2941

### Exemple 176. Chlorhydrate de 1-{4-hydroxy-5-méthoxy-2-[(3S)-3-(pipéridin-1-ylméthyl)-1,2,3,4-tétrahydroisoquinoléine-2-carbonyl]phényl}-N-(4-hydroxyphényl)-N-phényl-1H-indole-3-carboxamide

Le mode opératoire est le même que dans le procédé de l'Exemple 165, en remplaçant la (3*S*)-3-[(4-méthylpipérazin-1-yl)méthyl]-1,2,3,4-tétrahydroisoquinoléine issue de la Préparation 3' par la (3*S*)-3-(pipéridin-1-ylméthyl)-1,2,3,4-tétrahydroisoquinoléine issue de la Préparation 11'.
**LC/MS** (C₄₄H₄₂N₄O₅) 707 [M+H]⁺ ; RT 2,17 (Méthode A)

### Masse haute révolution (ESI+) :

Formule empirique : C₄₄H₄₂N₄O₅
[M+H]⁺ calculé : 707,3228
[M+H]⁺ mesuré : 707,3206

### Exemple 177. Dichlorhydrate de 1-{6-[(3S)-3-[(4-méthylpipérazin-1-yl)méthyl]-1,2,3,4-tétrahydroisoquinoléine-2-carbonyl]-2H-1,3-benzodioxol-5-yl}-N,N-diphényl-1H-indole-3-carboxamide

Le mode opératoire est le même que dans le procédé de l'Exemple 164, en remplaçant le composé issu de la Préparation 3h par le composé issu de la Préparation 3p.
**LC/MS** (C₄₄H₄₁N₅O₄) 704 [M+H]⁺ ; RT 2,33 (Méthode A)

### Masse haute révolution (ESI+) :

Formule empirique : C₄₄H₄₁N₅O₄
[M+H]⁺ calculé: 704,3231
[M+H]⁺ mesuré : 704,3245

### Exemple 178. Dichlorhydrate de 1-{5-chloro-2-[(3S)-3-[(4-méthylpipérazin-1-yl)méthyl]-1,2,3,4-tétrahydroisoquinoléine-2-carbonyl]phényl}-N,N-diphényl-1H-indole-3-carboxamide

Le mode opératoire est le même que dans le procédé de l'Exemple 164, en remplaçant le composé issu de la Préparation 3h par le composé issu de la Préparation 3q.
**LC/MS** (C₄₃H₄₀N₅O₂) 694 [M+H]⁺ ; RT 2.89 (Méthode A)

### Masse haute révolution (ESI+) :

Formule empirique : C₄₃H₄₀N₅O₂Cl
[M+H]⁺ calculé : 694.2943
[M+H]⁺ mesuré: 694.2918

### Exemple 179. Chlorhydrate de 1-{5-chloro-2-[(3S)-3-[(diméthylamino)méthyl]-1,2,3,4-tétrahydroisoquinoléine-2-carbonyl]phényl}-N-(4-hydroxyphényl)-N-phényl-1H-indole-3-carboxamide

Le mode opératoire est le même que dans le procédé de l'Exemple 164, en remplaçant le composé issu de la Préparation 3h par le composé issu de la Préparation 3i, et en remplaçant le composé de la Préparation 3' par le composé de la Préparation 12'.
**LC/MS** (C₄₀H₃₅N₄O₃Cl) 655 [M+H]⁺ ; RT 2,3 (Méthode A)

### Masse haute révolution (ESI+) :

Formule empirique : C₄₀H₃₅N₄O₃Cl
[M+H]⁺ calculé : 655,2470
[M+H]⁺ mesuré : 655,2446

### Exemple 180. Chlorhydrate de 1-{6-[(3S)-3-[(diméthylamino)méthyl]-1,2,3,4-tétrahydroisoquinoléine-2-carbonyl]-2H-1,3-benzodioxol-5-yl}-N-(4-hydroxyphényl)-N-phényl-1H-indole-3-carboxamide

Le mode opératoire est le même que dans le procédé de l'Exemple 164, en remplaçant le composé de la Préparation 3' par le composé de la Préparation 12'.
**LC/MS** (C₄₁H₃₆N₄O₅) 665 [M+H]⁺ ; RT 2,16 (Méthode A)

### Masse haute révolution (ESI+) :

Formule empirique : C₄₁H₃₆N₄O₅
[M+H]⁺ calculé : 665,2758
[M+H]⁺ mesuré : 665,2756

### Exemple 181. Dichlorhydrate de 1-{4-hydroxy-5-méthoxy-2-[(3S)-3-[(4-méthylpipérazin-1-yl)méthyl]-1,2,3,4-tétrahydroisoquinoléine-2-carbonyl]phényl}-N,N-diphényl-1H-indole-3-carboxamide

Le mode opératoire est le même que dans le procédé de l'Exemple 165, en remplaçant le composé de la Préparation 3a utilisé au Stade A par le composé de la Préparation 3c ; ce qui donne le produit sous forme d'un solide.
**LC/MS** (C₄₄H₄₃N₅O₄) 706 [M+H]⁺ ; RT 1,78 (Méthode A)

### Masse haute résolution (ESI+) :

Formule empirique : C₄₄H₄₃N₅O₄
[M+H]⁺ calculé : 706,3388
[M+H]⁺ mesuré : 706,3398

### Exemple 182. 1-{2,2-Difluoro-6-[(3S)-3-[(4-méthylpipérazin-1-yl)méthyl]-1,2,3,4-tétrahydroisoquinoléine-2-carbonyl]-2H-1,3-benzodioxol-5-yl}-N-(4-hydroxyphényl)-N-phényl-1H-indole-3-carboxamide

### Stade A : 6-Bromo-2H-1,3-benzodioxole-5-carboxylate de méthyle

A une suspension sous agitation d'acide 6-bromo-1,3-benzodioxole-5-carboxylique (10,29 g, 0,04 mol) dans du méthanol est ajouté goutte à goutte de l'acide sulfurique concentré sur *ca* 1 minute. Le mélange est porté à reflux et laissé sous agitation toute la nuit. On laisse le milieu réactionnel refroidir jusqu'à la température ambiante, et une précipitation se produit. De la glace est ajoutée dans le milieu et agitée jusqu'à ce qu'elle soit fondue. Le milieu est ensuite filtré et lavé avec un mélange eau-méthanol (2:1). Le solide est séché par aspiration pour conduire à une poudre.
**LC/MS** (C₉H₇O₄Br) pas de m/z observé; RT 1,22 (Méthode B)

### Stade B : 2-Bromo-4,5-dihydroxybenzoate de méthyle

A une solution sous agitation du composé obtenu au Stade A (2 g, 7,72 mmol) dans du dichlorométhane anhydre (20 mL), refroidie à 0 °C sous azote, est ajouté le trichlorure de bore (1 M dans le dichlorométhane; 15,5 mL, 0,02 mol) par portions sur 5 minutes, puis on laisse le milieu réactionnel se réchauffer jusqu'à la température ambiante toute la nuit. Le mélange réactionnel est lentement versé sur du méthanol (20 mL) sous agitation refroidi avec de la glace, puis il est agité pendant 1 week-end. Le solvant est éliminé sous vide et le résidu est dissous dans un mélange dichlorométhane-méthanol, séché sur sulfate de magnésium, filtré, concentré et séché sous vide pour conduire à un solide. Le produit est directement engagé dans le stade suivant sans autre purification.
**LC/MS** (C₈H₇O₄Br) 245 [M-H]⁻; RT 0,94 (Méthode B)

### Stade C : 2-Bromo-4,5-bis(méthoxyméthoxy)benzoate de méthyle

A une solution du composé obtenu au Stade B (200 mg, 0,81 mmol) dans du chloroforme (5 mL) et du diméthoxyméthane (5 mL), refroidie à 0 °C, est ajouté du P₂O₅ (1,15 g, 8,1 mmol). Le milieu réactionnel est agité sous azote pendant 5 minutes, puis on le laisse se réchauffer jusqu'à la température ambiante. Au bout de 5 heures, la réaction est refroidie de nouveau à 0°C, et 200 mg supplémentaires de P₂O₅ sont ajoutés. Le milieu réactionnel est agité pendant une nuit à température ambiante. Il est ensuite versé sur de la glace, extrait avec de l'acétate d'éthyle, séché sur sulfate de magnésium, filtré et concentré. Le matériau brut est purifié par chromatographie sur gel de silice selon un gradient allant d'iso-hexane jusqu'à un mélange 2:1 iso-hexane/ acétate d'éthyle pour livrer une huile.
**LC/MS** (C₁₂H₁₅O₆Br) 337,1 [M+H]⁺; RT 2,38 (Méthode A)

### Stade D : 2-(3-{[4-(Benzyloxy)phényl](phényl)carbamoyl}-1H-indol-1-yl)-4,5-dihydroxybenzoate de méthyle

Le composé obtenu dans le Stade C est traité selon la procédure de la Préparation 3a, et chauffé dans un appareil à micro-ondes à 160 °C pendant 2 heures. Le composé obtenu est repris dans du HCl 4M dans du dioxane (4 mL) et agité à température ambiante sous azote pendant 1 heure. Le milieu réactionnel est réparti selon sa solubilité entre l'acétate d'éthyle et une solution de NaOH 2N, puis les phases organiques sont séchées sur sulfate de magnésium, filtrées et concentrées pour livrer un solide, qui est utilisé dans le stade suivant sans autre purification.
**LC/MS** (C₃₆H₂₈N₂O₆) 585,4 [M+H]⁺; RT 2,69 (Méthode A)

### Stade E : 6-(3-{[4-(Benzyloxy)phényl](phényl)carbamoyl}-1H-indol-1-yl)-2,2-difluoro-2H-1,3-benzodioxole-5-carboxylate de méthyle

A une solution du composé obtenu au Stade D (90 mg, 0,15 mmol) dans le *N,N-*diméthylformamide (3 mL) est ajouté le dibromodifluorométhane (70 µL, 0,77 mmol), suivi de carbonate de césium (147 mg, 0,45 mmol), et le milieu réactionnel est chauffé dans un appareil à micro-ondes à 150 °C pendant 10 minutes. Le milieu réactionnel est réparti selon sa solubilité entre l'acétate d'éthyle et l'eau, séché sur sulfate de magnésium, filtré et concentré, puis purifié par chromatographie sur gel de silice selon un gradient allant d'iso-hexane jusqu'à un mélange 3:1 iso-hexane/ acétate d'éthyle pour conduire à une huile.
**LC/MS** (C₃₇H₂₆N₂O₆F₂) 633,3 [M+H]⁺; RT 2,94 (Méthode A)

### Stade F : Acide 6-(3-{(4-(benzyloxy)phényl)(phényl)carbamoyl}-1H-indol-1-yl)-2,2-difluoro-2H-1,3-benzodioxole-5-carboxylique

A une solution du composé obtenu au Stade E (39 mg, 0,06 mmol) dans du tétrahydrofurane (1 mL) et de l'éthanol (1 mL) est ajouté du NaOH 2N (1 mL, 2 mmol), et le milieu réactionnel est agité à température ambiante pendant 2 heures. Il est réparti selon sa solubilité entre l'acétate d'éthyle et l'eau, séché sur sulfate de magnésium, filtré et concentré pour conduire à une gomme qui est engagée dans le stade suivant sans autre purification.
**LC/MS** (C₃₆H₂₄N₂O₆F₂) 619,3 [M+H]⁺; RT 2,84 (Méthode A)

### Stade G : 1-{2,2-Difluoro-6-[(3S)-3-[(4-méthylpipérazin-1-yl)méthyl]-1,2,3,4-tétrahydroisoquinoléine-2-carbonyl]-2H-1,3-benzodioxol-5-yl}-N-(4-hydroxyphényl)-N-phényl-1H-indole-3-carboxamide

Le composé obtenu au Stade F est traité selon la procédure du Stade C de l'Exemple 166 en utilisant le composé de la Préparation 3', et purifié sur gel de silice dans du dichlorométhane jusqu'à un mélange méthanol (5%)/dichlorométhane, et finalement déprotégé selon la procédure du Stade D de l'Exemple 166, la réaction se poursuivant lentement sur 7 jours, et requérant plusieurs autres additions de palladium sur charbon à 10%, de formiate d'ammonium et d'éthanol. Le produit final est purifié par chromatographie sur gel de silice dans du dichlorométhane jusqu'à un mélange méthanol (6%)/dichlorométhane.
**LC/MS** (C₄₄H₃₉N₅O₅F₂) 756 [M+H]⁺; RT 2,33 (Méthode A)

### Masse haute révolution (ESI+) :

Formule empirique: C₄₄H₃₉N₅O₅F₂
[M+H]+ calculé: 756.2992
[M+H]+ mesuré: 756.2985

### Exemple 183. 1-{4,5-Dihydroxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

Le mode opératoire est le même que dans le procédé du Stade A de l'Exemple 131, en remplaçant le composé de la Préparation 1' par le composé de la Préparation 3', et en remplaçant le composé de la Préparation 3d par le composé de la Préparation 3r. La déprotection se fait selon le mode opératoire du Stade B de l'Exemple 155.
**LC/MS** (C₄₃H₄₁N₅O₅) 708 [M+H]⁺ ; RT 2,07 (Méthode A)

### Masse haute révolution (ESI+) :

Formule empirique : C₄₃H₄₁N₅O₅
[M+H]⁺ calculé : 708,3180
[M+H]⁺ mesuré: 708,3194

### Exemple 184. Chlorhydrate de N-(4-hydroxyphényl)-1-{6-[(3S)-3-(morpholin-4-ylméthyl)-1,2,3,4-tétrahydroisoquinoléine-2-carbonyl]-2H-1,3-benzodioxol-5-yl}-N-phényl-1H-indole-3-carboxamide

Le mode opératoire est le même que dans le procédé de l'Exemple 164, en remplaçant le composé de la Préparation 3' par le composé de la Préparation 2'.
**LC/MS** (C₄₃H₃₈N₄O₆) 707 [M+H]⁺ ; RT 2,24 (Méthode A)

### Masse haute révolution (ESI+) :

Formule empirique : C₄₃H₃₈N₄O₆
[M+H]⁺ calculé : 707,2864
[M+H]⁺ mesuré : 707,2870

### Exemple 185. 1-{2-Ethyl-7-[(3S)-3-[(4-méthylpipérazin-1-yl)méthyl]-1,2,3,4-tétrahydroisoquinoléine-2-carbonyl]-2,3-dihydro-1,4-benzodioxin-6-yl}-N-(4-hydroxyphényl)-N-phényl-1H-indole-3-carboxamide

Le mode opératoire est le même que dans le procédé du Stade A de l'Exemple 131, en remplaçant le composé de la Préparation 1' par le composé de la Préparation 3', et en remplaçant le composé de la Préparation 3d par le composé de la Préparation 3m. La déprotection se fait selon le mode opératoire du Stade B de l'Exemple 155.
**LC/MS** (C₄₇H₄₇N₅O₅) 762 [M+H]⁺ ; RT 1,84 (Méthode A)

### Masse haute révolution (ESI+) :

Formule empirique : C₄₇H₄₇N₅O₅
[M+H]⁺ calculé : 762,3650
[M+H]⁺ mesuré : 762,3614

### Exemple 186. Chlorhydrate de 1-{5-chloro-2-[(3S)-3-(morpholin-4-ylméthyl)-1,2,3,4-tétrahydroisoquinoléine-2-carbonyl]phényl}-N-(4-hydroxyphényl)-N-phényl-1H-indole-3-carboxamide

Le mode opératoire est le même que dans le procédé de l'Exemple 164, en remplaçant le composé issu de la Préparation 3h par le composé issu de la Préparation 3q, et en remplaçant le composé de la Préparation 3' par le composé de la Préparation 2'.
**LC/MS** (C₄₂H₃₇N₄O₄.HCl) 697 [M+H]⁺ ; RT 2,38 (Méthode A)

### Masse haute révolution (ESI+) :

Formule empirique : C₄₂H₃₇N₄O₄Cl
[M+H]⁺ calculé : 697,2576
[M+H]⁺ mesuré : 697,2569

### Exemple 187. Dichlorhydrate de N-cyclohexyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-phényl-1H-indole-3-carboxamide

### Stade A : Acide 5-(benzyloxy)-4-méthoxy-2-[3-(méthoxycarbonyl)-1H-indol-1-yl]benzoïque

Une solution d'acide 5-(benzyloxy)-2-bromo-4-méthoxybenzoïque (7,5 g, 22,24 mmol), de l'indole-3-carboxylate de méthyle (3,9 g, 22,24 mmol) et du carbonate de potassium (6,15 g, 44,48 mmol) dans du *N*,*N*-diméthylformamide (60 ml) est dégazée en faisant barboter de l'azote au travers. On ajoute de l'iodure de cuivre (490 mg, 2,22 mmol) et l'on agite le mélange réactionnel à 90°C sous azote pendant environ 16 h. Le mélange réactionnel est refroidi jusqu'à la température ambiante, dilué avec de l'eau et acidifié avec du HCl aqueux 2M. Le précipité résultant est filtré, lavé à l'eau, puis mis en suspension dans du dichlorométhane et lavé à la saumure. Les phases organiques sont séchées sur sulfate de magnésium, filtrées et évaporées et le matériau brut est purifié par chromatographie sur gel de silice selon un gradient allant de dichlorométhane à un mélange méthanol (5 %)/dichlorométhane pour livrer le produit sous forme d'une poudre.
**LC/MS** (C₂₅H₂₁NO₆) 432 [M+H]⁺ ; RT 2,62 (Méthode A)

### Stade B : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-1H-indole-3-carboxylate de méthyle

On ajoute de la DIPEA (4,7 ml, 27 mmol) et du HBTU (2 g, 5,4 mmol), puis le composé issu de la Préparation 3' (1,92 g, 5,4 mmol) à une solution de l'acide obtenu au Stade A (2,3 g, 5,4 mmol) dans du *N*,*N*-diméthylformamide (20 ml) et l'on agite le mélange réactionnel à température ambiante pendant environ 72 h. Le mélange réactionnel est dilué avec du dichlorométhane, lavé à l'eau, avec une solution saturée de bicarbonate de sodium, puis séché sur sulfate de magnésium, filtré et évaporé. Le matériau brut est purifié par chromatographie sur gel de silice selon un gradient allant de dichlorométhane jusqu'à un mélange méthanol (5 %)/dichlorométhane pour livrer le produit sous forme d'une mousse.
**LC/MS** (C₄₀H₄₂N₄O₅) 659 [M+H]⁺ ; RT 2,31 (Méthode A)

### Stade C : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-1H-indole-3-carboxylate de sodium

On ajoute une solution aqueuse de NaOH 2M (10 ml) à une solution de l'ester obtenu au Stade B (2,18 g, 3,31 mmol) dans du méthanol (20 ml) et du tétrahydrofurane (10 ml) et l'on agite la suspension à 50°C pendant 7 heures. Le mélange réactionnel est refroidi jusqu'à la température ambiante et concentré et le résidu est dissous dans de l'acétate d'éthyle, séché sur sulfate de magnésium, filtré et évaporé pour livrer un solide.
**LC/MS** (C₃₉H₃₉N₄O₅.Na) 645 [M+H]⁺ ; RT 1,77 (Méthode A) [masse de la molécule parente de type acide]

### Stade D : Chlorure de 1-[4-(benzyloxy)-5-méthoxy-2-[(3S)-3-[(4-méthylpipérazin-1-yl)méthyl]-1,2,3,4-tétrahydroisoquinoléine-2-carbonyl]phényl]-1H-indole-3-carbonyle

On ajoute une solution 2M de chlorure d'oxalyle dans du dichlorométhane (1,77 ml, 3,54 mmol), puis quelques gouttes de *N*,*N*-diméthylformamide à une solution du composé préparé au Stade C (1,18 g, 1,77 mmol) dans du dichlorométhane anhydre (15 ml) et l'on agite le mélange réactionnel sous azote à température ambiante pendant environ 16 h. Le solvant est éliminé pour donner un solide.
**LC/MS** (C₃₉H₃₉N₄O₄Cl) 659 [M+H]⁺ ; RT 1,85 [on observe de l'ester méthylique pour l'inactivation du méthanol]

### Stade E : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-N-cyclohexyl-N-phényl-1H-indole-3-carboxamide

On ajoute de la *N*-cyclohexylaniline (145 mg, 0,83 mmol) et de la pyridine (305 µl, 3,77 mmol) à une solution du chlorure d'acide obtenu au Stade D (500 mg, 0,75 mmol) dans du dichlorométhane anhydre (6 ml) refroidie jusqu'à 0°C sous azote, et on laisse le mélange réactionnel se réchauffer jusqu'à la température ambiante. On agite le mélange réactionnel pendant environ 16 h, après quoi on ajoute 70 mg supplémentaires de *N-*cyclohexylaniline (0,4 mmol) et l'on continue d'agiter le mélange réactionnel à température ambiante. Le mélange réactionnel est refroidi, dilué avec du dichlorométhane et lavé successivement à l'eau, avec du NaOH 2M, et à la saumure. Les phases organiques sont séchées sur sulfate de magnésium, filtrées et évaporées et le matériau brut est purifié par chromatographie sur gel de silice selon un gradient allant de dichlorométhane jusqu'à un mélange méthanol (5 %)/dichlorométhane pour livrer une gomme.
**LC/MS** (C₅₁H₅₅N₅O₄) 802 [M+H]⁺ ; RT 2,58 (Méthode A)

### Stade F : N-Cyclohexyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-phényl-1H-indole-3-carboxamide

On ajoute du formiate d'ammonium (150 mg, 2,2 mmol) et du palladium à 10 % sur carbone catalytique à une solution du composé obtenu au Stade E (180 mg, 0,22 mmol) dans du méthanol (10 ml). On chauffe le mélange réactionnel à reflux sous azote pendant 1 heure. Le mélange réactionnel est refroidi jusqu'à la température ambiante, filtré au travers de Célite, lavé au méthanol et concentré. Le matériau brut est purifié par chromatographie sur gel de silice selon un gradient allant de dichlorométhane jusqu'à un mélange méthanol (10 %)/ dichlorométhane pour livrer le produit sous forme d'un solide.
**LC/MS** (C₄₄H₄₉N₅O₄) 712 [M+H]⁺ ; RT 1,35 (Méthode B)

### Stade G : Dichlorhydrate de N-cyclohexyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-phéuyl-1H-iudole-3-carboxamide

On ajoute du HCl 2M dans de l'éther (0,35 ml, 0,7 mmol) à une solution du composé obtenu au Stade F (100 mg, 0,14 mmol) dans de l'alcool isopropylique (2 ml). On dilue la solution avec de l'éther (10 ml) et l'on agite la suspension résultante pendant 30 minutes, puis on la laisse se concentrer. Le résidu est trituré avec de l'éther, filtré, lavé avec une nouvelle quantité d'éther et le solide est séché sous vide.
**LC/MS** (C₄₄H₄₉N₅O₄) 712 [M+H]⁺ ; RT 2,37 (Méthode A)

### Masse haute résolution (ESI+) :

Formule empirique : C₄₄H₄₉N₅O₄
[M+H]⁺ calculé : 712,3857
[M+H]⁺ mesuré: 712,3828

### Exemple 188. 1-[(2S)-2-[(Benzyloxy)méthyl]-7-[(3S)-3-[(4-méthylpipérazin-1-yl)méthyl]-1,2,3,4-tétrahydroisoquinoléine-2-carbonyl]-2,3-dihydro-1,4-benzodioxin-6-yl]-N-(4-hydroxyphényl)-N-phényl-1H-indole-3-carboxamide

### Stade A : 2-Bromo-4,5-dihydroxybenzoate de méthyle

A une solution de 2-bromo-4,5-diméthoxybenzoate de méthyle (5,62 g, 20,43 mmol) dans du dichlorométhane (60 mL), refroidie à -78 °C, sous azote, est ajouté le tribromure de bore (1M, 11,62 mL, 122,6 mmol). On laisse le milieu réactionnel se réchauffer jusqu'à la à température ambiante, et on l'agite pendant une nuit. Le milieu réactionnel est versé sur 500 mL de méthanol froid sous agitation, puis il est concentré et réparti selon sa solubilité entre l'acétate d'éthyle et l'eau. Les phases organiques sont séchées sur sulfate de magnésium, filtrées et concentrées pour livrer un solide.
**LC/MS** (C₈H₇O₄Br) 247 [M+H]⁺; RT 1,82 (Méthode A)

### Stade B : (3S)-7-Bromo-3-(hydroxyméthyl)-2,3-dihydro-1,4-benzodioxine-6-carboxylate de méthyle

A une solution du composé obtenu au Stade A (1 g, 4,05 mmol) dans le *N,N-*diméthylformamide (16 mL) est ajouté le (2*R*)-oxiran-2-ylméthanesulfonate de 4-méthylbenzyle (1,11 g, 4,86 mmol), puis le carbonate de potassium (1,12 g, 8,1 mmol), et le milieu réactionnel est chauffé dans un appareil à micro-ondes à 120 °C pendant 20 minutes. Le milieu réactionnel est concentré et réparti selon sa solubilité entre l'acétate d'éthyle et l'eau, et les phases organiques sont séchées sur sulfate de magnésium, filtrées et concentrées. Le produit brut est purifié par chromatographie sur gel de silice dans l'iso-hexane, jusqu'à un mélange 1:1 acétate d'éthyle/iso-hexane pour livrer une huile.
**LC/MS** (C₁₁H₁₁O₅Br) no m/z observed; RT 1.62 (Méthode A)

### Stade C : (3S)-3-[(Benzyloxy)méthyl]-7-bromo-2,3-dihydro-1,4-benzodioxine-6-carboxylate de méthyle

A une solution du composé obtenu au Stade B (1,04 g, 3,44 mmol) dans le *N*,*N-*diméthylformamide (15 mL) sous azote, sont ajoutés l'hydrure de sodium 60% (165 mg, 4,13 mmol), et après 15 minutes, le bromure de benzyle (0,49 mL, 4,13 mmol), et le milieu réactionnel est agité à 50°C pendant 4 heures. Il est ensuite concentré et réparti selon sa solubilité entre l'acétate d'éthyle et l'eau. Les phases organiques sont séchées sur sulfate de magnésium, filtrées et concentrées. Le produit brut est purifié par chromatographie sur gel de silice dans l'iso-hexane, jusqu'à un mélange 7:1 acétate d'éthyle/iso-hexane pour livrer une huile.
**LC/MS** (C₁₈H₁₇O₅Br) 393,1 [M+H]⁺; RT 2,72 (Méthode A)

### Stade D : Acide (3S)-3-[(Benzyloxy)méthyl]-7-bromo-2,3-dihydro-1,4-benzodioxine-6-carboxylique

A une solution du composé obtenu au Stade C (0,5 g, 1,28 mmol) dissous dans un mélange méthanol/tétrahydrofurane (1,5 mL:1,5 mL) est ajouté du NaOH 2M (3 mL, 6 mmol), et le milieu réactionnel est agité à 50°C pendant 2 heures. Le milieu réactionnel est concentré, dilué dans de l'eau et acidifié avec de l'HCl 2M, extrait avec de l'acétate d'éthyle, séché sur sulfate de magnésium, filtré et concentré pour livrer un solide.
**LC/MS** (C₁₇H₁₅O₅Br) 379 [M+H]⁺; RT 2,48 (Méthode A)

### Stade E : 1-[(2S)-2-[(Benzyloxy)méthyl]-7-[(3S)-3-[(4-méthylpipérazin-1-yl)méthyl]-1,2,3,4-tétrahydroisoquinoléine-2-carbonyl]-2,3-dihydro-1,4-benzodioxin-6-yl]-1H-indole-3-carboxylate de méthyle

Le composé obtenu au Stade D est traité selon la procédure du Stade A de la Préparation 3d, et chauffé dans un appareil à micro-ondes à 130°C pendant 40 minutes, puis purifié par chromatographie sur gel de silice selon un gradient allant de dichlorométhane jusqu'à un mélange méthanol (5%)/dichlorométhane. Le composé obtenu est ensuite traité selon la procédure du Stade C de l'Exemple 166 en utilisant le composé de la Préparation 3', et purifié par chromatographie sur gel de silice selon un gradient allant de dichlorométhane jusqu'à un mélange méthanol (5%)/dichlorométhane pour livrer une huile.
**LC/MS** (C₄₂H₄₄N₄O₆) 701,4 [M+H]⁺; RT 2,42 (Méthode A)

### Stade F : Acide 1-[(2S)-2-[(benzyloxy)méthyl]-7-[(3S)-3-[(4-méthylpipérazin-1-yl)méthyl]-1,2,3,4-tétrahydroisoquinoléine-2-carbonyl]-2,3-dihydro-1,4-benzodioxin-6-yl]-1H-indole-3-carboxylique

Le composé obtenu au Stade E (301 mg, 0,43 mmol) dans du THF (1,5 mL) et du méthanol (1,5 mL) est agité avec du NaOH 2M (3 mL, 6 mmol), puis chauffé à 50 °C pendant une nuit. Le milieu réactionnel est concentré, repris dans de l'eau et acidifié avec de l'HCl 2M jusqu'à pH=4, et le produit est extrait avec de l'acétate d'éthyle. Les phases organiques sont séchées sur sulfate de magnésium, filtrées et concentrées pour livrer un solide.
**LC/MS** (C₄₁H₄₂N₄O₆) 697,3 [M+H]⁺; RT 2,3 (Méthode A)

### Stade G : 1-[(2S)-2-[(Benzyloxy)méthyl]-7-[(3S)-3-[(4-méthylpipérazin-1-yl)méthyl]-1,2,3,4-tétrahydroisoquinoléine-2-carbonyl]-2,3-dihydro-1,4-benzodioxin-6-yl]-N-{4-[(tert-butyldiméthylsilyl)oxy]phényl}-N-phényl-1H-indole-3-carboxamide

Le composé obtenu au Stade F (207 mg, 0,3 mmol) dans du dichlorométhane (5 mL) est refroidi à 0 °C, sous azote. On y additionne du chlorure d'oxalyle (2M, 0,45 mL, 0,9 mmol) et 1 goutte de *N*,*N*-diméthylformamide. Après 3 heures, le milieu réactionnel est concentré, puis co-évaporé deux fois de plus avec du dichlorométhane. Le résidu est remis en suspension dans du dichlorométhane (5 mL) et agité sous azote. On y additionne une solution de 4-[(*tert*-butyldiméthylsilyl)oxy]-*N*-phénylaniline (135 mg, 0,45 mmol) et de la pyridine (0,036 mL, 0,45 mmol) dans un minimum de dichlorométhane, et le milieu réactionnel est agité pendant une nuit. La réaction, incomplète, est inactivée avec du NaOH 2M, et la phase organique est séparée à l'aide d'un séparateur de phase, puis concentrée, chargée sur une colonne PE-AX, puis éluée avec du dichlorométhane suivi d'un mélange acide formique (5%)/dichlorométhane. Le matériau obtenu est soumis à une distillation azéotropique avec du toluène, puis engagé au stade suivant sans autre purification.
**LC/MS** (C₅₉H₆₅N₅O₆Si) 968,5 [M+H]⁺; RT 2,81 (Méthode A)

### Stade H : 1-[(2S)-2-[(Benzyloxy)méthyl)-7-[(3S)-3-[(4-méthylpiperazin-1-yl)méthyl)-1,2,3,4-tétrahydroisoquinoléine-2-carbonyl]-2,3-dihydro-1,4-benzodioxin-6-yl]-N-(4-hydroxyphéuyl)-N-phényl-1H-indole-3-carboxamide

Au composé obtenu au Stade G (103 mg, 0,11 mmol) dans du THF (3 mL) est ajouté de l'éthylène diamine (21,4 µL, 0,32 mmol) et du TBAF (1M dans le tétrahydrofurane, 0,32 mL, 0,32 mmol), et le milieu réactionnel est chauffé dans un appareil à micro-ondes à 120 °C pendant 20 minutes. Le milieu réactionnel est concentré et réparti selon sa solubilité entre l'acétate d'éthyle et une solution saturée de bicarbonate de sodium, puis les phases organiques sont séchées sur sulfate de magnésium, filtrées et concentrées. Le produit brut est purifié par chromatographie HPLC préparative.
**LC/MS** (C₅₃H₅₁N₅O₆) 854,4 [M+H]⁺; RT 1,35 (Méthode B)

### Exemple 189. 1-{3-Chloro-4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-N-phényl-1H-indole-3-carboxamide

Le mode opératoire est le même que dans le procédé du Stade A de l'Exemple 131, en remplaçant le composé de la Préparation 1' par le composé de la Préparation 3', et en remplaçant le composé de la Préparation 3d par le composé de la Préparation 3n. La déprotection se fait selon le mode opératoire du Stade B de l'Exemple 155.
**LC/MS** (C₄₄H₄₂N₅O₅Cl) 756,3 [M+H]⁺ ; RT 2,14 (Méthode A)

### Masse haute résolution (ESI+) :

Formule empirique : C₄₄H₄₂N₅O₅Cl
[M+H]⁺ calculé : 756,2947
[M+H]⁺ mesuré : 756,2948

### Exemple 190. Dichlorhydrate de N-(4-fluorophényl)-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-phényl-1H-indole-3-carboxamide

Le mode opératoire est le même que dans le procédé de l'Exemple 164, en remplaçant le composé issu de la Préparation 3h par le composé issu de la Préparation 3u.
**LC/MS** (C₄₄H₄₂N₅O₄F) 724 [M+H]⁺ ; RT 2,26 (Méthode A)

### Masse haute révolution (ESI+) :

Formule empirique : C₄₄H₄₂N₅O₄F
[M+H]⁺ calculé : 724,3294
[M+H]⁺ mesuré: 724,3301

### Exemple 191. Dichlorhydrate de N,N-dibutyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-1H-indole-3-carboxamide

Le mode opératoire est le même que dans le procédé de l'Exemple 187, en remplaçant la N-cyclohexylaniline utilisée au Stade E par de la dibutylamine.
**LC/MS** (C₄₀H₅₁N₅O₄) 666 [M+H]⁺ ; RT 2,31 (Méthode A)

### Masse haute révolution (ESI+) :

Formule empirique : C₄₀H₅₁N₅O₄
[M+H]⁺ calculé: 666,4014
[M+H]⁺ mesuré : 666,3987

### Exemple 192. N,N-Dibutyl-1-{2-[(3S)-3-[(4-méthylpipérazin-1-yl)méthyl]-1,2,3,4-tétrahydroisoquinoléine-2-carbonyl]-4-nitrophényl}-1H-indole-3-carboxamide

On ajoute le composé de la Préparation 3' (173 mg, 0,7 mmol), de la DIPEA (0,22 ml, 1,28 mmol) et du HBTU (291 mg, 0,77 mmol) au composé de la Préparation 3s (280 mg, 0,64 mmol) dans du *N*,*N*-diméthylformamide et l'on agite le mélange réactionnel à température ambiante pendant 16 heures. Le mélange réactionnel est concentré sous vide, dilué avec une solution aqueuse saturée de NaHCO₃ et extrait avec de l'acétate d'éthyle. Les extraits organiques sont lavés successivement avec une solution aqueuse saturée de NaHCO₃ et à la saumure, séchés sur sulfate de magnésium et concentrés sous vide. Le matériau brut est chargé à sec sur une colonne de silice de 20 g et élué avec de l'iso-hexane, puis de l'acétate d'éthyle, puis un mélange 19:1, puis 9:1 acétate d'éthyle:méthanol pour livrer le produit.
**LC/MS** (C₃₉H₄₈N₆O₄) 665 [M+H]⁺ ; RT 2,41 (Méthode A)

### Masse haute révolution (ESI+) :

Formule empirique : C₃₉H₄₈N₆O₄
[M+H]⁺ calculé : 665,3810
[M+H]⁺ mesuré: 665,3817

### Exemple 193. 1-{4-Hydroxy-5-méthoxy-2-[(3S)-3-[(4-méthylpipérazin-1-yl)méthyl]-1,2,3,4-tétrahydroisoquinoléine-2-carbonyl]phényl}-N-(4-méthylphényl)-N-phényl-1H-indole-3-carboxamide

Le mode opératoire est le même que dans le procédé du Stade A de l'Exemple 131, en remplaçant le composé de la Préparation 1' par le composé de la Préparation 3', et en remplaçant le composé de la Préparation 3d par le composé de la Préparation 3v. La déprotection se fait selon le mode opératoire du Stade B de l'Exemple 155.
**LC/MS** (C₄₅H₄₅N₅O₄) 720 [M+H]⁺ ; R.T 2,28 (Méthode A)

### Masse haute révolution (ESI+) :

Formule empirique : C₄₅H₄₅N₅O₄
[M+H]⁺ calculé : 720,3544
[M+H]⁺ mesuré : 720,3520

### Exemple 194. Dichlorhydrate de N-butyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-phényl-1H-indole-3-carboxamide

Le mode opératoire est le même que dans le procédé de l'Exemple 187, en remplaçant la *N-*cyclohexylaniline utilisée au Stade E par de la *N*-butylaniline.
**LC/MS** (C₄₂H₄₇N₅O₄.2HCl) 686,4 [M+H]⁺ ; RT 2,28 (Méthode A)

### Masse haute révolution (ESI+) :

Formule empirique : C₄₂H₄₇N₅O₄
[M+H]⁺ calculé : 686,3701
[M+H]⁺ mesuré : 686,3672

### Exemple 195. 5-Fluoro-N-(4-hydroxyphényl)-1-{6-[(3S)-3-[(4-méthylpipérazin-1-yl)méthyl]-1,2,3,4-tétrahydroisoquinoléine-2-carbonyl]-2H-1,3-benzodioxol-5-yl}-N-phényl-1H-indole-3-carboxamide

Le mode opératoire est le même que dans le procédé du Stade A de l'Exemple 131, en remplaçant le composé de la Préparation 1' par le composé de la Préparation 3', et en remplaçant le composé de la Préparation 3d par le composé de la Préparation 3o. La déprotection se fait selon le mode opératoire du Stade B de l'Exemple 155.
**LC/MS** (C₄₄H₄₀N₅O₅F) 738 [M+H]⁺ ; RT 2,22 (Méthode A)

### Masse haute révolution (ESI+) :

Formule empirique : C₄₄H₄₀N₅O₅F
[M+H]⁺ calculé : 738,3086
[M+H]⁺ mesuré : 738,3066

### Exemple 196. 1-{4-Amino-2-[(3S)-3-[(4-méthylpipérazin-1-yl)méthyl]-1,2,3,4-tétrahydroisoquinoléine-2-carbonyl]phényl}-N,N-dibutyl-1H-indole-3-carboxamide

On ajoute du palladium à 10 % sur carbone à une solution du composé obtenu à l'Exemple 192 (294 mg, 0,44 mmol) dans du tétrahydrofurane (15 ml), dégazée avec de l'azote, puis on agite le tout par oscillation sous une atmosphère d'hydrogène à température ambiante pendant 20 heures, après quoi l'on traite le mélange réactionnel avec une nouvelle quantité de palladium à 10 % sur carbone et on le réchauffe jusqu'à 45°C pendant 4 heures sous une atmosphère d'hydrogène. Le mélange réactionnel est refroidi jusqu'à la température ambiante, filtré au travers de Célite, qui est ensuite lavée au tétrahydrofurane, et les phases organiques combinées sont concentrées sous vide. Le matériau brut est chargé à sec sur une colonne de silice de 20 g et élué avec du dichlorométhane, puis un mélange 19:1, 15:1, puis 9:1 dichlorométhane:méthanol, puis un mélange 19:1, puis 15:1 acétate d'éthyle:ammoniaque 7M dans du méthanol pour livrer le produit.
**LC/MS** (C₃₉H₅₀N₆O₂) 635 [M+H]⁺ ; RT 2,31 (Méthode A)

### Masse haute résolution (ESI+) :

Formule empirique : C₃₉H₅₀N₆O₂
[M+H]⁺ calculé : 635,4068
[M+H]⁺ mesuré : 635,4056

### Exemple 197. 6-Fluoro-N-(4-hydroxyphényl)-1-{6-[(3S)-3-[(4-méthylpipérazin-1-yl)méthyl]-1,2,3,4-tétrahydroisoquinoléine-2-carbonyl]-2H-1,3-benzodioxol-5-yl}-N-phényl-1H-indole-3-carboxamide

On procède selon le mode opératoire du Stade A de la Préparation 2a, en utilisant le 6-fluoro-1*H*-indole-3-carboxylate de méthyle, puis selon le mode opératoire du Stade B, la réaction étant chauffée à 50 °C, suivi du mode opératoire du Stade C. Le composé obtenu est ensuite traité selon le mode opératoire de la Préparation 3a en utilisant l'acide 6-bromo-2H-1,3-benzodioxole-5-carboxylique, et chauffé dans un appareil à micro-ondes à 130 °C pendant 2 heures, puis purifié par chromatographie sur gel de silice selon un gradient allant de dichlorométhane jusqu'à un mélange méthanol (10%)/dichlorométhane. Le composé est ensuite traité selon le mode opératoire du Stade C de l'Exemple 166 en utilisant le composé de la Préparation 3', et purifié par chromatographie sur gel de silice dans le dichlorométhane, jusqu'à un mélange méthanol (5%)/dichlorométhane, et enfin déprotégé selon le mode opératoire du Stade D de l'Exemple 166 et purifié par chromatographie sur gel de silice dans le dichlorométhane, jusqu'à un mélange de méthanol (8%)/dichlorométhane.
**LC/MS** (C₄₄H₄₀N₅O₅F) 738 [M+H]⁺; RT 2,1 (Méthode A)

### Masse haute résolution (ESI+) :

Formule empirique: C₄₄H₄₀N₅O₅F
[M+H]+ calculé: 738.3086
[M+H]+ mesuré: 738.3077

### Exemple 198. 1-{4-Hydroxy-5-méthoxy-2-[(3S)-3-[(4-méthylpipérazin-1-yl)méthyl]-1,2,3,4-tétrahydroisoquinoléine-2-carbonyl]phényl}-N-(1H-indol-5-yl)-N-phényl-1H-indole-3-carboxamide

### Stade A : 5-{N-Phényl-1-[4-(benzyloxy)-5-méthoxy-2-[(3S)-3-[(4-méthylpipérazin-1-yl)méthyl]-1,2,3,4-tétrahydroisoquinoléine-2-carbonyl]phényl]-1H-indole-3-amido}-1H-indole-1-carboxylate de tert-butyle

Le mode opératoire est le même que dans le procédé de l'Exemple 204, en remplaçant la 4-[(*tert*-butyldiméthylsilyl)oxy]-*N*-cyclohexylaniline utilisée au Stade B par le 5-(phénylamino)-1*H*-indole-1-carboxylate de *tert*-butyle de la Préparation 3".
**LC/MS** (C₅₈H₅₈N₆O₆) 935 [M+H]⁺; RT 2,64 (Méthode A)

### Stade B : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-N-(1H-indol-5-yl)-N-phényl-1H-indole-3-carboxamide

On ajoute de l'acide trifluoroacétique (2 ml) à une solution du composé obtenu au Stade A (2,18 g, 2,33 mmol) dans du dichlorométhane (20 ml). On agite le mélange réactionnel à température ambiante pendant 5 heures, puis on le dilue avec du dichlorométhane et de l'eau, avant de l'alcaliniser avec du NaOH 2M aqueux. La phase organique est séparée et lavée à la saumure, séchée sur sulfate de magnésium, filtrée et évaporée. Le matériau brut est purifié par chromatographie sur gel de silice selon un gradient allant de dichlorométhane jusqu'à un mélange méthanol (5 %)/dichlorométhane pour livrer une mousse.
**LC/MS** (C₅₃H₅₀N₆O₄) 835 [M+H]⁺ ; RT. 2,41 (Méthode A)

### Stade C : 1-{4-Hydroxy-5-méthoxy-2-[(3S)-3-[(4-méthylpipérazin-1-yl)méthyl]-1,2,3,4-tétrahydroisoquinoléine-2-carbonyl]phényl}-N-(1H-indol-5-yl)-N-phényl-1H-indole-3-carboxamide

On ajoute du formiate d'ammonium (775 mg, 12,3 mmol) et du palladium à 10 % sur carbone (catalytique) à une solution du composé obtenu au Stade B (1,03 g, 1,23 mmol) dans du méthanol (15 ml) et l'on chauffe le mélange réactionnel jusqu'à 80°C sous azote pendant 6 heures, après quoi on ajoute une nouvelle portion de formiate d'ammonium (775 mg, 12,3 mmol) et de palladium à 10 % sur carbone (catalytique) et l'on continue de chauffer le mélange réactionnel pendant 16 h. On laisse le mélange réactionnel refroidir jusqu'à la température ambiante, puis il est filtré au travers de Célite, lavé avec du méthanol et le solvant est éliminé par évaporation. Le matériau brut est dissous dans du dichlorométhane et lavé à l'eau, puis les phases organiques sont séchées sur sulfate de magnésium, filtrées et évaporées. Le produit brut est purifié par chromatographie sur gel de silice selon un gradient allant de dichlorométhane jusqu'à un mélange méthanol (10 %)/ammoniaque/dichlorométhane.
**LC/MS** (C₄₆H₄₄N₆O₄) 745,4 [M+H]⁺ ; RT 2,19

### Masse haute résolution (ESI+) :

Formule empirique : C₄₆H₄₄N₆O₄
[M+H]⁺ calculé: 745.3497
[M+H]⁺ mesuré: 745.3465

### Exemple 199. N,N-Dibutyl-1-{2-[(3S)-3-[(4-méthylpipérazin-1-yl)méthyl]-1,2,3,4-tétrahydroisoquinoléine-2-carbonyl]-4-[2-(phénylsulfanyl)acétamido]phényl}-1H-indole-3-carboxamide

On ajoute de la DIPEA (45 µl, 0,26 mmol), à une solution du composé obtenu à l'Exemple 196 (82 mg, 0,13 mmol) dans du dichlorométhane (4 ml), refroidie jusqu'à 0°C sous azote, puis l'on ajoute lentement du chlorure de 2-(phénylsulfanyl)acétyle (21 µl, 0,14 mmol). Une fois l'addition terminée, on agite le mélange réactionnel à 0°C pendant 15 minutes, puis on le réchauffe jusqu'à la température ambiante et on l'agite pendant 3 heures. On traite le mélange réactionnel avec une nouvelle quantité de DIPEA (45 µl) et de chlorure d'acide (21 µl) et l'on agite le tout pendant 16 heures de plus. Le mélange réactionnel est dilué avec une solution aqueuse à 5 % d'hydroxyde d'ammonium et extrait avec de l'acétate d'éthyle. Les extraits organiques sont ensuite lavés successivement avec une solution aqueuse à 5 % d'hydroxyde d'ammonium et à la saumure, puis séchés sur sulfate de magnésium et concentrés sous vide. Une purification a lieu par HPLC préparative (pH9).
**LC/MS** (C₄₇H₅₆N₆O₃S) 785 [M+H]⁺ ; RT 2,5 (Méthode A)

### Masse haute résolution (ESI+) :

Formule empirique : C₄₇H₅₆N₆O₃S
[M+H]⁺ calculé : 785,4207
[M+H]⁺ mesuré : 785,4183

### Exemple 200. N,N-Dibutyl-1-{2-[(3S)-3-[(4-méthylpipérazin-1-yl)méthyl]-1,2,3,4-tétrahydroisoquinoléine-2-carbonyl]-4-(2-phénoxyacétamido)phényl}-1H-indole-3-carboxamide

Le mode opératoire est le même que dans le procédé de l'Exemple 199, en remplaçant le chlorure de 2-(phénylsulfanyl)acétyle par du chlorure de 2-phénoxyacétyle.
**LC/MS** (C₄₇H₅₆N₆O₄) 769 [M+H]⁺ ; RT 2,49 (Méthode A)

### Masse haute révolution (ESI+) :

Formule empirique : C₄₇H₅₆N₆O₄
[M+H]⁺ calculé : 769,4436
[M+H]⁺ mesuré : 769,4446

### Exemple 201. Dichlorhydrate de N-(4-fluorophényl)-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-1H-indole-3-carboxamide

Le mode opératoire est le même que dans le procédé de l'Exemple 204, en remplaçant la 4-[(*tert*-butyldiméthylsilyl)oxy]-*N*-cyclohexylaniline utilisée au Stade B par la 4-[(*tert-*butyldiméthylsilyl)oxy]-*N*-(4-fluorophényl)aniline.
**LC/MS** (C₄₄H₄₂N₅O₅F) 740 [M+H]⁺; RT 2,15 (Méthode A)

### Masse haute révolution (ESI+):

Formule empirique: C₄₄H₄₂N₅O₅F
[M+H]⁺ calculé: 740,3243
[M+H]⁺ mesuré: 740,3213

### Exemple 202. 1-{4-Hydroxy-5-méthoxy-2-[(31S)-3-[(4-méthylpipérazin-1-yl)méthyl]-1,2,3,4-tétrahydroisoquinoléine-2-carbonyl]phényl}-N-(4-hydroxyphényl)-N-(1H-indol-5-yl)-1H-indole-3-carboxamide

### Stade A : 5-(N-{4-[(tert-Butyldiméthylsilyl)oxy]phényl}-1-[4-(benzyloxy)-5-méthoxy-2-[(3S)-3-[(4-méthylpipérazin-1yl)méthyl]-1,2,3,4-tétrahydroisoquinoléine-2-carbonyl]phényl]-1H-indole-3-amido)-1H-indole-1-carboxylate de tert-butyle

Le mode opératoire est le même que dans le procédé de l'Exemple 204, en remplaçant la 4-[(*tert*-butyldiméthylsilyl)oxy]-*N*-cyclohexylaniline utilisée au Stade B par le 5-({4-[(*tert-*butyldiméthylsilyl)oxy]phényl}amino)-1*H*-indole-1-carboxylate de *tert*-butyle de la Préparation 5".
**LC/MS** (C₆₄H₇₂N₆O₇Si) 533 [M+2H]²⁺; RT 2,85 (Méthode A)

### Stade B : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-N-(4-hydroxyphényl)-N-(1H-indol-5-yl)-1H-indole-3-carboxamide

On ajoute de l'hydroxyde de potassium (1M, 2,1 ml, 2,1 mmol) à une solution du matériau obtenu au Stade A (1,49 g, 1,4 mmol) dans du méthanol (20 ml) et l'on agite le mélange réactionnel à température ambiante pendant *ca* 16 h. Le mélange réactionnel est concentré et le résidu repris dans du dichlorométhane et lavé au HCl dilué. Les phases organiques sont séchées sur sulfate de magnésium, filtrées et évaporées. Le résidu est dissous à nouveau dans du dichlorométhane (20 ml) et l'on y ajoute de l'acide trifluoroacétique (3 ml). Le mélange réactionnel est agité à température ambiante pendant *ca* 72 h. Le résidu est repris dans du dichlorométhane et neutralisé avec du NaOH 2M aqueux. Les phases organiques sont séparées et lavées à l'eau, séchées sur sulfate de magnésium, filtrées et évaporées. Le produit brut est purifié par chromatographie sur gel de silice selon un gradient allant de dichlorométhane jusqu'à un mélange méthanol (10 %)/ dichlorométhane pour livrer le produit sous forme d'un solide.
**LC/MS** (C₅₃H₅₀N₆O₅) 851 [M+H]⁺; RT 2,31 (Méthode A)

### Stade C : 1-{4-Hydroxy-5-méthoxy-2-[(3S)-3-[(4-méthylpipérazin-1-yl)méthyl)-1,2,3,4-tétrahydroisoquinoléine-2-carbonyl]phényl}-N-(4-hydroxyphényl)-N-(1H-indol-5-yl)-1H-indole-3-carboxamide

Le mode opératoire est le même que dans le procédé du Stade D de l'Exemple 204, et la purification est réalisée par HPLC préparative.
**LC**/**MS** (C₄₆H₄₄N₆O₅) 761,2 [M+H]⁺; RT 1,12 (Méthode B)

### Masse haute résolution (ESI+):

Formule empirique: C₄₆H₄₄N₆O₅
[M+H]⁺ calculé: 761.3446
[M+H]⁺ mesuré: 761.3429

### Exemple 203. Dichlorhydrate de N-butyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-1H-indole-3-carboxamide

Le mode opératoire est le même que dans le procédé de l'Exemple 204, en remplaçant la 4-[(*tert*-butyldiméthylsilyl)oxy]-*N*-cyclohexylaniline de la Préparation 4a par la 4-(benzyloxy)-*N*-butylaniline de la Préparation 4b.
**LC/MS** (C₄₂H₄₇N₅O₅) 702,4 [M+H]⁺; RT 2,19 (Méthode A)

### Masse haute révolution (ESI+):

Formule empirique: C₄₂H₄₇N₅O₅
[M+H]⁺ calculé: 702.3650
[M+H]⁺ mesuré: 702.3623

### Exemple 204. Dichlorhydrate de N-cyclohexyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-1H-indole-3-carboxamide

### Stade A : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-1H-indole-3-carboxylate de lithium

On ajoute du LiOH (1M, 7,28 ml, 7,28 mmol) à une solution de l'ester obtenu au Stade B de l'Exemple 187 (4 g, 6,07 mmol) dans du dioxane (30 ml) et l'on chauffe le mélange réactionnel à reflux pendant *ca* 16 h. On ajoute une nouvelle quantité de LiOH (1M, 9,1 ml) au mélange réactionnel, puis on le chauffe à reflux pendant *ca* 16 h. On laisse le mélange réactionnel refroidir jusqu'à la température ambiante, le solvant est éliminé sous vide et le résidu est soumis à une distillation azéotropique avec du toluène pour livrer un solide qui est utilisé sans autre purification pour l'étape suivante.
**LC/MS** (C₃₉H₃₉N₄O₅.Li) 645 [M+H]⁺; RT 2,23 (Méthode A) [masse de la molécule parente de type acide]

### Stade B : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-N-{4-[(tert-butyldiméthylsilyl)oxy]phényl}-N-cyclohexyl-1H-iudole-3-carboxamide

On ajoute du chlorure de thionyle (0,22 ml, 3,08 mmol) à une solution du sel de lithium obtenu au Stade A (1 g, 1,54 mmol) dans du dichlorométhane anhydre (16 ml) refroidie jusqu'à 0°C sous azote. On laisse le mélange réactionnel se réchauffer jusqu'à la température ambiante et on l'agite pendant 2 heures avant d'ajouter davantage de chlorure de thionyle (0,11 ml, 1,54 mmol), puis on l'agite pendant *ca* 16 h. Le solvant est éliminé et le résidu mis à nouveau en suspension dans du dichlorométhane et évaporé une fois encore. Une co-évaporation avec du dichlorométhane est répétée deux fois de plus avant une distillation azéotropique avec du toluène, pour obtenir un solide qui est dissous dans du dichlorométhane anhydre (10 ml). On ajoute une solution de l'aniline obtenue dans la Préparation 4a (640 mg, 2,07 mmol) et de pyridine (0,2 ml, 2,31 mmol) dans du dichlorométhane (6 ml) et l'on agite la solution à température ambiante sous azote pendant 4 heures. Le mélange réactionnel est dilué avec du dichlorométhane et lavé séquentiellement à l'eau, avec une solution saturée de bicarbonate de sodium et à la saumure. Les phases organiques sont séchées sur sulfate de magnésium, filtrées et évaporées et le matériau brut est purifié par chromatographie sur gel de silice selon un gradient allant de dichlorométhane jusqu'à un mélange méthanol (5 %)/dichlorométhane pour livrer le produit sous forme d'une mousse.
**LC/MS** (C₅₇H₆₉N₅O₅Si) 932 [M+H]⁺; RT 2,79 (Méthode A)

### Stade C : 1-[4-(Benzyloxy)-5-méthoxy-2-[(3S)-3-[(4-méthylpipérazin-1-yl)méthyl]-1,2,3,4-tétrahydroisoquinoléine-2-carbonyl]phényl]-N-cyclohexyl-N-(4-hydroxyphényl)-1H-indole-3-carboxamide

On ajoute de l'hydroxyde de potassium aqueux (1M, 1,22 ml, 1,22 mmol) à une solution du matériau obtenu au Stade B (758 mg, 0,81 mmol) dans du méthanol (10 ml) et l'on agite le mélange réactionnel à température ambiante pendant 3 heures. Le mélange réactionnel est concentré et la phase aqueuse est (légèrement) acidifiée avec du HCl 2M aqueux, et le produit est extrait dans de l'acétate d'éthyle, séché sur sulfate de magnésium, filtré et évaporé. Le produit brut est utilisé sans autre purification pour l'étape suivante.
**LC/MS** (C₅₁H₅₅N₅O₅) 818 [M+H]⁺; RT 2,39 (Méthode A)

### Stade D : N-Cyclohexyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-1H-indole-3-carboxamide

On ajoute du formiate d'ammonium (510 mg, 8,1 mmol) et du palladium à 10 % sur carbone (catalytique) à une solution du composé obtenu au Stade C (665 mg, 0,81 mmol) dans du méthanol (15 ml) et l'on chauffe le mélange réactionnel à reflux sous azote pendant 2 jours, durée au cours de laquelle on ajoute 2 autres portions de formiate d'ammonium (510 mg, 8,1 mmol) et de palladium à 10 % sur carbone (catalytique). On laisse le mélange réactionnel refroidir jusqu'à la température ambiante. Il est ensuite filtré au travers de Célite, lavé au méthanol et le solvant est éliminé par évaporation. Le matériau brut est purifié par HPLC préparative (pH4), puis le matériau purifié est dissous dans du dichlorométhane et lavé à l'eau et avec une solution saturée de bicarbonate de sodium, puis séché sur sulfate de magnésium.

### Stade E : Dichlorhydrate de N-cyclohexyl-1-{4-hydroxy-5-méthoxy-2-[(3S)-3-[(4-méthylpipérazin-1-yl)méthyl]-1,2,3,4-tétrahydroisoquinoléine-2-carbonyl]phényl}-N-(4-hydroxyphényl)-1H-indole-3-carboxamide

On ajoute du HCl dans l'éther (2M, 1,13 mL, 2,25 mmol) à une solution du composé obtenu au Stade D (330 mg, 0,45 mmol) dans l'isopropanol (5 mL). La solution ainsi obtenue est diluée dans l'éther (5 mL) et agitée à température ambiante pendant 20 minutes. Le solide est ensuite filtré, lavé avec de l'éther et séché sous vide.
**LC/MS** (C₄₄H₄₉N₅O₅) 728 [M+H]⁺; RT 2,21 (Méthode A)

### Masse haute résolution (ESI+):

Formule empirique: C₄₄H₄₉N₅O₅
[M+H]⁺ calculé: 728.3806
[M+H]⁺ mesuré: 728.3786

### Exemple 205. N-(4-Hydroxyphényl)-1-{5-méthoxy-2-[(3S)-3-[(4-méthylpipérazin-1-yl)méthyl]-1,2,3,4-tétrahydroisoquinoléine-2-carbonyl]phényl}-N-(1-méthyl-1H-indol-5-yl)-1H-indole-3-carboxamide

### Stade A : Acide 4-méthoxy-2-[3-(méthoxycarbonyl)-1H-indol-1yl]benzoïque

De l'indole-3-carboxylate de méthyle (2 g, 11,42 mmol), du carbonate de potassium (2,32 g, 16,8 mmol) et l'acide 2-iodo-4-méthoxybenzoïque (3,17 g, 11,42 mmol) sont combinés sous azote et mis en suspension dans du *N*,*N*-diméthylformamide (30 ml), dégazés avec de l'azote, puis l'on ajoute de l'iodure de cuivre (217 mg, 1,14 mmol). On chauffe le mélange réactionnel jusqu'à 80°C sous azote pendant *ca* 16h. On laisse le mélange réactionnel refroidir jusqu'à la température ambiante. Il est ensuite dilué avec de l'eau et acidifié avec du HCl 2M aqueux. Les phases organiques sont extraites dans du dichlorométhane et lavées à l'eau, séchées sur sulfate de magnésium, filtrées et évaporées. Le matériau brut est purifié par chromatographie sur gel de silice selon un gradient allant d'iso-hexane à un mélange 1:1 iso-hexane/acétate d'éthyle pour livrer le produit sous forme d'une huile.
**LC/MS** (C₁₈H₁₅NO₅) 326 [M+H]⁺; RT 2,36 (Méthode A)

### Stade B : 1-{5-Méthoxy-2-[(3S)-3-[(4-méthylpipérazin-1-yl)méthyl)-1,2,3,4-tétrahydroisoquinoléine-2-carbonyl]phényl}-1H-indole-3-carboxylate de méthyle

On ajoute de la DIPEA (8,25 ml, 47,35 mmol) et du HBTU (2,6 g, 7 mmol), puis le composé de la Préparation 3' (3 g, 8,52 mmol) à une solution de l'acide obtenu au Stade A (3,08 g, 9,47 mmol) dans du *N*,*N*-diméthylformamide (30 ml) et l'on agite le mélange réactionnel à température ambiante pendant *ca* 16h. Le mélange réactionnel est dilué avec du dichlorométhane et lavé successivement à l'eau et avec une solution saturée de bicarbonate de sodium. Les phases organiques sont séchées sur sulfate de magnésium, filtrées et évaporées et le matériau brut est purifié par chromatographie sur gel de silice selon un gradient allant de dichlorométhane jusqu'à un mélange méthanol (10 %)/dichlorométhane pour livrer le produit sous forme d'une huile.
**LC/MS** (C₃₃H₃₆N₄O₄) 553 [M+H]⁺; RT 2,14 (Méthode A)

### Stade C : 1-{5-Méthoxy-2-[(3S)-3-[(4-méthylpipérazin-1-yl)méthyl]-1,2,3,4-tétrahydroisoquinoléine-2-carbonyl]phényl}-1H-indole-3-carboxylate de lithium

Le mode opératoire est le même que dans le procédé du Stade A de l'Exemple 204 en remplaçant le 1-[4-(benzyloxy)-5-méthoxy-2-[(3*S*)-3-[(4-méthylpipérazin-1-yl)méthyl]-1,2,3,4-tétrahydroisoquinoléine-2-carbonyl]phényl]-1*H*-indole-3-carboxylate de méthyle par le composé du Stade B.
**LC/MS** (C₃₂H₃₃N₄O₄.Li) 539 [M+H]⁺; RT 2,04 (Méthode A) [masse pour la molécule parente de type acide]

### Stade D : Chlorure de 1-{5-méthoxy-2-[(3S)-3-[(4-méthylpipérazin-1-yl)méthyl]-1,2,3,4-tétrahydroisoquinoléine-2-carbonyl]phényl}-1H-indole-3-carbonyl

On ajoute du chlorure de thionyle (0,14 ml, 1,98 mmol) à une solution du sel de lithium obtenu au Stade C (540 mg, 0,99 mmol) dans du dichlorométhane anhydre (10 ml) refroidie jusqu'à 0°C sous azote. On laisse le mélange réactionnel se réchauffer jusqu'à la température ambiante et on l'agite pendant 5 heures avant d'ajouter une nouvelle quantité de chlorure de thionyle (0,035 ml, 0,5 mmol), puis on l'agite pendant *ca* 16 h. Le solvant est éliminé et le résidu est mis à nouveau en suspension dans du dichlorométhane et évaporé une fois encore. Une co-évaporation avec du dichlorométhane est répétée deux fois de plus avant une distillation azéotropique avec du toluène, pour obtenir un solide qui est utilisé sans autre purification pour l'étape suivante.
**LC**/**MS** (C₃₂H₃₃N₄O₃Cl) 553 [M+H]⁺; RT 2.13 (Méthode A) [on observe de l'ester méthylique obtenu par inactivation du méthanol]

### Stade E : N-{4-[(tert-Butyldiméthylsilyl)oxy]phényl}-1-{5-méthoxy-2-[(3S)-3-[(4-méthylpipérazin-1-yl)méthyl]-1,2,3,4-tétrahydroisoquinoléine-2-carbonyl]phényl}-N-(1-méthyl-1H-indol-5-yl)-1H-indole-3-carboxamide

On ajoute une solution de l'aniline obtenue dans la Préparation 4c (348 mg, 0,99 mmol) et de pyridine (0,13 ml, 1,5 mmol) dans du dichlorométhane (8 ml) à une solution du chlorure d'acide obtenu au Stade D (550 mg, 0,99 mmol) dans du dichlorométhane (10 ml) sous azote, et l'on agite le mélange réactionnel à température ambiante pendant *ca* 72 h. Le mélange réactionnel est dilué avec du dichlorométhane et lavé séquentiellement à l'eau, avec une solution saturée de bicarbonate de sodium et à la saumure. Les phases organiques sont séchées sur sulfate de magnésium, filtrées et évaporées et le matériau brut est purifié par chromatographie sur gel de silice selon un gradient allant de dichlorométhane jusqu'à un mélange méthanol (10 %)/ dichlorométhane pour livrer le produit sous forme d'un solide.
**LC/MS** (C₅₃H₆₀N₆O₄Si) 873 [M+H]⁺; RT 2,67 (Méthode A)

### Stade F : N-(4-Hydroxyphényl)-1-{5-méthoxy-2-[(3S)-3-[(4-méthylpipérazin-1-yl)méthyl]-1,2,3,4-tétrahydroisoquinoléine-2-carbonyl]phényl}-N-(1-méthyl-1H-indol-5-yl)-1H-indole-3-carboxamide

On ajoute de l'hydroxyde de potassium (1M, 0,78 ml, 0,78 mmol) à une solution du matériau obtenu au Stade E (451 mg, 0,52 mmol) dans du méthanol (10 ml) et l'on agite le mélange réactionnel à température ambiante toute une nuit. Le solvant est éliminé et le résidu est dissous dans du dichlorométhane et lavé à l'eau (plus quelques gouttes de HCl 2M), séché sur sulfate de magnésium, filtré et évaporé. Le produit brut est purifié par chromatographie sur gel de silice selon un gradient allant de dichlorométhane jusqu'à un mélange méthanol (10 %)/ dichlorométhane, puis trituré avec de l'éther, filtré et séché sous vide pour livrer une poudre.
**LC/MS** (C₄₇H₄₆N₆O₄) 759 [M+H]⁺; RT 2,2 (Méthode A)

### Masse haute révolution (ESI+) :

Formule empirique: C₄₇H₄₆N₆O₄
[M+H]⁺ calculé: 759.3653
[M+H]⁺ mesuré: 759.3628

### Exemple 206. Chlorhydrate de N-(4-hydroxyphényl)-5-méthyl-N-(1-méthyl-1H-pyrrolo[2,3-b]pyridin-5-yl)-1-(6-{[(3S)-3-(morpholin-4-ylméthyl)-3,4-dihydroisoquinoléin-2(1H)-yl]carbonyl}-1,3-benzodioxol-5-yl)-1H-pyrrole-3-carboxamide

***Microanalyse élémentaire : %mesuré (théorique)***
%C=66.28(66.27);%H=5.08(5.43);%N=10.70(11.04) ; %Cl-=5.41(4.66)

### Masse haute révolution (ESI+) :

Formule brute : C₄₂H₄₀N₆O₆
[M+H]⁺ calculé : 725.3082
[M+H]⁺ mesuré : 725.3089

### Exemple 207. Chlorhydrate de N-(4-hydroxyphényl)-N-(1-méthyl-1H-pyrrolo[2,3-b]pyridin-5-yl)-1-(6-{[(3S)-3-(morpholin-4-ylméthyl)-3,4-dihydroisoquinoléin-2(1H)-yl]carbonyl}-1,3-benzodioxol-5-yl)-4,5,6,7-tétrahydro-1H-indole-3-carboxamide

Le composé du titre est synthétisé selon le procédé de l'Exemple 12 en utilisant au Stade A l'acide obtenu à la Préparation 19, la (3S)-3-(4-morpholinylméthyl)-1,2,3,4-tétrahydroisoquinoléine obtenue à la Préparation 2', ainsi que la *N*-(4-{[*tert-*butyl(diméthyl)silyl]oxy}phényl)-1-méthyl-1*H*-pyrrolo[2,3-*b*]pyridin-5-amine obtenue à la Préparation 24".
***Microanalyse élémentaire : %mesuré (théorique)***
%C=67.26(67.45);%H=5.73(5.66);%N=10.22(10.49) ; %Cl-=4.70(4.42)

### Masse haute résolution (ESI+/FIA) :

Formule brute : C₄₅H₄₄N₆O₆
[M+H]⁺ calculé : 765.3395
[M+H]⁺ mesuré : 765.3398

### Exemple 208. 4-[(1-Méthyl-1H-pyrrolo[2,3-b]pyridin-5-yl){[1-(6-{[(3S)-3-(morpholin-4-ylméthyl)-3,4-dihydroisoquinoléin-2(1H)-yl]carbonyl}-1,3-benzodioxol-5-yl)-4,5,6,7-tétrahydro-1H-indol-3-yl]carbonyl}amino]phényl phosphate de disodium

### Stade A : 4-[(1-Méthyl-1H-pyrrolo(2,3-b)pyridin-5-yl){[1-(6-{[(3S)-3-(morpholin-4-ylméthyl)-3,4-dihydroisoquinoléin-2(1H)-yl]carbonyl}-1,3-benzodioxol-5-yl)-4,5,6,7-tétrahydro-1H-indol-3-yl]carbonyl}amino]phényl phosphate de dibenzyle

A une suspension de 80 mg d'hydrure de sodium (2 mmol) dans 8 mL de THF anhydre est ajouté par portions et à 0°C 719 mg (0,9 mmol) du composé de l'Exemple 207. Après 30 minutes d'agitation à 0°C et 30 min à température ambiante, le pyrophosphate de tétrabenzyle (580 mg ; 1 mmol) est ajouté à 0°C et le milieu réactionnel est agité une nuit à température ambiante. Après évaporation du solvant, le brut réactionnel est dilué au dichlorométhane (40 mL), lavé par une solution saturée de NaHCO₃, puis une solution saturée de NaCl. La phase organique est alors séchée sur MgSO₄, filtrée, concentrée à sec, et purifiée par chromatographie sur gel de silice (gradient CH₂Cl₂/MeOH). On obtient alors le produit du titre sous la forme d'un solide.

### Stade B : 4-[(1-Méthyl-1H-pyrrolo[2,3-b]pyridin-5-yl){[1-(6-{[(3S)-3-(morpholin-4-ylméthyl)-3,4-dihydroisoquinoléin-2(1H)-yl]carbonyl}-1,3-benzodioxol-5-yl)-4,5,6,7-tétrahydro-1H-indol-3-yl]carbonyl}amino]phényl phosphate de disodium

A une solution du produit obtenu au Stade A (395 mg ; 0,39 mmol) dans du méthanol (6 mL) est ajouté 40 mg de Pd/C à 10%, puis le milieu réactionnel est placé sous atmosphère d'hydrogène (1 bar) pendant 2 h. Apres filtration du catalyseur et concentration à sec, le brut réactionnel est solubilisé dans le méthanol (5 mL) et traité par 0.8 mL de soude 1N. Les solvants sont alors évaporés et le brut réactionnel est purifié par chromatographie sur phase OASIS® (gradient acétonitrile/H₂O) pour obtenir un solide.

### Exemple 209. Chlorhydrate de 1-(5-chloro-2-{[(3S)-3-(morpholin-4-ylméthyl)-3,4-dihydroisoquinoléin-2(1H)-yl]carbonyl}phényl)-N-(4-hydroxyphényl)-4,5-diméthyl-N-(1-méthyl-1H-pyrrolo[2,3-b]pyridin-5-yl)-1H-pyrrole-3-carboxamide

### Stade A : 1-(5-Chloro-2-{[(3S)-3-(morpholin-4-ylméthyl)-3,4-dihydroisoquinoléin-2(1H)-yl]carbonyl}phényl)-4,5-diméthyl-1H-pyrrole-3-carboxylate de méthyle

Le composé est obtenu suivant le protocole du Stade A de l'Exemple 8 en utilisant au Stade A l'acide obtenu à la Préparation 22 et la (3*S*)-3-(4-morpholinylméthyl)-1,2,3,4-tétrahydroisoquinoléine obtenue à la Préparation 2'.
**RMN ¹H** (400 MHz, dmso-d6) δ ppm : 9.85 (sl, OH), 7.77 (d, 1 H), 7.72 (d, 1 H), 7.63 (s, 1 H), 7.45-6.81 (m, 4 H), 6.88 (m, 1 H), 5.27 (m, 1 H), 4.51/4.28 (m, 2 H), 4/3.69 (m, 4 H), 3.61-3.01 (m, 6 H), 3.55 (m, 3 H), 2.57 (s, 2 H), 1.79 (m, 6 H)
***IR (ATR) cm-¹ :*** 1698 v -C=O ester aromatique, 1639 v -C=O amide

### Stade B : 1-(5-Chloro-2-{[(3S)-3-(morpholin-4-ylméthyl)-3,4-dihydroisoquinoléin-2(1H)-yl]carbonyl}phényl)-4,5-diméthyl-1H-pyrrole-3-carboxylate de lithium

Le composé est obtenu suivant le protocole du Stade B de l'Exemple 8 à partir du composé obtenu au stade précédent.
**RMN ¹H** (500 MHz, dmso-d6) δ ppm : 7.72-7.56 (m, 3 H), 7.66 (m, 1 H), 7.4-6.92 (m, 4 H), 4.85/3.72 (m, 1 H), 4.82/4.31/4.24/4.14 (m, 2 H), 3.5 (m, 4 H), 2.99/2.85/2.67/2.52 (m, 2 H), 2.62-1.94 (m, 6 H), 2.14/2.07/2.02/1.78/1.74 (m, 6 H)
***IR (ATR) cm¹ :*** 2575 v -OH, 1696-1670 v -C=O, 1626 v -C=O, 1595 v Ar, 1230-1180-1114 v -C-O-C, 867-833-743 v -CH-Ar

### Stade C : N-(4-{[tert-Butyl(diméthyl)silyl]oxy}phényl)-1-(5-chloro-2-{[(3S)-3-(morpholin-4-ylméthyl)-3,4-dihydroisoquinoléin-2(1H)-yl]carbonyl}phényl)-4,5-diméthyl-N-(1-méthyl-1H-pyrrolo[2,3-b]pyridin-5-yl)-1H-pyrrole-3-carboxamide

Le composé est obtenu suivant le protocole du Stade C de l'Exemple 8 en remplaçant le composé de la Préparation 1" par le composé de la Préparation 24".
**LC/MS** [M+H]⁺ = 717.45 + 719.45 pour 717.30 +719.30 en théorie

### Stade D : Chlorhydrate de 1-(5-chloro-2-{[(3S)-3-(morpholin-4-ylméthyl)-3,4-dihydroisoquinoléin-2(1H)-yl]carbonyl}phényl)-N-(4-hydroxyphényl)-4,5-diméthyl-N-(1-méthyl-1H-pyrrolo(2,3-b)pyridin-5-yl)-1H-pyrrole-3-carboxamide

Le composé obtenu au stade précédent (1,16g, 1,38 mmol) est directement mis en solution dans 15 mL de THF. Le TBAF 1M en solution dans le THF (1,51ml, 1,51 mmol) est ajouté goutte à goutte à la seringue. Le milieu réactionnel est ensuite agité à température ambiante pendant 12 h. L'avancement de la réaction est suivi par LC-MS. La solution est diluée avec du dichlorométhane, lavée avec une solution saturée de NaHCO₃ puis avec de l'eau. La phase organique est séchée sur sulfate de magnésium, filtrée puis évaporée à sec. Le composé est purifié sur colonne de gel de silice en utilisant le dichlorométhane et l'éthanol comme solvants, pour fournir la base libre.

450 mg de produit ainsi obtenu sont dissous dans 5 mL d'éthanol et 4 mL d'une solution d'acide chlorhydrique 1M dans l'éther sont lentement ajoutés ; le chlorhydrate correspondant précipite. Il est filtré, lavé avec de l'éther et lyophilisé dans un mélange acétonitrile : eau. On obtient 470 mg de chlorhydrate sous la forme d'une poudre blanche.
**RMN ¹H** (500 MHz, dmso-d6) δ ppm :11.46 (sl, NH), 9.5 (sl, OH), 8.14 (m, 1 H), 7.99 (m, 1 H), 7.63/7.51 (m, 1 H), 7.63 (m, 1 H), 7.51 (m, 1 H), 7.33-6.99 (m, 6 H), 6.8-6.6 (m, 2 H), 6.7/6.62 (m, 2 H), 6.41 (m, 1 H), 5.23 (m, 1 H), 4.82/4.11 (m, 2 H), 4.03/3.88 (m, 4 H), 3.8 (m, 3 H), 3.73/3.4/3.13/3.01 (m, 4 H), 3.28/3.15 (m, 2 H), 2.78/2.65 (m, 2 H), 1.93-1.56 (m, 6 H)
***IR (ATR) cm¹ :*** 2000 à 3500 v -NH+/OH, 1628 v -C=O, 1260-1230-1186 v -C-O-C, 830-736 v -CH-Ar

### Masse haute résolution (ESI+/FIA) :

Formule empirique: C₄₂H₄₁ClN₆O₄
[M+H]⁺ calculé: 729.2951
[M+H]⁺ mesuré: 759.2949
(rapports isotopiques en accord avec un atome de chlore)

### Exemple 210. Chlorhydrate de 1-(5-chloro-2-{[(3S)-3-(morpholin-4-ylméthyl)-3,4-dihydroisoquinoléin-2(1H)-yl]carbonyl}phényl)-N-(4-hydroxyphényl)-4,5-diméthyl-N-(1-méthyl-2,3-dihydro-1H-pyrrolo[2,3-b]pyridin-5-yl)-1H-pyrrole-3-carboxamide

La base libre du composé de l'Exemple 209 (490 mg, 0,671 mmol) est dissous dans 6,5 mL d'acide acétique. Le cyanoborohydrure de sodium (370 mg, 5,90 mmol) est ajouté par portions en trois fois. Le milieu réactionnel est agité à température ambiante pendant 70 h. L'avancement de la réaction est suivi par LC-MS. Le milieu réactionnel est évaporé, puis co-évoporé au toluène. Le composé ainsi obtenu est purifié sur colonne de gel de silice en utilisant le dichlorométhane et l'éthanol comme solvants. Ce composé est alors dissous dans 5 mL d'éthanol, et 4 mL d'une solution d'acide chlorhydrique 1M dans l'éther sont lentement ajoutés ; le chlorhydrate précipite. Il est filtré, lavé avec de l'éther et lyophilisé dans un mélange acétonitrile/eau. On obtient le chlorhydrate sous la forme d'une poudre blanche.
**RMN ¹H** (500 MHz, dmso-d6) δ ppm : 11.6 (sl, 1H), 8.16 (m, 1 H), 7.67 (m, 1 H), 7.55-7.01 (m, 11 H), 6.91 (m, 1 H), 6.65 (m, 1 H), 5.21 (m,1 H), 4.82/4.14 (m, 2 H), 4.14-3.81 (m,4 H), 3.85-2.94 (m, 4 H), 3.27/3.15 (m, 2 H), 3.11 (m, 3 H), 3.09 (m, 2 H), 2.62 (m, 2 H), 2.03/1.78/1.63 (m, 6 H)
***IR (ATR) cm-¹ :*** 2000 à 3500 v NH+/OH, 1628 v -C=O, 1361 v -C-O-C, 1264 v -CH-Ar

### Masse haute résolution (ESI+/FIA) :

Formule empirique: C₄₂H₄₃ClN₆O₄
[M+H]⁺ calculé: 731.3107
[M+H]⁺ mesuré: 731.3111

### Exemple 211. Chlorhydrate de 1-(5-chloro-2-{[(3S)-3-(morpholin-4-ylméthyl)-3,4-dihydroisoquinoléin-2(1H)-yl]carbonyl}phényl)-N-(5-cyano-1,2-diméthyl-1H-pyrrol-3-yl)-N-(4-hydroxyphényl)-4,5-diméthyl-1H-pyrrole-3-carboxamide

### Stade A : N-(4-{[tert-butyl(diméthyl)silyl]oxy}phényl)-1-(5-chloro-2-{[(3S)-3-(morpholin-4-ylméthyl)-3,4-dihydroisoquinoléin-2(1H)-yl]carbonyl}phényl)-N-(5-cyano-1,2-diméthyl-1H-pyrrol-3-yl)-4,5-diméthyl-1H-pyrrole-3-carboxamide

Le composé est obtenu suivant les protocoles des Stades A-C de l'Exemple 209 en remplaçant au Stade C le composé de la Préparation 24" par le composé de la Préparation 26".
**RMN ¹H** (500 MHz, dmso-d6) δ ppm : 7.67 (m, 1 H), 7.62/7.6 (m, 1 H), 7.44 (m, 1 H), 7.39 (m, 1 H), 7.25-6.95 (m, 4 H), 6.9 (m, 2 H), 6.78/6.63 (m, 1 H), 6.74/6.68/6.61 (m, 2 H), 5.03/4.79/3.56 (m, 1 H), 4.88/4.32/4.22/4.03 (m, 2 H), 3.64-3.41 (m, 4 H), 3.59 (s, 3 H), 2.97/2.82/2.7/2.63 (m, 2 H), 2.56-1.92 (m, 6 H), 2.1-1.68 (m, 9 H), 0.88 (m, 9 H), 0.1 (s, 6 H)
***IR (ATR) cm¹ :*** 2211 v -CN, 1637, v -C=O, 1253 v -C-O-C-, 910 v -Si-O-C-, 837 v -Si-C-, 782-744 v -CH-Ar

### Stade B : Chlorhydrate de 1-(5-chloro-2-{[(3S)-3-(morpholin-4-ylméthyl)-3,4-dihydroisoquinoléin-2(1H)-yl]carbonyl}phényl)-N-(5-cyano-1,2-diméthyl-1H-pyrrol-3-yl)-N-(4-hydroxyphényl)-4,5-diméthyl-1H-pyrrole-3-carboxamide

Le composé du Stade A (440 mg, 0,530 mmol) est mis en solution dans 10 mL de tétrahydrofurane, et on ajoute goutte à goutte le fluorure de tétrabutylammonium (580 µL, 1M dans le THF, 0,580 mmol). Le milieu réactionnel est agité à température ambiante pendant 1 heure. Une solution aqueuse saturée en hydrogénocarbonate est alors ajoutée au milieu qui est extrait 3 fois avec du dichlorométhane. La phase organique est séchée sur sulfate de magnésium, filtrée puis évaporée à sec. Le composé ainsi obtenu est purifié sur une colonne de gel de silice en utilisant le dichlorométhane et l'éthanol + 0.1% d'ammonaique comme solvants. On obtient le composé désiré sous la forme d'une poudre blanche. Ce dernier est dissous dans 5 mL d'éthanol et 4 mL d'une solution d'acide chlorhydrique 1M dans l'éther sont lentement ajoutées ; le chlorhydrate précipite. Il est filtré, lavé avec de l'éther et lyophilisé dans un mélange acétonitrile/eau.
**RMN ¹H** (500 MHz, dmso-d6) δ ppm : 11.3 (sl, 1 H), 9.4 (s, 1 H), 8.1 (sl, 1 H), 7.65 (d, 1 H), 7.4 (s, 1 H), 7.3-6.7 (plusieurs m, 7 H), 6.7 (m, 2 H), 6.5-5.5 (sl, 1? H), 5.2 (m, 1 H), 4.75/4.1 (m+m, 1+1 H), 4/3.9 (m+m, 2+2 H), 3.75/3.15 (m+m, 1+1 H), 3.6 (s, 3 H), 3.4/3 (m+m, 1+1 H), 3.3/3.15 (m+m, 1+1 H), 2.75-2.5 (m, 2 H), 2.1-1.55 (plusieurs s, 9 H)
***IR (ATR) cm-¹ :*** 3373 v -OH, 2700-2200 v -NH+, 2211 v -CN, 1625 v -C=O

### Masse haute résolution (ESI+/FIA) :

Formule empirique: C₄₁H₄₁ClN₆O₄
[M+H]⁺ calculé: 717.2951
[M+H]⁺ mesuré: 717.2941

### ETUDE PHARMACOLOGIOUE

### EXEMPLE A : Inhibition de Bcl-2 par la technique de polarisation de fluorescence

### Méthode A :

Les essais de polarisation de fluorescence ont été réalisés en microplaques (384 puits). La protéine Bcl-2 étiquetée (histag-Bcl-2 tel que Bcl-2 correspond au numéro d'ordre primaire UniProtKB®: P10415), à la concentration finale de 2,50.10⁻⁸M, est mélangée avec un peptide fluorescent (Fluorescein-REIGAQLRRMADDLNAQY), à la concentration finale de 1,50. 10⁻⁸M dans une solution tampon (NaPO₄ 20 mM, NaCl 50 mM, EDTA 1 mM, pH 7.4), en présence ou en absence de concentrations croissantes de composés à tester. Après incubation de 2 heures, la polarisation de fluorescence est mesurée.

### Méthode B :

Les essais de polarisation de fluorescence ont été réalisés en microplaques (384 puits). La protéine Bcl-2 étiquetée (histag-Bcl-2 tel que Bcl-2 correspond au numéro d'ordre primaire UniProtKB®: P10415), à la concentration finale de 2,50.10⁻⁸M, est mélangée avec un peptide fluorescent (Fluorescein-REIGAQLRRMADDLNAQY), à la concentration finale de 1,00. 10⁻⁸M dans une solution tampon (Hepes 10 mM, NaCl 150 mM, Tween20 0.05%, pH 7.4), en présence ou en absence de concentrations croissantes de composés à tester. Après incubation de 2 heures, la polarisation de fluorescence est mesurée.

Les résultats sont exprimés en IC₅₀ (concentration en composé qui inhibe à 50 % la polarisation de fluorescence) et sont présentés dans le tableau 1 ci-dessous. Les IC_{5O} de l'inhibition de Bcl-2 obtenues en utilisant la méthode B sont soulignées.

Les résultats montrent que les composés de l'invention inhibent l'interaction entre la protéine Bcl-2 et le peptide fluorescent décrit précédemment.

### EXEMPLE B : Cytotoxicité in vitro.

Les études de cytotoxicité ont été réalisées sur la lignée tumorale de leucémie RS4 ; 11. Les cellules sont réparties dans des microplaques et exposées aux composés à tester pendant 48 heures. La viabilité cellulaire est ensuite quantifiée par un essai colorimétrique, le Microculture Tétrazolium Assay (Cancer Res., 1987, 47, 939-942).
Les résultats sont exprimés en IC₅₀ (concentration en composé qui inhibe à 50 % la viabilité cellulaire) et sont présentés dans le tableau 1 ci-dessous.

Les résultats montrent que les composés de l'invention sont cytotoxiques.

**Tableau 1 : IC₅₀ d'inhibition de Bcl-2 (test de polarisation de fluorescence) et de cytotoxicité pour les cellules RS4 ; 11**

| *Note : Les IC₅₀ de l'inhibition de Bcl-2 obtenues en utilisant la méthode B sont soulignées.* | | | | | |
|---|---|---|---|---|---|
| | IC₅₀ (nM) Bcl-2 FP | IC₅₀ (nM) MTT RS4;11 | | IC₅₀ (nM) Bcl-2 FP | IC₅₀ (nM) MTT RS4;11 |
| **Exemple 5** | 212.7 | 2590 | **Exemple 38** | 238.2 | 1780,0 |
| **Exemple 6** | 50.5 | 915 | **Exemple 39** | 12.5 | 263,0 |
| **Exemple 10** | 145.8 | 2220 | **Exemple 40** | 7.8 | 167,0 |
| **Exemple 12** | 120.4 | 1810 | **Exemple 41** | 3.7 | 52,2 |
| **Exemple 13** | 16.2 | 462,0 | **Exemple 42** | 23.1 | 353,0 |
| **Exemple 14** | 24.8 | 1270,0 | **Exemple 43** | 7.2 | 101,0 |
| **Exemple 15** | 22.1 | 759,0 | **Exemple 44** | 6.8 | 164,0 |
| **Exemple 16** | 29.6 | 656,0 | **Exemple 45** | 6.7 | 36,5 |
| **Exemple 17** | 11.4 | 1010,0 | **Exemple 46** | 8.4 | 93,4 |
| **Exemple 18** | 44.6 | 1130,0 | **Exemple 47** | 9.2 | 50,3 |
| **Exemple 19** | 245.0 | 2360 | **Exemple 48** | 10.5 | 93,9 |
| **Exemple 20** | 74.3 | 900,0 | **Exemple 49** | 13.1 | 136,0 |
| **Exemple 21** | 24.6 | 256 | **Exemple 50** | 7.4 | 301,0 |
| **Exemple 22** | 10.7 | 142,0 | **Exemple 51** | 6.9 | 155,0 |
| **Exemple 23** | 26.1 | 445,0 | **Exemple 52** | 5.4 | 77,3 |
| **Exemple 24** | 15.6 | 140,0 | **Exemple 53** | 9.0 | 207,0 |
| **Exemple 25** | 87.6 | 1100,0 | **Exemple 54** | 20 | 103 |
| **Exemple 26** | 7.5 | 171,0 | **Exemple 55** | 57.2 | 1020,0 |
| **Exemple 27** | 20.5 | 479,0 | **Exemple 56** | 5.0 | 135,0 |
| **Exemple 28** | 8.0 | 378,0 | **Exemple 57** | 13.4 | 267,0 |
| **Exemple 29** | 44.6 | 1010,0 | **Exemple 58** | 20.5 | 190,0 |
| **Exemple 31** | 5.5 | 71,4 | **Exemple 59** | 19.2 | 206,0 |
| **Exemple 32** | 3.5 | 63,7 | **Exemple 60** | 206.4 | 89,5 |
| **Exemple 33** | 4.4 | 199,0 | **Exemple 61** | 7.7 | 204,0 |
| **Exemple 34** | 7.7 | 133,0 | **Exemple 62** | 7.5 | 205,0 |
| **Exemple 35** | 3.5 | 68,9 | **Exemple 63** | 168.3 | 1510,0 |
| **Exemple 36** | 1110.5 | 2420,0 | **Exemple 64** | 295.6 | 1360,0 |
| **Exemple 37** | 3.8 | 47,1 | **Exemple 65** | 5.1 | 130,0 |
| **Exemple 66** | 6.6 | 148,0 | **Exemple 97** | 6.1 | 73,2 |
| **Exemple 67** | 5.9 | 101,0 | **Exemple 98** | 45.1% @10 µM | 1880,0 |
| **Exemple 68** | 79.7 | 856,0 | **Exemple 99** | 4.8 | 41,7 |
| **Exemple 69** | 9.0 | 67,6 | **Exemple 100** | 6.9 | 43,4 |
| **Exemple 70** | 2.9 | 63,8 | **Exemple 101** | 99.1 | 1880,0 |
| **Exemple 71** | 8.3 | 196,0 | **Exemple 102** | 8.4 | 108,0 |
| **Exemple 72** | 4.5 | 43,4 | **Exemple 103** | 17.4 | 523,0 |
| **Exemple 73** | 3.9 | 51,4 | **Exemple 104** | 18.8 | 383,0 |
| **Exemple 74** | 4.2 | 162,0 | **Exemple 105** | 13.6 | 202,0 |
| **Exemple 75** | 7.1 | 184,0 | **Exemple 106** | 19.6 | 521,0 |
| **Exemple 76** | 8.7 | 151,0 | **Exemple 107** | 44.0 | 150,0 |
| **Exemple 77** | 4.9 | 207,0 | **Exemple 108** | 35.5 | 544,0 |
| **Exemple 78** | 3.7 | 81,4 | **Exemple 109** | 4.4 | 116,0 |
| **Exemple 79** | 85.5 | 344,0 | **Exemple 110** | 8.1 | 291,0 |
| **Exemple 80** | 6.8 | 120,0 | **Exemple 112** | 11.8 | 281,0 |
| **Exemple 81** | 83.8 | 652,0 | **Exemple 113** | 19.2 | 203,0 |
| **Exemple 82** | 17.6 | 373,0 | **Exemple 114** | 23.7 | 726,0 |
| **Exemple 83** | 3.7 | 350,0 | **Exemple 115** | 14.5 | 272,0 |
| **Exemple 84** | 6.7 | 146,0 | **Exemple 116** | 12.1 | 131,0 |
| **Exemple 85** | 3.7 | 129,0 | **Exemple 117** | 7.6 | 193,0 |
| **Exemple 86** | 5.4 | 67,7 | **Exemple 118** | 5.9 | 114,0 |
| **Exemple 87** | 4.4 | 138,0 | **Exemple 119** | 28.4 | 96,4 |
| **Exemple 88** | 19.6 | 541,0 | **Exemple 120** | 15 | 367,0 |
| **Exemple 89** | 4.2 | 59,8 | **Exemple 121** | 447.5 | 8770 |
| **Exemple 90** | 3.6 | 76,6 | **Exemple 122** | 53.3 | 2290 |
| **Exemple 91** | 5.3 | 172,0 | **Exemple 123** | 90 | 2370 |
| **Exemple 92** | 9.6 | 202,0 | **Exemple 131** | 18.9 | 469 |
| **Exemple 93** | 3.3 | 75,4 | **Exemple 132** | 31.7 | 1860 |
| **Exemple 94** | 7.9 | 118,0 | **Exemple 133** | 841.4 | ND |
| **Exemple 95** | 6.4 | 264,0 | **Exemple 134** | 72.1% @22.2 µM | 2100 |
| **Exemple 96** | 3.9 | 157,0 | **Exemple 135** | 10.4 | 461 |
| **Exemple 138** | 52.2 | 2020 | **Exemple 176** | 6.2 | 225 |
| **Exemple 140** | 25.0 | 1840 | **Exemple 177** | 18.8 | 568 |
| **Exemple 141** | 56.7 | 2280 | **Exemple 178** | 17.5 | 812 |
| **Exemple 142** | 70.6 | 2290 | **Exemple 179** | 18.1 | 886 |
| **Exemple 143** | 71.5 | 533 | **Exemple 180** | 11.0 | 210 |
| **Exemple 144** | 35.0 | 1380 | **Exemple 181** | 6.9 | 205 |
| **Exemple 145** | 12.2 | 1180 | **Exemple 182** | 110.7 | 1850 |
| **Exemple 146** | 17.2 | 346 | **Exemple 183** | 8.3 | ND |
| **Exemple 147** | 22.5 | 966 | **Exemple 184** | 14.1 | 154 |
| **Exemple 148** | 28.3 | 1170 | **Exemple 185** | 17.3 | 422 |
| **Exemple 149** | 21.6 | 340 | **Exemple 186** | 23.5 | 626 |
| **Exemple 150** | 51.0 | 1320 | **Exemple 187** | 21.6 | 1990 |
| **Exemple 152** | 217.5 | 2160 | **Exemple 188** | 16.8 | 68 |
| **Exemple 153** | 285.8 | 237 | **Exemple 190** | 13.6 | 645 |
| **Exemple 155** | 13.2 | 246 | **Exemple 193** | 5.6 | 499 |
| **Exemple 156** | 11.7 | 450 | **Exemple 194** | 6.7 | 534 |
| **Exemple 157** | 16.1 | 487 | **Exemple 195** | 31.1 | 507 |
| **Exemple 158** | 54.1 | 834 | **Exemple 197** | 9.8 | 328 |
| **Exemple 159** | 10.2 | 191 | **Exemple 198** | 3.4 | 616 |
| **Exemple 163** | 22.8 | 292 | **Exemple 199** | 57.7 | 2360 |
| **Exemple 164** | 7.8 | 132 | **Exemple 200** | 154.8 | 2080 |
| **Exemple 165** | 3.2 | 179 | **Exemple 201** | 3.1 | 124 |
| **Exemple 167** | 37.4 | 984 | **Exemple 202** | 4.4 | 798 |
| **Exemple 168** | 11.4 | 188 | **Exemple 203** | 10.0 | 454 |
| **Exemple 169** | 107.9 | 854 | **Exemple 204** | 4.7 | 780 |
| **Exemple 170** | 16.8 | 177 | **Exemple 205** | 4.3 | 123 |
| **Exemple 171** | 71.8 | 1050 | **Exemple 206** | 16.7 | 72,4 |
| **Exemple 172** | 4.2 | 1200 | **Exemple 207** | 20.3 | 22,2 |
| **Exemple 173** | 167.9 | 2040 | **Exemple 209** | 4.1 | 62,9 |
| **Exemple 174** | 41.2 | 402 | **Exemple 210** | 3.1 | 64,7 |
| **Exemple 175** | 25.1 | 460 | **Exemple 211** | 3.7 | 46,9 |

| | | | | | |
|---|---|---|---|---|---|
| ND : non déterminé | | | | | |

Pour les inhibiteurs partiels, le pourcentage d'inhibition de la polarisation de fluorescence pour une concentration donnée du composé testé est indiqué. Ainsi, 45.1% @ 10 µM signifie qu'on observe 45.1% d'inhibition de la polarisation de fluorescence pour une concentration de composé testé égale à 10 µM.

### EXEMPLE C : Induction de l'activité caspase in vivo.

La capacité des composés de l'invention à activer la caspase 3 est évaluée dans un modèle de xénogreffe de cellules leucémiques RS4 ; 11.
1.10⁷ cellules RS4;11 sont greffées par voie sous-cutanée dans des souris immunodéprimées (souche SCID). 25 à 30 jours après la greffe, les animaux sont traités par voie orale par les différents composés. Seize heures après le traitement, les masses tumorales sont récupérées, lysées et l'activité caspase 3 est mesurée dans les lysats tumoraux.
Cette mesure enzymatique est réalisée en dosant l'apparition d'un produit de clivage fluorigénique (activité DEVDase, Promega). Elle est exprimée sous la forme d'un facteur d'activation correspondant au rapport entre les deux activités caspases : celle pour les souris traitées divisée par celle pour les souris contrôles.

Les résultats montrent que les composés de l'invention sont capables d'induire l'apoptose dans les cellules tumorales RS4 ;11 *in vivo.*

### EXEMPLE D : Quantification de la forme clivée de la caspase 3 in vivo.

La capacité des composés de l'invention à activer la caspase 3 est évaluée dans un modèle de xénogreffe de cellules leucémiques RS4 ; 11.
1.10⁷ cellules RS4;11 sont greffées par voie sous-cutanée dans des souris immunodéprimées (souche SCID). 25 à 30 jours après la greffe, les animaux sont traités par voie orale par les différents composés. Après le traitement, les masses tumorales sont récupérées (au bout d'un temps T), lysées et la forme clivée (activée) de la caspase 3 est quantifiée dans les lysats tumoraux.

Cette quantification est réalisée en utilisant l'essai « Meso Scale Discovery (MSD) ELISA platform » qui dose spécifiquement la forme clivée de la caspase 3. Elle est exprimée sous la forme d'un facteur d'activation correspondant au rapport entre la quantité de caspase 3 clivée chez les souris traitées divisée par la quantité de caspase 3 clivée chez les souris contrôles.

Les résultats montrent que les composés de l'invention sont capables d'induire l'apoptose dans les cellules tumorales RS4 ; 11 *in vivo.*

**Tableau 2 : Facteurs d'activation des caspases (caspase 3 clivée essai MSD dans les tumeurs des souris traitées versus souris contrôles) in vivo, après traitement par voie orale (doses précisées entre parenthèses)**

| Composé testé | Temps au bout duquel est prélevé la tumeur (T) | Facteur d'activation ± SEM (versus contrôle) |
|---|---|---|
| **Exemple 184** | 16 h | 15,1 (100 mg/kg) |
| **Exemple 207** | 2 h | 14,6 ± 4,4 (25 mg/kg) |
| **Exemple 210** | 2 h | 36,3 ± 13,3 (12,5 mg/kg) |
| **Exemple 211** | 2 h | 94,7 ± 9,8 (25 mg/kg) |

### EXEMPLE E : Activité anti-tumorale in vivo.

L'activité anti-tumorale des composés de l'invention est évaluée dans un modèle de xénogreffe de cellules leucémiques RS4 ;11.
1.10⁷ cellules RS4;11 sont greffées par voie sous-cutanée dans des souris immunodéprimées (souche SCID). 25 à 30 jours après la greffe, lorsque la masse tumorale a atteint environ 150 mm³, les souris sont traitées par voie orale par les différents composés dans 2 schémas différents (traitement quotidien pendant cinq jours par semaine durant deux semaines, ou deux traitements par semaine pendant deux semaines). La masse tumorale est mesurée 2 fois par semaine depuis le début du traitement.

Les résultats obtenus montrent donc que les composés de l'invention sont capables d'induire une régression tumorale significative durant la période de traitement.

### EXEMPLE F : Composition pharmaceutique : Comprimées

| | |
|---|---|
| 1000 comprimés dosés à 5 mg d'un composé choisi parmi les exemples 1 à 211 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 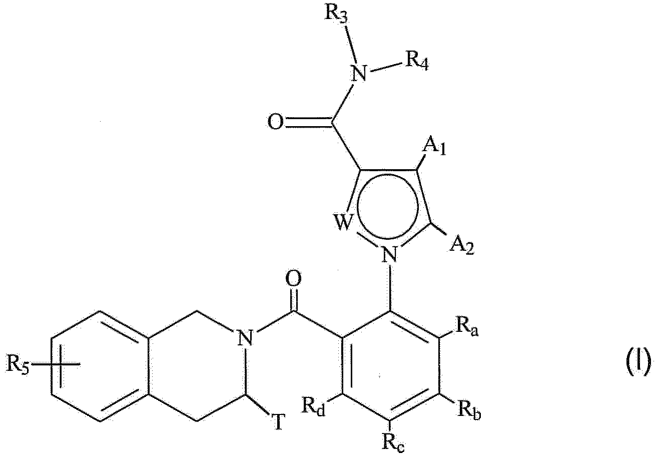 dans laquelle :
◆ W représente un groupe C-H tandis que A₁ et A₂ représentent respectivement un atome d'hydrogène et un groupement méthyle,
ou bien W représente un groupe C-H tandis que A₁ et A₂ forment avec les atomes de carbone qui les portent un cyclohexenyle ou un cycle benzo éventuellement substitué par un atome d'halogène,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
ou bien R₁ et R₂ forment avec l'atome d'azote qui les porte un hétérocycloalkyle,
◆ R₃ représente un groupement aryle ou hétéroaryle, étant entendu qu'un ou plusieurs atomes de carbone des groupements précédents, ou de leurs éventuels substituants, peut(vent) être deutéré(s),
◆ R₄ représente un groupement aryle ou hétéroaryle, étant entendu qu'un ou plusieurs atomes de carbone des groupements précédents, ou de leurs éventuels substituants, peut(vent) être deutéré(s),
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ, R_{b}, R_{c} et R_{d} représentent indépendamment les uns des autres R₇, un atome d'halogène, un groupement alkoxy (C₁-C₆) linéaire ou ramifié, un groupe hydroxy, un groupement polyhalogénoalkyle (C₁-C₆) linéaire ou ramifié, un groupe trifluorométhoxy, -NR₇R₇', nitro, ou bien les substituants de l'un des couples (Rₐ,R_{b}), (R_{b},R_{c}) ou (R_{c},R_{d}) forment ensemble avec les atomes de carbone qui les portent un cycle constitué de 5 à 7 chaînons, pouvant contenir de un à 2 hétéroatomes choisis parmi oxygène et soufre, étant aussi entendu qu'un ou plusieurs atomes de carbone du cycle précédemment défini peut(vent) être deutéré(s) ou substitués par un à 3 groupements choisis parmi halogène ou alkyle (C₁-C₆) linéaire ou ramifié,
ou bien Rₐ et R_{d} représentent chacun un atome d'hydrogène, R_{b} représente un hydrogène, un halogène, un groupement hydroxy ou méthoxy, et R_{c} est choisi parmi l'un des groupes suivants : hydroxy, méthoxy, amino, 3-phénoxyazétidine, 2-(phénylsulfanyl)acétamide, ou 2-(phénoxy)acétamide,
◆ R₇ et R₇ représentent indépendamment l'un de l'autre un hydrogène, un groupement alkyle (C₁-C₆) linéaire ou ramifié, alkényle (C₂-C₆) linéaire ou ramifié, alkynyle (C₂-C₆) linéaire ou ramifié, aryle ou hétéroaryle, ou bien R₇ et R₇' forment ensemble avec l'atome d'azote qui les porte un hétérocycle constitué de 5 à 7 chaînons,
étant entendu que lorsque le composé de formule (I) contient un groupement hydroxy, ce dernier peut être éventuellement substitué par l'un des groupes suivants : PO(OM)(OM'), -PO(OM)(O⁻M⁺), PO(O⁻M₁⁺)(O⁻M₂⁺), -PO(O⁻)(O⁻)M₃²⁺, -PO(OM)(O[CH₂CH₂O]ₙCH₃), ou -PO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5,
étant aussi entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique constitué de 3 à 10 chaînons et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié, spiro (C₃-C₆), alkoxy (C₁-C₆) linéaire ou ramifié, (C₁-C₆)alkyl-S-, hydroxy, oxo (ou *N-*oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié,
leurs énantiomères et diastéréoisomères, ainsi que leurs sels d'addition à un acide ou à une base pharmaceutiquement acceptable.

2. Composé de formule (I) selon la revendication 1 dans lequel R₄ représente un phényle substitué en position *para* par un groupement de formule -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), ou -OPO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyl (C₂-C₆) linéaire ou ramifié, un cycloalkyle ou un hétérocycloalkyle, tous deux constitués de 5 à 6 chainons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5, étant entendu que le groupement phényle peut être optionnellement substitué par un ou plusieurs atomes d'halogène.

3. Composé de formule (I) selon la revendication 1 ou 2 dans lequel T représente un groupement choisi parmi méthyle, aminométhyle, diméthylaminométhyle, morpholinylméthyle, (4-méthyl-1-pipérazinyl)méthyle, (3a*R*,6a*S*)-hexahydrocyclopenta[*c*]pyrrol-2(1*H*)-ylméthyle, (4,4-difluoropipéridin-1-yl)méthyle, (4-cyclopentylpipérazin-1-yl)méthyle, (4-cyclobutylpipérazin-1-yl)méthyle, pyrrolidin-1-ylméthyle, pipéridin-1-ylméthyle ou 2-(morpholin-4-yl)éthyle.

4. Composé de formule (I) selon l'une des revendications 1 à 3 dans lequel Rₐ et R_{d} représentent chacun un atome d'hydrogène et (R_{b},R_{c}) forment ensemble avec les atomes de carbone qui les portent l'un des groupes suivants : 1,3-dioxolane éventuellement substitué ; 1,4-dioxane éventuellement substitué; cyclopentane ; tétrahydrofurane ; 2,3-dihydrofurane ; ou bien Rₐ, R_{c} et R_{d} représentent chacun un atome d'hydrogène et R_{b} représente un groupement hydroxy ou méthoxy, un atome d'halogène, un groupement trifluorométhyle ou trifluorométhoxy.

5. Composé de formule (I) selon l'une des revendications 1 à 3 dans lequel un à deux des groupements Rₐ, R_{b}, R_{c}, R_{d} représentent un atome d'halogène, tandis que les autres représentent un hydrogène.

6. Composé de formule (I) selon l'une des revendications 1 à 5 dans lequel R₄ représente un groupement phényle, 4-hydroxyphényle, 4-méthoxyphényle, 4-méthylphényle, 3-chloro-4-hydroxyphényle ou 3-fluoro-4-hydroxyphényle.

7. Composé de formule (I) selon l'une des revendications 1 à 6 dans lequel R₃ représente un groupement choisi parmi phényle, 1*H*-indole, benzothiophène, benzofurane, 2,3-dihydro-1*H-*indole, 1*H*-indazole, 2,3-dihydro-1*H-*isoindole, 1*H*-pyrrolo[2,3-*b*]pyridine, phénoxyphényle, 2,3-dihydro-1*H*-pyrrolo[2,3-*b*]pyridine, 1*H*-pyrrole, ces groupements comportant éventuellement un ou plusieurs substituants choisis parmi halogène, alkyle (C₁-C₆) linéaire ou ramifié, trifluorométhoxy, 4-méthylpipérazinyle, alkoxy (C₁-C₆) linéaire ou ramifié, ou cyano.

8. Composé de formule (I) selon la revendication 1 choisi parmi le groupe suivant :
- *N-*(4-hydroxyphényl)-1-{6-[(3*S*)-3-(morpholin-4-ylméthyl)-1,2,3,4-tétrahydroisoquinoléine-2-carbonyl]-2*H*-1,3-benzodioxol-5-yl}-*N*-phényl-1*H-*indole-3-carboxamide,
- *N*-(4-hydroxyphényl)-*N*-(1-méthyl-1*H*-pyrrolo[2,3-*b*]pyridin-5-yl)-1-(6-{[(3*S*)-3-(morpholin-4-ylméthyl)-3,4-dihydroisoquinoléin-2(1*H*)-yl]carbonyl}-1,3-benzodioxol-5-yl)-4,5,6,7-tétrahydro-1*H*-indole-3-carboxamide,
- *N*-(4-hydroxyphényl)-*N-*(4-méthylphényl)-1-(6-{[(3*S*)-3-[(4-méthylpipérazin-1-yl)méthyl]-3,4-dihydroisoquinoléin-2(1*H*)-yl]carbonyl}-1,3-benzodioxol-5-yl)-1*H-*indole-3-carboxamide,
- *N-*(4-hydroxyphényl)-*N-*(1*H-*indol-5-yl)-1-(6-{[(3*S*)-3-[(4-méthylpipérazin-1-yl)méthyl]-3,4-dihydroisoquinoléin-2(1*H*)-yl]carbonyl}-1,3-benzodioxol-5-yl)-1*H-*indole-3-carboxamide,
- *N*-(4-hydroxyphényl)-*N*-(1-méthyl-1*H*-indol-5-yl)-1-(6-{[(3*S*)-3-[(4-méthylpipérazin-1-yl)méthyl]-3,4-dihydroisoquinoléin-2(1*H*)-yl]carbonyl}-1,3-benzodioxol-5-yl)-1*H*-indole-3-carboxamide,
- 1-(5-chloro-2-{[(3*S*)-3-[(4-méthylpipérazin-1-yl)méthyl]-3,4-dihydroisoquinoléin-2(1*H*)-yl]carbonyl]phényl)-*N-*(4-chlorophényl)-*N*-(4-hydroxyphényl)-5-méthyl-1*H*-pyrrole-3-carboxamide,
- 1-(5-chloro-2-{[(3*S*)-3-[(4-méthylpipérazin-1-yl)méthyl]-3,4-dihydroisoquinoléin-2(1*H*)-yl]carbonyl}phényl)-*N-*(4-hydroxyphényl)-*N-*(1*H-*indol-5-yl)-1*H-*indole-3-carboxamide,
- *N*-(4-hydroxyphényl)-1-{6-[(3*S*)-3-[(4-méthylpipérazin-1-yl)méthyl]-1,2,3,4-tétrahydroisoquinoléine-2-carbonyl]-2*H*-1,3-benzodioxol-5-yl}-*N*-phényl-1*H-*indole-3-carboxamide,
ainsi que leurs sels d'addition à un acide ou à une base pharmaceutiquement acceptable.

9. Composés choisis parmi le groupe suivant :
- 1-(5-chloro-2-{[(3*S*)-3-(morpholin-4-ylméthyl)-3,4-dihydroisoquinoléin-2(1*H*)-yl]carbonyl}phényl)-*N*-(4-hydroxyphényl)-4,5-diméthyl-*N*-(1-méthyl-2,3-dihydro-1*H-*pyrrolo[2,3-*b*]pyridin-5-yl)-1*H*-pyrrole-3-carboxamide,
- 1-(5-chloro-2-{[(3*S*)-3-(morpholin-4-ylméthyl)-3,4-dihydroisoquinoléin-2(1*H*)-yl]carbonyl}phényl)-*N*-(5-cyano-1,2-diméthyl-1*H*-pyrrol-3-yl)-*N*-(4-hydroxyphényl)-4,5-diméthyl-1*H-*pyrrole-3-carboxamide,
- 1-(5-chloro-2-{[(3S)-3-(morpholin-4-ylméthyl)-3,4-dihydroisoquinoléin-2(1*H*)-yl]carbonyl}phényl)-N-(4-hydroxyphényl)-4,5-diméthyl-N-(1-méthyl-1H-pyrrolo[2,3-b]pyridin-5-yl)-1H-pyrrole-3-carboxamide,
- 1-[(2*S*)-2-[(benzyloxy)méthyl]-7-[(35)-3-[(4-méthylpipérazin-1-yl)méthyl]-1,2,3,4-tétrahydroisoquinoléine-2-carbonyl]-2,3-dihydro-1,4-benzodioxin-6-yl]-*N-*(4-hydroxyphényl)-*N*-phényl-1*H*-indole-3-carboxamide.
ainsi que leurs sels d'addition à un acide ou à une base pharmaceutiquement acceptable.

10. Procédé de préparation d'un composé de formule (I) selon la revendication 1 **caractérisé en ce que** l'on utilise comme produit de départ le composé de formule (II) : 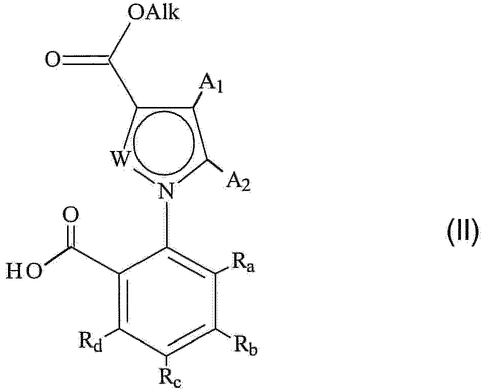 dans laquelle Alk représente un groupement alkyle (C₁-C₆) et W, A₁, A₂, Rₐ, R_{b}, R_{c}, R_{d} sont tels que définis dans la formule (I),
composé de formule (II) qui subit ensuite un couplage peptidique avec un composé de formule (III) : 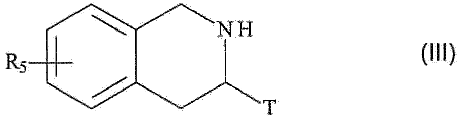 dans laquelle T et R₅ sont tels que définis dans la formule (I),
pour conduire au composé de formule (IV) : 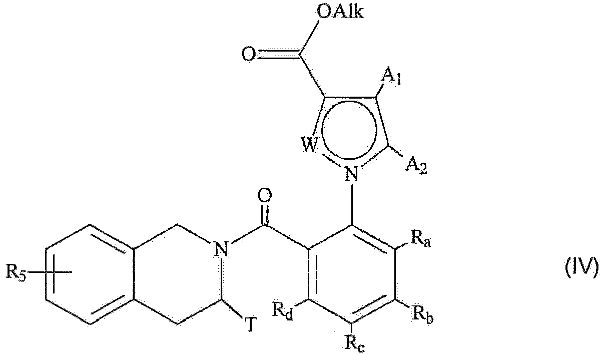 dans laquelle Alk est tel que défini précédemment et W, A₁, A₂, Rₐ, R_{b}, R_{c}, R_{d}, R₅ et T sont tels que définis dans la formule (I),
composé de formule (IV) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), qui peut être optionnellement soumis à l'action d'un dérivé pyrophosphate ou phosphonate dans des conditions basiques, pour conduire au composé de formule (I), composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

11. Procédé de préparation selon la revendication 10 d'un composé de formule (I) dans lequel l'un des groupements R₃ ou R₄ est substitué par une fonction hydroxy **caractérisé en ce que** l'amine NHR₃R₄ est préalablement soumise à une réaction de protection de la fonction hydroxy avant tout couplage avec l'acide carboxylique du composé de formule (IV), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

12. Procédé de préparation d'un composé de formule (I) selon la revendication 1 **caractérisé en ce que** l'on utilise comme produit de départ le composé de formule (V) : 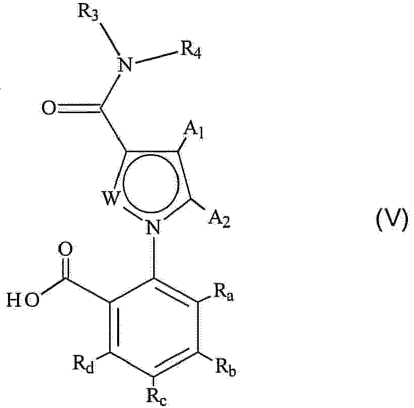 dans laquelle W, A₁, A₂, Rₐ, R_{b}, R_{c}, R_{d}, R₃ et R₄ sont tels que définis dans la formule (I),
composé de formule (V) qui subit ensuite un couplage peptidique avec un composé de formule (III) : 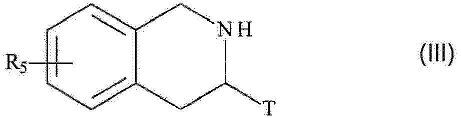 dans laquelle T et R₅ sont tels que définis dans la formule (I),
le composé ainsi obtenu étant optionnellement soumis à l'action d'un dérivé pyrophosphate ou phosphonate dans des conditions basiques, pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

13. Composition pharmaceutique contenant un composé de formule (I) selon l'une quelconque des revendications 1 à 9 ou un de ses sels d'addition avec un acide ou une base pharmaceutiquement acceptable en combinaison avec un ou plusieurs excipients pharmaceutiquement acceptables.

14. Composition pharmaceutique selon la revendication 13 pour son utilisation en tant qu'agent pro-apoptotique.

15. Composition pharmaceutique selon la revendication 13 pour son utilisation dans le traitement des cancers, des maladies auto-immunes et du système immunitaire.

16. Composition pharmaceutique selon la revendication 15 pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

17. Composé de formule (I) selon l'une des revendications 1 à 9, ou un de ses sels d'addition avec un acide ou une base pharmaceutiquement acceptable, pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

18. Association d'un composé de formule (I) selon l'une quelconque des revendications 1 à 9 avec un agent anticancéreux choisi parmi les agents génotoxiques, les poisons mitotiques, les anti-métabolites, les inhibiteurs du protéasome, les inhibiteurs de kinases ou les anticorps.

19. Composition pharmaceutique contenant une association selon la revendication 18 en combinaison avec un ou plusieurs excipients pharmaceutiquement acceptables.

20. Association selon la revendication 18 pour son utilisation dans le traitement des cancers.

21. Composé de formule (I) selon l'une quelconque des revendications 1 à 9 pour son utilisation en association avec une radiothérapie dans le traitement des cancers.

## Patentansprüche

1. Verbindung der Formel (I): 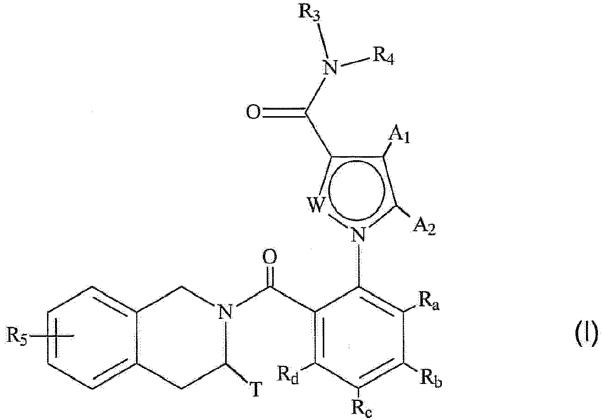 in der:
◆ W eine Gruppe C-H bedeutet, während A₁ und A₂ ein Wasserstoffatom beziehungsweise eine Methylgruppe darstellen,
oder W eine Gruppe CH bedeutet, während A₁ und A₂ zusammen mit den sie tragenden Kohlenstoffatomen einen Cyclohexenylring oder einen gegebenenfalls durch ein Halogenatom substituierten Benzoring bilden,
◆ T ein Wasserstoffatom, eine geradkettige oder verzweigte, gegebenenfalls durch ein bis drei Halogenatome substituierte (C₁-C₆)-Alkylgruppe, eine (C₁-C₄)-Alkyl-NR₁R₂-gruppe oder eine (C₁-C₄)-Alkyl-OR₆-gruppe bedeutet,
◆ R₁ und R₂ unabhängig voneinander ein Wasserstoffatom oder eine geradkettige oder verzweigte (C₁-C₆)-Alkylgruppe bedeuten,
oder R₁ und R₂ gemeinsam mit dem sie tragenden Stickstoffatom eine Heterocycloalkylgruppe bilden,
◆ R₃ eine Aryl- oder Heteroarylgruppe bedeutet, wobei es sich versteht, dass ein oder mehrere Kohlenstoffatome der vorstehenden Gruppen oder ihrer eventuellen Substituenten deuteriert sein kann (können),
◆ R₄ eine Aryl- oder Heteroarylgruppe bedeutet, wobei es sich versteht, dass ein oder mehrere Kohlenstoffatome der vorstehenden Gruppen oder ihrer eventuellen Substituenten deuteriert sein kann (können),
◆ R₅ ein Wasserstoff- oder Halogenatom, eine geradkettige oder verzweigte (C₁-C₆)-Alkylgruppe oder eine geradkettige oder verzweigte (C₁-C₆)-Alkoxygruppe bedeutet,
◆ R₆ ein Wasserstoffatom oder eine geradkettige oder verzweigte (C₁-C₆)-Alkylgruppe bedeutet,
◆ Rₐ, R_{b}, R_{c} und R_{d} unabhängig voneinander R₇, ein Halogenatom, eine geradkettige oder verzweigte (C₁-C₆)-Alkoxygruppe, eine Hydroxygruppe, eine geradkettige oder verzweigte (C₁-C₆)-Polyhalogenalkylgruppe, eine Trifluormethoxygruppe, - NR₇R₇', Nitrogruppe bedeuten oder die Substituenten eines der Paare (Rₐ,R_{b}), (R_{b},R_{c}) oder (R_{c},R_{d}) zusammen mit den sie tragenden Kohlenstoffatomen einen Ring mit 5 bis 7 Kettengliedern bilden, der ein bis 2 Heteroatome ausgewählt aus Sauerstoff und Schwefel enthalten kann, wobei es sich versteht, dass ein oder mehrere Kohlenstoffatome des oben definierten Ringes deuteriert oder durch eine bis 3 Gruppen ausgewählt aus Halogen oder geradkettigem oder verzweigtem (C₁-C₆)-Alkyl substituiert sein kann (können),
oder Rₐ und R_{d} jeweils ein Wasserstoffatom bedeuten, R_{b} ein Wasserstoff, Halogen, eine Hydroxy- oder Methoxygruppe bedeutet und R_{c} aus einer der folgenden Gruppen ausgewählt ist: Hydroxy, Methoxy, Amino, 3-Phenoxyazetidin, 2-(Phenylsulfanyl)-acetamid oder 2-(Phenoxy)-acetamid,
◆ R₇ und R₇' unabhängig voneinander Wasserstoff, eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, Aryl- oder Heteroarylgruppe bedeuten oder R₇ und R₇' gemeinsam mit dem sie tragenden Stickstoffatom einen Heterocyclus mit 5 bis 7 Kettengliedern bilden,
wobei es sich versteht, dass, wenn die Verbindung der Formel (I) eine Hydroxygruppe enthält, diese gegebenenfalls durch eine der folgenden Gruppen substituiert sein kann: - PO(OM)(OM'), -PO(OM)(O⁻M₁⁺), -PO(O⁻M₁⁺)(O⁻M₂⁺), -PO(O⁻)(O⁻)M₃²⁺, - PO(OM)(O[CH₂CH₂O]ₙCH₃) oder -PO(O⁻M₁⁺)(O[CH₂CH₂O]ₙCH₃), worin M und M' unabhängig voneinander ein Wasserstoffatom, eine geradkettige oder verzweigte (C₁-C₆)-Alkylgruppe, eine geradkettige oder verzweigte (C₂-C₆)-Alkenylgruppe, eine geradkettige oder verzweigte (C₂-C₆)-Alkinylgruppe, eine Cycloalkyl- oder Heterocycloalkylgruppe mit jeweils 5 bis 6 Kettengliedern bedeuten, während M₁⁺ und M₂⁺ unabhängig voneinander ein pharmazeutisch annehmbares einwertiges Kation bedeuten, M₃²⁺ ein pharmazeutisch annehmbares zweiwertiges Kation bedeutet und n eine ganze Zahl von 1 bis 5 bedeutet,
wobei es sich weiterhin versteht, dass man:
- unter "Aryl" eine Phenyl-, Naphthyl-, Biphenyl- oder Indenylgruppe versteht,
- unter "Heteroaryl" jede mono- oder bicyclische aus 5 bis 10 Kettengliedern gebildete Gruppe versteht, die mindestens einen aromatischen Teil umfasst und 1 bis 4 Heteroatome ausgewählt aus Sauerstoff, Schwefel oder Stickstoff (einschließlich quaternärem Stickstoff) enthält,
- unter "Cycloalkyl" jede carbocyclische, nicht-aromatische, mono- oder bicyclische, 3 bis 10 Kettenglieder enthaltende Gruppe versteht,
- unter "Heterocycloalkyl" jede nicht-aromatische, mono- oder bicyclische Gruppe versteht, die aus 3 bis 10 Kettengliedern gebildet ist und 1 bis 3 Heteroatome ausgewählt aus Sauerstoff, Schwefel, SO, SO₂ oder Stickstoff enthält,
wobei die in dieser Weise definierten Aryl-, Heteroaryl-, Cycloalkyl- und Heterocycloalkylgruppen und die Alkyl-, Alkenyl-, Alkinyl-, Alkoxygruppen durch 1 bis 3 Gruppen substituiert sein können ausgewählt aus geradkettigem oder verzweigtem (C₁-C₆)-Alkyl, (C₃-C₆)-Spiro, geradkettigem oder verzweigtem (C₁-C₆)-Alkoxy, (C₁-C₆-Alkyl-S-, Hydroxy, Oxo (oder gegebenenfalls *N-*Oxid), Nitro, Cyano, -COOR', - OCOR', NR'R", geradkettigem oder verzweigtem (C₁-C₆)-Polyhalogenalkyl, Trifluormethoxy, (C₁-C₆)-Alkylsulfonyl, Halogen, Aryl, Heteroaryl, Aryloxy, Arylthio, Cycloalkyl, Heterocycloalkyl, gegebenenfalls substituiert durch ein oder mehrere Halogenatome oder Alkylgruppen, wobei es sich versteht, dass R' und R" unabhängig voneinander ein Wasserstoffatom oder eine geradkettige oder verzweigte (C₁-C₆)-Alkylgruppe bedeuten,
deren Enantiomere und Diastereoisomere sowie deren Additionssalze mit einer pharmazeutisch annehmbaren Säure oder Base.

2. Verbindung der Formel (I) nach Anspruch 1, worin R₄ eine Phenylgruppe bedeutet, die in der *para*-Stellung durch eine Gruppe der Formel -OPO(OM)(OM'), - OPO(OM)(O⁻M₁⁺), -OPO(OM₁⁺)(OM₂⁺), -OPO(O⁻)(O⁻)M₃²⁺, - OPO(OM)(O[CH₂CH₂O]ₙCH₃) oder -OPO(O⁻M₁⁺)(O[CH₂CH₂O]ₙCH₃) substituiert ist, worin M und M' unabhängig voneinander ein Wasserstoffatom, eine geradkettige oder verzweigte (C₁-C₆)-Alkylgruppe, eine geradkettige oder verzweigte (C₂-C₆)-Alkenylgruppe, eine geradkettige oder verzweigte (C₂-C₆)-Alkinylgruppe, eine Cycloalkyl- oder Heterocycloalkylgruppe mit jeweils 5 bis 6 Kettengliedern bedeuten, während M₁⁺ und M₂⁺ unabhängig voneinander ein einwertiges pharmazeutisch annehmbares Kation, M₃²⁺ ein zweiwertiges pharmazeutisch annehmbares Kation bedeuten und n eine ganze Zahl von 1 bis 5 bedeutet, wobei es sich versteht, dass die Phenylgruppe gegebenenfalls durch ein oder mehrere Halogenatome substituiert sein kann.

3. Verbindung der Formel (I) nach Anspruch 1 oder 2, worin T eine Gruppe ausgewählt aus Methyl, Aminomethyl, Dimethylaminomethyl, Morpholinylmethyl, (4-Methyl-1-piperazinyl)-methyl, (3a*R,*6a*S*)-Hexahydrocyclopenta[*c*]pyrrol-2(1*H*)-yl-methyl, (4,4-Difluorpiperidin-1-yl)-methyl, (4-Cyclopentylpiperazin-1-yl)-methyl, (4-Cyclobutylpiperazin-1-yl)-methyl, Pyrrolidin-1-ylmethyl, Piperidin-1-ylmethyl oder (2-(Morpholin-4-yl)-ethyl bedeutet.

4. Verbindung der Formel (I) nach einem der Ansprüche 1 bis 3, worin Rₐ und R_{d} jeweils ein Wasserstoffatom bedeuten und (R_{b},R_{c}) gemeinsam mit den sie tragenden Kohlenstoffatomen eine der folgenden Gruppen bilden: gegebenenfalls substituiertes 1,3-Dioxolan; gegebenenfalls substituiertes 1,4-Dioxan; Cyclopentan; Tetrahydrofuran; 2,3-Dihydrofuran; oder Rₐ, R_{c} und R_{d} jeweils ein Wasserstoffatom bedeuten und R_{b} eine Hydroxy- oder Methoxygruppe, ein Halogenatom oder eine Trifluormethyl- oder Trifluormethoxygruppe bedeutet.

5. Verbindung der Formel (I) nach einem der Ansprüche 1 bis 3, worin eine bis zwei der Gruppen Rₐ, R_{b}, R_{c} und R_{d} ein Halogenatom darstellen, während die anderen ein Wasserstoffatom bedeuten.

6. Verbindung der Formel (I) nach einem der Ansprüche 1 bis 5, worin R₄ eine Phenyl-, 4-Hydroxyphenyl-, 4-Methoxyphenyl-, 4-Methylphenyl-, 3-Chlor-4-hydroxyphenyl- oder 3-Fluor-4-hydroxyphenylgruppe bedeutet.

7. Verbindung der Formel (I) nach einem der Ansprüche 1 bis 6, worin R₃ eine Gruppe bedeutet ausgewählt aus Phenyl, 1*H*-Indol, Benzothiophen, Benzofuran, 2,3-Dihydro-1*H*-indol, 1*H*-Indazol, 2,3-Dihydro-1*H*-isoindol, 1*H*-Pyrrolo[2,3-*b*]pyridin, Phenoxyphenyl, 2,3-Dihydro-1*H*-pyrrolo[2,3-*b*]pyridin oder 1*H*-Pyrrol, wobei diese Gruppen gegebenenfalls einen oder mehrere Substituenten ausgewählt aus Halogen, geradkettigem oder verzweigtem (C₁-C₆)-Alkyl, Trifluormethoxy, 4-Methylpiperazinyl, geradkettigem oder verzweigtem (C₁-C₆)-Alkoxy oder Cyano aufweisen.

8. Verbindung der Formel (I) nach Anspruch 1, ausgewählt aus der folgenden Gruppe:
- *N*-(4-Hydroxyphenyl)-1-{6-[(35)-3-(morpholin-4-ylmethyl)-1,2,3,4-tetrahydroisochinolin-2-carbonyl]-2*H*-1,3-benzodioxol-5-yl}-*N-*phenyl-1*H*-indol-3-carboxamid,
- *N*-(4-Hydroxyphenyl)-*N*-(1-methyl-1*H*-pyrrolo[2,3-*b*]pyridin-5-yl)-1-(6-{[(3*S*)-3-(morpholin-4-ylmethyl)-3,4-dihydroisochinolin-2(1*H*)-yl]-carbonyl}-1,3-benzo-dioxol-5-yl)-4,5,6,7-tetrahydro-1*H*-indol-3-carboxamid,
- *N-*(4-Hydroxyphenyl)-*N-*(4-methylphenyl)-1-(6-[(3*S*)-3-[4-methylpiperazin-1-yl)-methyl]-3,4-dihydroisochinolin-2(1*H*)-yl]-carbonyl}-1,3-benzodioxol-5-yl)-1*H-*indol-3-carboxamid,
- *N-*(4-Hydroxyphenyl)-*N-*(1*H*-indol-5-yl)-1-(6-[(3*S*)-3-[(4-methylpiperazin-1-yl)-methyl]-3,4-dihydroisochinolin-2(1*H*)-yl]-carbonyl}-1,3-benzodioxol-5-yl)-1*H-*indol-3-carboxamid,
- *N-*(4-Hydroxyphenyl)-*N-*(1-methyl-1*H*-indol-5-yl)-1-(6-{[(3*S*)-3-[(4-methylpiperazin-1-yl)-methyl]-3,4-dihydroisochinolin-2(1*H*)-yl]-carbonyl}-1,3-benzo-dioxol-5-yl)-1*H-*indol-3-carboxamid,
- 1-(5-Chlor-2-{[(3*S*)-3-[(4-methylpiperazin-1-yl)-methyl]-3,4-dihydroisochinolin-2(1*H*)-yl]-carbonyl}-phenyl)-*N-*(4-chlorphenyl)-*N-*(4-hydroxyphenyl)-5-methyl-1*H*-pyrrol-3-carboxamid,
- 1-(5-Chlor-2-{[(3*S*)-3-[(4-methylpiperazin-1-yl)-methyl]-3,4-dihydroisochinolin-2(1*H*)-yl]-carbonyl}-phenyl)-*N*-(4-hydroxyphenyl)-*N*-(1*H*-indol-5-yl)-1*H*-indol-3-carboxamid,
- *N*-(4-Hydroxyphenyl)-1-{6-[(3*S*)-3-[(4-methylpiperazin-1-yl)-methyl]-1,2,3,4-tetrahydroisochinolin-2-carbonyl]-2*H*-1,3-benzodioxol-5-yl}-*N*-phenyl-1*H*-indol-3-carboxamid
sowie deren Additionssalze mit einer pharmazeutisch annehmbaren Säure oder Base.

9. Verbindungen ausgewählt aus der folgenden Gruppe:
- 1-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-4,5-dimethyl-*N*-(1-methyl-2,3-dihydro-1*H*-pyrrolo[2,3-*b*]pyridin-5-yl)-1*H*-pyrrol-3-carboxamid,
- 1-(5-Chlor-2-{[(3*S*)-3-(morpholin-4-ylmethyl)-3,4-dihydroisochinolin-2(1*H*)-yl]-carbonyl}-phenyl)-*N*-(5-cyano-1,2-dimethyl-1*H*-pyrrol-3-yl)-*N*-(4-hydroxyphenyl)-4,5-dimethyl-1*H*-pyrrol-3-carboxamid,
- 1-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-4,5-dimethyl-*N*-(1-methyl-1*H-*pyrrolo[2,3-*b*]pyridin-5-yl)-1*H*-pyrrol-3-carboxamid,
- 1-[(2*S*)-2-[(Benzyloxy)-methyl]-7-[(3*S*)-3-[(4-methylpiperazin-1-yl)-methyl]-1,2,3,4-tetrahydroisochinolin-2-carbonyl]-2,3-dihydro-1,4-benzodioxin-6-yl]-*N-*(4-hydroxyphenyl)-*N-*phenyl-1*H*-indol-3-carboxamid
sowie deren Additionssalze mit einer pharmazeutisch annehmbaren Säure oder Base.

10. Verfahren zur Herstellung einer Verbindung der Formel (I) nach Anspruch 1, **dadurch gekennzeichnet, dass** man als Ausgangsprodukt die Verbindung der Formel (II) verwendet: 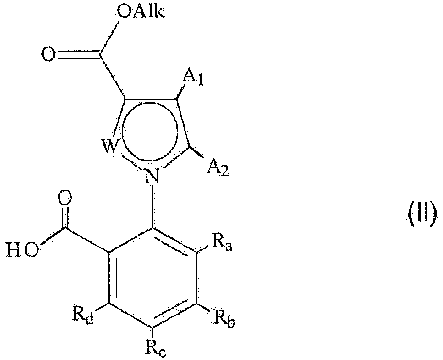 in der Alk eine (C₁-C₆)-Alkylgruppe bedeutet und W, A₁, A₂, Rₐ, R_{b}, R_{c} und R_{d} die bezüglich der Formel (I) angegebenen Bedeutungen besitzen,
welche Verbindung der Formel (II) einer Peptidkupplung mit einer Verbindung der Formel (III): 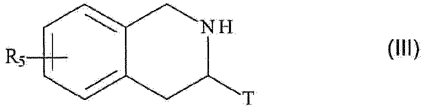 in der T und R₅ die bezüglich der Formel (I) angegebenen Bedeutungen besitzen, unterworfen wird
zur Bildung der Verbindung der Formel (IV): 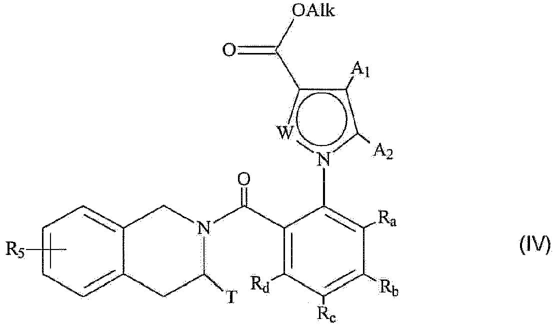 in der Alk die oben angegebene Bedeutung besitzt und W, A₁, A₂, Rₐ, R_{b}, R_{c}, R_{d}, R5 und T die bezüglich der Formel (I) angegebenen Bedeutungen besitzen,
wonach die Esterfunktion der Verbindung der Formel (IV) hydrolysiert wird zur Bildung der entsprechenden Carbonsäure oder des entsprechenden Carboxylats, welches in ein Säurederivat, wie das entsprechende Acylchlorid oder Anhydrid umgewandelt werden kann, bevor es mit einem Amin NHR₃R₄ gekuppelt wird, worin R₃ und R₄ die bezüglich der Formel (I) angegebenen Bedeutungen besitzen, und die gegebenenfalls der Einwirkung eines Pyrophosphat- oder Phosphonat-Derivats unter basischen Bedingungen unterworfen werden kann zur Bildung der Verbindung der Formel (I),
welche Verbindung der Formel (I) gemäß einer klassischen Trennungsmethode gereinigt werden kann, welche man gewünschtenfalls in ihre Additionssalze mit einer pharmazeutisch annehmbaren Säure oder Base umwandelt und die man gegebenenfalls mit Hilfe einer klassischen Trennmethode in ihre Isomeren auftrennt,
wobei es sich versteht, dass zu irgendeinem geeigneten Zeitpunkt im Verlaufe des oben beschriebenen Verfahrens bestimmte Gruppen (Hydroxy, Amino...) der Reaktionsteilnehmer oder Zwischenprodukte der Synthese zum Zwecke der Synthese geschützt und wieder von den Schutzgruppen befreit werden können.

11. Verfahren zur Herstellung gemäß Anspruch 10 einer Verbindung der Formel (I), in der eine der Gruppe R₃ oder R₄ durch eine Hydroxyfunktion substituiert ist, **dadurch gekennzeichnet, dass** das Amin NHR₃R₄ vor jeder Kupplung mit der Carbonsäure der Verbindung der Formel (IV) oder einem ihrer entsprechenden Säuerderivate einer Reaktion zum Schutz der Hydroxyfunktion unterworfen wird, wonach die gebildete geschützte Verbindung der Formel (I) anschließend einer Reaktion zur Abspaltung der Schutzgruppe unterworfen wird und gegebenenfalls in eines ihrer Additionssalze mit einer pharmazeutisch annehmbaren Säure oder Base umgewandelt wird.

12. Verfahren zur Herstellung einer Verbindung der Formel (I) nach Anspruch 1, **dadurch gekennzeichnet, dass** man als Ausgangsprodukt die Verbindung der Formel (V) verwendet: 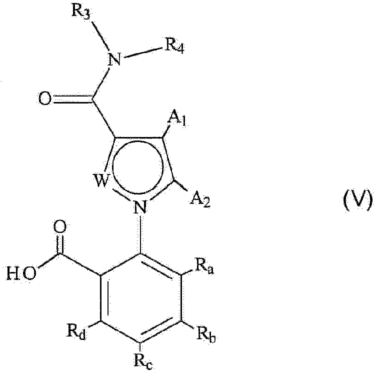 in der W, A₁, A₂, Rₐ, R_{b}, R_{c}, R_{d}, R₃ und R₄ die bezüglich der Formel (I) angegebenen Bedeutungen besitzen,
welche Verbindung der Formel (V) anschließend einer Peptidkupplung mit einer Verbindung der Formel (III): 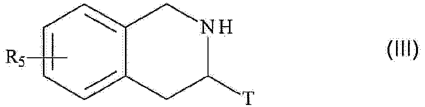 in der T und R₅ die bezüglich der Formel (I) angegebenen Bedeutungen besitzen, unterworfen wird,
wobei die in dieser Weise erhaltene Verbindung gegebenenfalls der Einwirkung eines Pyrophosphat- oder Phosphonat-Derivats unter basischen Bedingungen unterworfen wird zur Bildung der Verbindung der Formel (I),
welche Verbindung der Formel (I) gemäß einer klassischen Trennmethode gereinigt werden kann, welche man gewünschtenfalls in ihre Additionssalze mit einer pharmazeutisch annehmbaren Säure oder Base umwandelt und welche man gegebenenfalls mit Hilfe einer klassischen Trennmethode in ihre Isomeren aufspaltet,
wobei es sich versteht, dass zu irgendeinem geeigneten Zeitpunkt im Verlaufe des oben beschriebenen Verfahrens bestimmte Gruppen (Hydroxy, Amino...) der Reaktionsteilnehmer oder Zwischenprodukte der Synthese zum Zwecke der Synthese geschützt und wieder von den Schutzgruppen befreit werden können.

13. Pharmazeutische Zubereitung enthaltend eine Verbindung der Formel (I) nach einem der Ansprüche 1 bis 9 oder eines ihrer Additionssalze mit einer pharmazeutisch annehmbaren Säure oder Base in Kombination mit einem oder mehreren pharmazeutisch annehmbaren Hilfsstoffen.

14. Pharmazeutische Zubereitung nach Anspruch 13 zur Verwendung als proapoptotisches Mittel.

15. Pharmazeutische Zubereitung nach Anspruch 13 zur Verwendung bei der Behandlung von Krebs, Autoimmunerkrankungen und Erkrankungen des Immunsystems.

16. Pharmazeutische Zubereitung nach Anspruch 15 zur Verwendung bei der Behandlung von Blasenkrebs, Gehirnkrebs, Brustkrebs, Gebärmutterkrebs, chronischlymphatischen Leukämien, Kolorektalkrebs, Speiseröhrenkrebs, Leberkrebs, lymphoblastischen Leukämien, Nicht-Hodgkin Lymphomen, Melanomen, malignen Hämopathien, Myelomen, Ovarialkrebs, nicht-kleinzelligem Lungenkrebs, Prostatakrebs und kleinzelligem Lungenkrebs.

17. Verbindung der Formel (I) nach einem der Ansprüche 1 bis 9 oder eines ihrer Additionssalze mit einer pharmazeutisch annehmbaren Säure oder Base zur Verwendung bei der Behandlung von Blasenkrebs, Gehirnkrebs, Brustkrebs, Gebärmutterkrebs, chronisch- lymphatischen Leukämien, Kolorektalkrebs, Speiseröhrenkrebs, Leberkrebs, lymphoblastischen Leukämien, Nicht-Hodgkin Lymphomen, Melanomen, malignen Hämopathien, Myelomen, Ovarialkrebs, nicht-kleinzelligem Lungenkrebs, Prostatakrebs und kleinzelligem Lungenkrebs.

18. Kombination einer Verbindung der Formel (I) nach einem der Ansprüche 1 bis 9 mit einem Antikrebsmittel ausgewählt aus genotoxischen Mitteln, Mitosegiften, Antimetaboliten, Proteasominhibitoren, Kinaseinhibitoren oder Antikörpern.

19. Pharmazeutische Zubereitung umfassend eine Kombination nach Anspruch 18 in Kombination mit einem oder mehreren pharmazeutisch annehmbaren Hilfsstoffen.

20. Kombination nach Anspruch 18 zur Verwendung bei der Behandlung von Krebs.

21. Verbindung der Formel (I) nach einem der Ansprüche 1 bis 9 zur Verwendung in Kombination mit einer Strahlentherapie bei der Behandlung von Krebs.

## Claims

1. Compound of formula (I): 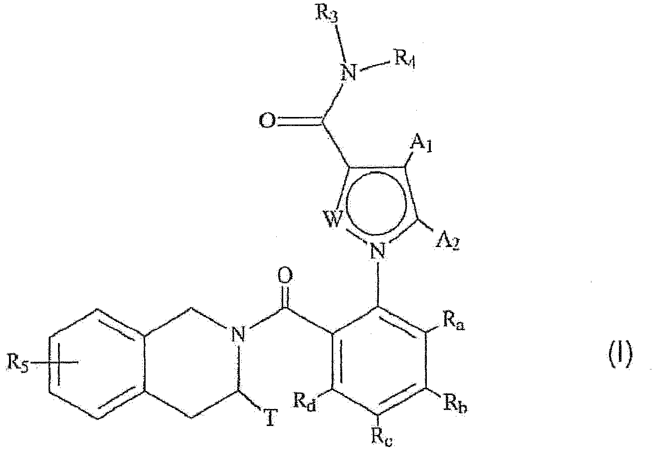 wherein:
◆ W represents a group C-H while A₁ and A₂ represent a hydrogen atom and a methyl group, respectively,
or W represents a group C-H while A₁ and A₂ form with the carbon atoms carrying them a cyclohexenyl or a benzo ring optionally substituted by a halogen atom,
◆ T represents a hydrogen atom, a linear or branched (C₁-C₆)alkyl optionally substituted by from one to three halogen atoms, a group (C₁-C₄)alkyl-NR₁R₂, or a group (C₁-C₄)alkyl-OR₆,
◆ R₁ and R₂ independently of one another represent a hydrogen atom or a linear or branched (C₁-C₆)alkyl group,
or R₁ and R₂ form with the nitrogen atom carrying them a heterocycloalkyl,
◆ R₃ represents an aryl or heteroaryl group, it being understood that one or more carbon atoms of the preceding groups, or, where appropriate, of their substituents, may be deuterated,
◆ R₄ represents an aryl or heteroaryl group, it being understood that one or more carbon atoms of the preceding groups, or, where appropriate, of their substituents, may be deuterated,
◆ R₅ represents a hydrogen or halogen atom, a linear or branched (C₁-C₆)alkyl group, or a linear or branched (C₁-C₆)alkoxy group,
◆ R₆ represents a hydrogen atom or a linear or branched (C₁-C₆)alkyl group,
◆ Rₐ, R_{b}, R_{c} and R_{d}, each independently of the others, represent R₇, a halogen atom, a linear or branched (C₁-C₆)alkoxy group, a hydroxy group, a linear or branched polyhalo-(C₁-C₆)alkyl group, a trifluoromethoxy group, -NR₇R₇', nitro, or the substituents of one of the pairs (Rₐ,R_{b}), (R_{b},R_{c}) or (R_{c},R_{d}) form together with the carbon atoms carrying them a ring composed of from 5 to 7 ring members, which may contain from one to 2 hetero atoms selected from oxygen and sulphur, it also being understood that one or more carbon atoms of the ring defined hereinbefore may be deuterated or substituted by from one to 3 groups selected from halogen and linear or branched (C₁-C₆)alkyl,
or Rₐ and R_{d} each represent a hydrogen atom, R_{b} represents a hydrogen, a halogen, a hydroxy or methoxy group, and R_{c} is selected from one of the following groups: hydroxy, methoxy, amino, 3-phenoxyazetidine, 2-(phenylsulphanyl)acetamide or 2-(phenoxy)acetamide,
◆ R₇ and R₇', each independently of the other, represent a hydrogen, a linear or branched (C₁-C₆)alkyl group, a linear or branched (C₂-C₆)alkenyl group, a linear or branched (C₂-C₆)alkynyl group, an aryl group or a heteroaryl group, or R₇ and R₇' form together with the nitrogen atom carrying them a heterocycle composed of from 5 to 7 ring members,
it being understood that when the compound of formula (I) contains a hydroxy group, the latter may be optionally substituted by one of the following groups: PO(OM)(OM'), -PO(OM)(O⁻M₁⁺), -PO(O⁻M₁⁺)(O⁻M₂⁺), -PO(O⁻)(O⁻)M₃²⁺, -PO(OM)(O[CH₂CH₂O]ₙCH₃), or PO(O⁻M₁⁺)(O[CH₂CH₂O]ₙCH₃), wherein M and M' independently of one another represent a hydrogen atom, a linear or branched (C₁-C₆)alkyl group, a linear or branched (C₂-C₆)alkenyl group, a linear or branched (C₂-C₆)alkynyl group, a cycloalkyl or a heterocycloalkyl, both composed of from 5 to 6 ring members, while M₁⁺ and M₂⁺ independently of one another represent a pharmaceutically acceptable monovalent cation, M₃²⁺ represents a pharmaceutically acceptable divalent cation, and n is an integer from 1 to 5,
it also being understood that:
- "aryl" means a phenyl, naphthyl, biphenyl or indenyl group,
- "heteroaryl" means any mono- or bi-cyclic group composed of from 5 to 10 ring members, having at least one aromatic moiety and containing from 1 to 4 hetero atoms selected from oxygen, sulphur and nitrogen (including quaternary nitrogens),
- "cycloalkyl" means any mono- or bi-cyclic, non-aromatic, carbocyclic group containing from 3 to 10 ring members,
- "heterocycloalkyl" means any mono- or bi-cyclic, non-aromatic group composed of from 3 to 10 ring members and containing from 1 to 3 hetero atoms selected from oxygen, sulphur, SO, SO₂ and nitrogen,
it being possible for the aryl, heteroaryl, cycloalkyl and heterocycloalkyl groups so defined and the groups alkyl, alkenyl, alkynyl and alkoxy to be substituted by from 1 to 3 groups selected from linear or branched (C₁-C₆)alkyl, (C₃-C₆)spiro, linear or branched (C₁-C₆)alkoxy, (C₁-C₆)alkyl-S-, hydroxy, oxo (or *N*-oxide where appropriate), nitro, cyano, -COOR', -OCOR', NR'R", linear or branched polyhalo-(C₁-C₆)alkyl, trifluoromethoxy, (C₁-C₆)alkylsulphonyl, halogen, aryl, heteroaryl, aryloxy, arylthio, cycloalkyl, heterocycloalkyl optionally substituted by one or more halogen atoms or alkyl groups, it being understood that R' and R", each independently of the other, represent a hydrogen atom or a linear or branched (C₁-C₆)alkyl group,
their enantiomers and diastereoisomers, and addition salts thereof with a pharmaceutically acceptable acid or base.

2. Compound of formula (I) according to claim 1, wherein R₄ represents a phenyl substituted in the *para*-position by a group of the formula -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), or -OPO(O⁻M₁⁺)(O[CH₂-CH₂O]ₙCH₃), wherein M and M' independently of one another represent a hydrogen atom, a linear or branched (C₁-C₆)alkyl group, a linear or branched (C₂-C₆)alkenyl group, a linear or branched (C₂-C₆)alkynyl group, a cycloalkyl or a heterocycloalkyl, both composed of from 5 to 6 ring members, while M₁⁺ and M₂⁺ independently of one another represent a pharmaceutically acceptable monovalent cation, M₃²⁺ represents a pharmaceutically acceptable divalent cation, and n is an integer from 1 to 5, it being understood that the phenyl group may be optionally substituted by one or more halogen atoms.

3. Compound of formula (I) according to claim 1 or 2, wherein T represents a group selected from methyl, aminomethyl, dimethylaminomethyl, morpholinylmethyl, (4-methyl-1-piperazinyl)methyl, (3a*R*,6a*S*)-hexahydrocyclopenta[*c*]pyrrol-2(1*H*)-ylmethyl, (4,4-difluoropiperidin-1-yl)methyl, (4-cyclopentylpiperazin-1-yl)methyl, (4-cyclobutylpiperazin-1-yl)methyl, pyrrolidin-1-ylmethyl, piperidin-1-ylmethyl and 2-(morpholin-4-yl)ethyl.

4. Compound of formula (I) according to any one of claims 1 to 3, wherein Rₐ and R_{d} each represent a hydrogen atom and (R_{b},R_{c}), together with the carbon atoms carrying them, form one of the following groups: optionally substituted 1,3-dioxolane; optionally substituted 1,4-dioxane; cyclopentane; tetrahydrofuran; 2,3-dihydrofuran; or Rₐ, R_{c} and R_{d} each represent a hydrogen atom and R_{b} represents a hydroxy or methoxy group, a halogen atom, a trifluoromethyl or trifluoromethoxy group.

5. Compound of formula (I) according to any one of claims 1 to 3, wherein one to two of the groups Rₐ, R_{b}, R_{c}, R_{d} represent a halogen atom, while the others represent a hydrogen.

6. Compound of formula (I) according to any one of claims 1 to 5, wherein R₄ represents a phenyl, 4-hydroxyphenyl, 4-methoxyphenyl, 4-methylphenyl, 3-chloro-4-hydroxyphenyl or 3-fluoro-4-hydroxyphenyl group.

7. Compound of formula (I) according to any one of claims 1 to 6, wherein R₃ represents a group selected from phenyl, 1*H-*indole, benzothiophene, benzofuran, 2,3-dihydro-1*H-*indole, 1*H*-indazole, 2,3-dihydro-1*H-*isoindole, 1*H*-pyrrolo[2,3-*b*]pyridine, phenoxyphenyl, 2,3-dihydro-1*H-*pyrrolo[2,3-*b*]pyridine, 1*H-*pyrrole, these groups optionally containing one or more substituents selected from halogen, linear or branched (C₁-C₆)alkyl, trifluoromethoxy, 4-methylpiperazinyl, linear or branched (C₁-C₆)alkoxy, and cyano.

8. Compound of formula (I) according to claim 1 selected from the following group:
- *N-*(4-hydroxyphenyl)-1-{6-[(3*S*)-3-(morpholin-4-ylmethyl)-1,2,3,4-tetrahydroisoquinoline-2-carbonyl]-2*H-*1,3-benzodioxol-5-yl}-*N*-phenyl-1*H* indole-3-carboxamide,
- *N-*(4-hydroxyphenyl)-*N-*(1-methyl-1*H-*pyrrolo[2,3-*b*]pyridin-5-yl)-1-(6-{[(3*S*)-3-(morpholin-4-ylmethyl)-3,4-dihydroisoquinolin-2(1*H*)-yl]carbonyl}-1,3-benzodioxol-5-yl)-4,5,6,7-tetrahydro-1*H-*indole-3-carboxamide,
- *N-*(4-hydroxyphenyl)-*N*-(4-methylphenyl)-1-(6-{[(3*S*)-3-[(4-methylpiperazin-1-yl)methyl]-3,4-dihydroisoquinolin-2(1*H*)-yl]carbonyl}-1,3-benzodioxol-5-yl)-1*H-*indole-3-carboxamide,
- *N-*(4-hydroxyphenyl)-*N*-(1*H*-indol-5-yl)-1-(6-{[(3*S*)-3-[(4-methylpiperazin-1-yl)methyl]-3,4-dihydroisoquinolin-2(1*H*)-yl]carbonyl}-1,3-benzodioxol-5-yl)-1*H-*indole-3-carboxamide,
- *N*-(4-hydroxyphenyl)-*N*-(1-methyl-1*H*-indol-5-yl)-1-(6-{[(3*S*)-3-[(4-methylpiperazin-1-yl)methyl]-3,4-dihydroisoquinolin-2(1*H*)-yl]carbonyl}-1,3-benzodioxol-5-yl)-1*H*-indole-3-carboxamide,
- 1-(5-chloro-2-{[(3*S*)-3-[(4-methylpiperazin-1-yl)methyl]-3,4-dihydroisoquinolin-2(1*H*)-yl]carbonyl}phenyl)-*N*-(4-chlorophenyl)-*N*-(4-hydroxyphenyl)-5-methyl-1*H*-pyrrole-3-carboxamide,
- 1-(5-chloro-2-{[(3*S*)-3-[(4-methylpiperazin-1-yl)methyl]-3,4-dihydroisoquinolin-2(1*H*)-yl]carbonyl}phenyl)-*N-*(4-hydroxyphenyl)-*N-*(1*H-*indol-5-yl)-1*H-*indole-3-carboxamide,
- *N*-(4-hydroxyphenyl)-1-{6-[(3*S*)-3-[(4-methylpiperazin-1-yl)methyl]-1,2,3,4-tetrahydroisoquinoline-2-carbonyl]-2*H-*1,3-benzodioxol-5-yl}-*N-*phenyl-1*H-*indole-3-carboxamide,
and addition salts thereof with a pharmaceutically acceptable acid or base.

9. Compounds selected from the following group:
- 1-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-4,5-dimethyl-*N*-(1-methyl-2,3-dihydro-1*H-*pyrrolo[2,3-*b*]pyridin-5-yl)-1*H-*pyrrole-3-carboxamide,
- 1-(5-chloro-2-{[(3*S*)-3-(morpholin-4-ylmethyl)-3,4-dihydroisoquinolin-2(1*H*)-yl]carbonyl}phenyl)-*N-*(5-cyano-1,2-dimethyl-1*H-*pyrrol-3-yl)-*N*-(4-hydroxyphenyl)-4,5-dimethyl-1*H-*pyrrole-3-carboxamide,
- 1-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-4,5-dimethyl-*N*-(1-methyl-1*H-*pyrrolo[2,3-*b*]pyridine-5-yl)-1*H-*pyrrole-3-carboxamide,
- 1-[(2*S*)-2-[(benzyloxy)methyl]-7-[(3*S*)-3[(4-methylpiperazin-1-yl)methyl]-1,2,3,4-tetrahydroisoquinoline-2-carbonyl]-2,3-dihydro-1,4-benzodioxin-6-yl]-*N-*(4-hydroxyphenyl)-*N*-phenyl-1*H*-indole-3-carboxamide,
and addition salts thereof with a pharmaceutically acceptable acid or base.

10. Process for the preparation of a compound of formula (I) according to claim 1, **characterised in that** there is used as starting material the compound of formula (II): 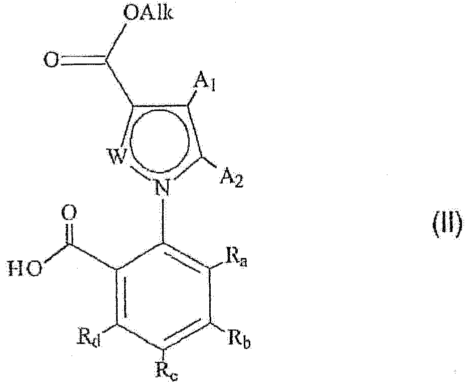 wherein Alk represents a (C₁-C₆)alkyl group and W, A₁, A₂, Rₐ, R_{b}, R_{c}, R_{d} are as defined for formula (I),
which compound of formula (II) is then subjected to peptide coupling with a compound of formula (III): 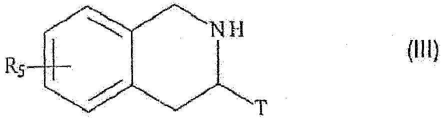 wherein T and R₅ are as defined for formule (I),
to yield the compound of formula (IV): 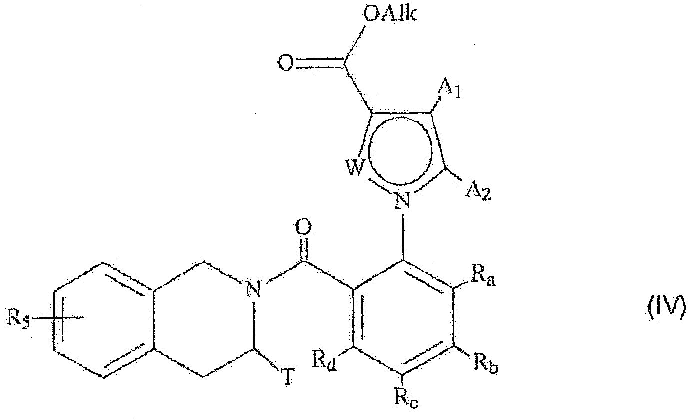 wherein Alk is as defined hereinbefore and W, A₁, A₂, Rₐ, R_{b}, R_{c}, R_{d}, R₅ and T are as defined for formula (I),
the ester function of which compound of formula (IV) is hydrolysed to yield the corresponding carboxylic acid or carboxylate, which may be converted into an acid derivative such as the corresponding acyl chloride or anhydride before being coupled with an amine NHR₃R₄ wherein R₃ and R₄ have the same meanings as for formula (I), which may optionally be subjected to the action of a pyrophosphate or phosphonate compound under basic conditions, to yield the compound of formula (I),
which compound of formula (I) may be purified according to a conventional separation technique, which is converted, if desired, into its addition salts with a pharmaceutically acceptable acid or base and which is optionally separated into its isomers according to a conventional separation technique,
it being understood that, at any time considered appropriate in the course of the above-described process, certain groups (hydroxy, amino...) of the reagents or intermediates of synthesis may be protected and then deprotected according to the requirements of synthesis.

11. Process according to claim 10 for the preparation of a compound of formula (I) wherein one of the groups R₃ or R₄ is substituted by a hydroxy function, **characterised in that** the amine NHR₃R₄ is subjected beforehand to a reaction protecting the hydroxy function prior to any coupling with the carboxylic acid formed from the compound of formula (IV), or with a corresponding acid derivative thereof, the resulting protected compound of formula (I) subsequently undergoes a deprotection reaction and is then optionally converted into one of its addition salts with a pharmaceutically acceptable acid or base.

12. Process for the preparation of a compound of formula (I) according to claim 1, **characterised in that** there is used as starting material the compound of formula (V): 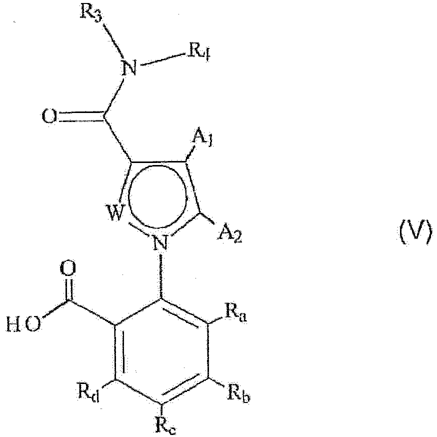 wherein W, A₁, A₂, Rₐ, R_{b}, R_{c}, R_{d}, R₃ and R₄ are as defined for formula (I),
which compound of formula (V) is then subjected to peptide coupling with a compound of formula (III): 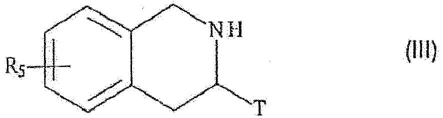 wherein T and R₅ are as defined for formula (I),
the compound so obtained optionally being subjected to the action of a pyrophosphate or phosphonate compound under basic conditions to yield the compound of formula (I), which compound of formula (I) may be purified according to a conventional separation technique, which is converted, if desired, into its addition salts with a pharmaceutically acceptable acid or base and which is optionally separated into its isomers according to a conventional separation technique,
it being understood that, at any time considered appropriate in the course of the above-described process, certain groups (hydroxy, amino...) of the reagents or intermediates of synthesis may be protected and then deprotected according to the requirements of synthesis.

13. Pharmaceutical composition comprising a compound of formula (I) according to any one of claims 1 to 9 or an addition salt thereof with a pharmaceutically acceptable acid or base in combination with one or more pharmaceutically acceptable excipients.

14. Pharmaceutical composition according to claim 13 for use as a pro-apoptotic agent.

15. Pharmaceutical composition according to claim 13 for use in the treatment of cancers, auto-immune diseases and diseases of the immune system.

16. Pharmaceutical composition according to claim 15 for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

17. Compound of formula (I) according to any one of claims 1 to 9, or an addition salt thereof with a pharmaceutically acceptable acid or base, for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

18. Association of a compound of formula (I) according to any one of claims 1 to 9 with an anti-cancer agent selected from genotoxic agents, mitotic poisons, antimetabolites, proteasome inhibitors, kinase inhibitors and antibodies.

19. Pharmaceutical composition comprising an association according to claim 18 in combination with one or more pharmaceutically acceptable excipients.

20. Association according to claim 18 for use in the treatment of cancers.

21. Compound of formula (I) according to any one of claims 1 to 9 for use in association with radiotherapy in the treatment of cancers.
